# EUROPEAN PATENT SPECIFICATION

(11) **EP 0 910 572 B1**
(45) Date of publication and mention of the grant of the patent: **27.04.2005**
(21) Application number: 97924707.9
(22) Date of filing: 13.05.1997
(51) Int. Cl.: C07K 5/06, C07C 237/22, C07D 521/00

(54) **INHIBITORS OF PICORNAVIRUS 3C PROTEASES AND METHODS FOR THEIR USE AND PREPARATION**
INHIBITOREN VON PICORNAVIRUS 3C PROTEASE UND VERFAHREN ZUR HERSTELLUNG UND VERWENDUNG
INHIBITEURS DE 3C PROTEASE ANTIPICORNOVIRALE ET PROCEDES CORRESPONDANTS D'UTILISATION ET DE PREPARATION

(30) Priority: 14.05.1996 US 17666 P; 14.05.1996 US 645687; 02.05.1997 US 850398
(43) Date of publication of application: 28.04.1999
(73) Proprietor: Agouron Pharmaceuticals, Inc., San Diego, California 92121 (US)
(72) Inventor: WEBBER, Stephen, E., San Diego, CA 92111 (US); DRAGOVICH, Peter, S., Encinitas, CA 92024 (US); PRINS, Thomas, J., Cardiff, CA 92007 (US); REICH, Siegfried, H., San Diego, CA 92104 (US); LITTLE, Thomas, L., Jr., Redmond, WA 98052 (US); LITTLEFIELD, Ethel, S., San Diego, CA 92126 (US); MARAKOVITS, Joseph, T., Encinitas, CA 92024 (US); BABINE, Robert, E., Carlsbad, CA 92009 (US); BLECKMAN, Ted, M., La Jolla, CA 92037 (US)
(74) Representative: Grünecker, Kinkeldey, Stockmair & Schwanhäusser Anwaltssozietät
(86) International application number: PCT/US1997/008112
(87) International publication number: WO 1997/043305

(56) References cited:
- VAILLANCOURT, MARC ET AL: "Synthesis of novel inhibitors of the HIV-1 protease:Difunctional enols of simple N-protected amino acids" BIOORG. MED. CHEM. , vol. 2, 1994, pages 343-355, XP002043058
- VAILLANCOURT, MARC ET AL: "Difunctional enols of N-protected amino acids as low molecular weight and novel inhibitors of HIV-1 protease" BIOORG. MED. CHEM. LETT. , vol. 3, no. 6, 1993, pages 1169-1174, XP002043059

## Description

The invention pertains to the discovery and use of new compounds that inhibit the enzymatic activity of picomaviral 3C proteases, specifically rhinovirus proteases (RVPs), as well as retard viral growth in cell culture.

The picomaviruses are a family of tiny non-enveloped positive stranded RNA containing viruses that infect humans and other animals. These viruses include the human rhinoviruses, human polioviruses, human coxsackieviruses, human echoviruses, human and bovine enteroviruses, encephalomyocarditis viruses, menigovirus, foot and mouth viruses, hepatitis A virus and others. The human rhinoviruses are a major cause of the common cold. To date, there are no effective therapies to cure the common cold, only treatments that relieve the symptoms.

One strategy that may be useful to treat picomaviral infections is by inhibiting the proteolytic 3C enzymes. These enzymes are required for the natural maturation of the picomaviruses. They are responsible for the autocatalytic cleavage of the genomic, large polyprotein into the essential viral proteins. Members of the 3C protease family are cysteine proteases, where the sulfhydryl group most often cleaves the glutamine-glycine amide bond. In theory, inhibition of 3C proteases can block proteolytic cleavage of the polyprotein, which in turn can retard the maturation and replication of the viruses by interfering with viral particle production. Therefore, inhibiting the processing of this cysteine protease with selective, small molecules that are specifically recognized, may represent an important and useful approach to treat and cure viral infections of this nature and, in particular, the common cold. Bioorg. Med. Chem. (1994), 2(5), 343-355 and Bioorg. Med. Chem. (1993), 3(6), 1165-1174 disclose a series of HIV-1 protease inhibitors which are enol-derivatives of simple amino acids.

### SUMMARY OF THE INVENTION

The present invention is directed to compounds that functions as picomaviral 3C protease inhibitors, particularly those that have antiviral activity. It is further directed to the preparation and use of such 3C protease inhibitors. The Inventors demonstrate that the compounds of the present invention bind to rhinovirus 3C proteases and preferably have antiviral cell culture activity. The enzymatic inhibition assays used reveal that these compounds can bind irreversibly, and the cell culture assays demonstrate that these compounds can possess antiviral activity.

The present invention is directed to compounds of the formula (I): 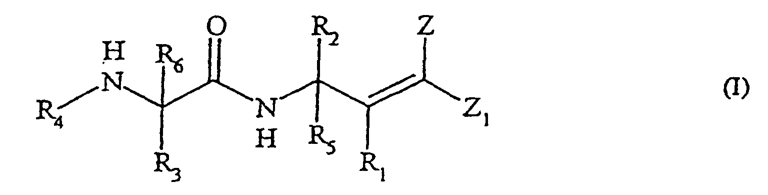 wherein
R₁ is H, F, an alkyl group, SH, an O-alkyl group, or an S-alkyl group;
R₂ and R₅ are independently selected from H, 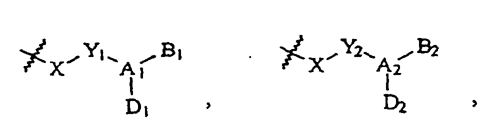 or an alkyl group, wherein said alkyl group is different from 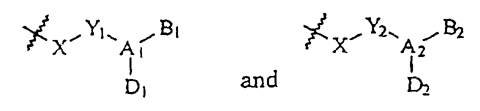 with the proviso that at least one of R₂ or R₅ must be 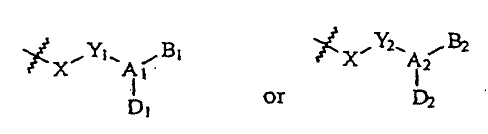 and wherein, when R₂ or R₅ is 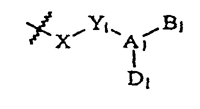 X is =CH or =CF and Y₁ is =CH or =CF
or X and Y₁ together with Q' form a three-membered ring in which Q' is -C(R₁₀)(R₁₁)- or -O-, X is -CH- or -CF-, and Y₁ is -CH-, -CF-, or -C(alkyl)-, where R₁₀ and R₁₁ independently are H, a halogen, or an alkyl group, or, together with the carbon atom to which they are attached, form a cycloalkyl group or a heterocycloalkyl group,
or X is -CH₂-, -CF₂-, -CHF-, or -S-,
and
Y₁ is -O-, -S-, -NR₁₂-, -C(R₁₃)(R₁₄)-, -C(O)-, -C(S)-, or -C(CR₁₃R₁₄)- wherein R₁₂ is H or alkyl, and R₁₃ and R₁₄ independently are H, F, or an alkyl group, or, together with the atoms to which they are bonded, form a cycloalkyl group or a heterocycloalkyl group;
and A₁ is C, CH, CF, S, P, Se, N, NR₁₅, S(O), Se(O), P-OR₁₅, or P-NR₁₅R₁₆ wherein R₁₅ and R₁₆ independently are an alkyl group, a cycloalkyl group, a heterocycloalkyl group, an aryl group, or a heteroaryl group, or, together with the atom to which they are bonded, form a heterocycloalkyl group;
and D₁ is a moiety with a lone pair of electrons capable of forming a hydrogen bond;
and B₁ is H, F, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈ wherein R₁₇, R₁₈, and R₁₉ independently are H, an alkyl group, a cycloalkyl group, a heterocycloalkyl group, an aryl group, a heteroaryl group, or an acyl group, or, wherein any two of R₁₇, R₁₈, and R₁₉, together with the atom(s) to which they are bonded, form a heterocycloalkyl group;
and with the provisos that when D₁ is the moiety ≡N with a lone pair of electrons capable of forming a hydrogen bond, B₁ does not exist; and when A₁ is an sp³ carbon, B₁ is not -NR₁₇R₁₈ when D₁ is the moiety -NR₂₅R₂₆ with a lone pair of electrons capable of forming a hydrogen bond, wherein R₂₅ and R₂₆ are independently H, an alkyl group, a cycloalkyl group, a heterocycloalkyl group, an aryl group, or a heteroaryl group;
and wherein D₁-A₁-B₁ optionally forms a nitro group where A₁ is N;
and wherein, when R₂ or R₅ is 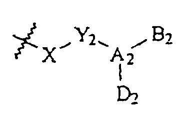 X is =CH or =CF and Y₂ is =C, =CH or =CF,
or X and Y₂ together with Q' form a three-membered ring in which Q' is -C(R₁₀)(R₁₁)- or -O-, X is -CH- or -CF-, and Y₂ is -CH-, -CF-, or -C(alkyl)-, where R₁₀ and R₁₁ independently are H, a halogen, or an alkyl group, or, together with the carbon atom to which they are attached, form a cycloalkyl group or a heterocycloalkyl group,
or X is -CH₂-, -CF₂-, -CHF-, or -S-,
and
Y₂ is -O-, -S-, -N(R'₁₂)-, -C(R'₁₃)(R'₁₄)-, -C(O)-, -C(S)-, or -C(CR'₁₃R'₁₄)- wherein R'₁₂ is H, an alkyl group, a cycloalkyl group, a heterocycloalkyl group, an aryl group, a heteroaryl group, -OR'₁₃, -NR'₁₃R'₁₄, -C(O)-R'₁₃, -SO₂R'₁₃, or -C(S)R'₁₃, and R'₁₃ and R'₁₄, independently are H, F, or an alkyl group, a cycloalkyl group, a heterocycloalkyl group, an aryl group, or a heteroaryl group or, together with the atom to which they are attached, form a cycloalkyl group or a heterocycloalkyl group;
and wherein any combination of Y₂, A₂, B₂, and D₂ forms a cycloalkyl group, a heterocycloalkyl group, an aryl group, or a heteroaryl group;
and A₂ is C, CH, CF, S, P, Se, N, NR₁₅, S(O), Se(O), P-OR₁₅, or P-N'R₁₅R₁₆ wherein R₁₅ and R₁₆ independently are an alkyl group, a cycloalkyl group, a heterocycloalkyl group, an aryl group, or a heteroaryl group or, together with the atom to which they are bonded, form a heterocycloalkyl group;
and D₂ is a moiety with a lone pair of electrons capable of forming a hydrogen bond;
and B₂ is H, F, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈ wherein R₁₇, R₁₈, and R₁₉ independently are H, an alkyl group, a cycloalkyl group, a heterocycloalkyl group, an aryl group, a heteroaryl group, or an acyl group, or, wherein any two of R₁₇, R₁₈, and R₁₉, together with the atom(s) to which they are bonded, form a heterocycloalkyl group;
R₃ and R₆ are independently H, F, or an alkyl group;
R₄ is as defined herein and in claim 1
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃, or-C(S)NR₂₁NR₂₂R₂₃, wherein R₂₁, R₂₂, R₂₃, and R₂₄ are independently H, an alkyl group, a cycloalkyl group, a heterocycloalkyl group, an aryl group, a heteroaryl group, an acyl group, or a thioacyl group, or wherein any two of R₂₁, R₂₂, R₂₃, and R₂₄, together with the atom(s) to which they are bonded, form a heterocycloalkyl group;
or Z₁, as defined above, together with R₁, as defined above, and the atoms to which Z₁ and R₁ are bonded, form a cycloalkyl or heterocycloalkyl group,
or Z and Z₁, both as defined above, together with the atoms to which they are bonded, form a cycloalkyl or heterocycloalkyl group;
and pharmaceutically acceptable salts, and solvates thereof; wherein the residues are as defined in claim 1.

These compounds preferably have antipicomaviral activity with an EC₅₀ less than or equal to 100 µM in the HI-HeLa cell culture assay, and more preferably antirhinoviral activity with an EC₅₀ less than or equal to 100 µM in the HI-HeLa cell culture assay and/or anticoxsachieviral activity with an EC₅₀ less than or equal to 100 µM in the HI-HeLa cell culture assay.

The present invention is also directed to several methods of preparing compounds of formula (I), defined above. One method according to the invention involves converting a compound of formula **Q** 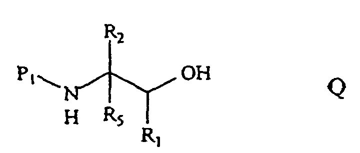 wherein R₁, R₂ and R₅ are as defined above, and P₁ is a protective group, preferably benzyloxy carbonyl or t-butoxycarbonyl, or a salt or solvate thereof, to a compound of formula I, as defined above, or a pharmaceutically acceptable salt or solvate thereof.

Preferred embodiments of the present invention are defined in claims 2 to 46.

Another method according to the invention involves converting a compound of the formula **B**: 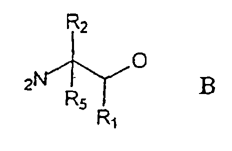 wherein R₁, R₂ and R₅ are as defined above, or a salt or solvate thereof, to a compound of formula I, as defined above, or a pharmaceutically acceptable prodrug, salt or solvate thereof.

Another method according to the invention involves converting a compound of formula **O**: 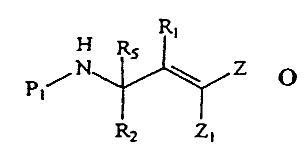 wherein R₁, R₂, R₅, Z and Z₁ are as defined above and P₁ is a protective group, preferably benzyloxy carbonyl or t-butoxycarbonyl, or a salt or solvate thereof, to a compound of formula I, as defined above, or a pharmaceutically acceptable salt or solvate thereof.

Another method according to the present invention involves converting a compound of formula **P**: 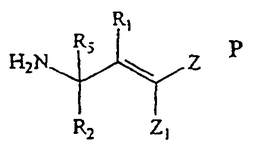 wherein R₁, R₂, R₅, Z and Z₁ are as defined above, or a salt or solvate thereof, to a compound of formula I, as defined above, or a phannaceutically acceptable, salt or solvate thereof.

### DETAILED DESCRIPTION OF THE INVENTION

The present invention relates to compounds of the formula I 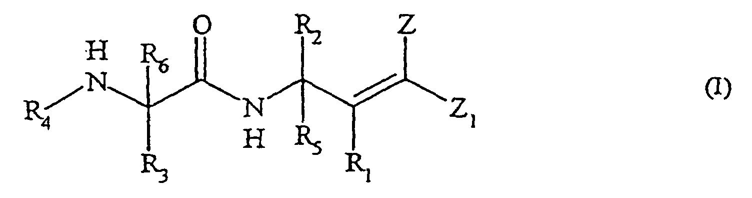 wherein R₁, R₂, R₃, R₄, R₅, R₆, Z and Z₁ are as defined above, and to the pharmaceutically acceptable salts, and solvates thereof, where these compounds, pharmaceutically acceptable salts, and solvates preferably have antipicornaviral activity with an EC₅₀ less than or equal to 100 µM in the HI-HeLa cell culture assay, and more preferably antirhinoviral activity with an EC₅₀ less than or equal to 100 µM in the HI-HeLa cell culture assay and/or anticoxsachieviral activity with an EC₅₀ less than or equal to 100 µM in the HI-HeLa cell culture assay.

The present invention preferably relates to compounds of the formula **II**: 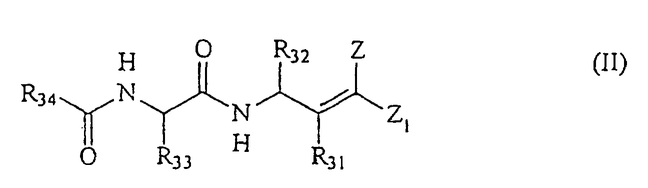 wherein
R₃₁ is H, F or an alkyl group;
R₃₂ is selected from one of the following moieties: 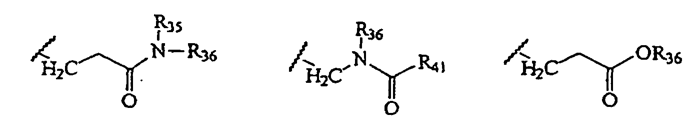 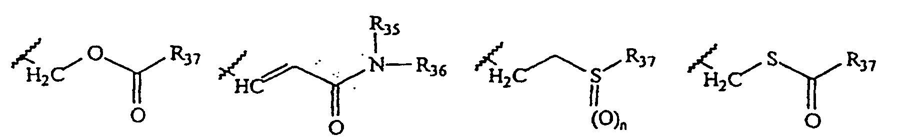 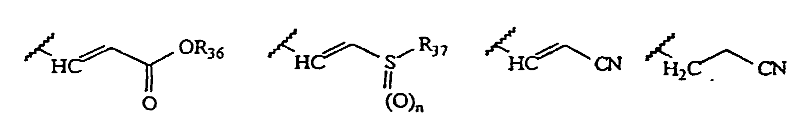 wherein
R₃₅ is H, an alkyl group, an aryl group, -OR₃₈, or -NR₃₈R₃₉, and
R₃₆ is H or an alkyl group,
or R₃₅ and R₃₆, together with the atom(s) to which they are attached, form a heterocycloalkyl group or a heteroaryl group;
R₄₁ is H, an alkyl group, an aryl group, -OR₃₈, -SR₃₉, -NR₃₈R₃₉, -NR₄₀NR₃₈R₃₉, or -NR₃₈OR₃₉, or R₄₁ and R₃₆, together with the atom(s) to which they are attached, form a heterocycloalkyl group;
R₃₇ is an alkyl group, an aryl group, or -NR₃₈R₃₉;
wherein R₃₈, R₃₉, and R₄₀ independently are H, an alkyl group, a cycloalkyl group, a heterocycloalkyl group, an aryl group, a heteroaryl group, or an acyl group, or, wherein any two of R₃₈, R₃₉, and R₄₀, together with the atom(s) to which they are bonded, form a heterocycloalkyl group;
n is 0, 1 or 2;
R₃₃ is H or an alkyl group;
R₃₄ is an alkyl group, a cycloalkyl group, a heterocycloalkyl group, an aryl group, a heteroaryl group, an O-alkyl, an O-cycloalkyl group, an O-heterocycloalkyl group, an O-aryl group, an O-heteroaryl group, an S-alkyl group, an NH-alkyl group, an NH-aryl group, an N,N-dialkyl group, or an N,N-diaryl group; and
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR_{21,}R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃, or -C(S)NR₂₁NR₂₂R₂₃, wherein R₂₁, R₂₂, R₂₃, and R₂₄ are independently H, an alkyl group, a cycloalkyl group, a heterocycloalkyl group, an aryl group, a heteroaryl group, an acyl group, or a thioacyl group, or wherein any two of R₂₁, R₂₂, R₂₃, and R₂₄, together with the atom(s) to which they are bonded, form a heterocycloalkyl group,
or Z and Z₁, both as defined above, together with the atoms to which they are bonded, form a heterocyclo alkyl group;
and pharmaceutically acceptable salts, and solvates thereof.

As used in the present application, the following definitions apply:

An "alkyl group" is intended to mean methyl, ethyl, propyl, isopropyl, butyl, isobutyl, t-butyl, ethenyl, pentenyl, butenyl, propenyl, ethynyl, butynyl, propynyl, pentynl, hexynyl, which may be unsubstituted (i.e., containing only carbon and hydrogen) or substituted by one suitable substituent as defined below.

A "cycloalkyl group" is intended to mean a non-aromatic, monovalent monocyclic, bicyclic, or tricyclic radical containing 3, 4, 5, 6, 7, 8, 9, 10, 11, 12, 13, or 14 carbon ring atoms, each of which may be saturated or unsaturated, and which may be unsubstituted or substituted by one suitable substituent as defined below, and to which may be fused one or more heterocycloalkyl groups, aryl groups, or heteroaryl groups, which themselves may be unsubstituted or substituted by one suitable substituent. Illustrative examples of cycloalkyl groups include, but are not limited to, the following moieties:

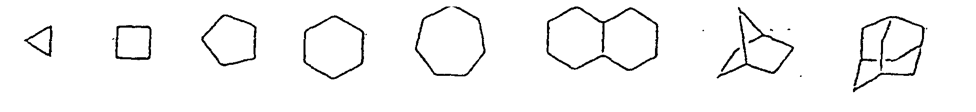

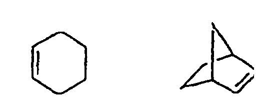

A "heterocycloalkyl group" is intended to mean a non-aromatic, monovalent monocyclic, bicyclic, or tricyclic radical, which is saturated or unsaturated, containing 3, 4, 5, 6, 7, 8, 9, 10, 11, 12, 13, 14, 15, 16, 17, or 18 ring atoms, and which includes 1, 2, 3, 4, or 5 heteroatoms selected from nitrogen, oxygen and sulfur, wherein the radical is unsubstituted or substituted by one suitable substituent as defined below, and to which may be fused one or more cycloalkyl groups, aryl groups, or heteroaryl groups, which themselves may be unsubstituted or substituted by one suitable substituent. Illustrative examples of heterocycloalkyl groups include, but are not limited to the following moieties:

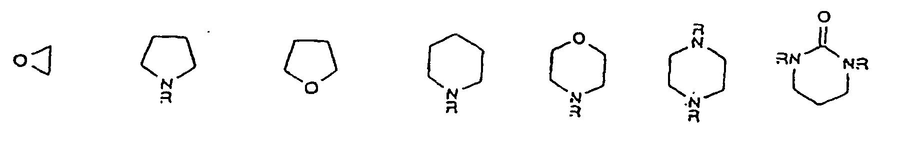

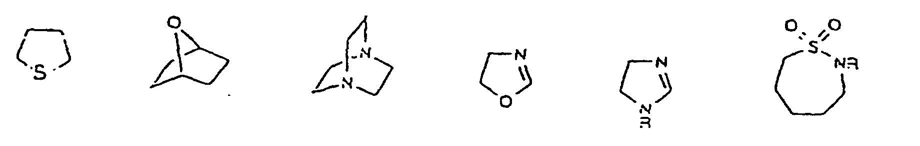

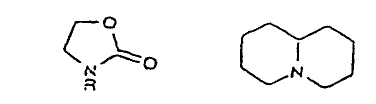

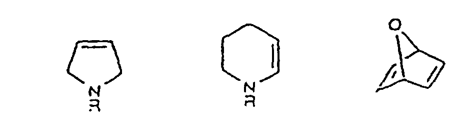

An "aryl group" is intended to mean an aromatic, monovalent monocyclic, bicyclic, or tricyclic radical containing 6, 10, 14, 18 carbon ring atoms, which may be unsubstituted or substituted by one suitable substituent as defined below, and to which may be fused one or more cycloalkyl groups, heterocycloalkyl groups, or heteroaryl groups, which themselves may be unsubstituted or substituted by one suitable substituent. Illustrative examples of aryl groups include, but are not limited to, the following moieties:

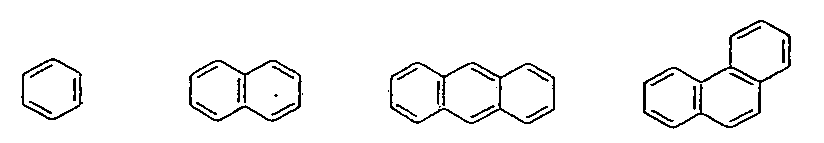

A "heteroaryl group" is intended to mean an aromatic monovalent monocyclic, bicyclic, or tricyclic radical containing 4, 5, 6, 7, 8, 9, 10, 11, 12, 13, 14, 15, 16, 17, or 18 ring atoms, including 1, 2, 3, 4, or 5 heteroatoms selected from nitrogen, oxygen and sulfur, which may be unsubstituted or substituted by one suitable substituent as defined below, and to which may be fused one or more cycloalkyl groups, heterocycloalkyl groups, or aryl groups, which themselves may be unsubstituted or substituted by one suitable substituent. Illustrative examples of heteroaryl groups include, but are not limited to, the following moieties:

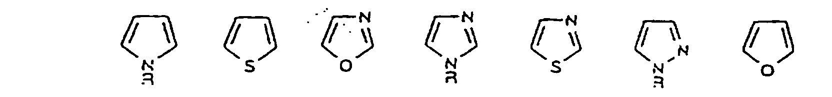

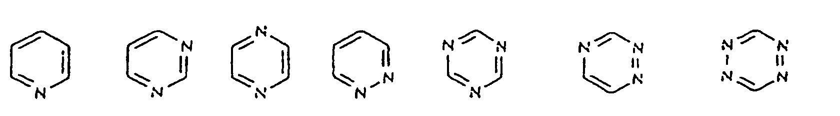

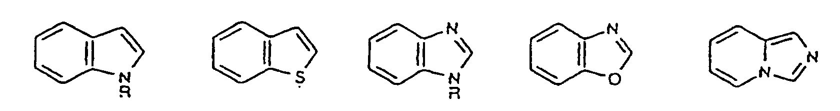

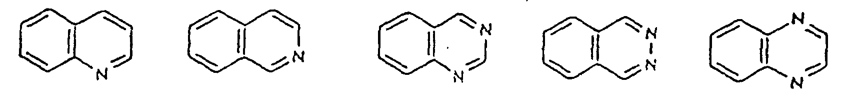

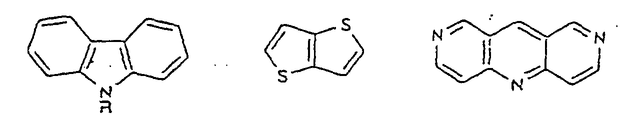

An "acyl group" is intended to mean a -C(O)-R radical, wherein R is any suitable substituent as defined below.

A "thioacyl group" is intended to mean a -C(S)-R radical, wherein R is any suitable substituent as defined below.

A "sulfonyl group" is intended to mean a -SO₂R radical, wherein R is any suitable substituent as defined below.

The term "suitable substituent" is intended to mean any of the substituents hydroxy groups, oxo groups, alkyl groups, acyl groups, sulfonyl groups, mercapto groups, alkylthio groups, alkoxy groups, cycloalkyl groups, heterocycloalkyl groups, aryl groups, heteroaryl groups, carboxy groups, amino groups, alkylamino groups, dialkylamino groups, carbamoyl groups, aryloxy groups, heteroarlyoxy groups, arylthio groups, heteroarylthio groups.

The term "suitable organic moiety" is intended to mean any organic moiety recognizable, such as by routine testing, to those skilled in the art as not adversely affecting the inhibitory activity of the inventive compounds. Illustrative examples of suitable organic moieties include, hydroxy groups, alkyl groups, oxo groups, cycloalkyl groups, heterocycloalkyl groups, aryl groups, heteroaryl groups, acyl groups, sulfonyl groups, mercapto groups, alkylthio groups, alkoxy groups, carboxy groups, amino groups, alkylamino groups, dialkylamino groups, carbamoyl groups, arylthio groups, heteroarylthio groups.

A "hydroxy group" is intended to mean the radical -OH.

An "amino group" is intended to mean the radical -NH₂.

An "alkylamino group" is intended to mean the radical -NHR where R is an alkyl group as defined above.

A "dialkylamino group" is intended to mean the radical -NRₐR_{b} where Rₐ and R_{b} are each independently an alkyl group as defined above.

An "alkoxy group" is intended to mean the radical -OR where R is an alkyl group as defined above, for example, methoxy, ethoxy, propoxy and the like.

An "alkoxycarbonyl group" is intended to mean the radical -C(O)OR where R is an alkyl group as defined above.

An "alkylsulfonyl group" is intended to mean the radical -SO₂R where R is an alkyl group as defined above.

An "alkylaminocarbonyl group" is intended to mean the radical -C(O)NHR where R is an alkyl group as defined above.

A "dialkylaminocarbonyl group" is intended to mean the radical -C(O)NRₐR_{b} where Rₐ and R_{b} are each independently an alkyl group as defined above.

A "mercapto group" is intended to mean the radical -SH.

An "alkylthio group" is intended to mean the radical -SR where R is an alkyl group as defined above.

A "carboxy group" is intended to mean the radical -C(O)OH.

A "carbamoyl group" is intended to mean the radical -C(O)NH₂.

An "aryloxy group" is intended to mean the radical -OR_{c} where R_{c} is an aryl group as defined above.

A "heteroarlyoxy group" is intended to mean the radical -OR_{d} where R_{d} is a heteroaryl group as defined above.

An "arylthio group" is intended to mean the radical -SR_{c} where R_{c} is an aryl group as defined above.

A "heteroarylthio group" is intended to mean the radical -SR_{d} where R_{d} is a heteroaryl group as defined above.

A "pharmaceutically acceptable solvate" is intended to mean a solvate that retains the biological effectiveness and properties of the biologically active components of compounds of formulas I and II.

Examples of pharmaceutically acceptable solvates include, water, isopropanol, ethanol, methanol, DMSO, ethyl acetate, acetic acid, and ethanolamine.

A "pharmaceutically acceptable salt" is intended to a mean a salt that retains the biological effectiveness and properties of the free acids and bases of compounds of formulas I and II and that is not biologically or otherwise undesirable.

Examples of pharmaceutically acceptable salts include, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formatessaveobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycolates, tartrates, methane-sulfonates, propanesulfonates, naphthalene-1-sulfonates, naphthalene-2-sulfonates, and mandelates.

If the inventive compound is a base, the desired salt may be prepared by any suitable method known to the art, including treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, pyranosidyl acids such as glucuronic acid and galacturonic acid, alpha-hydroxy acids such as citric acid and tartaric acid, amino acids such as aspartic acid and glutamic acid, aromatic acids such as benzoic acid and cinnamic acid, sulfonic acids such a p-toluenesulfonic acid or ethanesulfonic acid,

If the inventive compound is an acid, the desired salt may be prepared by any suitable method known to the art, including treatment of the free acid with an inorganic or organic base, such as an amine (primary, secondary or tertiary), an alkali metal or alkaline earth metal hydroxide or the like. Illustrative examples of suitable salts include organic salts derived from amino acids such as glycine and arginine, ammonia, primary, secondary and tertiary amines, and cyclic amines such as piperidine, morpholine and piperazine, and inorganic salts derived from sodium, calcium, potassium, magnesium, manganese, iron, copper, zinc, aluminum and lithium.

In the case of compounds, salts, or solvates that are solids, it is understood by those skilled in the art that the inventive compounds, salts, and solvates may exist in different crystal forms, all of which are intended to be within the scope of the present invention.

The inventive compounds may exist as single stereoisomers, racemates and/or mixtures of enantiomers and/or diastereomers. All such single stereoisomers, racemates and mixtures thereof are intended to be within the scope of the present invention. Preferably, the inventive compounds are used in optically pure form.

As generally understood by those skilled in the art, an optically pure compound is one that is enantiomerically pure. As used herein, the term "optically pure" is intended to mean a compound which comprises at least a sufficient amount of a single enantiomer to yield a compound having the desired pharmacological activity. Preferably, "optically pure" is intended to mean a compound that comprises at least 90% of a single isomer (80% enantiomeric excess), preferably at least 95% (90% e.e.), more preferably at least 97.5% (95% e.e.), and most preferably at least 99% (98% e.e.).

Preferably in the above formulas I and II, R₁ and R₃₁ are H or F. Preferably in formula I, R₄ is an acyl group or a sulfonyl group. Preferably in formulas I and II, D₁ and D₂ are -OR₂₅, =O, =S, ≡N, =NR₂₅, or -NR₂₅R₂₆, wherein R₂₅ and R₂₆ are independently H, an alkyl group, a cycloalkyl group, a heterocycloalkyl group, an aryl group, or a heteroaryl group, or, together with the nitrogen atom to which they are bonded, form a heterocycloalkyl group, and more preferably D₁ and D₂ are =O. Preferably A₁ and A₂ are C, CH, S, or S(O), and more preferably A₁ and A₂ are C.

Preferably B₁ and B₂ are NR₁₇R₁₈, wherein R₁₇ and R₁₈ are independently H, an alkyl group, a cycloalkyl group, a heterocycloalkyl group, an aryl group, a heteroaryl group, an acyl group, or wherein R₁₇ and R₁₈, together with the atom(s) to which they are bonded, form a heterocycloalkyl group.

Preferably Z and Z₁ are independently H, an aryl group, or a heteroaryl group, -C(O)R₂₁, -CO₂R₂₁, -CN, -C(O)NR_{21,}R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃, or -C(S)NR₂₁NR₂₂R₂₃; wherein R₂₁, R₂₂, and R₂₃ are independently H, an alkyl group, a cycloalkyl group, a heterocycloalkyl group, an aryl group, a heteroaryl group, or an acyl group, or wherein any two of R₂₁, R₂₂, and R₂₃, together with the atom(s) to which they are bonded, form a heterocycloalkyl group, or Z and Z₁, together with the atoms to which they are attached, form a heterocycloalkyl group.

Preferably R₃₂ is one of the folowing moieties: 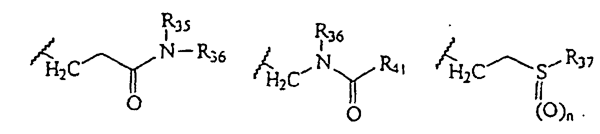 wherein R₃₅, R₃₆, R₃₇, R₄₁ and n are as defined above.

Compounds according to formula I include the following, where * indicates point of attachment:
Compounds 2, 3, 4, 5, 7, 11, 12, 13, 14, 16, 17, 18, 19, 21, 22, 24, 25, 41-43, 74, and 75 having the formula **III**: 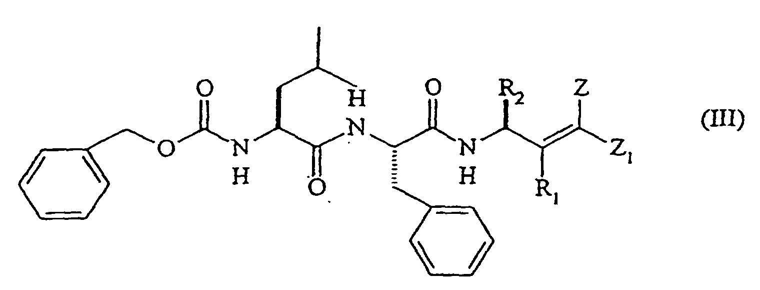
2. R₂ is CH₂CH₂C(O)NHCPh₃, R₁ is H, Z is H, and Z₁ is CO₂CH₂CH₃
3. R₂ is CH₂CH₂C(O)NH₂, R₁ is H, Z is H, and Z₁ is CO₂CH₂CH₃
4. R₂ is CH₂NHC(O)CH₃; R₁ is H, Z is H, and Z₁ is CO₂CH₂CH₃
5. R₂ is 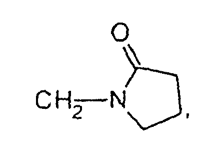 R₁ is H, Z is H, and Z₁ is CO₂CH₂CH₃
7. R₂ is CH₂CH₂C(O)NH₂, R₁ is H, Z is CO₂CH₃, and Z₁ is H
11. R₂ is CH₂CH₂C(O)NH₂, R₁ is H, Z is H, and Z₁ is CO₂CH₃
12. R₂ is CH₂CH₂S(O)CH₃, R₁ is H, Z is H, and Z₁ is CO₂CH₂CH₃
13. R₂ is CH₂CH₂C(O)NH₂, R₁ is H, Z is H, and Z₁ is C(O)CH₃
14. R₂ is CH₂CH₂C(O)NH₂, R₁ is H, Z is H, and Z₁ is CN
16. R₂ is CH₂NHC(O)NH₂, R₁ is H, Z is H, and Z₁ is CO₂CH₂CH₃
17. R₂ is CH₂CH₂C(O)NH₂, R₁ is H, Z is H, and Z₁ is CO₂CH(CH₃)₂
18. R₂ is CH₂CH₂C(O)NH₂, R₁ is H, Z is H, and Z₁ is 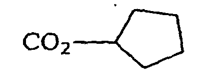
19. R₂ is CH₂CH₂C(O)NH₂, R₁ is H, Z is H, and Z₁ is 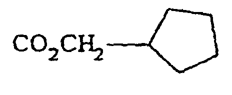
21. R₂ is CH₂CH₂C(O)NH₂, R₁ is H, Z is H, and Z₁ is 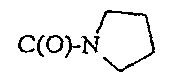
22. R₂ is CH₂CH₂C(O)NH₂, R₁ is H, Z is H, and Z₁ is C(O)N(CH₃)₂
24. R₂ is CH₂CH₂C(O)NH₂; R₁ is H, Z is H, and Z₁ is C(O)Ph
25. R₂ is CH₂CH₂C(O)NH₂; R₁ is H, Z is H, and Z₁ is 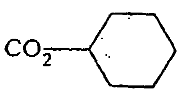
41. R₁ is H; R₂ is CH₂CH₂C(O)NH₂; Z is H; and Z₁ is 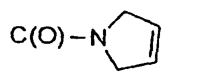
42. R₂ is CH₂CH₂C(O)NH₂, R₁ is H, Z is H, and Z₁ is 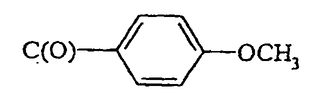
43. R₁ is H; R₂ is CH₂CH₂C(O)NH₂; Z is H; and Z₁ is 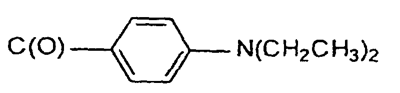
74. R₁ is H; R₂ is CH₂CH,C(O)NH₂; Z is H; and Z₁ is CH₂Cl
75. R₁ is H; R₂ is CH₂CH₂C(O)NH₂; Z is H; and Z₁ is

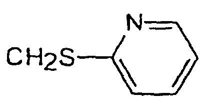

Compounds (26, 27, and 28) having the formula IV: 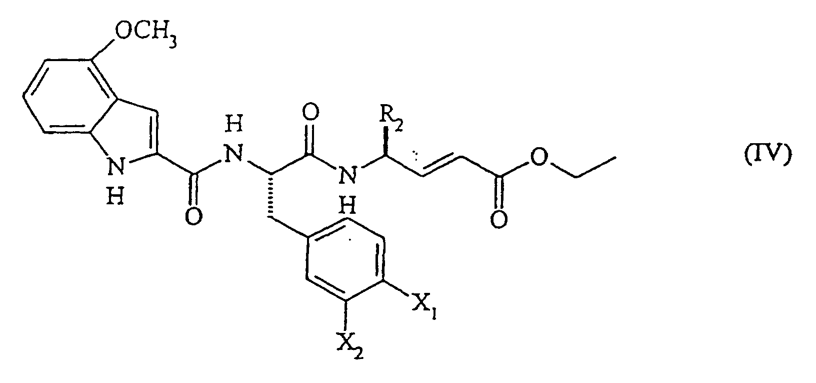 where X₁ and X₂ independently are H, F, or Cl,
26. R₂ is CH₂CH₂C(O)NH₂, X₁ is Cl and X₂ is H
27. R₂ is CH₂CH₂C(O)NH₂, X₁ is F and X₂ is H
28. R₂ is CH₂CH₂C(O)NH₂, X₁ is H and X₂ is F
Compounds (30-34) having the formula V: 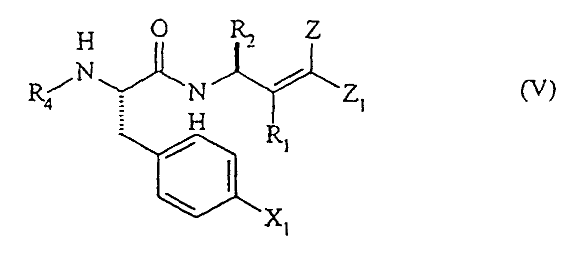
30. R₄ is PhCH₂OC(O), X₁ is H, R₂ is CH₂CH₂C(O)NH₂, R₁ is H, Z is H, and Z₁ is CO₂CH₂CH₃
31. R₄ is CH₃CH₂CH₂SO₂, X₁ is H, R₂ is CH₂CH₂C(O)NH₂, R₁ is H, Z is H, and Z₁ is CO₂CH₂CH₃
32. R₄ is PhCH₂SO₂, X₁ is H, R₂ is CH₂CH₂C(O)NH₂, R₁ is H, Z is H, and Z₁ is CO₂CH₂CH₃
33. R₄ is CH₃CH₂SO₂, X₁ is H, R₂ is CH₂CH₂C(O)NH₂, R₁ is H, Z is H, and Z₁ is CO₂CH₂CH₃
34. R₄ is PhSO₂, X₁ is H, R₂ is CH₂CH₂C(O)NH₂, R₁ is H, Z is H, and Z₁ is CO₂CH₂CH₃
Compound 29 having the formula VI: 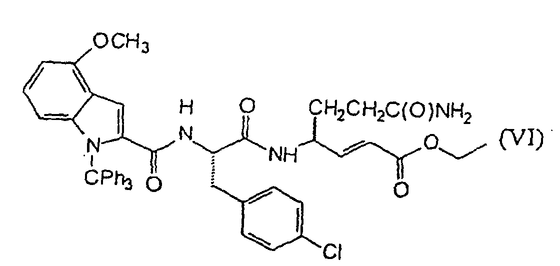 Compound 44 having the formula VII: 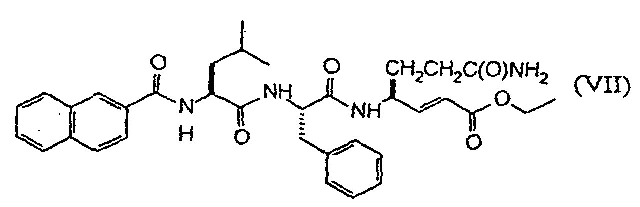 Compounds (35-37) having the formula VIII: 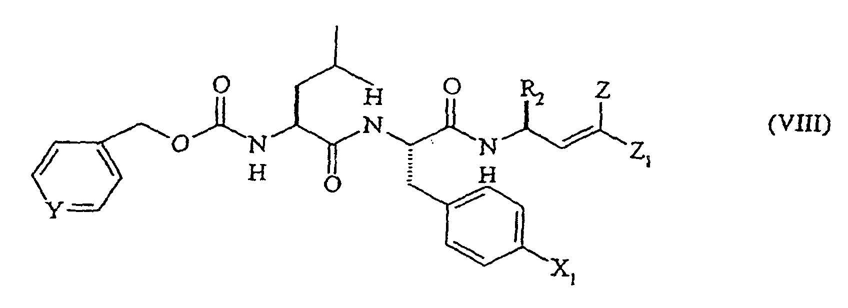
35. X₁ is F, R₂ is CH₂CH₂C(O)NH₂, Y is CH, Z is H, and Z₁ is CO₂CH₂CH₃
36. X₁ is H, R₂ is CH₂CH₂C(O)NH₂, Y is N, Z is H, and Z₁ is CO₂CH₂CH₃
37. X₁ is H, R₂ is CH₂CH₂C(O)NH₂, Y is CH, Z is H, and Z₁ is C(O)N(CH₃)OCH₃
Compounds 46-66 and 78 having the formula IX: 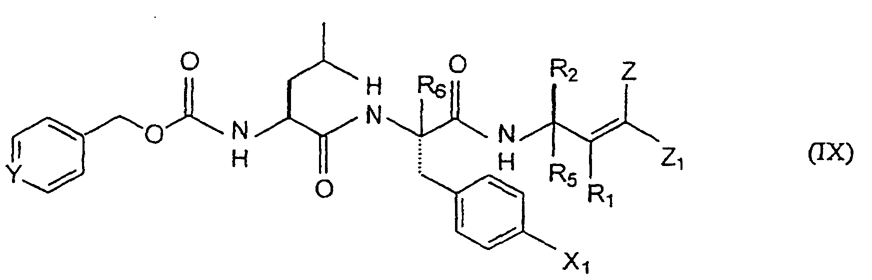
46. R₁ is H; R₂ is CH₂CH₂C(O)NH₂; R₅, R₆, and X₁ are H; Y is CH; Z is H; and Z₁ is 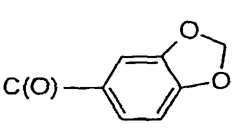
47. R₁ is H; R₂ is CH₂CH₂C(O)NH₁₂; R₅, R₆ and X₁ are H; Y is CH; Z is H; and Z₁ is 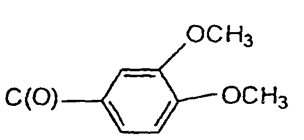
48. R₁ is H; R₂ is CH₂CH₂C(O)NH₂; R₅, R₆, and X₁ are H; Y is CH; Z is H; and Z₁ is 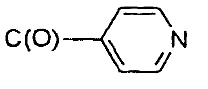
49. R₁ is H; R₂ is CH₂CH₂C(O)NH₂; R₅, R₆, and X₁ are H; Y is CH; Z is H; and Z₁ is 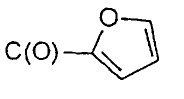
50. R₁ is H; R₂ is CH₂CH₂C(O)NH₂; R₅, R₆, and X₁ are H; Y is CH; Z is H; and Z₁ is 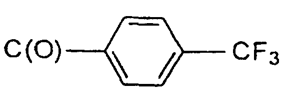
51. R₁ is H; R₂ is CH₂CH₂C(O)NH₂; R₅, R₆, and X₁ are H; Y is CH; Z is H; and Z₁ is 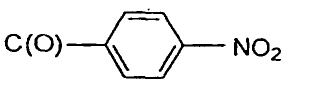
52. R₁ is H; R₂ is CH₂CH₂C(O)NH₂; R₅, R₆, and X₁ are H; Y is CH; Z is H; and Z₁ is C(O)tBu
53. R₁ is H; R₂ is CH₂CH₂C(O)NH₂; R₅ and R₆ are H; X₁ is OH; Y is CH; Z is H; and Z₁ is CO₂CH₂CH₃
54. R₁ is H; R₂ is CH₂CH₂C(O)NH₂; R₅, R₆, and X₁ are H; Y is CH; Z is H; and Z₁ is C(O)C(O)CH₃
55. R₁ is H; R₂ is CH₂CH₂C(O)NH₂; R₅, R₆, and X₁ are H; Y is CH; Z is H; and Z₁ is C(O)C(O)N(CH₃)₂
56. R₁ is H; R₂ is CH₂OC(O)NH₂; R₅, R₆, and X₁ are H; Y is CH; Z is H; and Z₁ is CO₂CH₂CH₃
57. R₁ is H; R₂ is CH₂CH₂C(O)NH₂; R₅, R₆, and X₁ are H; Y is CH; Z is H; and Z and Z₁ together form 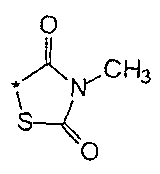 where the S is preferably trans to the R₁ group
58. R₁ is H; R₂ is CH₂CH₂C(O)NH₂; R₅, R₆, and X₁ are H; Y is CH; and Z and Z₁ together form 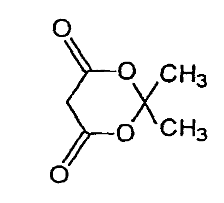
59. R₁ is H; R₂ is CH₂CH₂C(O)NH₂; R₅, R₆, and X₁ are H; Y is CH; Z is H; and Z₁ is C(O)NHPh
60. R₁ is H; R₂ is CH₂CH₂C(O)NH₂; R₅, R₆, and X₁ are H; Y is CH; Z is H; and Z₁ is C(O)N(CH₃)Ph
61. R₁ is H; R₂ is CH₂CH₂C(O)NH₂; R₅, R₆, and X₁ are H; Y is CH; Z is H; and Z₁ is 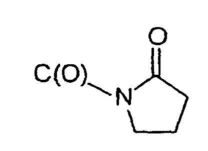
62. R₁ is H; R₂ is CH₂CH₂C(O)NH₂; R₅, R₆, and X₁ are H; Y is CH; Z is H; Z₁ is 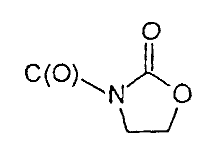
63. R₁, R₅, R₆, X₁, and Z are H; Y is CH; R₂ is CH₂CH₂C(O)NH₂; and Z₁ is 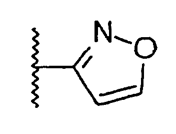
64. R₁, R₅, R₆, X₁, and Z are H; Y is CH; R₂ is CH₂CH₂C(O)NH₂; and Z₁ is 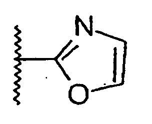
65. R₁, R₅, R₆, X₁, and Z are H; Y is CH; R₂ is CH₂CH₂C(O)NH₂; and Z₁ is 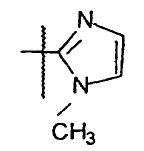
66. R₁, R₅, R₆, X₁, and Z are H; Y is CH; R₂ is CH₂CH₂C(O)NH₂; and Z₁ is 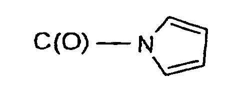
78. R₁, R₅, R₆ and X₁ are H; Y is CH; R₂ is CH₂CH₂C(O)NH₂; Z is CH₂Cl; and Z₁ is H
Compounds 67-69 having the formula X: 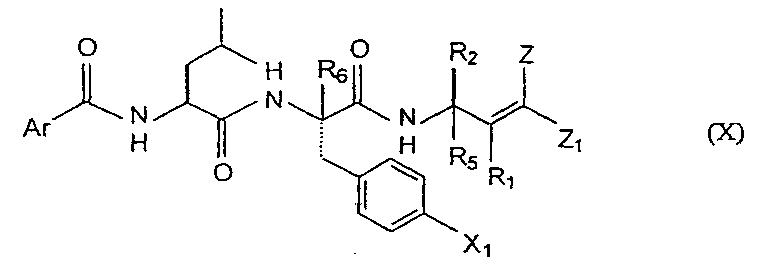
67. R₁, R₅, R₆, X₁, and Z are H; R₂ is CH₂CH₂C(O)NH₂; Z₁ is CO₂CH₂CH₃; and Ar is Ph
68. R₁, R₅, R₆, X₁, and Z are H; R₂ is CH₂CH₂C(O)NH₂; Z₁ is CO₂CH₃; and Ar is 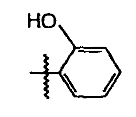
69. R₁, R₅, R₆, X₁, and Z are H; R₂ is CH₂CH₂C(O)NH₂; Z₁ is CO₂CH₂CH₃; and Ar is 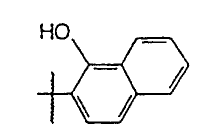
Compounds 70-73 having the formula XI: 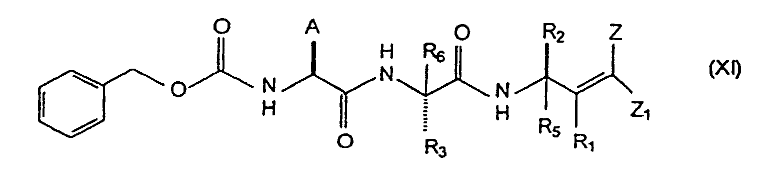
70. R₁, R₅, R₆, and Z are H; R₂ is CH₂CH₂C(O)NH₂; R₃ is CH₂Ph; Z₁ is CO₂CH₂CH₃; and A is 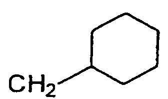
71. R₁, R₅, R₆, and Z are H; R₂ is CH₂CH₂C(O)NH₂; R₃ is CH₂Ph; Z₁ is CO₂CH₂CH₃; and A is Ph
72. R₁, R₅, R₆, and Z are H; R₂ is CH₂CH₂C(O)NH₂; A is CH₂CH(CH₃)₂; Z₁ is CO₂CH₂CH₃; and R₃ is 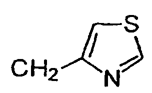
73. R₁, R₅, R₆, and Z are H; R₂ is CH₂CH₂C(O)NH₂; A is CH₂CH(CH₃)₂; Z₁ is CO₂CH₂CH₃; and R₃ is 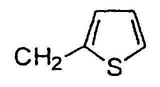
Compounds 1, 6, 8-10, 15, 20, 23, 38-40, 76, and 77 having the formula XII: 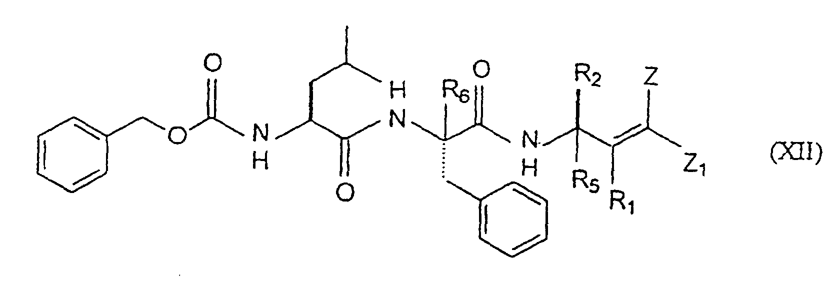
1. R₁ is H; R₂ is CH₂CH₂CN; R₅ is H; R₆ is H; Z is F; and Z₁ is CO₂CH₂CH₃
6. R₁ is H; R₂ is CH₂CH₂C(O)NH₂; R₅ is H; R₆ is H; Z is H; and Z₁ is C(O)NHCH₂CH₃
8. R₁ is H; R₂ is CH₂CH₂C(O)NH₂; R₅ is H; R₆ is H; Z is F; and Z₁ is CO₂CH₂CH₃
9. R₁ is H; R₂ is CH₂CH₂C(O)NH₂; R₅ is H; R₆ is H; Z is H; and Z₁ is SO₂CH₃
10. R₁ is H; R₂ is CH₂CH₂C(O)NH₂; R₅ is H; R₆ is H; Z is H; and Z₁ is SO₂Ph
15. R₁ is H; R₂ is CH₂CH₂C(O)NH₂; R₅ is H; R₆ is H; Z is H; and Z₁ is CO₂H
20. R₁ is H; R₂ is CH₂CH₂C(O)NH₂; R₅ is H; R₆ is H; Z is H; and Z₁ is PO(OCH₂CH₃)₂
23. R₁ is H; R₂ is CH₂CH₂C(O)NH₂; R₅ is H; R₆ is H; Z is H; and Z₁ is 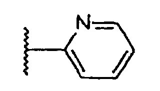
38. R₁ is H; R₂ is CH₂CH₂C(O)NH₂; R₅ is H; R₆ is H; Z is H; and Z₁ is 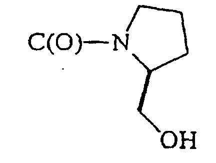
39. R₁ is H; R₂ is CH₂CH₂C(O)NH₂; R₅ is H; R₆ is H; Z is H; and Z₁ is 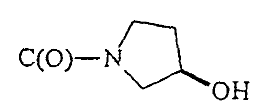
40. R₁ is H; R₂ is CH₂CH₂C(O)NH₂; R₅ is H; R₆ is H; Z is H; and Z₁ is 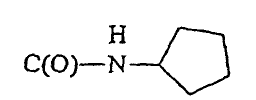
76. R₁ is H; R₂ is CH₂CH₂C(O)NH₂; R₅ is H; R₆ is H; Z is H; and Z₁ is CH₂OAc
77. R₁ is H; R₂ is CH₂CH₂C(O)NH₂; R₅ is H; R₆ is H; Z is H; and Z₁ is 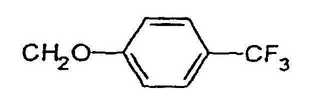
Compound 45 having the formula XIII:
45. 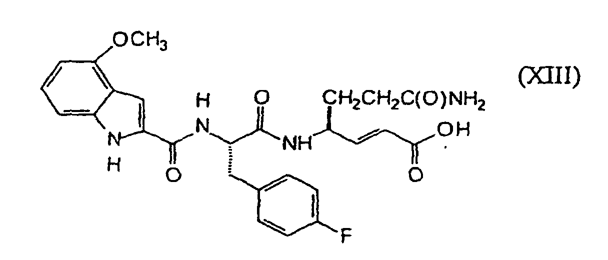
Compounds 79-97, also having the formula III: 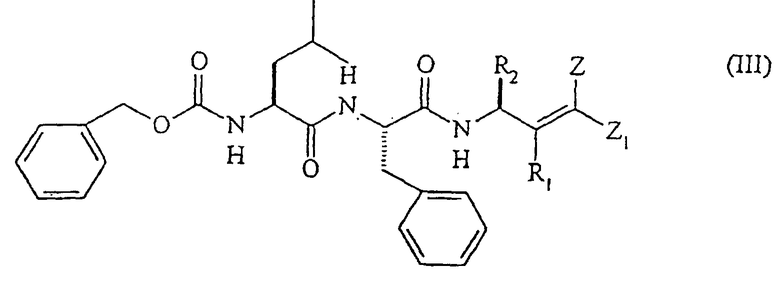
82. R₂ is CH₂CH₂C(O)NH₂, R₁ is H, Z is CH₃ and Z₁ is CO₂CH₂CH₃
90. R₂ is CH₂CH₂C(O)NH₂, R₁ is H, and Z and Z₁ together form 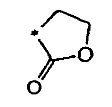 where C=O is preferably cis to the R₁ group
   or wherein R₂ is CH₂CH₂C(O)NH₂, R₁ is H, Z is H, and Z₁ is selected from: 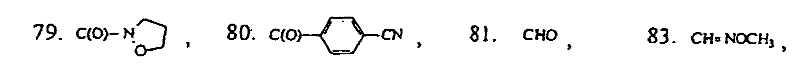 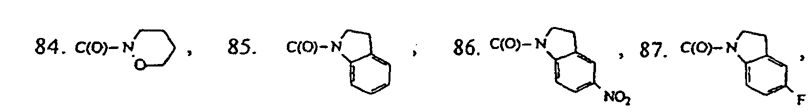 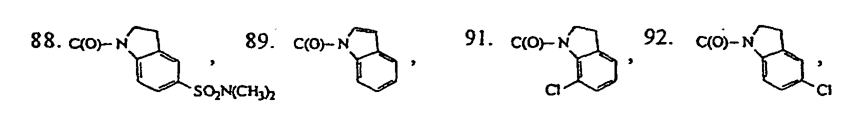 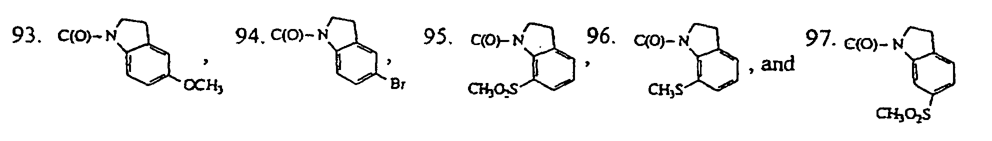
Compounds 98-121 having formula XIV: 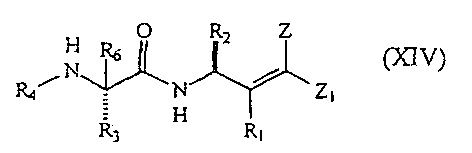 wherein R₆ is H, R₁ is H, R₂ is CH₂CH₂C(O)NH₂, Z is H, Z₁ is CO₂CH₂CH₃, and
98. R₃ is CH₂Ph and R₄ is 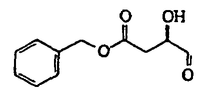
99. R₃ is H and R₄ is 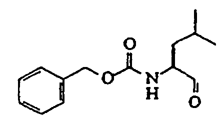
100. R₃ is 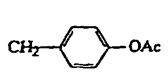 and R₄ is 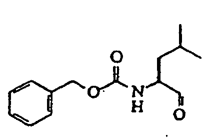
101. R₃ is CH₂Ph and R₄ is 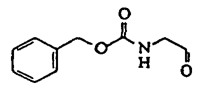
102. R₃ is CH₂Ph and R₄ is 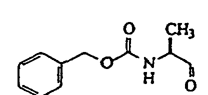
103. R₃ is 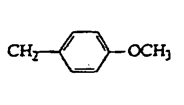 and R₄ is 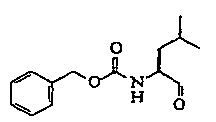
104. R₃ is CH₂Ph and R₄ is 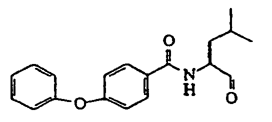
105. R₃ is 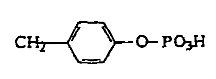 and R₄ is 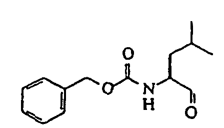
106. R₃ is CH₂Ph and R₄ is 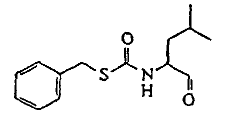
107. R₃ is CH₂Ph and R₄ is 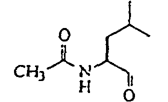
108. R₃ is CH₂CH₃ and R₄ is 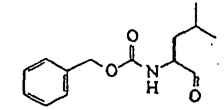
109. R₃ is CH₃ and R₄ is 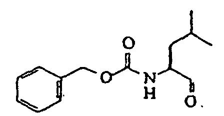
110. R₃ is CH₂Ph and R₄ is 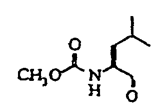
111. R₃ is CH₂Ph and R₄ is 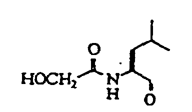
112. R₃ is 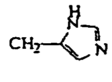 and R₄ is 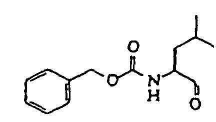
113. R₃ is 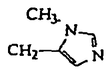 and R₄ is 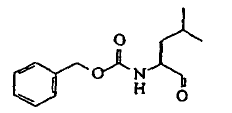
114. R₃ is 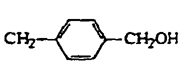 and R₄ is 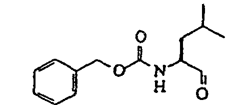
115. R₃ is CH₂Ph and R₄ is 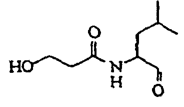
116. R₃ is CH₂Ph and R₄ is 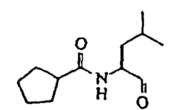
117. R₃ is CH₂Ph and R₄ is 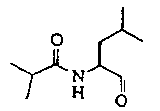
118. R₃ is CH₂Ph and R₄ is 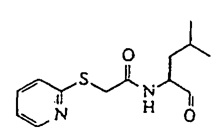
119. R₃ is CH₂Ph and R₄ is 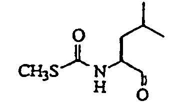
120. R₃ is CH₂Ph and R₄ is 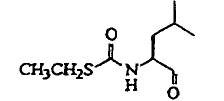
121. R₃ is CH₂CH₂CO₂H and R₄ is 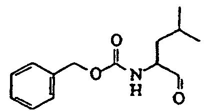 Compounds 122-130, also having the formula XIV: 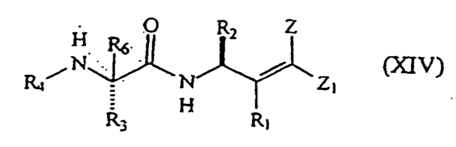 wherein R₆ is H, R₁ is H, R₃ is CH₂Ph and
122. R₂ is CH₂OC(O)NHC(O)CH₂Cl, Z is H, Z₁ is CO₂CH₂CH₃ and R₄ is 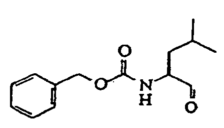
123. R₂ is CH₂CH₂C(O)NH₂, Z is H, Z₁ is CO₂CH₂CH₃ and R₄ is 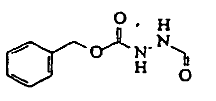
124. R₂ is CH₂CH₂C(O)NH₂, Z is H, Z₁ is 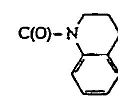 and R₄ is 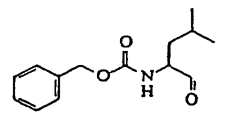
125. R₂ is CH₂CH₂C(O)NH₂, Z is H, Z₁ is NO₂, and R₄ is 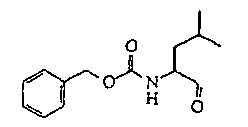
126. R₂ is CH₂CH₂C(O)NH₂, Z is H, Z₁ is 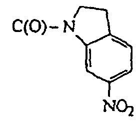 and R₄ is 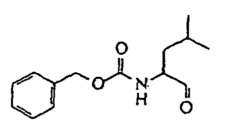
127. R₂ is CH₂CH₂C(O)NH₂, Z is H, Z₁ is 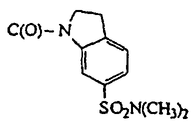 and R₄ is 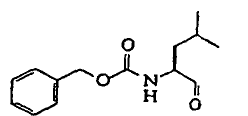
128. R₂ is CH₂CH₂C(O)NH₂, Z is H, Z₁ is 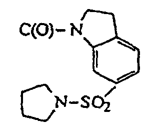 and R₄ is 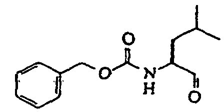
129. R₂ is CH₂CH₂C(O)NH₂, Z is H, Z₁ is CO₂CH₂CH_{3,} and R₄ is 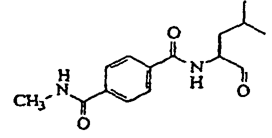
130. R₂ is CH₂CH₂C(O)NH₂, Z and Z₁ together form 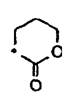 and R₄ is 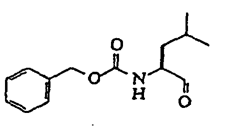 where C=O is preferably cis to the R₁ group.
Compounds 131-145, also having the formula XIV: 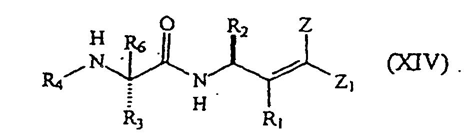 wherein R₆ is H, R₁ is H, R₂ is CH₂CH₂C(O)NH₂, R₄ is 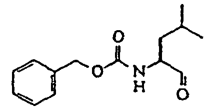 and
131. R₃ is CH₂Ph, Z is H and Z₁ is 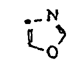
132. R₃ is 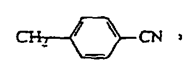 Z is H and Z₁ is CO₂CH₂CH₃
133. R₃ is 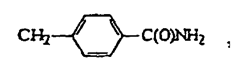 Z is H and Z₁ is CO₂CH₂CH₃
134. R₃ is CH(OH)CH₃, Z is H and Z₁ is CO₂CH₂CH₃
135. R₃ is 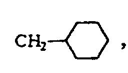 Z is H and Z₁ is CO₂CH₂CH₃
136. R₃ is 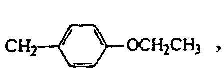 Z is H and Z₁ is CO₂CH₂CH₃
137. R₃ is CH₂CH₂CH₃, Z is H and Z₁ is CO₂CH₂CH₃
138. R₃ is CH₂Ph, Z is H and Z₁ is C(O)N(OH)CH₃
139. R₃ is 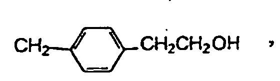 Z is H and Z₁ is CO₂CH₂CH₃
140. R₃ is 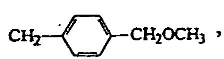 Z is H and Z₁ is CO₂CH₂CH₃
141. R₃ is CH₂CH(CH₃)₂, Z is H and Z₁ is CO₂CH₂CH₃
142. R₃ is CH₂SCH₃, Z is H and Z₁ is CO₂CH₂CH₃
143. R₃ is CH₂SCH₂CH₃, Z is H, and Z₁ is CO₂CH₂CH₃
144. R₃ is CH₂Ph, Z is CH₃, and Z₁ is CO₂H,
145. R₃ is CH₂Ph, Z is H, and Z₁ is 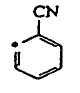
Compounds 146-155, also having the formula XIV: 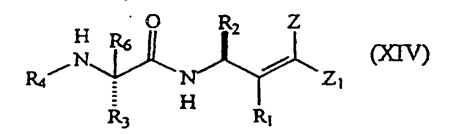 wherein R₆ is H, R₁ is H, R₂ is CH₂CH₂C(O)NH₂, Z is H, and
146. Z₁ is CO₂CH₂CH₃, R₃ is CH₂Ph, and R₄ is 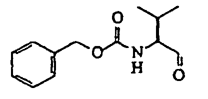
147. Z₁ is CO₂CH₂CH₃, R₃ is CH₂Ph, and R₄ is 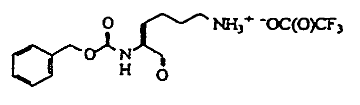
148. Z₁ is CO₂CH₂CH₃, R₃ is CH₂Ph, and R₄ is 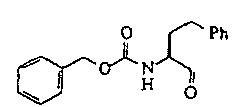
149. Z₁ is CO₂CH₂CH₃, R₃ is CH₂Ph, and R₄ is 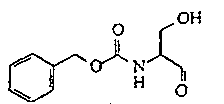
150. Z₁ is 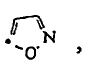 R₃ is CH₂Ph, and R₄ is 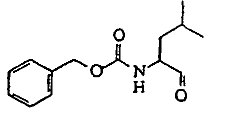
151. Z₁ is CO₂CH₂CH₃, R₃ is CH₂Ph, and R₄ is 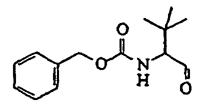
152. Z₁ is CO₂CH₂CH₃, R₃ is CH₂Ph, and R₄ is 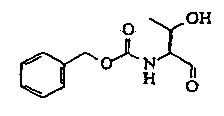
153. Z₁ is CO₂CH₂CH₃, R₃ is CH₂Ph, and R₄ is 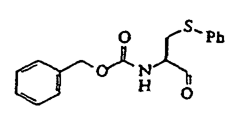
154. Z₁ is CO₂CH₂CH₃, R₃ is CH₂Ph, and R₄ is 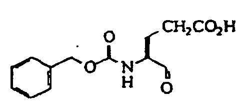
155. Z₁ is CO₂CH₂CH₃, R₃ is 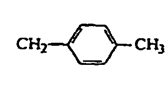 and R₄ is 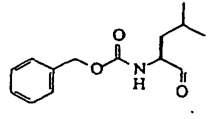
Compounds 156-173, also having formula XIV: 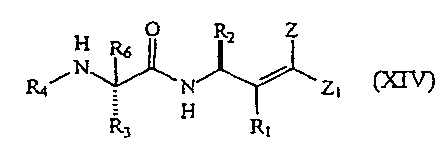 wherein R₆ is H, R₃ is CH₂Ph, R₂ is CH₂CH₂C(O)NH₂, and
157. R₁ is H, Z is H, Z₁ is CO₂CH₂CH₃, and R₄ is 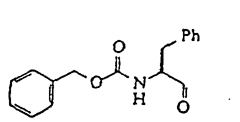
158. R₁ is H, Z is H, Z₁ is CO₂CH₂CH₃, and R₄ is 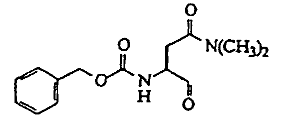
159. R₁ is H, Z is H, Z₁ is CO₂CH₂CH₃, and R₄ is 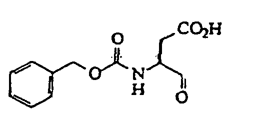
160. R₁ is H, Z is H, Z₁ is 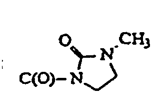 and R₄ is 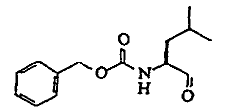
161. R₁ is H, Z is H, Z₁ is CO₂CH₂CH₃, and R₄ is 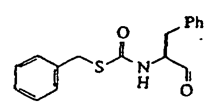
162. R₁ is H, Z is H, Z₁ is CO₂CH₂CH₃, and R₄ is 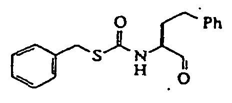
163. R₁ is H, Z is H, Z₁ is CO₂CH₂C(CH₃)₃, and R₄ is 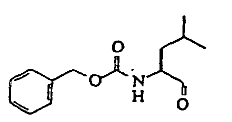
164. R₁ is H, Z and Z₁ together form 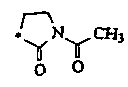 and R₄ is 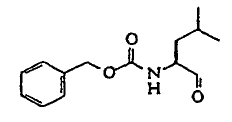 where C=O is preferably cis to the R₁ group
165. R₁ is H, Z is H, Z₁ is CO₂CH₂CH₃, and R₄ is 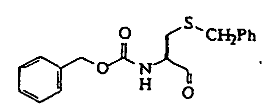
166. R₁ is H, Z is H, Z₁ is CO₂CH₂CH₃, and R₄ is 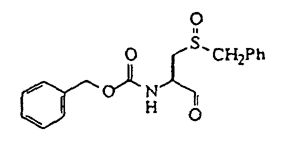
167. R₁ is H, Z is H, Z₁ is CO₂CH₂CH_{3,} and R₄ is 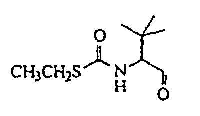
168. R₁ is H, Z is CH₃, Z₁ is CO₂CH₂CH₃, and R₄ is 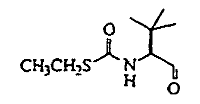
169. R₁ is H, Z and Z₁ together form 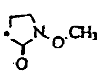 and R₄ is 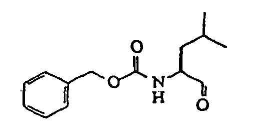 where C=O is preferably cis to R₁
170. R₁ is H, Z is H, Z₁ is CO₂CH₂CH₃, and R₄ is 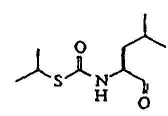
171. R₁ is H, Z is CH₃, Z₁ is CO₂CH₂CH₃, and R₄ is 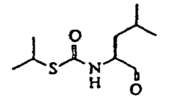
172. R₁ is H, Z is H, Z₁ is CO₂CH₂CH₃, and R₄ is 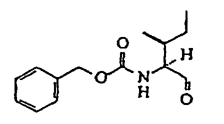
173. R₁ is H, Z is H, Z₁ is CO₂CH₂CH₃, and R₄ is 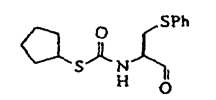
Compounds 174-188, also having the formula XIV: 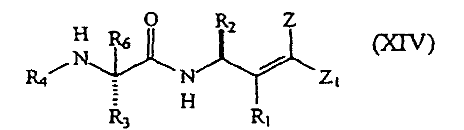 wherein R₆ is H, R₂ is CH₂CH₂C(O)NH₂, R₁ is H, and
174. Z is H, Z₁ is CO₂CH₂CH₃, R₃ is 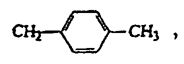 and R₄ is 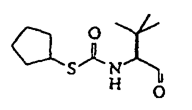
175. Z is CH₃, Z₁ is CO₂CH₂CH₃, R₃ is 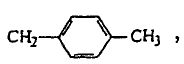 and R₄ is 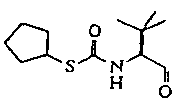
176. Z is H, Z₁ is CO₂CH₂CH₃, R₃ is 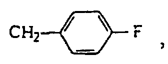 and R₄ is 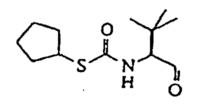
177. Z is CH₃, Z₁ is CO₂CH₂CH₃, R₃ is 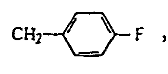 and R₄ is 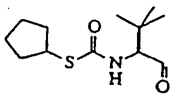
178. Z is H, Z₁ is CO₂CH₂CH₃, R₃ is CH₂Ph, and R₄ is 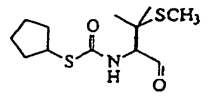
179. Z is H, Z₁ is CO₂CH₂CH₃, R₃ is CH₂Ph, and R₄ is 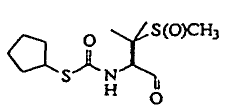
180. Z is H, Z₁ is CO₂CH₂CH₃, R₃ is CH₂Ph, and R₄ is 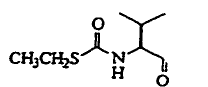
181. Z and Z₁ together form 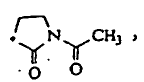 R₃ is 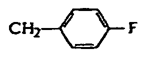 and R₄ is 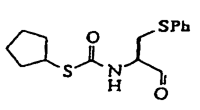 where C=O is preferably cis to the R₁ group
182. Z is H, Z₁ is CO₂CH₂CH₃, R₃ is CH₂Ph, and R₄ is 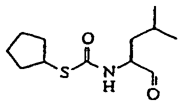
183. Z is H, Z₁ is CO₂CH₂CH₃, R₃ is 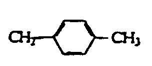 and R₄ is 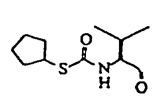
184. Z is H, Z₁ is CO₂CH₂CH₃, R₃ is 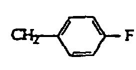 and R₄ is 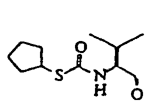
185. Z is H, Z₁ is CO₂CH₂CH₃, R₃ is 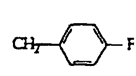 and R₄ is 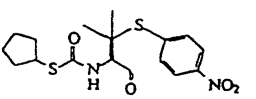
186. Z is H, Z₁ is CO₂CH₂Ph, R₃ is 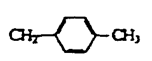 and R₄ is 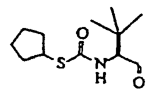
187. Z is CH₃, Z₁ is CO₂CH₂CH₃, R₃ is CH₂Ph and R₄ 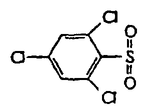 is
188. Z is H, Z₁ is CO₂CH₂CH₂OCH₃, R₃ is 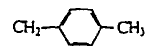 and R₄ is 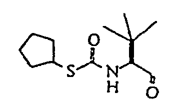
189. R₃ is 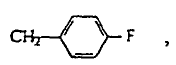 R₄ is 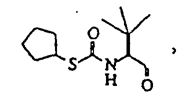 and Z and Z₁ together form 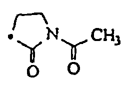 where C=O is preferably cis to the R₁ group
190. Z is H, Z₁ is CO₂CH₂CH₃, R₃ is 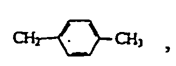 and R₄ is

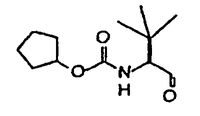

Other compounds according to the invention include the following compounds of formula III: 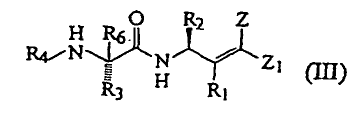 wherein R₆ is H, R₁ is H, R₃ is CH₂Ph, R₂ is CH₂CH₂C(O)NH₂, Z is H, Z₁ is CO₂CH₂CH₃, and R₄ is selected from the following: 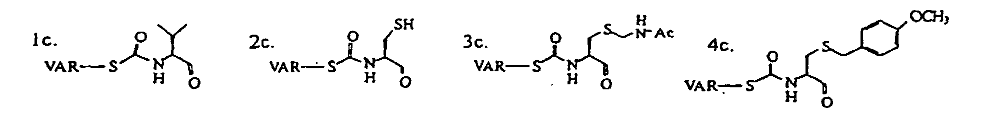 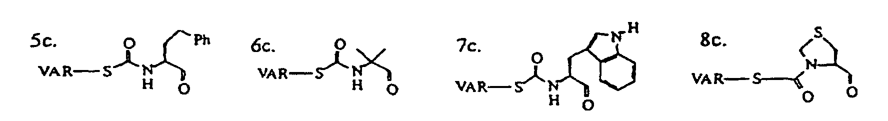 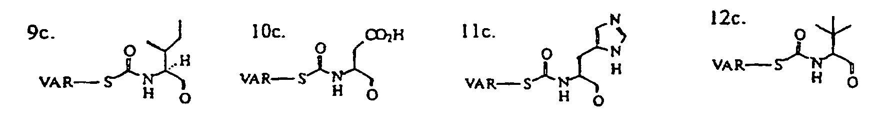 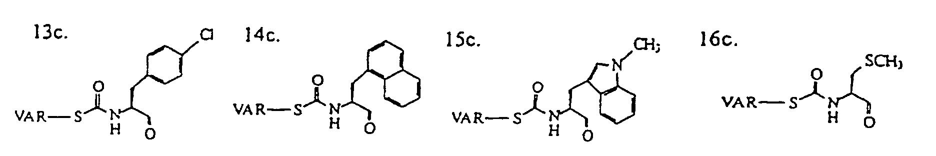 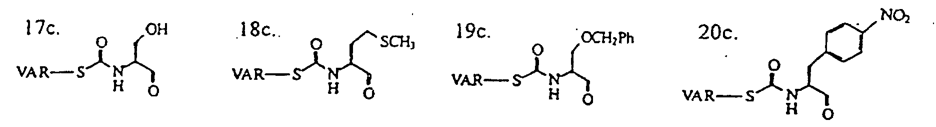 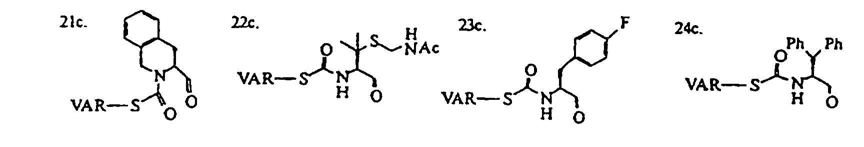 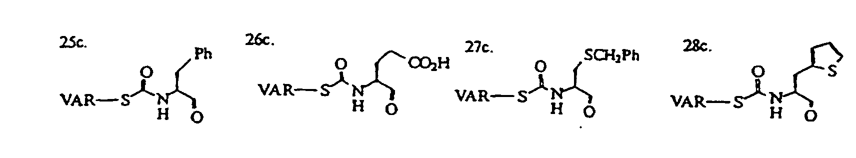 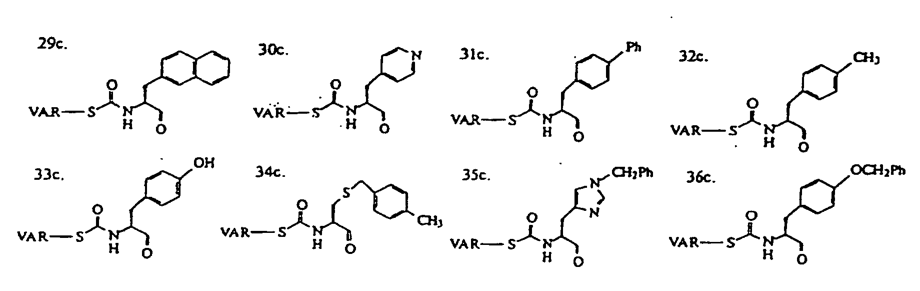 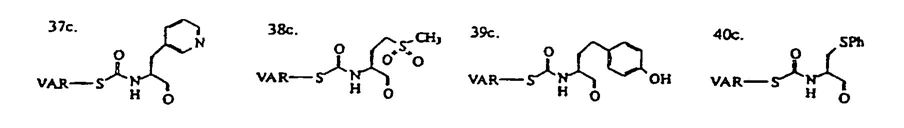 wherein VAR is selected from -CH₂CH₃, -CH(CH₃)₂, -CH₂CH(CH₃)₂, -CH₂-Ph,

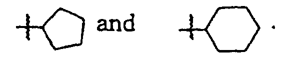

The present invention is further directed to methods of inhibiting picornaviral 3C protease activity that comprises contacting the protease for the purpose of such inhibition with an effective amount of a compound of formula I or a pharmaceutically acceptable salt, or solvate thereof. For example, one can inhibit picornaviral 3C protease activity in mammalian tissue by administering a compound of formula I or II or a pharmaceutically acceptablebe salt, or solvate thereof. More particularly, the present invention is directed to methods of inhibiting rhinoviral protease activity.

The activity of the inventive compounds as inhibitors of picomaviral 3C protease activity may be measured by any of the methods available to those skilled in the art, including in vivo and in vitro assays. Examples of suitable assays for activity measurements include the Antiviral HI-HeLa Cell Culture Assay and the Normal Human Bronchial Epithelial Cell Assay, both described herein.

Administration of the compounds of the formulas I and II, or their pharmaceutically acceptable salts, and solvates, may be performed according to any of the accepted modes of administration available to those skilled in the art. Illustrative examples of suitable modes of administration include, but are not limited to, oral, nasal, parenteral, topical, transdermal and rectal.

The inventive compounds of formulas I and II, and their pharmaceutically acceptable salts, and solvates, may be administered as a pharmaceutical composition in any suitable pharmaceutical form recognizable to the skilled artisan. suitable pharmaceutical forms include, but are not limited to, solid, semisolid, liquid, or lyopholized formulations, such as tablets, powders, capsules, suppositories, suspensions and aerosols. The pharmaceutical composition may also include suitable excipients, diluents, vehicles and carriers, as well as other pharmaceutically active agents, depending upon the intended use.

Acceptable methods of preparing suitable pharmaceutical forms of the pharmaceutical compositions are known to those skilled in the art. For example, pharmaceutical preparations may be prepared following conventional techniques of the pharmaceutical chemist involving steps such as mixing, granulating and compressing when necessary for tablet forms, or mixing, filling and dissolving the ingredients as appropriate, to give the desired products for oral, parenteral, topical, intravaginal, intranasal, intrabronchial, intraocular, intraural and/or rectal administration.

Solid or liquid pharmaceutically acceptable carriers, diluents, vehicles or excipients may be employed in the pharmaceutical compositions. Illustrative solid carriers include starch, lactose, calcium sulphate dihydrate, terra alba, sucrose, talc, gelatin, pectin, acacia, magnesium stearate, and stearic acid. Illustrative liquid carriers may include syrup, peanut oil, olive oil, saline solution, and water. The carrier or diluent may include a suitable prolonged-release material, such as glyceryl monostearate or glyceryl distearate, alone or with a wax. When a liquid carrier is used, the preparation may be in the form of a syrup, elixir, emulsion, soft gelatin capsule, sterile injectable liquid (e.g. solution), or a nonaqueous or aqueous liquid suspension.

A dose of the pharmaceutical composition contains at least a therapeutically effective amount of the active compound (i.e., a compound of formula I or II or a pharmaceutically acceptable salt, or solvate thereof) and preferably is made up of one or more pharmaceutical dosage units. The selected dose may be administered to a mammal, for example, a human patient, in need of treatment mediated by inhibition of 3 C protease activity, by any known method of administering the dose including topical, for example, as an ointment or cream; orally, rectally, for example, as a suppository; parenterally by injection; or continuously by intravaginal, intranasal, intrabronchial, intraaural or intraocular infusion.

A "therapeutically effective amount" is intended to mean that amount of a compound of formula I or II that, when administered to a mammal in need thereof, is sufficient to effect treatment for disease conditions alleviated by the inhibition of the activity of one or more picarrioviral 3C proteases, such as human rhinoviruses, human poliovirus, human coxsackieviruses, encephalomyocarditis viruses, menigovirus, and hepatitis A virus. The amount of a given compound of formula I or II that will correspond to a "therapeutically effective amount" will vary depending upon factors such as the particular compound, the disease condition and the severity thereof, the identity of the mammal in need thereof, but can nevertheless be readily determined by one of skill in the art.

"Treating" or "treatment" is intended to mean at least the mitigation of a disease condition in a mammal, such as a human, that is alleviated by the inhibition of the activity of one or more picarnoviral 3C proteases, such as human rhinoviruses, human poliovirus, human coxsackieviruses, encephalomyocarditis viruses, menigovirus, and hepatitis A virus, and includes:
(a) prophylactic treatment in a mammal, particularly when the mammal is found to be predisposed to having the disease condition but not yet diagnosed as having it;
(b) inhibiting the disease condition; and/or
(c) alleviating, in whole or in part, the disease condition.

The inventive compounds, and their salts, and solvates, may be prepared by employing the techniques available in the art using starting materials that are readily available. Certain novel and exemplary methods of preparing the inventive compounds are described below.

Preferably, the inventive compounds of formulas I and II are prepared by the novel methods of the present invention, including the four general methods shown below. In each of these general methods, R₁, R₂, R₃, R₄, R₅, R₆, Z, and Z₁ are as defined above.

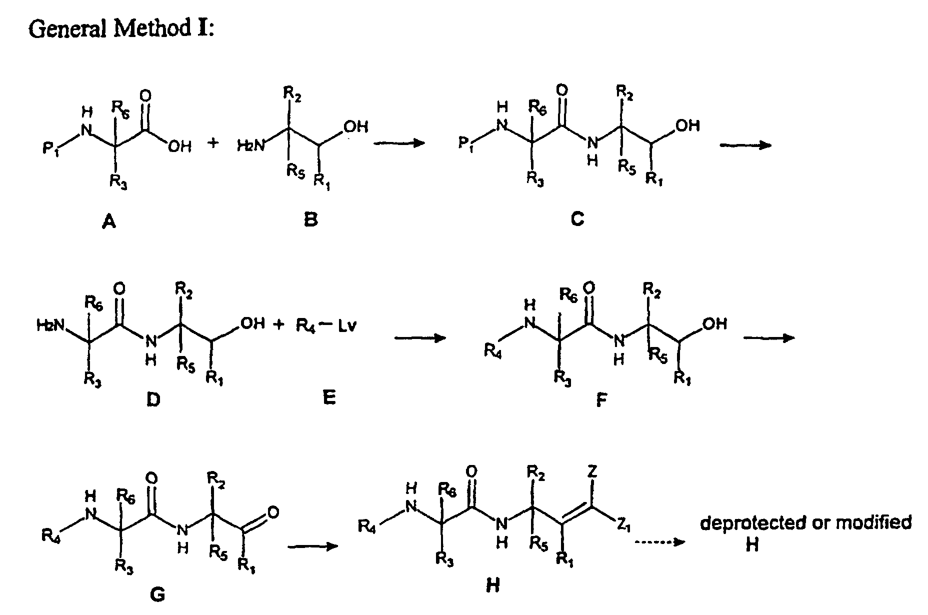

In General Method **I**, protected amino acid **A**, where P₁ is an appropriate protecting group for nitrogen, is subjected to an amide forming reaction with amino alcohol (or salt thereof) **B** to produce amide **C**. Amide **C** is then deprotected to give free amine (or salt thereof) **D**. Amine **D** and compound **E**, where "Lv" is an appropriate leaving group, are subjected to a bond forming reaction generating compound **F**. Compound **F** is oxidized to intermediate **G**, which is then transformed into unsaturated product **H**. If protecting groups are used on any R groups (R₁-R₆) and/or on Z and/or Z₁, product **H** is deprotected and/or further modified to yield "deprotected or modified **H**."

An alternative method to prepare intermediate **F** is described as follows:

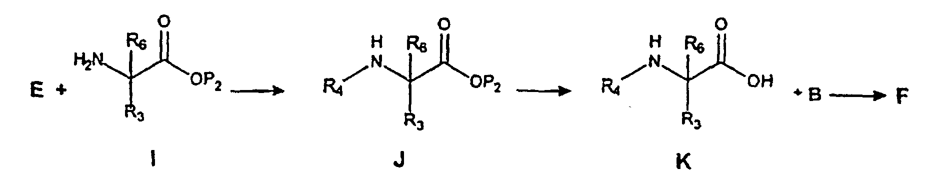

Compound **E** and amino acid (or salt thereof) **I**, where P₂ is an appropriate protecting group for oxygen, are subjected to a bond forming reaction to produce intermediate **J**. Intermediate **J** is deprotected to yield free carboxylic acid **K**, which is subsequently subjected to an amide forming reaction with amino alcohol (or salt thereof) **B** to generate intermediate **F**.

Amino alcohol **B** can be prepared as follows:

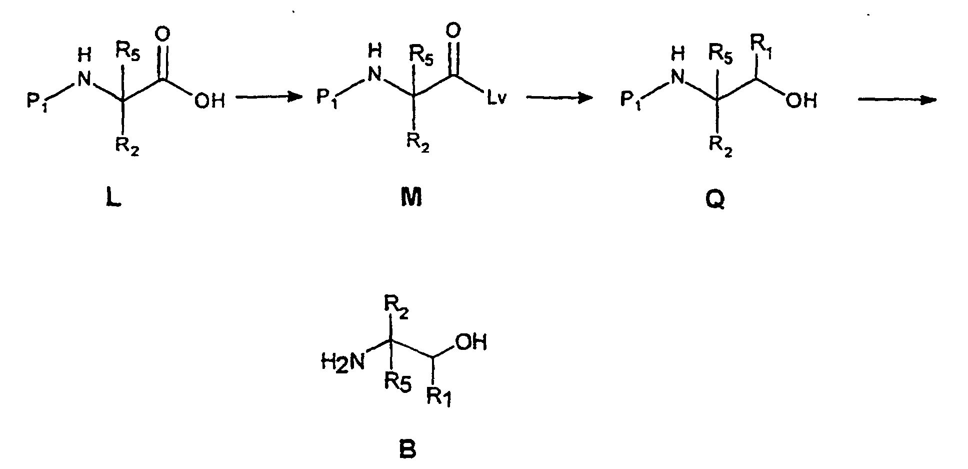

Amino acid **L**, where P₁ is an appropriate protecting group for nitrogen, is converted to carbonyl derivative **M**, where "Lv" is a leaving group. Compound **M** is subjected to a reaction where "Lv" is reduced to protected amino alcohol **Q**. Amino alcohol **Q** is deprotected to give amino alcohol **B**.

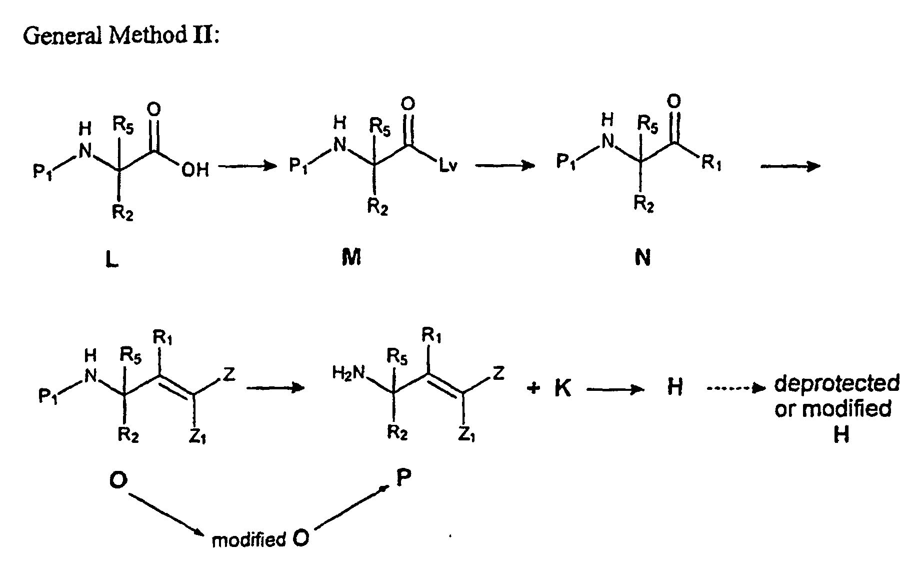

In General Method **II**, amino acid **L**, where P₁ is an appropriate protecting group for nitrogen, is converted to a carbonyl derivative **M**, where "Lv" is a leaving group. Compound **M** is subjected to a reaction where "Lv" is replaced by R₁ to give derivative **N**. Derivative **N** is then transformed into unsaturated product **O**. Unsaturated compound **O** is deprotected to give free amine (or salt thereof) **P**, or modified one or more times at R₂, R₅, Z and/or Z₁ to give one or more modified **O** compounds.

Modified **O** is then deprotected to give amine (or salt thereof) **P**. Amine **P** is subsequently subjected to an amide forming reaction with carboxylic acid **K**, prepared as described in General Method **I**, to give final product **H**. If protecting groups were used on any R group (R₁-R₆) and/or on Z and/or Z₁, product **H** is deprotected and/or further modified to yield "deprotected or modified **H**."

An alternative method to prepare intermediate **N** is described as follows:

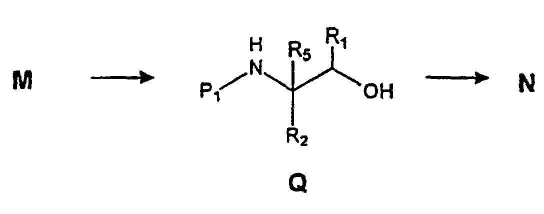

Compound **M** is subjected to a reaction where "Lv" is reduced to protected amino alcohol **Q**. Amino alcohol **Q** is subsequently oxidized to derivative **N**.

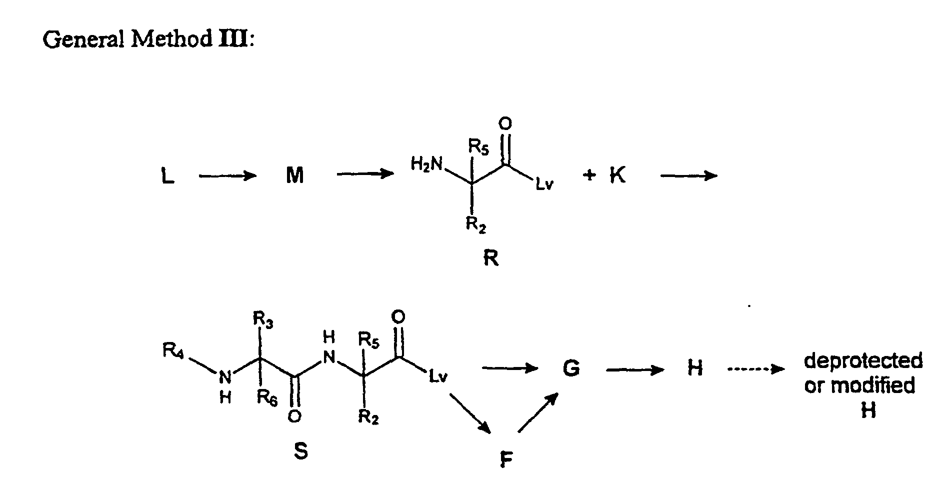

In General Method **III**, amino acid **L**, where P₁ is an appropriate protecting group for nitrogen, is converted to a carbonyl derivative **M**, where "Lv" is a leaving group. Derivative **M** is deprotected to give free amine (or salt thereof) R₁ which subsequently is subjected to an amide forming reaction with carboxylic acid **K** to give intermediate **S**. Intermediate **S** is then either converted directly to carbonyl intermediate **G,** or successively reduced to alcohol **F,** which is then oxidized to **G.** Intermediate **G** is subjected to a reaction to yield the unsaturated final product **H.** If protecting groups were used on any R groups (R₁-R₆) and/or on Z and/or Z₁, product **H** is deprotected and/or further modified to yield "deprotected or modified **H.**"

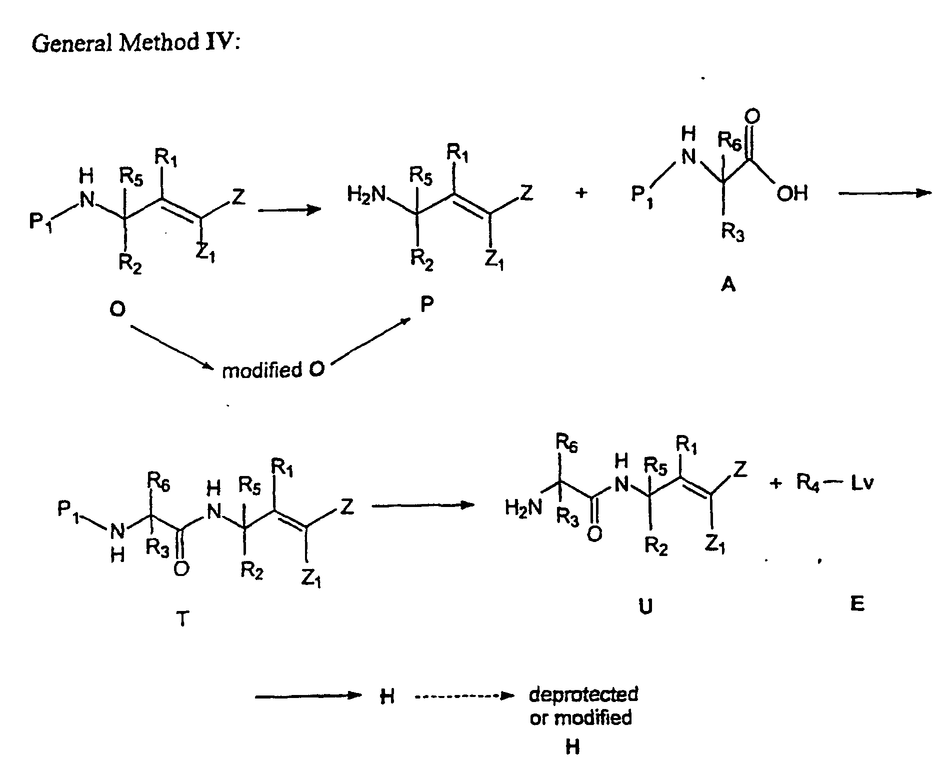

In General Method **IV**, free amine (or salt thereof) **P**, prepared from intermediate **O** as described in General Method **II**, is converted to amide **T** by reaction with amino acid **A**, where P₁ is an appropriate protecting group for nitrogen. Compound **T** is further deprotected to free amine (or salt thereof) **U**, which is subsequently converted to **H** with reactive intermediate **E**. If protecting groups were used on any R groups (R₁-R₆) and/or on Z and/or Z₁, product **H** is deprotected and/or further modified to yield "deprotected or modified **H**."

Preferably the compound of formulas I or II can be prepared by one of four specific methods. For example, compounds 4, 12, 14, 16, 20, 23, 24, 26-30, 35, and 36 can be prepared by Specific Method I:

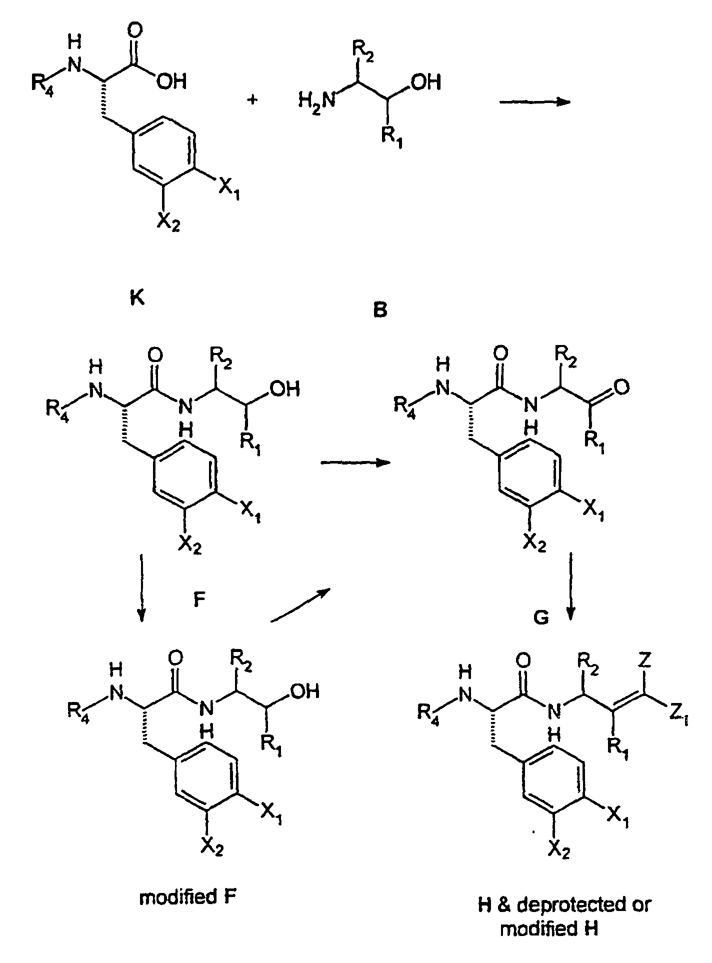

In Specific Method I, carboxylic acid **K**, CBZ-L-Leu-L-Phe, which can be purchased from Bachem or prepared as described in General Method I, is subjected to an amide forming reaction with amino alcohol (or salt thereof) **B** to generate intermediate **F**. Intermediate **F** is oxidized to intermediate **G**, which is then transformed into unsaturated product **H**. In the case of Compound 12, intermediate **F** is oxidized to modified **F**, which is then oxidized to intermediate **G**. If protecting groups were used on any R groups (R₁-R₆) and/or on Z and/or Z₁, product **H** is deprotected and/or further modified to yield "deprotected or modified **H**."

For example, compounds 1-3, 6-11, 17-19, 21, 22, 25, 37-40, and 74-77 can be prepared by Specific Method **II**:

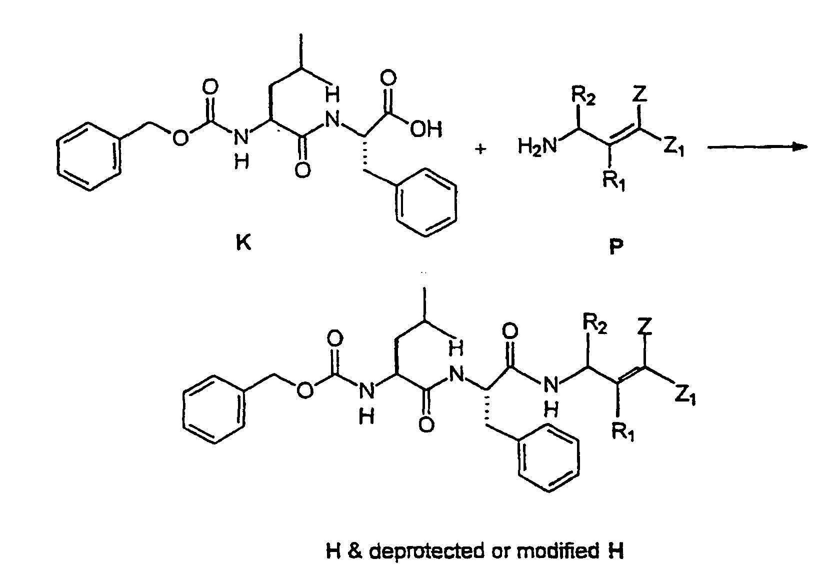

In Specific Method **II**, intermediate **P** (or salt thereof), prepared as described in General Method **II**, is subjected to an amide forming reaction with carboxylic acid **K**, CBZ-L-Leu-L-Phe, which can be purchased from Bachem or prepared as described in General Method **I**, to give final product **H**. If protecting groups were used on any R group (R₁-R₆) and/or on Z and/or Z₁, product **H** is deprotected and/or further modified to yield "deprotected or modified **H**."

For example, compounds 5, 13, and 15 can be prepared by Specific Method **III**:

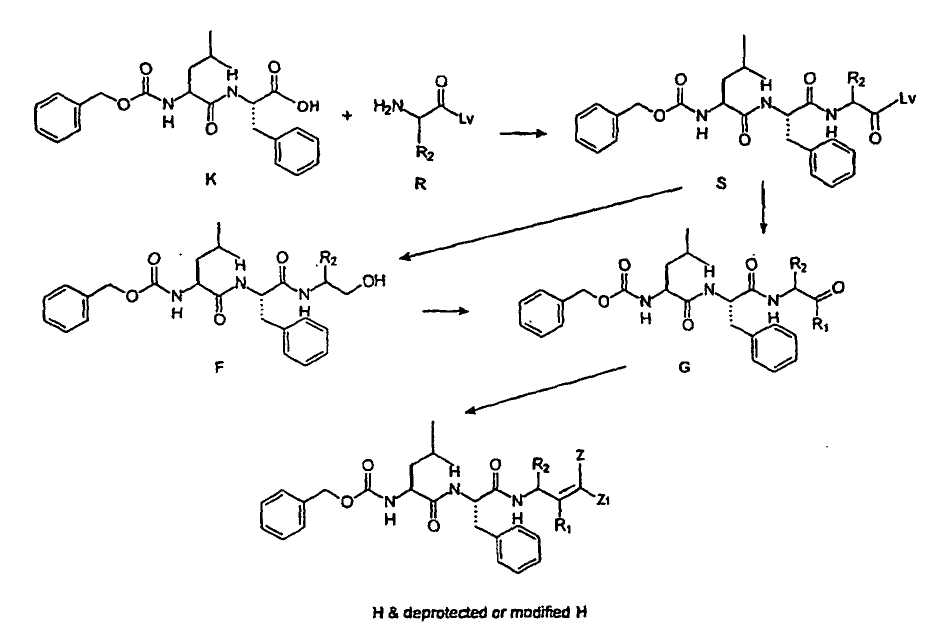

In Specific Method **III**, free amine (or salt thereof) R₁ prepared as described in General Method **III**, is subjected to an amide forming reaction with carboxylic acid **K**, CBZ-L-Leu-L-Phe, which can be purchased from Bachem or prepared as described in General Method **I**, to give intermediate **S**. Intermediate **S** is then either converted directly to carbonyl intermediate **G**, in the case of compounds 13 and 15, or reduced to alcohol **F**, which is then oxidized to intermediate **G**, in the case of compound 5. Intermediate **G** is subjected to a reaction to yield the unsaturated final product **H**. If protecting groups were used on any R groups (R₁-R₆) and/or on Z and/or Z₁, product **H** is deprotected and/or further modified to yield "deprotected or modified **H**."

For example, compounds 31-34 can be prepared by Specific Method **IV**:

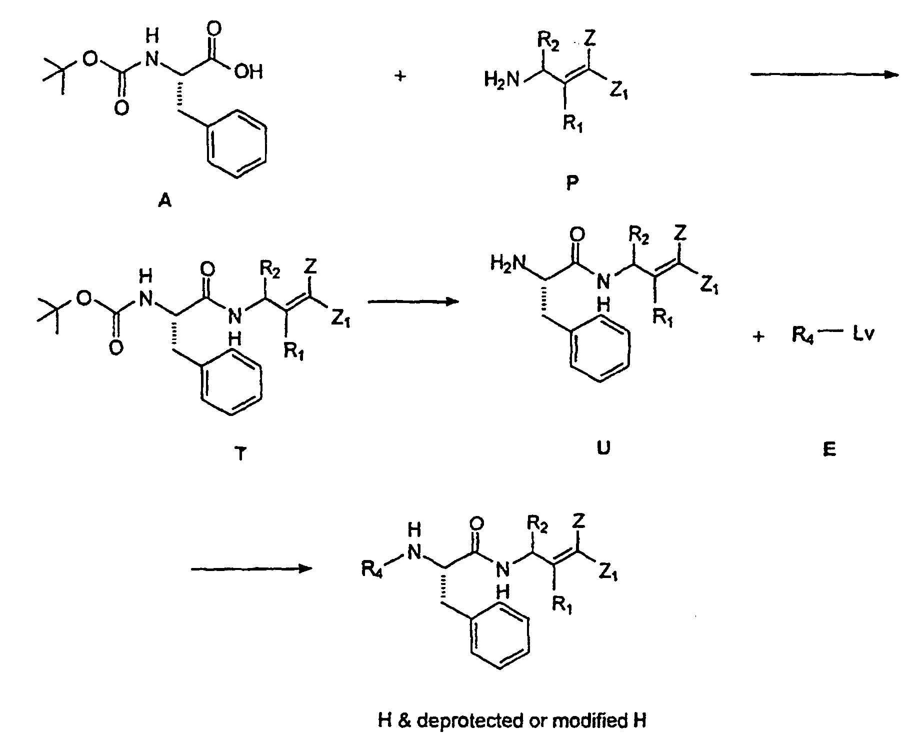

In Specific Method **IV**, free amine (or salt thereof) **P**, prepared as described in General Method **II**, is converted to amide **T** by reaction with protected amino acid **A**, which can be purchased from Bachem, Advanced Chemtech, and Synthetech. Compound **T** is further deprotected to free amine (or salt thereof) **U**, which is subsequently converted to **H** with reactive intermediate **E**. If protecting groups were used on any R groups (R₁-R₆) and/or on Z and/or Z₁, product **H** is deprotected and/or further modified to yield "deprotected or modified **H**."

Suitable protecting groups for nitrogen are recognizable to those skilled in the art and include, but are not limited to benzyloxycarbonyl, t-butoxycarbonyl, 9-fluorenylmethoxycarbonyl, p-methoxybenxyloxycarbonyl, trifluoroacetamide, and p-toluenesulfonyl. Suitable protecting groups for oxygen are recognizable to those skilled in the art and include, but are not limited to -CH₃, -CH₂CH₃, tBu, -CH₂Ph, -CH₂CH=CH₂, -CH₂OCH₂CH₂Si(CH₃)₃, and -CH₂CCl₃. Other examples of suitable protecting groups for nitrogen or oxygen can be found in T. Green & P. Wuts, Protective Groups in Organic Synthesis (2nd ed. 1991), which is incorporated herein by reference.

Suitable leaving groups are recognizable to those skilled in the art and include, but are not limited to, Cl, Br, I, sulfonates, O-alkyl groups, 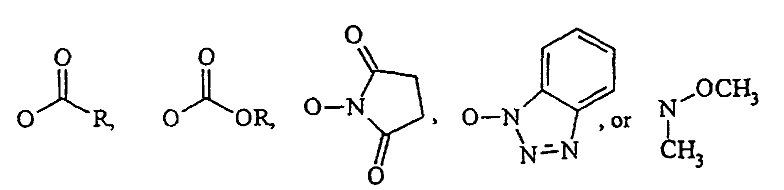 Other examples of suitable leaving groups are described in J. March, Advanced Organic Chemistry, Reactions, Mechanisms, and Structure (4th ed. 1992) at pages 205, 351-56, 642-43, 647, 652-53, 666, 501, 520-21, 569, 579-80, 992-94, 999-1000, 1005, and 1008, which are incorporated herein by reference.

### EXAMPLES

Examples of the processes used to make several of the compounds of formulas I and II are set forth below. The structures of the compounds of the following Examples were confirmed by one or more of the following: proton magnetic resonance spectroscopy, infrared spectroscopy, elemental microanalysis, mass spectrometry, thin layer chromatography and melting point.

Proton magnetic resonance (NMR) spectra were determined using a Tech-Mag or Varian UNITY*plus* 300 spectrometer operating at a field strength of 300 megahertz (MHz). Chemical shifts are reported in parts per million (δ) and setting the references such that in CDCI, the CHCl₃ is at 7.26 ppm, in acetone-*d*₆ the acetone is at 2.02 ppm, and in DMSO-*d*₆ the DMSO is at 2.49 ppm. Peak multiplicities are designated as follows: s, singlet; d, doublet; dd, doublet of doublets; ddd, doublet of doublet of doublets; t, triplet; q, quartet; bs, broad singlet; bt, broad triplet; m, multiplet. Mass spectra (FAB; fast atom bombardment) were determined at the Scripps Research Institute Mass Spectometry Facility, San Diego, CA. Infrared absorption (IR) spectra were taken on a MIDAC Corporation FTIR or a Perkin-Elmer 1600 series FTIR spectrometer.

Elemental microanalysis were performed by Atlantic Microlab Inc. Norcross, Georgia and gave results for the elements stated with ± 0.4% of the theoretical values. Flash chromatography was performed using Silica gel 60 (Merck Art 9385). Thin layer chromatographs (TLC) were performed on precoated sheets of silica 60 F₂₅₄ (Merck Art 5719). Melting points were determined on a Met-Temp apparatus and are uncorrected. Anhydrous *N*,*N*-Dimethylformamide (DMF), *N*,*N*-dimethylacetamide (DMA), dimethysulfoxide (DMSO), were used as is. Tetrahydrofuran (THF) was distilled from sodium benzophenone ketyl under nitrogen.

Et₂O refers to diethyl ether. Pet. ether refers to petroleum ether having a boiling range of 36-53 °C. TFA refers to trifluoroacetic acid. Et₃N refers to triethylamine. Other abbreviations include: methanol (MeOH), ethanol (EtOH), ethyl acetate (EtOAc), acetyl (Ac), methyl (Me), phenyl (Phe), triphenylmethyl (Tr), benzyloxycarbonyl (CBZ), tert-butoxycarbonyl (BOC), *m*-chloroperoxybenzoic acid (*m*-CPBA), alanine (Ala), glutamine (Gln), leucine (Leu), methionine (Met), phenylalanine (Phe), penicillamine (Pen). Additionally, "L" represents natural amino acids, "D" represent unnatural amino acids, and "DL" represents racemic mixtures.

A simplified naming system was used to identify intermediates and final products. Amino acid and peptide alcohols are given the suffix 'ol' (for example methioninol). Amino acid and peptide aldehydes are given the suffix 'al' (for example methioninal). When naming final products, italicized amino acid abbreviations represent modifications at the C-terminus of that residue where the following apply:
1. acrylic acid esters are reported as either "E" (trans) or "Z" (cis) propenoates,
2. acrylonitriles are reported as either E or Z propenonitriles,
3. acrylamides are reported as either E or Z propenamides, except in the case of the compound 21, which is reported as 1-Pyrrolidin-1-yl-3-(CBZ-L-Leu-L-Phe-L-*Gln*)-E-Propenone,
4. vinyl sulfones, vinyl phosphonates, or vinyl aryls are reported as E or Z vinyl sulfones, vinyl phosphonates or aryls, and
5. vinyl ketones are reported as either E or Z en-2-ones.

### Example 1 - Preparation of Compound 12: Ethyl-3-[CBZ-L-Leu-L-Phe-L-Met(sulfoxide)]-E-Propenoate

### Preparation of Intermediate CBZ-L-Leu-L-Phe-L-Methioninol

CBZ-L-Leu-L-Phe (3.02 g, 7.3 mmol) was dissolved in 75 mL of CH₂Cl₂. To this solution was added N-hydroxysuccinimide (0.91 g, 7.7 mmol) and 2 mL of DMF, and stirring was continued until all solids had gone into solution. N,N-Dicyclohexylcarbodiimide (1.60 g, 7.7 mmol) was added to the reaction mixture, and the reaction was stirred at room temperature for one hour. The mixture was then filtered into a separate flask containing S-(-)-methioninol (1.06 g, 7.7 mmol) dissolved in a minimum of DMF, removing the N.N'-dicyclohexylurea precipitate. The reaction was allowed to stir overnight at room temperature. The solvents were removed under vacuum, and the resulting crude product was purified by flash chromatography (anhydrous NH₃/ MeOH/ CHCl₃, 0.5:4.5:9.5) on silica gel to give 3.72 g (96%) of white solid: IR (KBr) 3293, 3065, 2955, 1696, 1645, 1539, 1236, 698 cm⁻¹; ¹H NMR (DMSO-*d*₆) δ 0.80 (m, 6H), 1.31 (m, 2H), 1.51 (m, 2H), 1.82 (m, 1H), 2.00 (s, 3H), 2.43 (m, 2H), 2.78-3.29 (m, 4H), 3.72 (m, 1H), 3.97 (m, 1H), 4.45 (m, 1H), 4.66 (t, 1H, *J* = 5.5 Hz), 5.01 (s, 2H), 7.15-7.39 (m, 10H), 7.43 (d, 1H, *J* = 8.1Hz), 7.62 (d, 1H, *J* = 8.5 Hz), 7.95 (d, 1H, *J* = 8.1Hz). Anal. (C₂₈H₃₉N₃O₅S) C, H, N.

### Preparation of Intermediate CBZ-L-Leu-L-Phe-L-Methioninol (sulfoxide)

CBZ-L-Leu-L-Phe-L-methioninol (1.50 g, 2.80 mmol) was dissolved in 50 mL of CH₂Cl₂. A total amount of 0.61 g (3.5 mmol) of *m*-CPBA was added portionwise over a period of five hours as the reaction was stirred at room temperature. After an additional hour, the reaction was poured into saturated NaHCO₃/CH₂Cl₂. The organic layer was separated, washed with brine, and dried (Na₂SO₄). After removal of the solvent, the crude residue was flash chromatographed on a short flash silica gel column eluting with 5% MeOH/CHCl₃. The product was obtained as a white glassy solid (1.38 g, 90%): IR (KBr) 3295, 3063, 2955, 1694, 1644, 1541, 1263, 1234, 1043, 698 cm⁻¹; ¹H NMR (DMSO-*d*₆) δ 0.81 (m, 6 H), 1.32 (m, 2H), 1.59 (m, 2H), 1.92 (m, 1H), 2.47 (s, 3 H), 2.55-3.29 (m, 6 H), 3.73 (m, 1H), 3.97 (m, 1H), 4.42 (m, 1H), 4.75 (t, 1H, *J* = 5.5 Hz), 5.01 (m, 2H), 7.16-7.39 (m, 10 H), 7.44 (d, 1H, *J* = 7.7 Hz), 7.73 (d, 1H, *J* = 8.8 Hz), 7.98 (m, 1H). Anal. (C₂₈H₃₉N₃O₆S) C, H, N, S.

### Preparation of Intermediate CBZ-L-Leu-L-Phe-L-Methioninal (sulfoxide)

CBZ-L-Leu-L-Phe-L-methioninol (sulfoxide) (1.38 g, 2.53 mmol) was dissolved in DMSO. o-Iodoxybenzoic acid (2.12 g, 7.59 mmol) was added, requiring a few minutes of stirring at room temperature to dissolve. After three hours, the DMSO was removed under reduced pressure. The residue was twice diluted with CH₂Cl₂, and the solvent was evaporated to remove any residual DMSO. The residue was diluted with a minimum of acetone, and the white precipitate was filtered off. The filtrate was concentrated to near dryness and dissolved in EtOAc, which produced more of the white precipitate, which was again filtered off. The filtrate was washed with a 10% Na₂S₂O₃/10% NaHCO₃ solution, water, and brine before drying over Na₂SO₄. Upon removal of the organic solvent, the residue was twice taken up in benzene and evaporated to remove any residual water, giving 0.98 g (71%) of a white glassy solid which was used immediately without further purification: ¹H NMR (DMSO-*d*_{*6*}) 8 0.81 (m, 6H), 1.30 (m, 2H), 1.50 (m, 1H), 1.97 (m, 1H), 2.48 (s, 3H), 2.55-3.27 (m, 5H), 3.70 (m, 1H), 4.47 (m, 1H), 4.71 (m, 1H), 5.00 (s, 2H), 7.20-7.40 (m, 10H), 7.93 (m, 1H), 8.08 (m, 1H), 8.51 (m, 1H), 9.22 (s, 1H); (M+H) 544.

### Preparation of Product - Ethyl-3-[CBZ-L-Leu-L-Phe-L-Met(sulfoxide)]-E-Propenoate

CBZ-L-Leu-L-Phe-L-Methioninal (sulfoxide) (0.98 g, 1.80 mmol) was dissolved in 50 mL of THF. (Carbethoxymethylene)triphenyl-phosphorane (1.11 g, 2.16 mmol) was added, and the reaction was stirred at room temperature overnight. The solvent was removed in vacuo, and the residue subjected to flash column chromatography eluting with 2% MeOH/CHCl₃. The product was obtained (0.82 g, 74%) as a white solid: ¹H NMR (DMSO-*d*₆) δ 0.81 (m, 6H), 1.21 (t, 3H, *J* = 7 Hz), 1.34 (m, 2H), 1.54 (m, 1H), 1.78 (m, 1H), 1.93 (m, 1H), 2.49 (s, 3H), 2.50-3.05 (m, 4H), 3.99 (m, 1H), 4.10 (q, 2H, *J* = 7 Hz), 4.51 (m, 2H), 5.00 (dd, 2H, *J* = 17.3, 4.4Hz), 5.62 (m, 1H), 6.72 (m, 1H), 7.19 (m, 5H), 7.34 (m, 5H), 7.43 (d, 1H, *J* = 8.1Hz), 8.08 (d, 1H, *J* = 7.4 Hz), 8.13 (d, 1H, *J* = 8.5Hz); (M+H) 614; HRMS calcd for C₃₂H₄₃N₃O₇S+Cs 746.1876 (M+Cs), found 746.1850. Anal. (C₃₂H₄₃N₃O₇S) C, H, N, S.

### Example 2 - Preparation of Compound 4: Ethyl-3-[CBZ-L-Leu-L-Phe-L-(N-Ac-amino)-Ala]-E-Propenoate

### Preparation of Intermediate CBZ-L-(N-Ac-amino)-Ala

CBZ-L-Amino-Ala (1.5 g, 6.3 mmol) was suspended in 50 mL of H₂O with stirring. Acetic anhydride (5.0 mL) was added slowly to this suspension over a 30 minute period, during which time the starting material dissolved. The reaction mixture was stirred for an additional 1 hour at room temperature and then evaporated to dryness under vacuum. The resulting oil was dissolved in 30 mL CHCl₃ and left for 12 hours. The solid that formed was collected by filtration, washed with 30 mL of CHCl₃ and dried yielding 1.29 g (73%) of product as a white solid: IR (KBr) 3271, 3125, 3065, 1734, 1703, 1614, 1545,1289,1244, 1053, 727 cm⁻¹; ¹H NMR (DMSO-*d*₆) δ 1.84 (s, 3H), 3.2-3.55 (m 2H), 4.13 (m, 1H), 5.08 (s, 2H), 7.12-7.41 (m, SH), 7.54 (d, 1H, *J* = 8.1 Hz), 8.02 (bt, 1H, *J* = 5.5 Hz), 12.78 (bs, 1H); Anal. (C₁₃H₁₆N₂O₅) C, H, N.

### Preparation of Intermediate CBZ-L-(N-Ac-amino)-Ala-OMe

Anhydrous HCl gas was slowly bubbled at 0 °C into a stirred suspension of CBZ-L-(N-Ac-amino)-Ala (1.21 g, 4.3 mmol) in MeOH (43 mL) until the solid was dissolved. Stirring was continued for 30 minutes at 0 °C whereupon the methanolic HCl was carefully evaporated to dryness. The methyl ester was formed as a white solid in quantitative yield and used without further purification: IR (KBr) 3323, 3285, 3094, 2957, 1755, 1736, 1686, 1651, 1531, 1277, 1057, 736, 600 cm⁻¹; ¹H NMR (DMSO-*d*₆) δ 1.78 (s, 3H), 3.22-3.47 (m, 2H), 3.61 (s, 3H), 4.15 (m, 1H), 5.02 (s, 2H), 7.24-7.36 (m, 5H), 7.64 (d, 1H, *J* = 7.7 Hz), 7.97 (bt, 1H, *J* = 6.3 Hz); Anal. (C₁₄H₁₁N₂O₅) C, H, N.

### Preparation of Intermediate CBZ-L-(N-Ac-amino)-Alaninol

To a solution of CBZ-L-(N-Ac-amino)-Ala-OMe (1.8 g, 6.12 mmol) in 50 mL anhydrous THF/EtOH (2:1) was added LiCl (0.52 g, 12.24 mmol). Upon dissolution, NaBH₄ (0.46 g, 12.24 mmol) was added, and the mixture was stirred at room temperature for 12 hours. The reaction mixture was evaporated to near dryness, whereupon 45 mL of H₂O was added. The pH of this mixture was adjusted to 2-3 using concentrated HCl, followed by extraction with EtOAc (300 mL). The organic layer was washed with H₂O (50 mL), dried over Na₂SO₄, filtered and concentrated. The residue was purified by flash column chromatography (10% MeOH/CHCl₃) to give 1.38 g (85%) of a white solid: IR (KBr) 3303, 3082, 2951, 2926, 1689, 1645, 1547, 1284, 1061, 1046, 756, 698 cm⁻¹; ¹H NMR (DMSO-*d*₆) δ 1.78 (s, 3H), 3.03 (m, 1H), 3.16-3.28 (m, 3H), 3.49 (m, 1H), 5.00 (s, 2H), 6.95 (d, 1H, *J* = 8.1 Hz), 7.29-7.3 8 (m, 5H), 7.83 (bt, 1H, *J* = 5.5 Hz); Anal. (C₁₃H₁₈N₂O₄) C, H, N.

### Preparation of Intermediate L-(N-Ac-amino)-Alaninol

To a solution of CBZ-L-(N-Ac-amino)-alaninol (1.36 g, 5.11 mmol) in 40 mL MeOH, 10% Pd on carbon (0.15 g) was added with stirring while under an argon atmosphere. The reaction vessel was evacuated under vacuum and then put under an atmosphere of hydrogen using a balloon. The mixture was stirred for 2 hours. At this time the hydrogen gas was evacuated, and the catalyst was removed by filtration. The solvent was removed under vacuum. Addition of EtOAc and reconcentration gave a white hygroscopic solid in quantitative yield which was used without further purification: mp = 80-82 °C; ¹H NMR (DMSO-*d*₆) δ 1.79 (s, 3H), 2.66 (m, 1H), 2.86 (m, 1H), 3.06 (m, 1H), 3.21 (2H, m), 3.4 (bs, 2H), 4.55 (bs, 1H), 7.76 (bs, 1H). Anal. (C₅H₁₂N₂O₂) C, H, N.

### Preparation of Intermediate CBZ-L-Leu-L-Phe-L-(N-Ac-amino)-Alaninol

This compound was prepared from CBZ-L-Leu-L-Phe and L-(N-Ac-amino)-alaninol using the procedure described in Example 1 for the preparation of CBZ-L-Leu-L-Phe-L-methioninol. The compound was purified by column chromatography (7% MeOH/CHCl₃) to give a white solid (81%): IR (KBr) 3302, 2955, 1694, 1651, 1539, 1454, 1236, 1047, 698 cm⁻¹; ¹H NMR (DMSO-*d*₆) δ 0.80 (s, 6H), 1.32 (m, 2H), 1.47 (m, 1H), 1.79 (s, 3H), 2.81 (m, 1H), 2.97 (m, 2H), 3.14-3.25 (m, 3H), 3.71 (m, 1H), 3.95 (m, 1H), 4.42 (m, 1H), 4.67 (t, 1H, *J* = 5.5 Hz), 5.00 (m, 2H), 7.16-7.34 (m, 10H), 7.45 (d, 1H, *J* = 8.1 Hz), 7.70 (m, 2H), 7.88 (d, 1H, *J* = 8.1 Hz); Anal. (C₂₈H₃₈N₄O₆) C, H, N.

### Preparation of Intermediate CBZ-L-Leu-L-Phe-L-(N-Ac-amino)-Alaninal

This compound was prepared in 73% yield as a white solid from CBZ-L-Leu-L-Phe-L-(N-Ac-amino)-alaninol using the procedure described in Example 1 for the preparation of CBZ-L-Leu-L-Phe-L-methioninal (sulfoxide). The product was used immediately without further purification. The product existed as a mixture of aldehyde and aldehyde hydrate. IR (KBr) 3294, 2957, 1695, 1649, 1539, 1263, 698 cm⁻¹; ¹H NMR (DMSO-*d*₆) δ 0.81 (dd, 6H, *J* = 8.8, 6.2 Hz), 1.31 (m, 2H), 1.50 (m, 1H), 1.76 (s, hydrate), 1.78 (s, 3H), 2.83 (m, 1H), 3.00 (m, 1H), 3.20 (d, *J* = 9.6 Hz, hydrate), 3.35 (m, 1H), 3.80 (m, hydrate), 3.97 (m, 2H), 4.16 (m, 1H), 4.37 (m, hydrate), 4.44 (m, hydrate), 4.54 (m, 1H), 5.01 (s, 2H), 6.28 (d, 1H, *J* = 7.0 Hz, hydrate), 6.41 (d, 1H, *J* = 6.6 Hz, hydrate), 7.12-7.50 (m, 10H), 7.63 (t, 1H, *J* = 7.9 Hz), 7.87 (m, 1H), 7.98 (d, 1H, *J* = 8.1 Hz), 8.40 (d, 1H, *J* = 7.0 Hz), 9.26 (s, 1H); Anal. (C₂₈H₃₆N₄O₆·0.5 H₂O) C, H, N.

### Preparaton of Product - Ethyl-3-[CBZ-L-Leu-L-Phe-L-(N-Ac-amino)-Ala]-E-Propenoate

This compound was prepared in 55% yield as a white solid from CBZ-L-Leu-L-Phe-L-(N-Ac-amino)-alaninal and (carbethoxymethylene)triphenylphosphorane using the procedure described in Example I for the preparation of compound 12, ethyl-3-[CBZ-L-Leu-L-Phe-L-Met(sulfoxide)-E-propenoate. The product was purified by flash column chromatography (3% MeOH/CHCl₃). ¹H NMR (DMSO-*d*₆) δ 0.81 (dd, 6H, *J* = 9.2, 6.6 Hz), 1.21 (t, 3H, *J* = 7.2 Hz), 1.34 (m, 2H), 1.53 (m, 1H), 1.78 (s, 3H), 2.80-3.28 (m, 4H), 3.99 (m, 1H), 4.10 (q, 2H, *J* = 7.0 Hz), 4.43 (m, 2H), 5.01 (m, 2H), 5.61 (d, 1H, *J* = 15.4 Hz), 6.61 (dd, 1H, *J* = 15.4, 5.2 Hz), 7.10-7.34 (m, 10H), 7.44 (d, 1H, *J* = 7.7 Hz), 7.70 (m, 2H), 7.82 (t, 1H, *J* = 5.5 Hz), 8.05 (m, 2H); HRMS calcd for C₃₂H₄₂N₄O₁+Cs 727.2108 (M+Cs), found 727.2137. Anal. (C₃₂H₄₂O₇) C, H, N.

### Example 3 - Preparation of Compound 2: Ethyl-3-[CBZ-L-Leu-L-Phe-L-(Tr-Gln)]-E-Propenoate

### Preparation of Intermediate BOC-L-(Tr-Gln)-N(Me)OMe

Isobutyl chloroformate (0.611 mL, 4.71 mmol) was added to a solution of BOC-L-(Tr-Gln) (2.30 g, 4.71 mmol) and 4-methylmorpholine (1.04 mL, 9.46 mmol) in CH₂Cl₂ at 0 °C. The reaction mixture was stirred at 0 °C for 20 minutes then *N,O*-dimethylhydroxylamine hydrochloride (0.459 g, 4.71 mmol) was added. The resulting solution was stirred at 0 °C for 15 minutes and at 23 °C for 4 hours, then was partitioned between water (150 mL) and a 1:1 mixture of EtOAc and hexanes (2 x 150 mL). The combined organic layers were dried over Na₂SO₄ and were concentrated. Purification of the residue by flash column chromatography (40% hexanes in EtOAc) afforded the product (2.22 g, 89%) as a white foam: R_{*f*} = 0.22 (50% EtOAc in hexanes); IR (KBr) 3411, 3329, 3062, 1701, 1659 cm⁻¹; ¹H NMR (CDCl₃) δ 1.42 (s, 9H), 1.63-1.77 (m, 1H), 2.06-2.17 (m, 1H), 2.29-2.43 (m, 2H), 3.17 (s, 3H), 3.64 (s, 3H), 4.73 (bs, 1H), 5.38-5.41 (m, 1H), 7.20-7.31 (m, 15H); Anal. (C₃₁H₃₇N₃O₅) C, H, N.

### Preparation of Intermediate BOC-L-(Tr-Glutaminal)

Diisobutylaluminum hydride (7.84 mL of 1.5 M solution in toluene, 11.76 mmol) was added to a solution of BOC-L-(Tr-Gln)-N(Me)OMe (2.50 g, 4.70 mmol) in THF at -78°C, and the reaction mixture was stirred at -78 °C for 4 hours. Methanol (3 mL) and 1.0 M HCl (6 mL) were added sequentially, and the mixture was warmed to 23 °C. The resulting suspension was diluted with Et₂O (150 mL) and was washed with 1.0 M HCl (3 x 100 mL), half-saturated NaHCO₃ (100 mL), and water (100 mL). The organic layer was dried over MgSO₄, filtered, and concentrated to give crude aldehyde (2.01 g, 91%) as a white solid: mp = 114-116 °C; R_{*f*} = 0.42 (50% EtOAc in hexanes); IR (KBr) 3313, 1697, 1494 cm⁻¹; ¹H NMR (CDCl₃) δ 1.44 (s, 9H), 1.65-1.75 (m, 1H), 2.17-2.23 (m, 1H), 2.31-2.54 (m, 2H), 4.11 (bs, 1H), 5.38-5.40 (m, 1H), 7.11 (s, 1H), 7.16-7.36 (m, 15H), 9.45 (s, 1H).

### Preparation of Intermediate Ethyl-3-[BOC-L-(Tr-Gln)]-E-Propenoate

Sodium bis(trimethylsilyl)amide (3.38 mL of a 1.0 M solution in THF, 3.3 mmol) was added to a solution of triethyl phosphonoacetate (0.732 mL, 3.39 mmol) in THF (100 mL) at -78 °C, and the resulting solution was stirred for 20 minutes at that temperature. BOC-L-(Tr-Glutaminal) (1.60 g, 3.39 mmol) in THF (20 mL) was added via cannula, and the reaction mixture was stirred for 4 hours at -78 °C then was partitioned between 1.0 M HCl (150 mL) and a 1:1 mixture of EtOAc and hexanes (2 x 150 mL). The organic layers were dried over Na₂SO₄ and concentrated. Purification of the residue by flash column chromatography (40% EtOAc in hexanes) provided ethyl-3-[BOC-L-(Tr-*Gln*)]-E-propenoate (1.53 g, 83%) as a white foam: R_{*f*} = 0.60 (50% EtOAc in hexanes); IR (cm⁻¹) 3321, 1710; ¹H NMR (CDCl₃) δ 1.27 (t, 3 H, *J* = 7.2 Hz), 1.42 (s, 9H), 1.70-1.78 (m, 1H), 1.80-1.96 (m, 1H), 2.35 (t, 2H, *J* = 7.0 Hz), 4.18 (q, 2H, *J* = 7.2 Hz), 4.29 (bs, 1H), 4.82-4.84 (m, 1H), 5.88 (dd, 1H, *J* = 15.7, 1.6 Hz), 6.79 (dd, 1H, *J* = 15.7, 5.3 Hz), 6.92 (s, 1H), 7.19-7.34 (m, 15H); Anal. (C₃₃H₃₈N₂O₅) C, H, N.

### Preparation of Product Ethyl-3-[CBZ-L-Leu-L-Phe-L-(Tr-Gln)]-E-Propenoate

Ethyl-3-[BOC-L-(Tr-*Gln*)]-E-propenoate (0.224 g, 0.422 mmol) was dissolved in 1,4-dioxane (3 mL) and cooled to 0 °C. A solution of HCl in 1,4-dioxane (4.0 M, 3 mL, 12 mmol) was added dropwise, and the reaction solution was allowed to warm to room temperature. After being stirred for 2 hours, the solution was diluted with 1: 1 CH₂Cl₂/EtOAc (50 mL) and added to a solution of NaOH (16 mmol) in saturated aqueous NaHCO₃ (50 mL). After vigorous shaking, the phases were separated, and the aqueous phase was washed 2 more times with 1:1 CH₂Cl₂/EtOAc (50 mL). The combined organic phases were dried over Na₂SO₄ and concentrated to give 0.164 g (88%) of the crude free amine, which was used without further purification.

The crude amine (0.371 mmol, 1.0 equiv) was dissolved in dry CH₂Cl₂ (5 mL). CBZ-L-Leu-L-Phe (0.176 g, 0.427 mmol), 1-hydroxybenzotriazole hydrate (0.081 g, 0.599 mmol), 4-methylmorpholine (0.175 mL, 1.59 mmol), and 1-(3-dimethylaminopropyl)-3-ethylcarbodiimide hydrochloride (0.114 g, 0.595 mmol) were added sequentially. After being stirred for 18 hours at 23 °C, the reaction mixture was poured into water (40 mL) and extracted with 1:1 CH₂Cl₂/EtOAc (3 x 50 mL). The combined organic layers were dried over Na₂SO₄ and were concentrated. The residue was purified by flash column chromatography (50% EtOAc in hexanes) to give the product (0.163 g, 49%) as a white solid: mp = 192-194 °C; IR (KBr) 3295, 3049, 1696, 1654 cm⁻¹; ¹H NMR (CDCl₃) δ 0.84 (d, 3H, *J* = 6.5 Hz), 0.86 (d, 3H, *J* = 6.5 Hz), 1.24-1.32 (m, 1H), 1.28 (t, 3H, *J* = 7.2 Hz), 1.43-1.75 (m, 3H), 1.91-2.06 (m, 1H), 2.20-2.38 (m, 2H), 2.93-3.02 (m, 1H), 3.07-3.18 (m, 1H), 3.95-4.02 (m, 1H), 4.17 (q, 2H, *J* = 7.2 Hz), 1.43-4.55 (m, 2H), 4.82-4.95 (m, 2H), 5.69 (d, 1H, *J* = 15.7 Hz), 6.46 (d, 1H, *J* = 7.5 Hz), 6.60 (d, 1H, *J* = 8.1 Hz), 6.69 (dd, 1H, *J* = 15.7, 5.1 Hz), 7.09-7.38 (m, 27 H); Anal. (C₅₁H₅₆N₄O₇) C, H, N.

### Example 4 - Preparation of Compound 3: Ethyl-3-(CBZ-L-Leu-L-Phe-L-Gln)-E-Propenoate

### Preparation of Product - Ethyl-3-(CBZ-L-Leu-L-Phe-L-Gln)-E-Propenoate

Compound 2, ethyl-3-[CBZ-L-Leu-L-Phe-L-(Tr-*Gln*)]-E-propenoate (0.15 g, 0.18 mmol), prepared as described in Example 3, was dissolved in 1:1 CH₂Cl₂/TFA (5 mL) at 23 °C and the bright yellow solution was stirred 30 minutes, whereupon the solvent was evaporated. CCl₄ (10 mL) was added, and the resulting solution was concentrated twice. Addition of Et₂O (10 mL) to the oily residue quickly gave a white precipitate. After stirring 10 minutes, the solid was collected by filtration and washed sequentially with acetone (2 x 10 mL) and Et₂O (2 x 10 mL) then was dried *in vacuo* to give ethyl-3-(CBZ-L-Leu-L-Phe-L-Gln)-E-propenoate (0.057 mg, 53%) as a white solid: mp = 219-221 °C; IR (KBr) 3300, 3065, 1672 cm⁻¹; ¹H NMR (DMSO-*d*₆) δ 0.78 (d, 3H, *J* = 6.8 Hz), 0.82 (d, 3H, *J* = 6.5 Hz), 1.21 (t, 3H, *J* = 7.0 Hz), 1.25-1.37 (m, 2H), 1.42-1.54 (m, 1H), 1.58-1.80 (m, 2H), 2.02-2.09 (m, 2H), 2.84 (dd, 1H, *J* = 13.2, 8.9 Hz), 2.97 (dd, 1H, *J* = 13.2, 5.8 Hz), 3.93-4.01 (m, 1H), 4.11 (q, *2H, J* = 7.0 Hz), 4.33-4.52 (m, 2H), 4.97 (d, 1H, *J* = 12.3 Hz), 5.04 (d, 1H *J* = 12.3 Hz), 5.64 (d, 1H, *J* = 15.9 Hz), 6.69 (dd, 1H, *J* = 15.9, 5.4 Hz), 6.76 (s, 1H), 7.13-7.37 (m, 11H), 7.43 (d, 1H, *J* = 7.8 Hz), 7.99 (d, 1H, *J* = 8.1 Hz), 8.04 (d, 1H, *J* = 8.1 Hz); Anal. (C₃₂H₄₂N₄O₇) C, H, N.

### Example 5 - Preparation of Compound 7: Methyl-3-(CBZ-L-Leu-L-Phe-L-Gln)-Z-Propenoate

### Preparation of Intermediate Methyl-3-[BOC-L-(Tr-Gln)]-Z-Propenoate

18-crown-6 (0.867 g, 3.28 mmol) was evaporated from toluene (40 mL) and then dissolved in dry THF (14 mL) under argon. Bis(2,2,2-trifluoroethyl)(methoxycarbonylmethyl)phosphonate (0.111 mL, 0.525 mmol) was added, and the reaction mixture was cooled to -78 °C. After dropwise addition of a solution of potassium bis(trimethylsilyl)-amide in toluene (0.5 M, 1.26 mL, 0.63 mmol), the reaction mixture was stirred for 25 minutes. A solution of BOC-L-(Tr-glutaminal) (0.310 g, 0.656 mmol) in dry THF (4 mL) was added dropwise, and, after stirring 1 hour more, saturated aqueous NH₄Cl (2 mL) was added. The reaction mixture was allowed to warm to room temperature, and the THF was evaporated. Water (10 mL) was added to the residue, which was then extracted with CH₂Cl₂ (3x30 mL). The combined organic phases were dried over Na₂SO₄ and concentrated. The residue was purified by flash column chromatography (35% EtOAc/hexanes) to give the product (0.181 g, 52%) as a glass: IR (thin film) 3326, 1713, 1690, 1666, 1514 cm⁻¹; ¹H NMR (CDCl₃) δ 1.41 (s, 9H), 1.84-1.93 (m, 2H), 2.37-2.44 (m, 2H), 3.68 (s, 3H), 5.10 (m, 2H), 5.80 (d, 1H, *J* = 11.8 Hz), 6.03 (m, 1H), 6.88 (bs, 1H), 7.18-7.32 (m, 15H).

### Preparation of Intermediate Methyl-3-[CBZ-L-Leu-L-Phe-L-(Tr-Gln)]-Z-Propenoate

Methyl-3-[BOC-L-(Tr-*Gln*)]-Z-prapenoate (0.143 g, 0.271 mmol) was dissolved in 1,4-dioxane (3 mL) at room temperature. A solution of HCl in 1,4-dioxane (4.0 M, 3 mL) was added dropwise, and the reaction solution was stirred for 2 hours under an argon balloon. Then the solvent was evaporated to give the crude amine salt as a glassy residue, which was used without further purification. This amine salt, CBZ-L-Leu-L-Phe (0.112 g, 0.272 mmol), and 1-hydroxybenzotriazole hydrate (0.055 g, 0.40 mmol) were dissolved in dry CH₂Cl₂ (5 mL) under argon at room temperature. 4-Methylmorpholine (0.149 mL, 1.36 mmol), and 1-(3-dimethylaminopropyl)-3-ethylcarbodiimide hydrochloride (0.078 g, 0.40 mmol) were then added sequentially. After stirring for 3 hours, water (10 mL) was added, and the mixture was extracted with CH₂Cl₂ (3 x 30 mL). The combined organic phases were dried over Na₂SO₄ and concentrated. The residue was purified by flash column chromatography (33% acetone in hexanes) to give the product (0.132 g, 59%) as a white foam: IR (thin film) 3296,1708,1650,1517 cm⁻¹; Anal. (C₅₀H₅₄N₄O₇) C, H, N.

### Preparation of Product - Methyl-3-(CBZ-L-Leu-L-Phe-L-Gln)-Z-Propenoate

Methyl-3-[CBZ-L-Leu-L-Phe-L-(Tr-*Gln*)]-Z-propenoate (0.110 g, 0.134 mmol) was dissolved in 1:1 CH₂Cl₂/TFA (4 mL), giving a bright yellow solution, which was stirred for 30 minutes under an argon balloon. CCl₄ (7 mL) was added, and the solution was concentrated twice. The residue was triturated with Et₂O (3 mL) to give a white solid, which was collected by filtration and dried *in vacuo* (0.040 g, 51%): mp = 185-188 °C; IR (KBr) 3401, 3283, 1719, 1690, 1643, 1538 cm⁻¹; ¹H NMR (DMSO-*d*₆) δ 0.78 (d, 3H, *J* = 6.6 Hz), 0.82 (d, 3H, *J* = 6.5 Hz), 1.22-1.38 (m, 2H), 1.43-1.54 (m, 1H), 1.58-1.75 (m, 2H), 1.92-2.09 (m, 2H), 2.77-2.90 (m, 2H), 3.65 (s, 3H), 3.91-4.00 (m, 1H), 4.37-4.46 (m, 1H), 4.99 (d, 1H, *J* = 12.6 Hz), 5.04 (d, 1H, *J* = 12.6 Hz), 5.18-5.25 (m, 1H), 5.79 (d, 1H, *J* = 11.5 Hz), 5.92 (dd, 1H, *J* = 11.5, 8.7 Hz), 6.72 (s, 1H), 7.14-7.36 (m, 11H), 7.43 (d, 1H, *J* = 8.0 Hz), 7.76 (d, 1H, *J* = 8.1 Hz), 8.01 (d, 1H, *J* = 8.0 Hz); Anal. (C₃₁H₄₀N₄O₇) C, H, N.

### Example 6 - Preparation of Compound 11: Methyl-3-(CBZ-L-Leu-L-Phe-L-Gln)-E-Propenoate

### Preparation of Intermediate Methyl-3-[BOC-L-(Tr-Gln)]-E-Propenoate

Sodium bis(trimethylsilyl)amide (0.978 mL of a 1.0 M solution in THF, 0.978 mmol) was added to a solution of trimethyl phosphonoacetate (0.144 mL, 0.890 mmol) in THF (20 mL) at -78 °C, and the resulting solution was stirred for 15 minutes at that temperature. BOC-L-(Tr-Glutaminal) (0.420 g, 0.889 mmol) in THF (10 mL) was added via cannula, and the reaction mixture was stirred for 2 hours at -78 °C, then was partitioned between 0.5 M HCl (100 mL) and a 1:1 mixture of EtOAc and hexanes (2 x 100 mL). The organic layers were dried over Na₂SO₄ and were concentrated. Purification of the residue by flash column chromatography (gradient elution, 30-40% EtOAc in hexanes) provided methyl-3-[BOC-L-(Tr-*Gln*)]-E-propenoate (0.460 g, 96%) as a white solid: mp 110-112 °C; IR (thin film) 3318, 1708, 1665 cm⁻¹; ¹H NMR (CDCl₃) δ 1.42 (s, 9H), 1.72-1.82 (m, 1H), 1.91-1.98 (m, 1H), 2.34-2.41 (m, 2H), 3.72 (s, 3H), 4.29 (s, br, 1H), 4.78-4.81 (m, 1H), 5.89 (dd, 1H, *J* = 15.6, 1.6 Hz), 6.80 (dd, 1H, *J* = 15.6, 5.3 Hz), 6.87 (s, 1H), 7.19-7.33 (m, 15H).

### Preparation of Intermediate Methyl-3-[CBZ-L-Leu-L-Phe-L-(Tr-Gln)]-E-Propenoate

Using the procedure described in Example 28 for the preparation of ethyl-2-fluoro-3-[CBZ-L-Leu-L-Phe-L-(Tr-*Gln*)]-E-propenoate, methyl-3-[BOC-L-(Tr-*Gln*)]-E-propenoate (0.157 g, 0.297 mmol) was deprotected and coupled with CBZ-L-Leu-L-Phe (0.123 g, 0.298 mmol) to provide methyl-3-[CBZ-L-Leu-L-Phe-L-(Tr-*Gln*)]-E-propenoate (0.176 g, 72%) as a white foam: ¹H NMR (CDCl₃) δ 0.84 (d, 3H, *J* = 6.7 Hz), 0.86 (d, 3H, *J* = 6.7 Hz), 1.45-1.61 (m, 3H), 1.67-1.75 (m, 1H), 1.94-1.96 (m, 1H), 2.20-2.35 (m, 2H), 2.95-3.15 (m, 2H), 3.72 (s, 3H), 3.94-4.01 (m, 1H), 4.46-4.49 (m, 1H), 4.83-4.93 (m, 3H), 5.72 (d, 1H, *J* = 15.8 Hz), 6.45 (d, 1H, *J* = 7.2 Hz), 6.63 (d, 1H, *J* = 8.1 Hz), 6.71 (dd, 1H, *J* = 15.8, 5.1 Hz), 7.01-7.38 (m, 27H).

### Preparation of Product - Methyl-3-(CBZ-Leu-L-Phe-L-Gln)-E-Propenoate

Using the procedure described in Example 4 for the preparation of compound 3, methyl-3-[CBZ-L-Leu-L-Phe-L-(Tr-*Gln*)]-E-propenoate (0.087 g, 0.106 mmol) was deprotected to provide methyl-3-(CBZ-L-Leu-L-Phe-L-*Gln*)-E-propenoate (0.015 g, 25%) as a white solid: mp = 220 °C (dec); ¹H NMR (DMSO-*d*₆) δ 0.79 (d, *3*H, *J* = 10.9 Hz), 0.81 (d, 3H, *J* = 10.9 Hz), 1.26-1.34 (m, 2H), 1.47-1.49 (m, 1H), 1.61-1.76 (m, 2H), 2.06 (t, 2H, *J* = 7.6 Hz), 2.84 (dd, 1H, *J* = 13.5,9.0 Hz), 2.97 (dd, 1H, *J* = 13.5, 5.6 Hz), 3.65 (s, 3H), 3.93-3.97 (m, 1H), 4.38 (s, br, 1H), 4.44-4.49 (m, 1H), 4.97 (d, 1H, *J* = 12.5 Hz), 5.04 (d, 1H, *J* = 12.5 Hz), 5.68 (d, 1H, *J* = 15.6 Hz), 6.70 (dd, 1H, *J* = 15.6, 5.5 Hz), 6.76 (s, 1H), 7.19 (s, br, 7H), 7.34 (s, br, 4H), 7.44 (d, 1H, *J* = 7.5 Hz), 7.99 (d, 1H, *J* = 8.1 Hz), 8.05 (d, 1H, *J* = 8.1 Hz).

### Example 7 - Preparation of Compound 13: 4-(CBZ-L-Leu-L-Phe-L-Gln)-E-3-Butene-2-one

### Preparation of Intermediate CBZ-L-Leu-L-Phe-L-(Tr-Gln)-N(Me)OMe

BOC-L-(Tr-Gln)-N(Me)OMe (0.807 g, 1.52 mmol) was dissolved in 1,4-dioxane (4.5 mL) at room temperature. A solution of HCl in 1,4-dioxane (4.0 M, 4.5 mL) was added dropwise, and the reaction solution was stirred for 2.5 hours under an argon balloon. The solvent was evaporated to give the crude amine salt as a white foam, which was used without further purification. This amine salt, CBZ-L-Leu-L-Phe (0.626 g, 1.52 mmol) and 1-hydroxybenzotriazole hydrate (0.308 g, 2.28 mmol) were stirred in dry CH₂Cl₂ (12 mL) under argon at room temperature. 4-Methylmorpholine (0.840 mL, 7.64 mmol), and 1-(3-dimethylaminopropyl)-3-ethylcarbodiimide hydrochloride (0.436 g, 2.27 mmol) were added sequentially. After stirring for 3 hours, the reaction solution was poured into water (25 mL), and the aqueous layer was extracted 3 times with CH₂Cl₂ (70 mL, 40 mL, and 30 mL). The combined organic phases were dried over Na₂SO₄ and concentrated. The residue was purified by flash column chromatography (40% acetone in hexanes) to give the product (0.826 g, 66%) as a white foam: IR (thin film) 3300, 1643, 1525 cm⁻¹.

### Preparation of Intermediate CBZ-L-Leu-L-Phe-L-(Tr-Glutaminal)

CBZ-L-Leu-L-Phe-L-(Tr-Gln)-N(Me)OMe (0.768 g, 0.930 mmol) was dissolved in dry THF (12 mL) under argon and cooled to -78 °C. A solution of diisobutylaluminum hydride in toluene (1.5 M, 2.17 mL, 3.26 mmol) was added dropwise. After stirring 3 hours, methanol (0.7 mL) was added slowly, followed by 1 N HCl (1 mL). The reaction mixture was allowed to warm to nearly room temperature and was then diluted with 5:1 CH₂Cl₂/EtOAc (120 mL). The resulting mixture was washed with 1 N HCl (2 x 15 mL), half-saturated NaHCO₃ (15 mL) and brine (25 mL). The organic phase was dried over MgSO₄ and concentrated to give the product as an off-white foam (0.606 g, 85%), which was used without further purification. An analytical sample was purified by column chromatography (36% acetone in hexanes): IR (thin film) 3295, 1708, 1660, 1531 cm⁻¹; ¹H NMR (CDCl₃) δ 0.80 (d, 3H, *J* = 6.2 Hz), 0.87 (d, 3H, *J* = 6.4 Hz), 1.27-1.59 (m, 3H), 1.71-1.83 (m, 1H), 2.07-2.15 (m, 1H), 2.22-2.29 (m, 2H), 2.96 (dd, 1H, *J* = 13.7, 7.4 Hz), 3.08 (dd, 1H, *J* = 13.7, 6.2 Hz), 3.99-4.08 (m, 1H), 4.11-4.20 (m, 1H), 4.55-4.64 (m, 1H), 4.92 (bs, 2H), 5.17 (d, 1H, *J* = 6.7 Hz), 6.70 (d, 1H, *J* = 7.4 Hz), 7.08-7.35 (m, 27H), 9.26 (s, 1H); Anal. (C₄₇H₅₀N₄O₆) C, H, N.

### Preparation of Intermediate 4-[CBZ-L-Leu-L-Phe-L-(Tr-Gln)]-E-3-Butene-2-one

CBZ-L-Leu-L-Phe-L-(Tr-Glutaminal) (0.605 g, 0.789 mmol) and 1-triphenylphosphoranylidene-2-propanone (0.251 g, 0.788 mmol) were stirred in dry THF (7 mL) at room temperature, under argon, giving a yellow solution. After stirring 20 hours, the solvent was evaporated, and the residue was purified by flash column chromatography (36% acetone in hexanes) to give the product (0.425 g, 67%) as a white foam: IR (thin film) 3299,1666, 1519 cm⁻¹.

### Preparation of Product - 4-(CBZ-L-Leu-L-Phe-L-Gln)-E-3-Butene-2-one

This compound was prepared in 54% yield from 4-[CBZ-L-Leu-L-Phe-L-(Tr-*Gln*)]-E-3-butene-2-one using the procedure described in Example 26 for the preparation of compound 14, 3-(CBZ-L-Leu-L-Phe-DL-*Gln*)-E-propenonitrile: mp = 194-196 °C (dec); IR (KBr) 3413, 3284, 1684, 1643, 1537 cm ⁻¹; ¹H NMR (DMSO-*d*_{*6*}) δ 0.79 (d, 3H, *J* = 6.6 Hz), 0.82 (d, 3H, *J* = 6.6 Hz), 1.23-1.39 (m, 2H), 1.44-1.55 (m, 1H), 1.60-1.84 (m, 2H), 2.05-2.12 (m, 2H), 2.17 (s, 3H), 2.84 (dd, 1H, *J* = 13.6, 8.7 Hz), 2.99 (dd, 1H, *J* = 13.6, 5.7 Hz), 3.93-4.02 (m, 1H), 4.34-4.44 (m, 1H), 4.46-4.55 (m, 1H), 4.98 (d, 1H, *J* = 12.6 Hz), 5.04 (d, 1H, *J* = 12.6 Hz), 5.84 (d, 1H, *J* = 16.0 Hz), 6.64 (dd, 1H, *J* = 16.0, 5.4 Hz), 6.77 (s, 1H), 7.15-7.37 (m, 11H), 7.43 (d, 1H, *J* = 7.9 Hz), 7.99 (d, 1H, *J* = 8.1 Hz), 8.06 (d, 1H, *J* = 8.1 Hz); Anal. (C₃₁H₄₀N₄O₆) C, H, N.

### Example 8 - Preparation of Compound 5: Ethyl-3-[CBZ-L-Leu-L-Phe-L-[N-(2-pyrrolidinone)]-Ala]-E-Propenoate

### Preparation of Intermediate CBZ-L-[N-(4-Chlorobutyryl)-amino]-Ala-OMe

Acetyl chloride (19.6 g, 250 mmol) was slowly added to a solution of MeOH (300 mL) at 0 °C. After 10 minutes, CBZ-L-amino-Ala (10 g., 42 mmol) was added, and the reaction was allowed to stir for 12 hours at room temperature. Removal of solvent under vacuum provided 13.5 g of crude CBZ-L-amino-Ala-OMe as the hydrochloride salt. The crude ester was taken up in 200 mL CH₂Cl₂, to which was added Et₃N (10.6 g, 105 mmol) and then 4-chlorobutyryl chloride (7.1 g, 50.4 mmol) at 0 °C. The reaction was allowed to warm to room temperature and was stirred for 4 hours. At this time the reaction mixture was added to brine. The organic layer was extracted, washed with 1 N HCl, brine, dried over MgSO₄, and concentrated yielding 19 g of crude material. The material was purified by flash column chromatography (50% EtOAc-hexanes), giving an 87% yield of product. ¹H NMR (CDCl₃) δ 2.07 (m, 2H), 2.35 (t, 2H, *J* = 7.0 Hz), 3.57 (t, 2H, *J* = 6.3 Hz), 3.67 (t, 2H, *J* = 5.9 Hz), 3.77 (s, 3H), 4.45 (m, 1H), 5.12 (s, 2H), 5.84 (d, 1H, *J* = 6.3 Hz), 6.00 (bs, 1H), 7.37 (s, 5H).

### Preparation of Intermediate CBZ-L-[N-(2-pyrrolidinone)]-Ala-OMe

A solution of CBZ-L-[N-(4-chlorobutyryl)-amino]-Ala-OMe (14.6 g, 39 mmol) in DMF (400 mL) was cooled to 0 °C. To the solution was added NaH (1.87 g of a 60% dispersion in oil, 46.8 mmol), and the mixture was stirred at room temperature for 4 hours. The DMF was removed under high vacuum, and the residue was taken up in EtOAc, washed with 1 N HCl, saturated aqueous NaHCO₃, brine, dried over MgSO₄ and concentrated. The material was purified by flash column chromatography (100% EtOAc), giving 7.0 g (56%) of product. ¹H NMR δ (CDCl₃) 1.97 (m, 2H), 2.35 (m, 2H), 3.36 (m, 1H), 3.40-3.60 (m, 3H), 3.77 (s, 3H), 4.52 (m, 1H), 5.13 (d, 2H, *J* = 5.6 Hz), 5.83 (d, 1H, *J* = 6.3 Hz), 7.37 (m, 5H).

### Preparation of Intermediate L-[N-(2-pyrrolidinone)]-Ala-OMeHCl

This compound was prepared from CBZ-L-[N-(2-pyrtolidinone)]-Ala OMe by catalytic hydrogenation as described in Example 2 for the preparation of L-(N-Ac-amino)-alaninol, except methanolic HCl was used in order to isolate the product as the HCl salt. ¹H NMR (CDCl₃), δ 2.03 (m, 2H), 2.39 (m, 2H), 3.14 (bs, 2H), 3.40-3.70 (m, 5H), 3.75 (s, 3H).

### Preparation of Intermediate CBZ-L-Leu-L-Phe-L-[N-(2-pyrrolidinone)]-Ala-OMe

This compound was prepared from CBZ-L-Leu-L-Phe and L-[N-(2-pyrrolidinone)]-Ala-OMe·HCl using the procedure described in Example 1 for the preparation of CBZ-L-Leu-L-Phe-L-methioninol. ¹H NMR (CDCl₃), δ 0.89 (m, 6H), 1.36 (m, 2H), 1.56 (m, 1H), 1.61 (m, 2H), 2.04 (m, 3H), 2.31 (m, 2H), 3.07-3.70 (m, 6H), 3.75 (s, 3H), 4.11 (m, 1H), 4.71 (m, 1H), 5.13 (bs, 1H), 5.18 (bs, 1H), 6.76-6.88 (m, rotomers, 1H), 7.10-7.45 (m, 10H).

### Preparation of Intermediate CBZ-L-Leu-L-Phe-L-[N-(2-pyrrolidinone)]-Alaninol

This compound was prepared by the reduction of CBZ-L-Leu-L-Phe-L-[N-(2-pyrrolidinone)]-Ala-OMe with NaBH₄ and LiCl using the procedure described in Example 2 for the preparation of CBZ-L-(N-Ac-amino)-alaninol.

### Preparation of Intermediate CBZ-L-Leu-L-Phe-L-[N-(2-pyrrolidinone)]-Alaninal

This compound was prepared from CBZ-L-Leu-L-Phe-L-[N-(2-pyrrolidinone)]-alaninol using the procedure described in Example 1 for the preparation of CBZ-L-Leu-L-Phe-L-methioninal (sulfoxide). Anal. (C₃₀H₃₈N₄O₆·1.4 H₂O) C, H, N.

### Preparation of Product - Ethyl-3-[CBZ-L-Leu-L-Phe-L-[N-(2-pyrrolidinone)]-Ala]-E-Propenoate

This compound was prepared from CBZ-L-Leu-L-Phe-L-[N-(2-pyrrolidinone)]-alaninal and (carbethoxymethylene)triphenylphosphorane using the procedure described in Example 1 for the preparation of compound 12, ethyl-3-(CBZ-L-Leu-L-Phe-L-*Met*(sulfoxide)-E-propenoate. ¹H NMR (DMSO-*d*₆) δ 0.80 (d, 6H, *J* =7.0 Hz), 0.95-1.40 (m, 7H), 1.49 (m, 1H), 1.82 (m, 2H), 2.12 (m, 2H), 2.60-3.10 (m, 2H), 3.20 (m, 2H), 3.81 (m, 1H), 4.00 (m, 1H), 4.10 (m, 2H), 4.49 (m, 1H), 4.72 (m, 1H), 5.01 (bs, 1H), 5.70 (d, 0.5H -rotomer- *J* = 16.5 Hz), 5.97 (d, 0.5H -rotomer- *J* = 16.5 Hz), 6.70 (d, 0.5H -rotomer- *J* = 16.5 Hz), 6.80 (d, 0.5H -rotomer- *J* = 16.5 Hz), 7.20 (d, 2H, *J* = 7.4 Hz), 7.34 (m, 3H), 7.60 (m, 5H), 8.04 (m, 1H), 8.23 (m, 1H). HRMS calcd for C₃₄H₄₄N₄O₇+Cs 753.2264 (M + Cs), found 753.2295.

### Example 9 - Preparation of Compound 16: Ethyl-3-[CBZ-L-Leu-L-Phe-L-(N-carbamyl-amino)-Ala]-E-Propenoate

### Preparation of Intermediate CBZ-L-(N-BOC-amino)-Ala

To a stirred solution of NaOH (1.23 g, 30.76 mmol) in 36 mL of H₂O and 24 mL *tert*-butanol was added CBZ-L-amino-Ala (7.15 g, 30 mmol). To this solution was added di-*tert*-butyl dicarbonate (6.88 g, 31.5 mmol). Stirring was continued at room temperature for 12 hours, at which time the solution was washed with pet. ether (2 x 150 mL). The organic layers were washed with saturated aqueous NaHCO₃ (3 x 20 mL), and the aqueous layers were combined and acidified at 0 °C with 25% aqueous KHSO₄ to pH 2-3. This milky white mixture was then extracted with a large excess of Et₂O, dried over anhydrous Na₂SO₄, and concentrated to yield 9.13 g (90%) of product as a white solid, which was used without further purification. ¹H NMR (DMSO-*d*₆) δ 1.35 (s, 9H), 3.21 (m, 2H), 4.05 (m, 1H), 5.02 (s, 2H), 6.83 (bt, 1H, *J* = 6.6 Hz), 7.34 (m, 5H), 7.41 (d, 1H, *J* = 8.1 Hz), 12.65 (bs, 1H). This compound was further characterized as its corresponding methyl ester.

### Preparation of Intermediate CBZ-L-(N-BOC-amino)-Ala-OMe.

A solution of diazomethane in Et₂O, generated from N-methyl-N-nitroso-p-toluenesulfonamide (7.7 g, 36.0 mmol), 70 mL Et₂O, 16 mL EtOH, 12 mL H₂O and KOH (7.65 g, 13.6 mmol) was carefully distilled into a stirred solution of CBZ-L-(N-BOC-amino)-Ala (7.8 g, 23.0 mmol) in 50 mL Et₂O and 10 mL EtOH at 0 °C. The yellow solution was stirred for 30 minutes. The cold solution was then brought to room temperature, and argon was bubbled into the reaction flask to remove any excess diazomethane. After the solution turned colorless, it was concentrated to give the methyl ester as a white solid in quantitative yield. mp = 72-74 °C; IR (KBr) 3418, 3331, 3005, 2955, 1753, 1724,1676, 1552, 1525, 1298, 1045, 699 cm⁻¹; ¹H NMR (CDCl₃) δ 1.41 (s, 9H), 3.55 (m, 2H), 3.76 (s, 3H), 4.40 (m, 2H), 4.82 (m, 1H), 5.11 (s, 2H), 5.77 (m, 1H), 7.35 (m, 5H). Anal. (C₁₇H₂₄N₂O₆) C, H, N.

### Preparation of Intermediate CBZ-L-(N-BOC-amino)-Alaninol

Using the borohydride reduction procedure described in Example 2 for the preparation of CBZ-L-(N-Ac-amino)-alaninol, CBZ-L-(N-BOC-amino)-Ala-OMe was converted to the corresponding alcohol and isolated in 96% yield without column chromatography purification. mp = 116-119 °C; IR (KBr) 3327,3277,3065,2976,1699, 1682, 1543, 1315, 1250, 1062, 1001, 696 cm⁻¹; ¹H NMR (DMSO-*d*₆) δ 1.35 (s, 9H), 2.90-3.10 (m, 4H), 3.55 (m 1H), 4.60 (bt, 1H, *J* = 5.5 Hz), 4.99 (s, 2H), 6.72 (bt, 1H, *J* = 5.5 Hz), 6.86 (d, 1H, *J* = 8.1 Hz), 7.34 (m, 5H). Anal. (C₁₆H₂₄N₂O₅) C, H, N.

### Preparation of Intermediate L-(N-BOC-amino)-Alaninol

Using the hydrogenation procedure described in Example 2 for the preparation of L-(N-Ac-amino)-alaninol, the CBZ group was removed from CBZ-L-(N-BOC-amino)-alaninol to give the amino alcohol in 98% yield. mp = 61-64 °C; IR (KBr) 3362, 2980, 2935, 1680, 1534, 1370, 1287, 1175, 1059, 642 cm⁻¹; ¹H NMR (DMSO-*d*₆) δ 1.36 (s, 9H), 2.64 (m, 1H), 2.72 (m, 1H), 2.93 (m, 1H), 3.13 (m, 1H), 3.32 (m, 2H), 4.45 (bs, 1H), 6.67 (bs, 1H); Anal. (C₈H₁₈N₂O₃) C, H, N.

### Preparation of Intermediate CBZ-L-Leu-L-Phe-L-(N-BOC-amino)-Alaninol

This compound was prepared from L-(N-BOC-amino)-alaninol and CBZ-L-Leu-L-Phe using the coupling procedure described in Example 2 for the preparation of CBZ-L-Leu-L-Phe-L-(N-Ac-amino)-alaninol. The reaction mixture was purified by flash column chromatography (5% saturated anhydrous NH₃ in MeOH/CH₂Cl₂) to give a white solid in 90% yield. IR (KBr) 3420, 3327, 3289, 3032, 2953, 1694, 1643, 1535, 1284, 1036, 696 cm⁻¹; ¹H NMR (DMSO-*d*₆) δ 0.80 (dd, 6H, *J* = 11.2, 6.4 Hz), 1.35 (s, 9H), 1.55 (m 2H), 1.72 (m, 1H), 2.89 (m, 2H), 3.19 (m, 2H), 3.78 (m, 1H), 3.92 (m, 1H), 4.44 (m, 1H), 4.62 (t, 1H, *J* = 5.5 Hz), 5.01 (d, 2H, *J* = 5.9 Hz), 6.63 (bt, 1H, *J* = 5.5 Hz), 7.18 (m, 5H), 7.34 (m, 5H), 7.45 (d, 1H, *J* = 8.1 Hz), 7.60 (d, 1H, *J* = 7.7 Hz), 7.85 (d, 1H, *J* = 8.1 Hz). Anal. (C₃₁H₄₄N₄O₇) C, H, N.

### Preparation of Intermediate CBZ-L-Leu-L-Phe-L-(N-BOC-amino)-Alaninal

This compound was prepared in 90% yield as a white solid from CBZ-L-Leu-L-Phe-L-(N-BOC-amino)-alaninol using the procedure described in Example 1 for the preparation of CBZ-L-Leu-L-Phe-L-methioninal (sulfoxide). The product was used immediately without further purification. The product existed as a mixture of aldehyde and aldehyde hydrate. IR (KBr) 3299, 3067, 2959, 2934, 1696, 1647, 1535, 1254, 1171, 747, 698 cm⁻¹; ¹H NMR (DMSO-*d*₆) δ 0.80 (dd, 6H, *J* = 9.0, 6.8 Hz), 1.35 (s, 9H), 1.41 (m, 2H), 1.69 (m, 1H), 2.80-3.01 (m, 2H), 3.29 (m, 2H), 3.97 (m, 1H), 4.10 (m, 1H), 4.60 (m, 1H), 5.00 (s, 2H), 5.56 (d, *J* = 7.4 Hz, hydrate), 6.78 (t, 1H, *J* = 6.3 Hz), 7.20 (m, 5H), 7.33 (m, 5H), 7.40 (d, 1H, *J* = 8.1 Hz), 7.97 (d, 1H, *J* = 8.1 Hz), 8.39 (d, 1H, *J* = 6.6 Hz), 9.26 (s, 1H); HRMS calcd for C₃₁H₄₂N₄O₇+Cs 715.2108 (M+Cs), found 715.2133. Anal. (C₃₁H₄₂N₄O₇·0.5 H₂O) C, H, N.

### Preparation of Intermediate Ethyl-3-[CBZ-L-Leu-L-Phe-L-(N-BOC-amino)-Ala]-E-Propenoate

This compound was prepared in approximately 40% yield as a white foaming solid from CBZ-L-Leu-L-Phe-L-(N-BOC-amino)-alaninal and (carbethoxymethylene)-triphenylphosphorane using the procedure described in Example 1 for the preparation of compound 12, ethyl-3-[CBZ-L-Leu-L-Phe-L-*Met*(sulfoxide)-E-propenoate. The product was partially purified (impure with triphenylphosphine oxide as determined by NMR) by flash column chromatography (4% MeOH/CH₂Cl₂). ¹H NMR (DMSO-*d*₆) 8 0.80 (dd, 6H, *J* = 9.6, 6.3 Hz), 1.19 (t, 3H, *J* = 6.8 Hz), 1.34 (s, 9H), 1.45-1.70 (m, 3H), 2.82-3.05 (m, 4H), 3.99 (m, 1H), 4.08 (q, 2H, *J* = 7.0 Hz), 4.46 (m, 2H), 5.01 (m, 2H), 5.64 (d, 1H, *J* = 16.2 Hz), 6.61 (dd, 1H, *J* = 16.2,5.5 Hz), 6.85 (bt, 1H, *J* = 5.2 Hz), 7.18 (m, 5H), 7.34 (m, 5H), 7.42 (d, 1H, *J* = 5.5 Hz), 7.96 (d, 1H, *J* = 7.4 Hz), 8.01 (d, 1H, *J* = 7.4 Hz); HRMS calcd for C₃₅H₄₈N₄O₂+Na 675.3370 (M+Na), found 675.3363.

### Preparation of Intermediate Ethyl-3-(CBZ-L-Leu-L-Phe-L-amino-Ala)-E-Propeuoate

To a stirred solution of ethyl-3-[CBZ-L-Leu-L-Phe-L-(N-BOC-amino)-*Ala*]-E-propenoate (0.14 g, 0.215 mmol) in 12 mL CH₂Cl₂, cooled to 0 °C, was added 0.65 mL TFA dropwise. The reaction was followed by TLC (silica, 10% MeOH/CH₂Cl₂) until there was a disappearance of starting material. At this time the reaction mixture was taken up in 100 mL EtOAc and washed with saturated NaHCO₃ (3 x 10 mL). The organic layer was then washed with H₂O then saturated brine and dried over anhydrous Na₂SO₄. Concentration of the solution gave a residue, which was purified by flash column chromatography (8% MeOH/CH₂Cl₂) to give a beige foam in 84% yield. ¹H NMR (DMSO-*d*₆) δ 0.80 (dd, 6H, *J* = 9.4, 6.8 Hz), 1.22 (t, 3H, *J* = 7.2 Hz), 1.31 (m, 2H), 1.51 (m, 1H), 2.64 (m, 2H), 2.91 (m, 2H), 3.99 (m, 1H), 4.10 (q, 2H, *J* = 7.4 Hz), 4.36 (m, 1H), 4.49 (m, 1H), 5.02 (m, 2H), 5.60 (d, 1H, *J* = 16.2 Hz), 6.76 (dd, 1H, *J* = 15.6, 5.0 Hz), 7.20 (m, 5H), 7.34 (m, 5H), 7.46 (d, 1H, *J* = 7.0 Hz), 7.95 (d, 1H, *J* = 8.5 Hz), 8.05 (d, 1H, *J* = 5.9 Hz); MS calcd for C₃₅H₄₈N₄O₃+H 553 (M+H), found 553.

### Preparation of Product - Ethyl-3-[CBZ-L-Leu-L-Phe-L-(N-carbamyl-amino)-Ala]-E-Propenoate

To a stirred solution of bis (4-nitrophenyl) carbonate (66 mg, 0.22 mmol) in 2 mL CH₂Cl₂, was added a solution of ethyl-3-[CBZ-L-Leu-L-Phe-L-amino-*Ala*]-E-propenoate (0.10 g, 0.18 mmol) in 2 mL CH₂Cl₂. The mixture was stirred for 3 hours at which time 2 mL of saturated anhydrous methanolic ammonia was added. The yellow solution was allowed to stir for 30 minutes longer, diluted with 100 mL CH₂Cl₂, and washed repeatedly with 1N NaOH to remove 4-nitrophenol. The organic layer was washed with dilute HCl, H₂O, and brine, and dried over anhydrous Na₂SO₄. This solution was concentrated, and the residue was subjected to flash column chromatography (5% MeOH/CH₂Cl₂) to yield a white solid in 20% yield. IR (KBr) 3470, 3291, 2978, 2926, 1715, 1645, 1539, 1281, 1045, 698 cm⁻¹; ¹H NMR (DMSO-*d*₆) δ 0.81 (dd, 6H, *J* = 9.0, 6.8 Hz), 1.21 (t, 3H, *J* = 7.0 Hz), 1.30 (m, 2H), 1.48 (m, 1H), 2,92 (m, 2H), 3.10 (m, 2H), 3.97 (m, 1H), 4.10 (q, 2H, *J* = 7.0 Hz), 4.40 (m, 2H), 5.01 (m, 2H), 5.54 (bs, 2H), 5.61 (d, *J* = 16.5 Hz), 6.04 (t, 1H, *J* = 7.7 Hz), 6.71 (dd, *J* = 15.8, 5.2 Hz), 7.20 (m, 5H), 7.34 (m, 5H), 7.46 (d, 1H, *J* = 7.4 Hz), 8.01 (d, 1H, *J* = 7.0 Hz), 8.11 (d, 1H, *J* = 8.5 Hz); HRMS calcd for C₃₁H₄₁N₅O₇+Cs 728.2060 (M+Cs), found 728.2078 Anal. (C₃₁H₄₃N₅O₇) C, H, N.

### Example 10 - Preparation of Compound 17: Isopropyl-3-(CBZ-L-Leu-L-Phe-L-Gln)-E-Propenoate

### Preparation of Intermediate 3-[BOC-L-(Tr-Gln)]-E-Propenoic Acid

Ethyl-3-[BOC-L-(Tr-*Gln*)]-E-propenoate (1.874 g, 3.46 mmol), prepared as decribed in Example 3, was taken up in 20 mL EtOH and treated with 1N NaOH solution (7.95 mL, 7.95 mmol) dropwise, via addition funnel, over 2 hours. The resulting solution was stirred at room temperature for 1.5 hours, whereupon the reaction mixture was poured into water and washed with ether. The aqueous layer was acidified to pH 3 with 1N HCl, and extracted 3 times with EtOAc. The organic phase was separated and dried over MgSO₄ and concentrated to provide 3-[BOC-L-(Tr-*Gln*)]-E-propenoic acid (1.373 g, 77%) as an off-white foam. No further purification was needed: IR (thin film) 3315, 1698, 1666 cm⁻¹; ¹H NMR(CDCl₃) δ 1.42 (s, 9H), 1.76 (m, 1H), 1.83-1.98 (m, 1H), 2.37 (t, 2H, *J* = 7.0 Hz), 4.30 (m, 1H), 4.88 (m, 1H), 5.85 (d, 1H, *J* = 15.3 Hz), 6.86 (dd, 1H, *J* = 15.5, 5.1 Hz), 6.92 (s, 1H), 7.25 (m, 15H).

### Preparation of Intermediate Isopropyl-3-[BOC-L-(Tr-Gln)]-E-Propenoate

3-[BOC-L-(Tr-*Gln*)]-E-Propenoic acid (0.500 g, 0.973 mmol), isopropanol (0.008 mL, 1.07 mmol), and 4-dimethylaminopyridine (0.012 g, 0.0973 mmol) were taken up in 10 mL dry CH₂Cl₂ and treated with 1-(3-dimethylaminopropyl)-3-ethylcarbodiimide hydrochloride (0.196 g, 1.07 mmol). The resulting solution was stirred at room temperature overnight, concentrated in vacuo, and purified by flash column with 50% EtOAc/hexanes to provide isopropyl-3-[BOC-L-(Tr-*Gln*)]-E-propenoate (0.106 g, 20%) as a white foam: R_{*f*} = 0.8 (50% EtOAc/hexanes); IR 3320, 1711 cm⁻¹; ¹H NMR(CDCl₃) δ 1.25 (d, 6H, *J* = 6.23 Hz), 1.43 (s, 9H), 1.72 (m, 1H), 1.96 (m, 1H), 2.37 (t, 2H, *J* = 7.16 Hz), 4.30 (bs, 1H), 4.74 (m, 1H), 5.05 (m, 1H), 5.86 (dd, 1H, *J* = 15.9,5.0 Hz), 6.78 (dd, 1H, *J* = 15.6, 5.0 Hz), 6.89 (bs, 1H), 7.26 (m, 15H); Anal. (C₃₄H₄₀N₂O₅) C, H, N.

### Preparation of Intermediate Isopropyl-3-[CBZ-L-Leu-L-Phe-L-(Tr-Gln)]-E-Propenoate

Isopropyl-3-[BOC-L-(Tr-*Gln*)]-E-propenoate (0.087 g, 0.191 mmol) was deprotected and coupled with CBZ-L-Leu-L-Phe (0.079 g, 0.191 mmol) using the procedure described in Example 3 for the preparation of ethyl-3-[BOC-L-(Tr-*Gln*)]-E-propenoate, to provide the product (0.064 g, 40%) as a white foam: R_{*f*}= 0.7 (50% EtOAc/hexanes); IR (thin film) 3283, 1707 cm⁻¹; ¹H NMR (CDCl₃) δ 0.86 (m, 6H), 1.03 (m, 1H), 1.23 (m, 6H), 1.72 (m, 1H), 1.96 (m, 1H), 2.28 (m, 2H), 2.54 (m, 1H), 2.70 (m, 1H), 2.78 (m, 1H), 2.95-3.25 (m, 4H), 3.99 (m, 1H), 4.85-5.13 (m, 4H), 5.66 (d, 1H, *J* = 15.9 Hz), 6.45 (d, 1H, *J* = 7.5 Hz), 6.55 (d, 1H, *J* = 7.5 Hz), 6.68 (m, 1H), 7.12-7.36 (m, 25H); MS (M+Cs) 983.

### Preparation of Product - Isopropyl-3-(CBZ-L-Leu-L-Phe-L-Gln)-E-Propenoate

Using the procedure described in Example 4 for the preparation of compound 3, ethyl-3-(CBZ-L-Leu-L-Phe-L-*Gln*)-E-propenoate, isopropyl-3-[CBZ-L-Leu-L-Phe-L-(Tr-Gln)]-E-propenoate (0.059 g, 0.0694 mmol) was deprotected to provide the product (0.024 g, 57%) as a white solid: mp = 180-182 °C; R_{*f*}= 0.6 (10% MeOH/CHCl₃); IR (KBr) 3272, 1705 cm⁻¹; ¹H NMR (DMSO-*d*₆) δ 0.70 (m, 1H), 0.80 (dd, 6H, *J* = 10.6, 6.5 Hz), 1.21 (dd, 6H, *J* = 6.2, 2.5 Hz), 1.32 (m, 1H), 1.70 (m, 1H), 2.05 (t, 2H, *J* = 7.6 Hz), 2.83 (m, 1H), 2.97 (m, 1H), 3.99 (m, 1H), 4.37-4.49 (m, 4H), 4.91-5.06 (m, 4H), 5.60 (d, 1H, *J* = 15.3 Hz), 6.67 (dd, 1H, *J* = 15.6, 5.6 Hz), 6.76 (bs, 1H), 7.19 (m, 5H), 7.34 (m, 5H), 7.44 (d, 1H, *J* = 7.2 Hz), 8.01 (m, 2H); Anal. (C₃₃H₄₄N₄O₇·1.0 CH₂Cl₂) C, H, N.

### Example 11 - Preparation of Compound 18: Cyclopentyl-3-(CBZ-L-Leu-L-Phe-L-Gln)-E-Propenoate

### Preparation of Intermediate Cyclopentyl-3-[BOC-L-(Tr-Gln)]-E-Propenoate

Using the procedure described in Example 10 for the preparation of isopropyl-3-[BOC-L-(Tr-*Gln*)]-E-propenoate, 3-[BOC-L-(Tr-*Gln*)]-E-propenoic acid (0.50 g, 0.973 mmol) was coupled with cyclopentanol (0.1 mL, 1.07 mmol) to provide cyclopentyl-3-[BOC-L-(Tr-*Gln*)]-E-propenoate (0.123 g, 22%) as a white foam: R_{*f*}= 0.7 (EtOAc/hexanes); IR (thin film) 3319,1708 cm⁻¹; ¹H NMR (CDCl₃) δ 1.27 (m, 2H), 1.44 (s, 9H), 1.59-1.89 (m, 8H), 2.38 (t, 2H, *J* = 7.2 Hz), 4.32 (bs, 1H), 4.55 (m, 1H), 5.22 (m, 1H), 5.87 (d, 1H, *J* = 15.6 Hz), 6.77 (dd, 1H, *J* = 15.1,4.1 Hz), 6.90 (bs, 1H), 7.20-7.33 (m, 15H).

### Preparation of Intermediate Cyclopentyl-3-[CBZ-L-Leu-L-Phe-L-(Tr-Gln)]-E-Propenoate

Using the procedure described in Example 4 for the preparation of compound 3, ethyl-3-[CBZ-L-Leu-L-Phe-L-(Tr-*Gln*)]-E-propenoate, cyclopentyl-3-[BOC-L-(Tr-*Gln*)]-E-propenoate (0.077 g, 0.160 mmol) was deprotected and coupled with CBZ-L-Leu-L-Phe (0.068 g, 0.160 mmol) to provide cyclopentyl-3-[CBZ-L-Leu-L-Phe-L-(Tr-*Gln*)]-E-propenoate (0.052 g, 36%) as a white foam: R_{*f*} = 0.4 (50% EtOAc/hexanes); IR (thin film) 3401, 3319, 1708 cm⁻¹; ¹H NMR (CDCl₃) δ 0.84 (m, 6H), 1.05 (m, 1H), 1.28 (m, 1H), 1.46-1.71 (m, 9H), 1.85 (m, 1H), 2.28 (m, 2H), 2.98-3.12 (m, 4H), 3.99 (m 1H), 4.47 (m, 2H), 4.83-5.21 (m, 4H), 5.65 (d 1H, *J* = 15.9 Hz), 6.50 (d, 1H, *J* = 7.2 Hz), 6.59 (d, 1H, *J* = 8.1 Hz), 6.65 (dd, 1H, *J* = 15.9,5.4 Hz), 7.04-7.35 (m 25H); MS (M+Cs) 1009.

### Preparation of Product - Cyclopentyl-3-(CBZ-L-Leu-L-Phe-L-Gln)-E-Propenoate

Using the procedure described in Example 4 for the preparation of compound 3, ethyl-3-(CBZ-L-Leu-L-Phe-L-*Gln*)-E-propenoate, cyclopentyl-3-[CBZ-L-Leu-L-Phe-L-(Tr-*Gln*)]-E-propenoate (0.052 g, 0.059 mmol) was deprotected to provide the product (0.014 g, 36%) as a white solid: mp = 182-185 °C; R_{*f*}= 0.5 (10% MeOH/CHCl₃); IR (thin film) 3389, 3295, 1707 cm⁻¹; ¹H NMR (Acetone-*d*₆) δ 0.85 (dd, 6H, *J* = 10.6, 6.5 Hz), 1.08 (m, 1H), 1.48 (m, 1H), 1.60-1.70 (m, 11H), 1.89 (m, 1H), 2.22 (m, 2H), 2.96 (m, 1H), 3.18 (dd, 1H, *J* = 13.9, 5.8 Hz), 4.00 (d, 1H, *J* = 6.8 Hz), 4.08 (m, 1H), 4.59 (m, 2H), 4.97-5.16 (m, 4H), 5.76 (d, 1H, *J* = 15.3 Hz), 6.71 (m, 2H), 7.15-7.41 (m, 10H), 7.51 (d, 1H, *J* = 7.8 Hz); HRMS calcd for C₃₅H₄₆N₇O₇+Cs 767.2421 (M+Cs) found 767.2435.

### Example 12 - Preparation of Compound 19: Cyclopentylmethyl-3-(CBZ-L-Leu-L-Phe-L-Gln)-E-Propenoate

### Preparation of Intermediate Cyclopentylmethyl-3-[BOC-L-(Tr-Gln)]-E-Propenoate

Using the procedure described in Example 10 for the preparation ofisopropyl-3-[BOC-L-(Tr-*Gln*)]-E-propenoate, 3-[BOC-L-(Tr-*Gln*)]-E-propenoic acid (0.50 g, 0.973 mmol) was coupled with cyclopentylmethanol (0.12 mL, 1.07 mmol) to provide this ester (0.298 g, 51%) as a pale yellow oil: R_{*f*} = 0.7 (50% EtOAc/hexanes); IR (thin film) 3336, 1707 cm⁻¹; ¹H NMR (CDCl₃) δ 1.28 (m, 2H), 1.43 (s, 9H), 1.54-1.62 (m, 5H), 1.72-1.78 (m, 4H), 2.37 (t, 2H, *J* = 7.2 Hz), 4.01 (d, 2H, *J* = 7.2 Hz), 4.31 (bs, 1H), 4.78 (m, 1H), 5.90 (dd, 1H, *J* = 15.9, 1.6 Hz), 6.80 (dd, 1H, *J* = 15.9, 53 Hz), 6.90 (bs, 1H), 7.19-7.34 (m, 15H); Anal (C₃₇H₄₄N₂O₅) C, H, N.

### Preparation of Intermediate Cyclopentylmethyl-3-[CBZ-L-Leu-L-Phe-L-(Tr-Gln)]-E-Propenoate

Using the procedure described in Example 4 for the preparation of compound 3, ethyl-3-[CBZ-L-Leu-L-Phe-L-(Tr-*Gln*)]-E-propenoate, cyclopentylmethyl-3-[BOC-L-(Tr-*Gln*)]-E-propenoate (0.150 g, 0.310 mmol) was deprotected and coupled with CBZ-L-Leu-L-Phe (0.128 g, 0.310 mmol) to provide the product (0.062 g, 22%) as an off-white foam: R_{*f*} = 0.4 (50% EtOAc/hexanes); IR (thin film) 3413, 3295, 1708 cm⁻¹; ¹H NMR (CDCl₃) δ 0.84 (m, 6H), 1.05 (m, 1H), 1.46-1.65 (m, 10H), 1.74 (m, 1H), 2.25 (m, 2H), 2.93-3.11 (m, 4H), 3.93-4.02 (m, 3H), 4.20 (m, 1H), 4.48 (m, 1H), 4.86-5.11 (m, 4H), 5.70 (d, 1H, *J* = 15.0 Hz), 6.46 (d, 1H, *J* = 6.9 Hz), 6.54 (d, 1H, *J* = 8.4 Hz), 6.70 (m, 1H), 6.78 (m, 1H), 7.14-7.36 (m, 25H); MS (M+Cs) 1023.

### Preparation of Product - Cyclopentylmethyl-3-(CBZ-L-Leu-L-Phe-L-Gln)-E-Propenoate

Using the procedure described in Example 4 for the preparation of compound 3, ethyl-3-(CBZ-L-Leu-L-Phe-L-*Gln*)-E-propenoate, cyclopentylmethyl-3-[CBZ-L-Leu-L-Phe-L-(Tr-*Gln*)]-E-propenoate (0.062 g, 0.070 mmol) was deprotected to provide compound 11 (0.021 g, 47%) as a white solid: mp = 145-148 °C; R_{*f*}= 0.4 (10% MeOH/CHCl₃); IR (thin film) 3401, 3295, 1713 cm⁻¹; ¹H NMR (acetone-*d*₆) δ 0.86 (dd, 6H, *J* = 10.6, 6.5 Hz), 1.09 (m, 1H), 1.20-1.85 (m, 13H), 2.21 (m, 2H), 2.99 (m, 1H), 3.18 (m, 1H), 3.99 (m, 2H), 4.10 (m, 2H), 4.59 (m, 2H), 4.98-5.16 (m, 4H), 5.83 (d, 1H, *J* = 14.6 Hz), 6.67-6.98 (m, 2H), 7.20-7.45 (m, 10H), 7.55 (m, 1H); HRMS calcd for C₃₆H₄₈N₇O₇+Cs 781.2577 (M+Cs) found 781.2559.

### Example 13 - Preparation of Compound 21: 1-Pyrrolidin-L-yl-3-(CBZ-L-Leu-L-Phe-L-Gln)-E-Propenone

### Preparation of Intermediate 1-Pyrrolidin-1-yl-3-[BOC-L-(Tr-Gln)]-E-Propenone

3-[BOC-L-(Tr-*Gln*)]-E-Propenoic acid (1.09 g, 2.12 mmol) was coupled with pyrrolidine (0.18 mL, 2.12 mmol) by dissolving both in 30 mL dry CH₂Cl₂ and treating with 1-(3-dimethylaminopropyl)-3-ethylcarbodiimide hydrochloride (0.610 g, 3.18 mmol), 1-hydroxybenzotriazole hydrate (0.430 g, 3.18 mmol), Et₃N (1.18 mL, 8.48 mmol) and stirring at room temperature overnight. The reaction mixture was poured into 50 mL 1N HCl, and the layers were separated. The organic layer was washed with 1N HCl and then a saturated NaHCO₃ solution. The organic layer was dried over MgSO₄ and concentrated to give a yellow residue, which was then subjected to column chromatography using a 5% MeOH/CHCl₃ to yield the product (0.661 g, 55%) as a white foam: R_{*f*} = 0.5 (5% MeOH/CHCl₃); IR (thin film) 3291, 1696 cm⁻¹; ¹H NMR (CDCl₃) δ 1.42 (s, 9H), 1.89 (m, 6H), 2.37 (m, 2H), 3.44-3.53 (m, 4H), 4.28 (bs, 1H), 4.82 (d, 1H, *J* = 7.8 Hz), 6.17 (dd, 1H, *J* = 15.3, 1.6 Hz), 6.71 (dd, 1H, *J* = 15.4, 6.1 Hz), 6.93 (bs, 1H), 7.19-7.32 (m, 15H); Anal (C₃₅H₄₁N₃O₄·CH₂Cl₂) C, H, N.

### Preparation of Intermediate 1-Pyrrolidin-1-yl-3-[CBZ-L-Leu-L-Phe-L-(Tr-Gln)]-E-Propenone

Using the procedure described in Example 3 for the preparation of compound 2, ethyl-3-[CBZ-L-Leu-L-Phe-L-(Tr-*Gln*)]-E-propenoate, 1-pyrrolidin-1-yl-3-[BOC-L-(Tr-Gln)]-E-propenone (0.613 g, 1.166 mmol) was deprotected and coupled with CBZ-L-Leu-L-Phe (0.481 g, 1.166 mmol), yielding 1-pyrrolidin-1-yl-3-[CBZ-L-Leu-L-Phe-L-(Tr-*Gln*)]-E-propenone (0.668 g, 67%) as a white foam: R_{*f*} = 0.5 (10% MeOH/CHCl₃); IR (thin film) 3294, 1702 cm⁻¹; ¹H NMR (CDCl₃) δ 0.84 (m, 6H), 1.31 (m, 1H), 1.46 (m, 1H), 1.81-1.94 (m, 6H), 2.28 (m, 2H), 2.96 (m, 1H), 3.15 (m, 1H), 3.39-3.50 (m, 4H), 3.95 (m, 2H), 4.87-5.11 (m, 4H), 6.14 (d, 1H, *J* = 15.3 Hz), 6.45 (d, 1H, *J* = 7.8 Hz), 6.67 (dd, 1H, *J* = 14.8, 4.8 Hz), 6.82 (d, 1H, *J* = 8.1 Hz), 7.08-7.33 (m, 25H), 7.44 (d, 1H, *J* = 8.1 Hz); MS (M+H) 862.

### Preparation of Product - 1-Pyrrolidin-1-yl-3-(CBZ-L-Leu-L-Phe-L-Gln)-E-Propenone

Using the procedure described in Example 4 for the preparation of compound 3, ethyl-3-(CBZ-L-Leu-L-Phe-L-*Gln*)-E-propenoate, 1-pyrrolidin-1-yl-3-[CBZ-L-Leu-L-Phe-L-(Tr-*Gln*)]-E-propenone (0.668 g, 0.776 mmol) was deprotected to provide this final product (0.320 g, 67%) as a white solid: mp = 195-196 °C (dec); R_{*f*} = 0.4 (10% MeOH/CHCl₃); IR (thin film) 3289, 1684 cm⁻¹; ¹H NMR (DMSO-*d*₆) δ 0.79 (dd, 6H, *J* = 12.1, 6.5 Hz), 1.29 (m, 1H), 1.47 (m, 1H), 1.68-1.87 (m, 6H), 2.05 (m, 2H), 2.84 (m, 1H), 3.01 (m, 1H), 3.29-3.40 (m, 4H), 3.94 (m, 1H), 4.44 (m, 2H), 5.01 (m, 2H), 6.14 (d, 1H, *J* = 14.9 Hz), 6.507 (dd, 1H, *J* = 15.4, 5.8 Hz), 6.76 (bs, 1H), 7.14-7.35 (m, 10H), 7.46 (d, 1H, *J* = 7.8 Hz), 7.95-8.02 (m, 2H); HRMS calcd for C₃₄H₄₅N₅O₆ 620.3448 (M+H), found 620.3437; Anal. (C₃₄H₄₅N₅O₆·0.2 CH₂Cl₂) C, H, N.

### Example 14 - Preparation of Compound 22: N,N-Dimethyl-3-(CBZ-L-Leu-L-Phe-L-Gln)-E-Propenamide

### Preparation of Intermediate N,N-Dimethyl-3-[BOC-L-(Tr-Gln)]-E-Propenamide

Using the procedure described in Example 13 for the preparation of 1-pyrrolidin-1-yl-3-[BOC-L-(Tr-*Gln*)]-E-propenone, 3-[BOC-L-(Tr-*Gln*)]-E-propenoic acid (1.05 g, 2.04 mmol) was coupled with N,N-dimethylamine (0.167 g, 2.04 mmol) to provide the amide (0.848 g, 77%) as a white foam: R_{*f*} = 0.6 (10% MeOH/CHCl₃); IR (thin film) 3297, 1690 cm⁻¹; ¹H NMR (CDCl₃) δ 1.42 (s, 9H), 1.81 (m, 1H), 1.93 (m, 1H), 2.38 (m, 2H), 2.98 (s, 3H), 3.03 (s, 3H), 4.27 (bs, 1H), 4.84 (d, 1H, *J* = 7.2 Hz), 6.31 (dd, 1H, *J* = 15.1, 1.4 Hz), 6.65 (dd, 1H, *J* = 15.3, 5.9 Hz), 6.94 (bs, 1H), 7.19-7.33 (m, 15H); Anal (C₃₃H₃₉N₃O₄·0.9 CH₂Cl₂) C, H, N.

### Preparation of Intermediate N,N-Dimethyl-3-[CBZ-L-Leu-L-Phe-L-(Tr-Gln)]-E-Propenamide

Using the procedure described in Example 3 for the preparation of compound 2, ethyl-3-[CBZ-L-Leu-L-Phe-L-(Tr-*Gln*)]-E-propenoate, N,N-dimethyl-3-[BOC-L-(Tr-*Gln*)]-E-propenamide (0.726 g, 1.567 mmol) was deprotected and coupled with CBZ-L-Leu-L-Phe (0.646 g, 1.567 mmol) to provide the product (0.417 g, 32%) as a white foam: R_{*f*}= 0.5 (10% MeOH/CHCl₃); IR (thin film) 3291, 1702 cm⁻¹; ¹H NMR (CDCl₃) δ 0.84 (m, 6H), 1.30 (m,1H), 1.47 (m, 1H), 1.74 (m, 1H), 1.94 (m, 3H), 2.56 (s, 3H), 2.96 (m, 1H), 3.15 (m, 1H], 2.99 (d, 6H, *J* = 13.4 Hz), 3.94 (m, 1H), 4.54 (m, 2H), 4.87 (s, 2H), 5.00 (d, 2H, *J* = 5.3 Hz), 6.28 (d, 1H, *J* = 14.9 Hz), 6.42 (d, 1H, *J* = 7.8 Hz), 6.63 (dd, 1H, *J* = 15.3, 5.0 Hz), 6.81 (d, 1H, *J* = 8.4 Hz), 7.06 (bs 1H), 7.10-7.36 (m, 25H); Anal (C₅₁H₅₇N₅O₆·3.0 H₂O) C, H, N.

### Preparation of Product - N,N-Dimethyl-3-(CBZ-L-Leu-L-Phe-L-Gln)-E-Propenamide

Using the procedure described in Example 4 for the preparation of compound 3, ethyl-3-(CBZ-L-Leu-L-Phe-L-*Gln*)-E-propenoate, N,N-dimethyl-3-[CBZ-L-Leu-L-Phe-L-(Tr-*Gln*)]-E-propenamide (0.417 g, 0.5 mmol) was deprotected to provide N,N-dimethyl-3-(CBZ-L-Leu-L-Phe-L-*Gln*)-E-propenamide (0.214 g, 72%) as a white solid: mp = 174-175 °C (dec); R_{*f*} = 0.34 (MeOH/CHCl₃); IR (thin film) 3284,1684 cm⁻¹; ¹H NMR (DMSO-*d*₆) δ 0.79 (dd, 6H, *J* = 12.1, 6.5 Hz), 1.30 (m, 1H), 1.47 (m, 1H), 1.70 (m, 2H), 2.06 (m, 2H), 2.84 (m, 1H), 2.98 (s, 3H), 3.03 (s, 3H), 3.94 (m, 1H), 4.44 (m, 2H), 4.95-5.07 (m, 4H), 6.27 (d, 1H, *J* = 15.3 Hz), 6.47 (dd, 1H, *J* = 15.3, 5.6 Hz), 6.75 (bs, 1H), 7.14-7.35 (m, 10H), 7.46 (d, 1H, *J* = 7.5 Hz), 7.96-8.01 (m, 2H); HRMS calcd for C₃₂H₄₃N₅O₆ 594.3291 (M+H), found 594.3281. Anal. (C₃₂H₄₃N₅O₆·1.0 CH₂Cl₂) C, H, N.

### Example 15 - Preparation of Compound 24: 1-Phenyl-3-(CBZ-L-Leu-L-Phe-L-Gln)-E-Propenone

### Preparation of Intermediate 2-(2-[CBZ-L-Leu-L-Phe-L-(Tr-Gln)]-E-Vinyl) Pyridine

2-Picolyltriphenylphosphonium chloride/NaNH₂ (0.345 g, 0.76 mmol) was dissolved in 10 mL of THF. CBZ-L-Leu-L-Phe-L-(Tr-Glutaminal) (0.53 g, 0.69 mmol) was dissolved in 5 mL of THF and added dropwise to the yield solution at room temperature, which was allowed to stir overnight. The solvent was removed in vacuo, and the crude product purified by column chromatography eluting with a gradient of 1-5% MeOH in CHCl₃ to give 0.353 g (61 %) of a white glassy solid: IR (KBr) 3295, 3061, 2953, 1952, 1881, 1649, 1539, 1234, 1045, 972, 750, 696 cm⁻¹; ¹H NMR (DMSO-*d*₆) δ 0.78 (t, 6H, *J* = 7.0 Hz), 1.30 (m, 2H), 1.46 (m, 1H), 1.70 (m, 2H), 2.27 (m, 2H), 2.78 (m, 1H), 3.03 (m, 1H), 3.97 (m, 1H), 4.42 (m, 1H), 4.52 (m, 1H), 4.96 (d, 1H, *J* = 12.0 Hz), 5.03 (d, 1H, *J* = 12.0 Hz), 6.38 (d, 1H, *J* = 16.0 Hz), 6.60 (dd, 1H, *J* = 16.0, 6.0 Hz), 7.10-7.34 (m, 27H), 7.42 (d, 1H, *J* = 8.0 Hz), 7.73 (t, 1H, *J* = 7.5 Hz), 7.92 (d, 1H, *J* = 8.5 Hz), 8.07 (d, 1H, *J* = 8.5 Hz), 8.49 (d, 1H, *J* = 5.0 Hz), 8.59 (s, 1H); MS (M+H) 842. Anal. (C₅₃H₅₅N₅O₅·0.75 H₂O) C, H, N.

### Preparation of Intermediate 2-[2-(CBZ-L-Leu-L-Phe-L-Gln)-E-Vinyl] Pyridine

Using the procedure described in Example 32 for the preparation of compound 20, diethyl-[2-(CBZ-L-Leu-L-Phe-L-*Gln*)-E-vinyl] phosphonate, 2-[2-(CBZ-L-Leu-L-Phe-L-*Gln*)-E-vinyl] pyridine was synthesized from 2-(CBZ-L-Leu-L-Phe-L-Tr-*Gln*)-E-vinyl pyridine in 69% yield as a white solid: IR (KBr) 3291, 3059, 2955, 2359, 1694, 1641, 1539, 1234, 1119, 1047, 970, 743, 698 cm⁻¹; ¹H NMR (DMSO-*d*₆) δ 0.78 (m, 6H), 1.32 (m, 2H), 1.49 (m, 1H), 1.77 (m, 2H), 2.11 (t, 2H, *J* = 7.0 Hz), 2.86 (m, 1H), 3.01 (m, 1H), 3.96 (m, 1H), 4.41 (m, 1H), 4.51 (m, 1H), 4.98 (d, 1H, *J* = 13.0 Hz), 5.04 (d, 1H, *J* = 13.0 Hz), 6.39 (d, 1H, *J* = 16.0 Hz), 6.60 (dd, 1H, *J* = 16.0,6.0 Hz), 6.75 (bs, 1H), 7.08-7.34 (m, 13H), 7.45 (d, 1H, *J* = 8.0 Hz), 7.73 (dt, 1H, *J* = 7.5, 1.5 Hz), 7.97 (d, 1H, *J* = 8.0 Hz), 8.07 (d, 1H, *J* = 8.0 Hz), 8.50 (d, 1H, *J* = 4.0 Hz); HRMS calcd for C₃₄H₄₁N₅O₅ 600.3186 (M+H), found 600.3198. Anal. (C₃₄H₄₁N₅O₅·1.0 H₂O) C, H, N.

### Preparation of Intermediate 1-Phenyl-3-[CBZ-L-Leu-L-Phe-L-(Tr-Gln)]-E-Propenone

Using the procedure described in Example 1 for the preparation of compound 12, ethyl-3-[CBZ-L-Leu-L-Phe-L-*Met*(sulfoxide)-E-propenoate, this compound was synthesized from CBZ-L-Leu-L-Phe-L-Tr-glutaminal and (benzoylmethylene)triphenylphosphorane to give 0.38 g of crude material (impure with triphenylphosphine oxide), which was used without further purification.

### Preparation of Product - 1-Phenyl-3-(CBZ-L-Leu-L-Phe-L-Gln)-E-Propenone

To 0.38 g of 1-phenyl-3-[CBZ-L-Leu-L-Phe-L-(Tr-*Gln*)]-E-propenone, impure with triphenylphosphine oxide, was added 10 mL of CH₂Cl₂. TFA (1 mL) was added to this solution, and the reaction was stirred at room temperature for four hours. The reaction was poured into an EtOAc/saturated NaHCO₃ solution and agitated until white solids began to precipitate out of the organic layer. The aqueous layer was separated, and the solids filtered and washed with EtOAc to give compound 14 (0.0795 g, 20% yield from the aldehyde; 2 steps) as a white solid: IR (KBr) 3408, 3293, 3063, 2955, 1653, 1539, 1449, 1283, 1234, 1121, 1047, 970, 698 cm⁻¹; ¹H NMR (DMSO-*d*₆) δ 0.78 (m, 6H), 1.31 (m, 2H), 1.45 (m, 1H), 1.76 (m, 2H), 2.11 (t, 2H, *J* = 8.0 Hz), 2.89 (m, 1H), 3.01 (m, 1H), 3.97 (m, 1H), 4.51 (m, 2H), 4.97 (d, 1H, *J* = 13.0 Hz), 5.05 (d, 1H, *J* = 13.0 Hz), 6.76 (dd, 1H, *J* = 15.0,5.0 Hz), 6.77 (bs, 1H), 6.91 (d, 1H, *J* = 15.0 Hz), 7.02-7.34 (m, 11H), 7.47 (d, 1H, *J* = 7.0 Hz), 7.54 (m, 2H), 7.66 (t, 1H, *J* = 7.0 Hz), 7.93 (d, 2H, *J* = 7.0 Hz), 8.04 (d, 1H, *J* = 8.0 Hz), 8.10 (d, 1H, *J* = 8.5 Hz); HRMS calcd for C₃₆H₄₃N₄O₆ 627.3182 (M+H), found 627.3199. Anal. (C₃₆H₄₃N₄O₆) C, H, N.

### Example 16 - Preparation of Compound 26: Ethyl-3-[N-(4-Methoxyindole-2-Carbonyl)-L-(4-Cl-Phe)-L-Gln]-E-Propenoate

### Preparation of Intermediate BOC-L-(4-Cl-Phe)-L-(Tr-Glutaminol)

BOC-L-4-Cl-Phe (0.90 g, 3.0 mmol) was dissolved in 30 mL of THF. Carbonyldiimidazole (0.49 g, 3.0 mmol) was added, and the reaction was allowed to stir at room temperature for one hour. L-(Tr-Glutaminol) (1.12 g, 3 mmol) was added, and the reaction was stirred overnight at room temperature. The solvent was removed in vacuo, and the product was purified by flash column chromatography eluting with 3% MeOH/CHCl₃ to yield 1.57 g (80%) of a white solid: IR (KBr) 3416, 3302, 3057, 3024, 2978, 2934, 1663, 1491, 1447, 1366, 1250, 1165, 752, 700 cm⁻¹; ¹H NMR (DMSO-*d*₆) δ 1.28 (s, 9H), 1.44 (m, 1H), 1.66 (m, 1H), 2.26 (m, 2H), 2.72 (m, 1H), 2.91 (m, 1H), 3.18 (m, 2H), 3.64 (m, 1H), 4.07 (m, 1H), 4.67 (t, 1H, *J* = 5.0 Hz), 7.05-7.32 (m, 19H), 6.86 (d, 1H, *J* = 8.5 Hz), 7.62 (d, 1H, *J* = 8.5 Hz), 8.48 (s, 1H). Anal. (C₃₈H₄₂N₃O₅Cl·1.0 H₂O) C, H, N.

### Preparation of Intermediate L-(4-Cl-Phe)-L-(Tr-Glutaminol) Hydrochloride Salt

BOC-L-(4-Cl-Phe)-L-(Tr-Glutaminol) (1.57 g., 2.4 mmol) was dissolved in a minimum amount of CH₂Cl₂ (∼ 5 mL) followed by 50 mL of Et₂O. Anhydrous HCl gas was bubbled into the solution until a white solid precipitated from solution. The reaction was allowed to stir at room temperature overnight, and the resulting solid was filtered and washed with Et₂O, giving 1.19 g (84%) of a white crystalline material: IR (KBr) 3246, 3057, 3028, 2934, 1668, 1494, 1447, 1089, 700 cm⁻¹; ¹H NMR (DMSO-*d*₆) δ 1.48 (m, 1H), 1.71 (m, 1H), 2.30 (m, 2H), 2.94-3.17 (m. 3H), 3.27 (m, 1H), 3.67 (br, 2H), 3.98 (m, 1H), 7.07-7.40 (m, 19H), 8.28 (bs, 3H), 8.34 (d, 1H, *J* = 8.8 Hz), 8.54 (s, 1H). Anal. (C₃₃H₃₄N₃O₃Cl·1.0 HCl·0.75 H₂O) C, H, N.

### Preparation of Intermediate N-(4-Methoxyindole-2-Carbonyl)-L-(4-Cl-Phe)-L-(Tr-Glutaminol)

4-Methoxyindole-2-carboxylic acid (0.36 g, 1.87 mmol) was suspended in 10 mL of CH₂Cl₂. To this suspension was added N-hydroxysuccinimide (0.23 g, 1.97 mmol) and 2 mL ofDMF to dissolve all solids. Dicyclohexylcarbodiimide (0.41 g, 1.97 mmol) was added, and the reaction mixture was stirred at room temperature for 4 hours. At this time the mixture was then filtered into a separate flask containing (1.17 g, 1.97 mmol) of L-(4-Cl-Phe)-L-(Tr-glutaminol)·HCl salt, 0.41 mL (2.95 mmol) of Et,N, 10 mL of CH₂Cl₂, and 2 mL of DMF, removing the N,N'-dicyclohexylurea precipitate. The reaction was allowed to stir overnight at room temperature. The solvents were removed in vacuo, and the resulting crude product was purified by flash column chromatography eluting with 3% (anhydrous NH₃/MeOH)/CHCl₃ to afford 0.53 g (39%) of a white solid: IR (KBr) 3290, 3057, 2933, 1653, 1491, 1360, 1257,1098, 754, 698 cm⁻¹; ¹H NMR (DMSO-*d*₆) δ 1.50 (m, 1H), 1.74 (m, 1H), 2.28 (m 2H), 3.02 (m, 2H), 3.24 (m, 2H), 3.66 (m, 1H), 3.87 (s, 3H), 4.65 (m, 1H), 4.70 (m, 1H), 6.49 (m, 1H, *J* = 7.3 Hz), 6.94-7.38 (m, 22H), 7.86 (d, 1H, *J* = 8.8 Hz), 8.49 (d, 1H, *J* = 8.8 Hz), 8.53 (s, 1H), 11.50 (s, 1H). Anal. (C₄₃H₄₁N₄O₅Cl·0.75 H₂O) C, H, N.

### Preparation of Intermediate N-(4-Methoxyindole-2-Carbonyl)-L-(4-Cl-Phe)-L-(Tr-Glutaminal)

N-(4-Methoxyindole-2-carbonyl)-L-(4-Cl-Phe)-L-(Tr-glutaminol) (1.13 g, 1.55 mmol) was dissolved in 15 mL of DMSO. o-Iodoxybenzoic acid (1.30 g, 4.66 mmol) was added to this solution, and dissolved after a few minutes of stirring at room temperature. After two hours the DMSO was removed under reduced pressure. The residue was twice diluted with CH₂Cl₂, and the solvent was evaporated to remove any residual DMSO. The residue was diluted with EtOAc, and the white precipitate was triturated and filtered off. The organic solvent was washed with 10% Na₂S₂O₃/10% NaHCO₃ solution, water, and brine before drying over Na₂SO₄. The solvent was removed to give 0.85 g (76%) of a white glassy solid which was used immediately without further purification: ¹H NMR (DMSO-*d*₆) δ 1.72 (m, 2H), 2.32 (m, 2H), 3.04 (m, 1H), 3.11 (m, 1H), 3.87 (m, 3H), 4.05 (m, 1H), 4.81 (m, 1H), 6.49 (d, 1H, *J* = 7.3 Hz), 6.94-7.39 (m, 22H), 8.60 (m, 2H), 8.63 (s, 1H), 9.34 (s, 1H), 11.48 (s, 1H).

### Preparation of Intermediate Ethyl-3-[N-(4-Methoxyindole-2-Carbonyl)-L-(4-Cl-Phe)-L-(Tr-Gln)]-E-Propenoate

Using the procedure described in Example 1 for the preparation of compound 12, ethyl-3-[CBZ-L-Leu-L-Phe-L-*Met*(sulfoxide)-E-propenoate, this compound was synthesized from N-(4-methoxyindole-2-carbonyl)-L-(4-Cl-Phe)-L-(Tr-glutaminal) in 59% yield as a white solid: IR (KBr) 3302,3057,2934,1958,1896,1659,1491,1260,1096,1036,833, 756, 700 cm⁻¹; ¹H NMR (DMSO-*d*₆) δ 1.22 (t, 3H, *J* = 6.0 Hz), 1.72 (m, 2H), 2.24 (m, 2H), 3.05 (m, 2H), 3.88 (s, 3H), 4.12 (q, 2H, *J* = 6.0 Hz),4.43 (m, 1H), 4.78 (m, 1H), 5.74 (d, 1H, *J* = 14.0 Hz), 6.50 (d, 1H, *J* = 7.7 Hz), 6.77 (dd, 1H, *J* = 16.0,5.0 Hz), 6.93-7.57 (m, 22H), 8.33 (d, 1H, *J* = 7.7 Hz), 8.56 (d, 1H, *J* = 7.7 Hz), 8.60 (s, 1H), 11.51 (s, 1H). Anal. (C₄₇H₄₅N₄O₆Cl·0.5 H₂O) C, H, N.

### Preparation of Product - Ethyl-3-[N-(4-Methoxyindole-2-Carbonyl)-L-(4-Cl-Phe)-L-Gln]-E-Propenoate

Using the procedure described in Example 32 for the preparation of compound 20, diethyl-[2-(CBZ-L-Leu-L-Phe-L-*Gln*)-E-vinyl] phosphonate, this compound was synthesized by deprotection of ethyl-3-[N-(4-methoxyindole-2-carbonyl)-L-(4-Cl-Phe)-L-(Tr-*Gln*)]-E-propenoate. The product was purified by flash silica gel chromatography eluting with 2-3% MeOH/CHCl, to give 0.16 g (73%) of an off-yellow solid: IR (KBr) 3420, 3289, 2930, 2838, 1722, 1663, 1622,1541, 1261,1184, 1101, 976, 754 cm⁻¹; ¹H NMR (DMSO-*d*₆) δ 1.21 (t, 3H, *J* = 7.0 Hz), 1.74 (m, 2H), 2.11 (t, 2H, *J* = 8.0 Hz), 3.02 (m, 2H), 3.88 (s, 3H), 4.12 (q, 2H, *J* = 7.0 Hz), 4.42 (m, 1H), 4.68 (m, 1H), 5.74 (dd, 1H, *J* = 16.0, 1.5 Hz), 6.47 (d, 1H, *J* = 5.0 Hz), 6.75 (bs, 1H), 6.76-6.81 (m, 2H), 6.96 (d, 1H, *J* = 8.5 Hz), 7.07 (t, 1H, *J* = 8.0 Hz), 7.24-7.38 (m, 5H), 8.33 (d, 1H, *J* = 8.0 Hz), 8.58 (d, 1H, *J* = 8.5 Hz), 11.52 (s, 1H); HRMS calcd for C₂₈H₃₁N₄O₆Cl+Cs 687.0986 (M+Cs), found 687.0976. Anal. (C₂₈H₃₁N₄O₆Cl) C, H, N.

### Example 17 - Preparation of Compound 27: Ethyl-3-[N-(4-Methoxyindole-2-Carbonyl)-L-(4-F-Phe)-L-Gln]-E-Propenoate

### Preparation of Intermediate BOC-L-(4-F-Phe)-L-(Tr-Glutaminol)

Using the procedure described in Example 16 for the preparation of BOC-L-(4-Cl-Phe)-L-(Tr-glutaminol), this compound was synthesized from BOC-L-4-F-Phe and L-(Tr-glutaminol) in 80% yield. White solid: IR (KBr) 3416, 3308, 3057, 2978, 2932, 1663, 1510, 1368, 1223, 1167, 1051, 752, 700 cm⁻¹; ¹H NMR (DMSO-*d*₆) δ 1.28 (s, 9H), 1.44 (m, 1H), 1.68 (m, 1H), 2.25 (m, 2H), 2.70 (m, 1H), 2.90 (m, 1H), 3.25 (m, 2H), 3.63 (m, 1H), 4.10 (m, 1H), 4.67 (t, 1H, *J* = 5.0 Hz), 7.04-7.28 (m, 19H), 6.85 (d, 1H, *J* = 8.5 Hz), 7.61 (d, 1H, *J* = 8.0 Hz), 8.48 (s, 1H). Anal. (C₃₈H₄₂N₃O₅F·0.75 H₂O) C, H, N.

### Preparation of Intermediate L-(4-F-Phe)-L-(Tr-Glutaminol) Hydrochloride Salt

Using the procedure described in Example 16 for the preparation of L-(4-Cl-Phe)-L-(Tr-glutaminol)hydxochloride salt, this salt was synthesized from BOC-L-(4-F-Phe)-L-(Tr-glutaminol) in 79% yield. White crystalline solid: IR (KBr) 3245, 3057, 2361, 1668, 1510, 1447, 1223, 766, 700 cm⁻¹; ¹H NMR (DMSO-*d*₆) δ 1.47 (m, 1H), 1.72 (m, 1H), 2.30 (m, 2H), 2.94-3.16 (m, 3H), 3.23 (m, 1H), 3.65 (bs, 2H), 3.95 (m, 1H), 7.09-7.32 (m, 19H), 8.28 (m, 4H), 8.54 (s, 1H). Anal. (C₃₃H₃₄N₃O₃F·1.0HCl·1.0 H₂O) C, H, N.

### Preparation of Intermediate N-(4-Methoxyindole-2-Carbonyl)-L-(4-F-Phe)-L-(Tr-Glutaminol)

Using the procedure described in Example 16 for the preparation of N-(4-methoxyindole-2-carbonyl)-L-(4-Cl-Phe)-L-(Tr-glutaminol), this intermediate was synthesized from 4-methoxyindole-2-carboxylic acid and L-(4-F-Phe)-L-(Tr-glutaminol)·HCl salt, in 40% yield. White solid: IR (KBr) 3314, 3059, 2938, 1956, 1888, 1653, 1510, 1361, 1255, 1097, 835, 756, 700 cm⁻¹; ¹H NMR (DMSO-*d*₆) δ 1.58 (m, 1H), 1.81 (m, 1H), 2.28 (m, 2H), 3.02 (m, 2H), 3.23 (m, 2H), 3.67 (m, 1H), 3.87 (s, 3H), 4.69 (m, 2H), 6.49 (m, 1H, *J* = 7.3 Hz), 6.94-7.3 9 (m, 22H), 7.84 (d, 1H, *J* = 8.5 Hz), 8.48 (d, 1H, *J* = 8.5 Hz), 8.53 (s, 1H), 11.49 (s, 1H). Anal. (C₄₃H₄₁N₄O₅F·1.0H₂O) C, H, N.

### Preparation of Intermediate N-(4-Methoxyindole-2-Carbonyl)-L-(4-F-Phe)-L-(Tr-Glutaminal)

Using the oxidation procedure described in Example 16 for the preparation of N-(4-methoxyindole-2-carbonyl)-L-(4-Cl-Phe)-L-(Tr-glutaminal), this aldehyde was prepared in 80% yield from N-(4-methoxyindole-2-carbonyl)-L-(4-F-Phe)-L-(Tr-glutaminol). Glassy white solid: ¹H NMR (DMSO-*d*₆) δ 1.72 (m, 2H), 2.37 (m, 2H), 3.03 (m, 1H), 3.17 (m, 1H), 3.87 (s, 3H), 4.09 (m, 1H), 4.74 (m. 1H), 6.49 (d, 1H, *J* = 7.7 Hz), 6.94-7.41 (m, 22H), 8.58 (m, 2H), 8.63 (s, 1H), 9.32 (s, 1H), 11.49 (s, 1H).

### Preparation of Intermediate Ethyl-3-[N-(4-Methoxyindole-2-Carbonyl)-L-(4-F-Phe)-L-(Tr-Gln)]-E-Propenoate

Using the procedure described in Example 1 for the preparation of compound 12, ethyl-3-[CBZ-L-Leu-L-Phe-L-Met(sulfoxide)-E-propenoate, this vinyl ester was synthesized from N-(4-methoxyindole-2-carbonyl)-L-(4-F-Phe)-L-(Tr-glutaminal) and (carbethoxymethylene)triphenyl-phosphorane in 60% yield. White solid: IR (KBr) 3300, 3061, 2938, 1958, 1890, 1653, 1510, 1368, 1260, 1100, 1036, 835, 756, 700 cm⁻¹; ¹H NMR (DMSO-*d*₆) δ 1.20 (t, 3H, *J* = 7.0 Hz), 1.70 (m, 2H), 2.35 (m, 2H), 3.01 (m, 2H), 3.87 (s, 3H), 4.11 (q, 2H, *J* = 7.0 Hz), 4.41 (m, 1H), 4.67 (m, 1H), 5.68 (d, 1H, *J* = 16.0 Hz), 6.49 (d, 1H, *J* = 7.7 Hz), 6.74 (dd, 1H, *J* = 16.0, 5.0 Hz), 6.97-7.38 (m, 22H), 8.31 (d, 1H, *J* = 8.5 Hz), 8.55 (d, 1H, *J* = 8.5 Hz), 8.58 (s, 1H), 11.51 (s, 1H). Anal. (C₄₇H₄₅N₄O₆F·1.0H₂O) C, H, N.

### Preparation of Product - Ethyl-3-[N-(4-Methoxyindole-2-Carbonyl)-L-(4-F-Phe)-L-Gln]-E-Propenoate

Using the procedure described in Example 32 for the preparation of compound 20, diethyl-[2-(CBZ-L-Leu-L-Phe-L-*Gln*)-E-vinyl] phosphonate, this compound was synthesized by deprotection of ethyl-3-[N-(4-methoxyindole-2-carbonyl)-L-(4-F-Phe)-L-(Tr-*Gln*)]-E-propenoate in 50% yield: White crystalline solid: IR (KBr) 3422, 3293, 2932, 1719, 1665, 1620, 1541, 1510, 1369, 1261, 1182,1101, 752 cm⁻¹; ¹H NMR (DMSO-*d*_{*6*}) δ 1.21 (t, 3H, *J* = 7.0 Hz), 1.73 (m, 2H), 2.10 (t, 2H, *J* = 8.0 Hz), 3.02 (m, 2H), 3.88 (s, 3H), 4.13 (q, 2H, *J* = 7.0 Hz), 4.43 (m, 1H), 4.67 (m, 1H), 5.67 (dd, 1H, *J* = 16.0, 1.5 Hz), 6.49 (d, 1H, *J* = 7.0 Hz), 6.75 (bs, 1H), 6.76 (dd, 1H, *J* = 16.0, 5.5 Hz), 6.96 (d, 1H, *J* = 8.5 Hz), 7.03-7.10 (m, 3H), 7.23 (bs, 1H), 7.31-7.39 (m, 3H), 8.31 (d, 1H, *J* = 8.0 Hz), 8.57 (d, 1H, *J* = 8.0 Hz), 11.51 (s, 1H); HRMS calcd for C₂₈H₃₁N₄O₆F+Cs 671.1282 (M+Cs), found 671.1288. Anal. (C₂₈H₃₁N₄O₆F) C, H, N.

### Example 18 - Preparation of Compound 28: Ethyl-3-[N-(4-Methoxyindole-2-Carbonyl)-L-(3-F-Phe)-L-Gln]-E-Propenoate

### Preparation of Intermediate BOC-L-(3-F-Phe)-L-(Tr-Glutaminol)

Using the procedure described in Example 16 for the preparation of BOC-L-(4-Cl-Phe)-L-(Tr-glutaminol), this compound was synthesized from BOC-L-3-F-Phe and L-(Tr-glutaminol) in 74% yield. White solid: IR (KBr) 3410, 3302, 3059, 3030, 2974, 2934, 1663, 1491, 1448, 1250, 1167, 1051, 752, 700 cm⁻¹; ¹H NMR (DMSO-*d*_{*6*}) δ 1.28 (s, 9H), 1.46 (m, 1H), 1.71 (m, 1H), 2.26 (m, 2H), 2.74 (m, 1H), 2.95 (m, 1H), 3.19 (m, 2H), 3.65 (m, 1H), 4.11 (m, 1H), 4.67 (t, 1H, *J* = 5.0 Hz), 6.97-7.32 (m, 19H), 6.89 (d, 1H, *J* = 8.5 Hz), 7.58 (d, 1H, *J* = 8.5 Hz), 8.48 (s, 1H). Anal. (C₃₈H₄₂N₃O₅F·1.0 H₂O) C, H, N.

### Preparation of Intermediate L-(3-F-Phe)-L-(Tr-Glutaminol) Hydrochloride Salt

Using the procedure described in Example 16 for the preparation of L-(4-Cl-Phe)-L-(Tr-glutaminol)hydrochloride salt, this salt was synthesized from BOC-L-(3-F-Phe)-L-(Tr-glutaminol) in 88% yield. White crystalline solid: IR (KBr) 3231,3047,1668, 1491, 1447, 1254, 1145, 1036, 752, 700 cm⁻¹; ¹H NMR (DMSO-*d*_{*6*}) δ 1.45 (m, 1H), 1.72 (m, 1H), 2.30 (m, 2H), 2.96-3.11 (m, 3H), 3.25 (m, 1H), 3.70 (m, 1H), 4.03 (m, 1H), 7.06-7.38 (m, 19H), 8.30 (bs, 4H), 8.54 (s, 1H). Anal. (C₃₃H₃₄N₃O₃F·1.0 HCl·0.5 H₂O) C, H, N.

### Preparation of Intermediate N-(4-Methoxyindole-2-Carbonyl)-L-(3-F-Phe)-L-(Tr-Glutaminol)

Using the procedure described in Example 16 for the preparation of N-(4-methoxyindole-2-carbonyl)-L-(4-Cl-Phe)-L-(Tr-glutarninol), this intermediate was synthesized from 4-methoxyindole-2-carboxylic acid and L-(3-F-Phe)-L-(Tr-glutaminol)HCl salt, in 60% yield. White solid: IR (KBr) 3291,3057,2936,1956,1890, 1653, 1361, 1256, 1100, 754, 698 cm⁻¹; ¹H NMR (DMSO-*d*₆) δ 1.58 (m, 1H), 1.81 (m, 1H), 2.28 (m, 2H), 3.02 (m, 2H), 3.28 (m, 2H), 3.70 (m, 1H), 3.87 (s, 3H), 4.68 (m, 2H), 6.49 (m, 1H, *J* = 7.7 Hz), 6.94-7.28 (m, 22H), 7.85 (d, 1H, *J* = 8.5 Hz), 8.50 (d, 1H, *J* = 8.5 Hz), 8.53 (s, 1H), 11.50 (s, 1H). Anal. (C₄₃H₄₁N₄O₅F·1.0H₂O) C, H, N.

### Preparation of Intermediate N-(4-Methoxyindole-2-Carbonyl)-L-(3-F-Phe)-L-(Tr-Glutaminal)

Using the oxidation procedure described in Example 16 for the preparation of N-(4-methoxyindole-2-carbonyl)-L-(4-Cl-Phe)-L-(Tr-glutaminal), this aldehyde was prepared in 77% yield from N-(4-methoxyindole-2-carbonyl)-L-(3-F-Phe)-L-(Tr-glutaminol) and was used immediately. Glassy white solid: ¹H NMR (DMSO-*d*₆) δ 1.68 (m, 2H), 2.37 (m, 2H), 3.04 (m, 1H), 3.18 (m, 1H), 3.87 (m, 3H), 4.05 (m, 1H), 4.81 (m, 1H), 6.49 (d, 1H, *J* = 7.7 Hz), 6.94-7.30 (m, 22H), 8.60 (m, 2H), 8.62 (s, 1H), 9.33 (s, 1H), 11.48 (s, 1H).

### Preparation of Intermediate Ethyl-3-[N-(4-Methoxyindole-2-Carbonyl)-L-(3-F-Phe)-L-(Tr-Gln)]-E-Propenoate

Using the procedure described in Example 1 for the preparation of compound 12, ethyl-3-[CBZ-L-Leu-L-Phe-L-*Met*(sulfoxide)-E-propenoate, this vinyl ester was synthesized from N-(4-methoxyindole-2-carbonyl)-L-(3-F-Phe)-L-(Tr-glutaminal) and (carbethoxymethylene)triphenyl-phosphorane in 68% yield. White solid: IR (KBr) 3293, 3057, 2934, 1956, 1894, 1657, 1491, 1368, 1260, 1100, 1036, 978, 756, 700 cm⁻¹; ¹H NMR (DMSO-*d*₆) δ 1.20 (t, 3H, *J* = 7.0 Hz), 1.69 (m, 2H), 2.25 (m, 2H), 3.02 (m, 2H), 3.87 (s, 3H), 4.11 (q, 2H, *J* = 7.0 Hz), 4.42 (m, 1H), 4.69 (m, 1H), 5.71 (d, 1H, *J* = 16.0 Hz), 6.49 (d, 1H, *J* = 8.0 Hz), 6.75 (dd, 1H, *J* = 16.0, 5.0 Hz), 6.91-7.29 (m, 22H), 8.32 (d, 1H, *J* = 8.0 Hz), 8.56 (d, 1H, *J* = 8.0 Hz), 8.59 (s, 1H), 11.51 (s, 1H). Anal. (C₄₇H₄₅N₄O₆F·0.5 H₂O) C, H, N.

### Preparation of Product - Ethyl-3-[N-(4-Methoxyindole-2-Carbonyl)-L-(3-F-Phe)-L-Gln]-E-Propenoate

Using the procedure described in Example 32 for the preparation of compound 20, diethyl-[2-(CBZ-L-Leu-L-Phe-L-*Gln*)-E-vinyl]phosphonate, this compound was synthesized by deprotection of ethyl-3-[N-(4-methoxyindole-2-carbonyl)-L-(3-F-Phe)-L-(Tr-*Gln*)]-E-propenoate in 52% yield. White solid: IR (KBr) 3283, 2932, 1663, 1539, 1370, 1256, 1188, 1098, 1036, 978, 752 cm⁻¹; ¹H NMR (DMSO-*d*₆) δ 1.21 (t, 3H, *J* = 7.0 Hz), 1.73 (m, 2H), 2.11 (t, 2H, *J* = 7.0 Hz), 3.07 (m, 2H), 3.88 (s, 3H), 4.11 (q, 2H, *J* = 7.0 Hz), 4.49 (m, 1H), 4.75 (m, 1H), 5.72 (dd, 1H, *J* = 16.0, 1.5 Hz), 6.49 (d, 1H, *J* = 7.7 Hz), 6.8 0 (m, 2H), 6.98-7.31 (m, 8H), 8.32 (d, 1H, *J* = 8.0 Hz), 8.58 (d, 1H, *J* = 8.0 Hz), 11.52 (s, 1H); HRMS calcd for C₂₈H₃₁N₄O₆F 539.2306 (M+H), found 539.2317. Anal. (C₂₈H₃₁N₄O₆F) C, H, N.

### Example 19 - Preparation of Compound 30: Ethyl-3-(CBZ-L-Phe-L-Gln)-E-Propenoate

### Preparation of Intermediate Ethyl-3-[CBZ-L-Phe-L-(Tr-Gln)]-E-Propenoate

Ethyl-3-[BOC-L-(Tr-*Gln*)]-E-propenoate (0.60 g, 1.1 mmol), prepared as in Example 3, was deprotected and coupled with CBZ-L-Phe (0.31 g, 1.04 mmol) using the procedure described in Example 28 for the preparation of ethyl-2-fluoro-3-[CBZ-L-Leu-L-Phe-L-(Tr-*Gln*)]-E-propenoate to provide ethyl-3-[CBZ-L-Phe-L-(Tr-*Gln*)]-E-propenoate (0.400 g, 53%) as a white foam: IR (thin film) 3298,1651 cm⁻¹; ¹H NMR (CDCl₃) δ 1.21 (t, 3H, *J* = 7.2 Hz), 1.65-1.75 (m, 1H), 1.90-1.93 (m, 1H), 2.29 (s, br, 2H), 2.98-3.00 (m, 2H), 4.12 (q, 2H, *J* = 7.2 Hz), 4.25-4.30 (m, 1H), 4.93 (d, 1H, *J* = 12.3 Hz), 4.50 (s, br, 1H), 5.01 (d, 1H, *J* = 12.3 Hz), 5.23 (d, 1H, *J* = 6.2 Hz), 5.63 (d, 1H, *J* = 15.6 Hz), 6.39 (d, 1H, *J* = 7.2 Hz), 6.61 (dd, 1H, *J* = 15,6, 5.6 Hz), 6.79 (s, 1H), 7.11-7.34 (m, 25H); Anal. (C₄₅H₄₅N₃O₆) C, H, N.

### Preparation of Product - Ethyl-3-(CBZ-L-Phe-L-Gln)-E-Propenoate

Using the procedure described in Example 4 for the preparation of compound 3, Ethyl-3-[CBZ-L-Phe-L-(Tr-*Gln*)]-E-propenoate (0.40 g, 0.58 mmol) was deprotected to provide ethyl-3-(CBZ-L-Phe-L-*Gln*)-E-propenoate (0.15 g, 78%) as a white solid: mp = 184-186 °C; IR (thin film) 3287, 1637, 1533 cm⁻¹; ¹H NMR (DMSO-*d*₆) δ 1.21 (t, 3H, *J* = 7.2 Hz), 1.64-1.80 (m, 2H), 2.08 (t, 2H, *J* = 7.6 Hz), 2.73-2.80 (m, 1H), 2.94 (dd, 1H, *J* = 13.7, 5.3 Hz), 4.11 (q, 2H, *J* = 7.2 Hz), 4.20-4.26 (m, 1H), 4.28-4.39 (m, 1H), 4.95 (s, 2H), 5.69 (d, 1H, *J* = 15.9 Hz), 6.70 (d, 1H, *J* = 5.3 Hz), 6.75-6.77 (m, 2H), 7.17-7.35 (m, 11H), 7.53 (d, 1H, *J* = 8.4 Hz), 8.20 (d, 1H, *J* = 8.1 Hz); Anal. (C₂₆H₃₁N₃O₆) C, H, N.

### Example 20 - Preparation of Compound 31: Ethyl-3-[N-(Propylsulfonyl)-L-Phe-L-Gln]-E-Propenoate

### Preparation of Intermediate Ethyl-3-[BOC-L-Phe-L-(Tr-Gln)]-E-Propenoate

Ethyl-3-[BOC-L-(Tr-*Gln*)]-E-propenoate (2.26 g, 4.16 mmol), prepared as in Example 3, was dissolved in 1,4-dioxane (15 mL). A solution of HCl in 1,4-dioxane (4.0 M, 15 mL) was added dropwise. The reaction solution was stirred at room temperature for 2 hours, then poured into a solution of aqueous NaOH (1 M, 80 mL) in saturated aqueous NaHCO₃ (120 mL). The resulting mixture was extracted with CH₂Cl₂ (2 x 200 mL). The combined organic phases were dried over Na₂SO₄ and concentrated to give the free amine intermediate as a slightly yellow solid, which was used without further purification. This crude amine, BOC-L-Phe (1.10 g, 4.15 mmol), and 1-hydroxybenzotriazole hydrate (0.843 g, 6.24 mmol) were stirred in dry CH₂Cl₂ (35 mL) under argon at room temperature. 4-Methylmorpholine (1.83 mL, 16.6 mmol) and 1-(3-dimethylaminopropyl)-3-ethylcarbodiimide hydrochloride (1.20 g, 6.26 mmol) were added sequentially. After stirring for 3.5 hours, the reaction mixture was poured into water (100 mL), and the mixture was extracted with CH₂Cl₂ (2 x 100 mL). The combined organic phases were dried over Na₂SO₄ and concentrated. The residue was purified by column chromatography (33% acetone in hexanes) to give the product (1.94 g, 68%) as a white foam: IR (thin film) 3413, 3310, 1708, 1660 cm⁻¹; ¹H NMR (CDCl₃) δ 1.30 (t, 3H, *J* = 7.2 Hz), 1.39 (s, 9H), 1.64-1.77 (m, 1H), 1.88-2.00 (m, 1H), 2.25-2.31 (m, 2H), 2.94-3.07 (m, 2H), 4.18 (q, 2H, *J* = 7.2 Hz), 4.49-4.59 (m, 1H), 4.95 (bs, 1H), 5.66 (d, 1H, *J* = 15.9 Hz), 6.29 (m, 1H), 6.64 (dd, 1H, *J* = 15.9, 5.3 Hz), 6.81 (bs, 1H), 7.14-7.34 (m, 21H); Anal. (C₄₂K₄₇N₃O₆) C, H, N.

### Preparation of Intermediate Ethyl-3-[L-Phe-L-(Tr-Gln)]-E-Propenoate

Ethyl-3-[BOC-L-Phe-L-(Tr-*Gln*)]-E-propenoate (0.300 g, 0.435 mmol) was dissolved in 1,4-dioxane (2 mL). A solution of HCl in 1,4-dioxane (4.0 M, 2 mL) was added dropwise. The reaction solution was stirred at room temperature for 2.5 hours, then poured into a solution of aqueous NaOH (1 M, 10 mL) in saturated aqueous NaHCO, (20 mL). The resulting mixture was extracted with CH₂Cl₂ (3 x 40 mL). The combined organic phases were dried over Na₂SO₄ and concentrated to give the product as a foam (0.257 g, quantitative) which was used without further purification.

### Preparation of Intermediate Ethyl-3-[N-(Propylsulfonyl)-L-Phe-L-(Tr-Gln)]-E-Propenoate

Ethyl-3-[L-Phe-L-(Tr-*Gln*)]-E-propenoate was dissolved in dry CH₂Cl₂ (7 mL) under argon and cooled to 0 °C. NEt₃ (0.067 mL, 0.48 mmol) and 1-propanesulfonyl chloride (0.054 mL, 0.48 mmol) were added sequentially. After stirring for 1 hour, the reaction mixture was allowed to warm to room temperature. More NEt, (0.100 mL, 0.714 mmol) and 1-propanesulfonyl chloride (0.086 mL, 0.76 mmol) were added. After 1.5 hours more, the solvent was evaporated and the residue was purified by column chromatography (50% EtOAc in hexanes) to give the product as a foam (0.121 g, 40%): IR (thin film) 3292, 1713, 1652, 1312, 1144 cm⁻¹; ¹H NMR (CDCl₃) δ 0.80 (t, 3H, *J* = 7.5 Hz), 1.28 (t, 3H, *J* = 7.2 Hz), 1.34-1.58 (m, 2H), 1.67-1.81 (m, 1H), 1.92-2.04 (m, 1H), 2.32-2.56 (m, 4H), 2.79 (dd, 1H, *J* = 13.9, 8.9 Hz), 3.05 (dd, 1H, *J* = 13.9, 5.5 Hz), 3.96-4.05 (m, 1H), 4.17 (q, 2H, *J* = 7.2 Hz), 4.49-4.59 (m, 1H), 5.14 (d, 1H, *J* = 8.7 Hz), 5.75 (dd, 1H, *J* = 15.9, 1.7 Hz), 6.72 (dd, 1H, *J* = 15.9,5.3 Hz), 6.94 (s, 1H), 7.02 (d, 1H, *J* = 8.1 Hz), 7.12-7.33 (m, 20H); HRMS (M+Cs) calcd for C₄₀H₄₅N₃O₆S 828.2083, found 828.2063.

### Preparation of Product - Ethyl-3-[N-(Propylsulfonyl)-L-Phe-L-Gln]-E-Propenoate

Ethyl-3-[N-(propylsulfonyl)-L-Phe-L-(Tr-*Gln*)]-E-propenoate (0.100 g, 0.143 mmol) was dissolved in CH₂Cl₂/TFA 1:1 (4 mL) under argon. The bright yellow solution was stirred at room temperature for 30 minutes. CCl₄ (4 mL) was added and the solution was concentrated to dryness. The residue was triturated with Et₂O (3 mL) to give a white precipitate which was collected by filtration and washed with Et₂O (2 x 2 mL) to give the product (0.048 g, 74%): mp = 161-162 °C; IR (KBr) 3284, 3213, 1708, 1666, 1543, 1314, 1138 cm⁻¹; ¹H NMR (acetone-*d*₆) δ 0.83 (t, 3H, *J* = 7.5 Hz), 1.25 (t, 3H, *J* = 7.2 Hz), 1.39-1.62 (m, 2H), 1.73-2.02 (m, 2H), 2.23-2.30 (m, 2H), 2.54-2.72 (m, 2H), 2.92 (dd, 1H, *J* = 13.5, 8.9 Hz), 3.15 (dd, 1H, *J* = 13.5, 6.1 Hz), 4.14 (q, 2H, *J* = 7.2 Hz), 4.12-4.21 (m, 1H), 4.53-4.63 (m, 1H), 5.79 (dd, 1H, *J* = 15.7, 1.7 Hz), 6.18 (bs, 1H), 6.30 (d, 1H, *J* = 8.7 Hz), 6.78 (dd, 1H, *J* = 15.7, 5.4 Hz), 6.75 (bs, 1H), 7.19-7.35 (m, 5H), 7.59 (d, 1H, *J* = 8.1 Hz); Anal. (C₂₁H₃₁N₃O₆S) C, H, N.

### Example 21 - Preparation of Compound 32: Ethyl-3-[N-(Benzylsulfonyl)-L-Phe-L-Gln]-E-Propenoate

### Preparation of Intermediate Ethyl-3-[N-(Benzylsulfonyl)-L-Phe-L-(Tr-Gln)]-E-Propenoate

Ethyl-3-[L-Phe-L-(Tr-*Gln*)]-E-propenoate (0.250 g, 0.424 mmol) was dissolved in dry CH₂Cl₂ (7 mL) under argon and cooled to 0 °C. Triethylamine (0.118 mL, 0.847 mmol) and α-toluenesulfonyl chloride (0.162 g, 0.850 mmol) were added sequentially. After stirring for 45 min, the solvent was evaporated and the residue was purified by column chromatography (47% EtOAc in hexanes) to give the product as a white foam (0.154 g, 49%): IR (thin film) 3296, 1708, 1663, 1316, 1154 cm⁻¹; ¹H NMR (CDCl₃) δ 1.29 (t, 3H, *J* = 7.2 Hz), 1.59-1.72 (m, 1H), 1.91-2.03 (m, 1H), 2.31-2.37 (m, 2H), 2.82 (dd, 1H, *J* = 13.7, 7.2 Hz), 2.92 (dd, 1H, *J* = 13.7, 7.2 Hz), 3.78-3.87 (m, 1H), 3.90 (d, 1H, *J* = 13.9 Hz), 3.97 (d, 1H, *J* = 13.9 Hz), 4.17 (q, 2H, *J* = 7.2 Hz), 4.44-4.54 (m, 1H), 4.96 (d, 1H, *J* = 7.8 Hz), 5.59 (dd, 1H, *J* = 15.7, 1.7 Hz), 6.51 (d, 1H, *J* = 7.5 Hz), 6.63 (dd, 1H, *J* = 15.7, 5.1 Hz), 6.91 (s, 1H), 7.03-7.07 (m, 2H), 7.17-7.40 (m, 23H); Anal. (C₄₄H₄₅N₃O₆S) C, H, N.

### Preparation of Product - Ethyl-3-[N-(Benzylsulfonyl)-L-Phe-L-Gln]-E-Propenoate

This compound was prepared in 72% yield from ethyl-3-[N-(benzylsulfonyl)-L-Phe-L-(Tr-*Gln*)]-E-propenoate using the procedure described in Example 20 for the preparation of ethyl-3-[N-(propylsulfonyl)-L-Phe-L-*Gln*]-E-propenoate: mp = 165-167 °C; IR (KBr) 3330, 3201, 1713, 1660, 1314 cm⁻¹; ¹H NMR (acetone-*d*₆) δ 1.25 (t, 3H, *J* = 7.2 Hz), 1.72-1.99 (m, 2H), 2.22-2.30 (m, 2H), 2.96 (dd, 1H, *J* = 13.5, 7.3 Hz), 3.10 (dd, 1H, *J* = 13.5, 7.0 Hz), 4.03-4.22 (m, 5H), 4.51-4.62 (m, 1H), 5.72 (dd, 1H, *J* = 15.6, 1.6 Hz), 6.18 (bs, 1H), 6.33 (d, 1H, *J* = 8.4 Hz), 6.72 (bs, 1H), 6.73 (dd, 1H, *J* = 15.6, 5.4 Hz), 7.19-7.35 (m, 10H), 7.55 (d, 1H, *J* = 8.1 Hz); Anal. (C₂₅H₃₁N₃O₆S) C, H, N.

### Example 22 - Preparation of Compound 33: Ethyl-3-[N-(Ethylsulfonyl)-L-Phe-L-Gln]-E-Propenoate

### Preparation of Intermediate Ethyl-3-[N-(Ethylsulfonyl)-L-Phe-L-(Tr-Gln)]-E-Propenoate

This compound was prepared in 46% yield from ethyl-3-[L-Phe-L-(Tr-*Gln*)]-E-propenoate and ethanesulfonyl chloride using the procedure described in Example 21 for the preparation of ethyl-3-[N-(benzylsulfonyl)-L-Phe-L-(Tr-*Gln*)]-E-propenoate. The material was purified by flash column chromatography (50% EtOAc in hexanes): IR (thin film) 3295, 1713, 1666, 1314, 1143 cm⁻¹; ¹H NMR (CDCl₃) δ 1.04 (t, 3H, *J* = 7.5 Hz), 1.29 (t, 3H, *J* = 7.2 Hz), 1.68-1.81 (m, 1H), 1.95-2.06 (m, 1H), 2.33-2.43 (m, 2H), 2.45-2.58 (m, 1H), 2.59-2.72 (m, 1H), 2.86 (dd, 1H, *J* = 13.7, 8.4 Hz), 3.09 (dd, 1H, *J* = 13.7, 5.6 Hz), 3.96-4.04 (m, 1H), 4.19 (q, 2H, *J* = 7.2 Hz), 4.50-4.59 (m, 1H), 4.91 (bs, 1H), 5.72 (dd, 1H, *J* = 15.9, 1.9 Hz), 6.71 (dd, 1H, *J* = 15.9, 5.3 Hz), 6.87 (s, 1H), 6.96 (d, 1H, *J* = 7.8 Hz), 7.13-7.34 (m, 20H); Anal. (C₃₉H₄₃N₃O₆S) C, H, N.

### Preparation of Product Ethyl-3-[N-(Ethylsulfonyl)-L-Phe-L-Gln]-E-Propenoate

This compound was prepared in 82% yield from ethyl-3-[N-(ethylsulfonyl)-L-Phe-L-(Tr-*Gln*)]-E-propenoate using the procedure described in Example 20 for the preparation of compound 31, ethyl-3-[N-(propylsulfonyl)-L-Phe-L-*Gln*]-E-propenoate: mp = 150-151 °C; IR (KBr) 3284, 3225, 1713, 1655, 1314,1138 cm⁻¹; ¹H NMR (acetone-*d*₆) δ 1.05 (t, 3H, *J* = 7.3 Hz), 1.26 (t, 3H, *J* = 7.2 Hz), 1.74-1.87 (m, 1H), 1.90-2.02 (m, 1H), 2.22-2.33 (m, 2H), 2.62-2.84 (m, 2H), 2.95 (dd, 1H, *J* = 13.7, 8.7 Hz), 3.15 (dd, 1H, *J* = 13.7, 6.2 Hz), 4.16 (q, 2H, *J* = 7.2 Hz), 4.13-4.23 (m, 1H), 4.54-4.64 (m, 1H), 5.78 (dd, 1H, *J* = 15.9, 1.6 Hz), 6.22 (bs, 1H), 6.34 (d, 1H, *J* = 9.0 Hz), 6.78 (bs, 1H), 6.78 (dd, 1H, *J* = 15.9, 5.6 Hz), 7.21-7.35 (m, 5H), 7.61 (d, 1H, *J* = 8.1 Hz); Anal. (C₂₀H₂₉N₃O₆S) C, H, N.

### Example 23 - Preparation of Compound 34: Ethyl-3-[N-(Phenylsulfonyl)-L-Phe-L-Gln]-E-Propenoate

### Preparation of Intermediate Ethyl-3-[N-(Phenylsulfonyl)-L-Phe-L-(Tr-Gln)]-E-Propenoate

This compound was prepared in 55% yield from ethyl-3-[L-Phe-L-(Tr-*Gln*)]-E-propenoate and benzenesulfonyl chloride using the procedure described in Example 21 for the preparation of ethyl-3-[N-(benzylsulfonyl)-L-Phe-L-(Tr-*Gln*)]-E-propenoate. The material was purified by flash column chromatography (47% EtOAc in hexanes): IR (thin film) 3295, 1713, 1660, 1308, 1161 cm⁻¹; ¹H NMR (CDCl₃) δ 1.29 (t, 3H, *J* = 7.2 Hz), 1.59-1.72 (m, 1H), 1.83-1.95 (m, 1H), 2.12-2.33 (m, 2H), 2.82-2.94 (m, 2H), 3.82-3.91 (m, 1H), 4.18 (q, 2H, *J* = 7.2 Hz), 4.31-4.41 (m, 1H), 5.05 (d, 1H, *J* = 7.8 Hz), 5.67 (dd, 1H, *J* = 15.7, 1.7 Hz), 6.60 (dd, 1H, *J* = 15.7, 5.4 Hz), 6.72 (d, 1H, *J* = 7.8 Hz), 6.79 (s, 1H), 6.91-6.97 (m, 2H), 7.13-7.40 (m, 20H), 7.48-7.54 (m, 1H), 7.58-7.62 (m, 2H); Anal. (C₄₃H₄₃N₃O₆S) C, H, N.

### Preparation of Product Ethyl-3-[N-(Phenylsulfonyl)-L-Phe-L-Gln]-E-Propenoate

This compound was prepared in 83% yield from ethyl-3-[N-(phenylsulfonyl)-L-Phe-L-(Tr-*Gln*)]-E-propenoate using the procedure described in Example 20 for the preparation of ethyl-3-[N-(propylsulfonyl)-L-Phe-L-*Gln*]-E-propenoate: mp = 173-175 °C; IR (KBr) 3284, 3201, 1708, 1660, 1314, 1161 cm⁻¹; ¹H NMR (acetone-*d*₆) δ 1.24 (t, 3H, *J* = 7.2 Hz), 1.59-1.85 (m, 2H), 2.07-2.19 (m, 2H), 2.85 (dd, 1H, *J* = 13.5, 7.6 Hz), 2.99 (dd, 1H, *J* = 13.5, 6.7 Hz), 4.03-4.16 (m, 1H), 4.13 (q, 2H, *J* = 7.2 Hz), 4.30-4.40 (m, 1H), 5.65 (dd, 1H, *J* = 15.7, 1.6 Hz), 6.21 (bs, 1H), 6.63 (dd, 1H, *J* = 15.7, 5.6 Hz), 6.74 (bs, 1H), 6.75 (d, 1H, *J* = 8.7 Hz), 7.07-7.29 (m, 5H), 7.42-7.61 (m, 4H), 7.67-7.80 (m, 2H); Anal. (C₂₄H₂₉N₃O₆S) C, H, N.

### Example 24 - Preparation of Compound 35: Ethyl-3-[CBZ-L-Leu-L-(4-F-Phe)-L-Gln]-E-Propenoate

### Preparation of Intermediate CBZ-L-Leu-L-(4-F-Phe)-L-(Tr-Glutaminol)

Using the procedure described in Example 16 for the preparation of N-(4-methoxyindole-2-carbonyl)-L-(4-Cl-Phe)-L-(Tr-glutaminol), this intermediate was synthesized from CBZ-L-Leu and the free base of L-(4-F-Phe)-L-(Tr-glutaminol)·HCl, in 68% yield as a white solid: IR (KBr) 3304, 3063, 2955, 1651, 1510, 1223, 1038, 752, 698 cm⁻¹; ¹H NMR (DMSO-*d*₆) δ 0.79 (m, 6H), 1.34 (m, 2H), 1.46 (m, 2H), 1.72 (m, 1H), 2.25 (m, 2H), 2.80 (m, 1H), 2.99 (m, 1H), 3.16 (m, 1H), 3.26 (m, 1H), 3.64 (m, 1H), 3.95 (m, 1H), 4.47 (m, 1H), 4.66 (t, 1H, *J* = 5.5 Hz), 4.97 (d, 1H, *J* = 12.5Hz), 5.02 (d, 1H, *J* = 12.5 Hz), 7.01 (t, 2H, *J* = 8.8 Hz), 7.15-7.37 (m, 22H), 7.42 (d, 1H, *J* = 7.7 Hz), 7.69 (d, 1H, *J* = 8.5 Hz), 7.87 (d, 1H, *J* = 8 Hz), 8.54 (s, 1H).

### Preparation of Intermediate CBZ-L-Leu-L-(4-F-Phe)-L-(Tr-Glutaminal)

Using the oxidation procedure described in Example 16 for the preparation of N-(4-methoxyindole-2-carbonyl)-L-(4-CI-Phe)-L-(Tr-glutaminal), this aldehyde was prepared from CBZ-L-Leu-L-(4-F-Phe)-L-(Tr-glutaminol) in 92% yield as a white glassy solid, which was used immediately without further purification.

### Preparation of Intermediate Ethyl-3-[CBZ-L-Leu-L-(4-F-Phe)-L-(Tr-Gln)]-E-Propenoate

Using the procedure described in Example 1 for the preparation of compound 12, ethyl-3-[CBZ-L-Leu-L-Phe-L-*Met*(sulfoxide)-E-propenoate, (carbethoxymethylene)triphenyl-phosphorane and CBZ-L-Leu-L-(4-F-Phe)-L-(Tr-glutaminal) were stirred together in THF giving 0.37 g of the crude material contaminated with triphenylphosphine oxide which was subsequently used without further purification. A small amount (27 mg) was purified by flash column chromatography (MeOH/CHCl₃) for spectral analysis: ¹H NMR (DMSO-*d*₆) δ 0.79 (t, 6H, *J* = 7.0 Hz), 1.20 (t, 3H, *J* = 7.0 Hz), 1.23-1.82 (m, 5H), 2.25 (m, 2H), 2.85 (m, 1H), 2.95 (m, 1H), 3.96 (m, 1H), 4.10 (q, 2H, *J* = 7.0 Hz), 4.34 (m, 1H), 4.48 (m, 1H), 4.96 (d, 1H, *J* = 13.0 Hz), 5.02 (d, 1H, *J* = 13.0 Hz), 5.57 (d, 1H, *J* = 15.0 Hz), 6.67 (dd, 1H, *J* = 15.0, 5.5 Hz), 7.01 (t, 2H, *J* = 9.0 Hz), 7.13-7.32 (m, 22H), 7.39 (d, 1H, *J* = 8.0 Hz), 7.99 (d, 1H, *J* = 8.0 Hz), 8.07 (d, 1H, *J* = 8.0 Hz), 8.58 (s, 1H).

### Preparation of Product Ethyl-3-[CBZ-L-Leu-L-(4-F-Phe)-L-Gln)-E-Propenoate

This compound was prepared by the deprotection of ethyl-3-[CBZ-L-Leu-L-(4-F-Phe)-L-(Tr-*Gln*)]-E-prapenoate using the procedure describe in Example 32 for the preparation of compound 20, but in the absence of triisopropylsilane. The product was isolated as a white solid in 58% yield (2 steps from CBZ-L-Leu-L-(4-F-Phe)-L-(Tr-glutaminal). IR (KBr) 3439, 3293, 3067, 2961, 1692, 1643, 1539, 1227, 1045, 984, 835, 698 cm⁻¹; ¹H NMR (DMSO-*d*₆) δ 0.80 (m, 6H), 1.21 (t, 3H, *J* = 7.0 Hz), 1.26 (m, 2H), 1.45 (m, 1H), 1.71 (m, 2H), 2.06 (t, 2H, *J* = 7.5 Hz), 2.81 (m, 1H), 2.94 (m, 1H), 3.97 (m, 1H), 4.10 (q, 2H, *J* = 7.0 Hz), 4.37 (m, 1H), 4.47 (m, 1H), 4.98 (d, 1H, *J* = 12.5 Hz), 5.04 (d, 1H, *J* = 12.5 Hz), 5.59 (d, 1H, *J* = 16.0 Hz), 6.68 (dd, 1H, *J* = 16.0, 5.5 Hz), 6.76 (bs, 1H), 7.01 (t, 2H, *J* = 8.8 Hz), 7.19-7.34 (m, 8H), 7.43 (d, 1H, *J* = 8.0 Hz), 8.05 (m, 2H); HRMS calcd for C₃₂H₄₃N₄O₇F+Cs 745.2014 (M+Cs), found 745.2040 Anal. (C₃₂H₄₁N₄O₇F·1.25 H₂O) C, H, N.

### Example 25 - Preparation of Compound 15: 3-(CBZ-L-Leu-L-Phe-L-Gln)-E-Propenoic Acid

### Preparation of Intermediate tert-Butyl-3-[CBZ-L-Phe-L-(Tr-Gln)]-E-Propenoate

To 0.20 g (0.261 mmol) of CBZ-L-Leu-L-Phe-L-(Tr-glutaminal) was added 3 mL of dry THF. To this stirred solution was added (*tert*-butoxycarbonylmethylene) triphenylphosphorane (0.098 g, 0.261 mmol). The reaction mixture was stirred at room temperature overnight. The solvent was removed in vacuo, and the residue was subjected to column chromatography with hexanes:EtOAc (6.5:3.5). The product was obtained in 69% yield as a white foam.

### Preparation of Product 3-(CBZ-L-Leu-L-Phe-L-Gln)-E-Propenoic Acid

*tert*-Butyl-3-[CBZ-L-Leu-L-Phe-L-(Tr-*Gln*)]-E-propenoate (0.157 g, 0.181 mmol) was dissolved in an excess of TFA, and 10 drops of water were added. The mixture was stirred at room temperature for 1 hour and evaporated to dryness. CCl₄ was added and the mixture was concentrated in vacuo to azeotrope any remaining water. The residue was slurried in Et₂O and the resulting white solid was filtered and dried to give 0.053 g (52%). mp = 219-220 °C (dec); IR (thin film); 2949, 1690, 3269, 1639 cm⁻¹; ¹H NMR (DMSO-*d*₆) δ 0.80 (dd, 6H, *J* = 9.0, 6.5 Hz), 1.23-1.38 (m, 2H), 1.41-1.56 (m, 1H), 1.61-1.79 (m, 2H), 2.0-2.1 (m, 2H), 2.84 (dd, 1H, *J* = 13.6, 8.9 Hz), 2.99 (dd, 1H, *J* = 13.5, 5.1 Hz), 3.91 (m, 1H), 4.32-4.41 (m, 1H), 4.44-4.54 (m, 1H), 5.01 (dd, 1H, *J* = 12.5, 12.1 Hz), 5.64 (d, 1H, *J* = 15.6 Hz), 6.64 (dd, 1H, *J* = 15.6, 5.6 Hz), 6.76 (bs, 1H), 7.14-7.3 (m, 11H), 7.43 (d, 1H, *J* = 7.5 Hz), 7.97 (d, 1H, *J* = 8.1 Hz), 8.04 (d, 1H, *J* = 8.1 Hz), 12.28 (bs, 1H).

### Example 26 - Preparation of Compound 14: 3-(CBZ-L-Leu-L-Phe-DL-Gln)-E-Propenonitrile

### Preparation of Intermediate 3-[BOC-DL-(Tr-Gln)]-E-Propenonitrile

A solution of diethyl cyanomethylphosphonate (0.202 mL, 1.25 mmol) in dry THF (25 mL) was cooled to -78 °C. After dropwise addition of a solution of sodium bis(trimethylsilyl)amide in THF (1.0 M, 1.25 mL), the reaction solution was stirred for 20 minutes. A solution of BOC-L-(Tr-glutaminal) (0.590 g, 1.25 mmol) in dry THF (5 mL) was added dropwise, and, after stirring 50 minutes more, saturated aqueous NH₄Cl (4 mL) was added. The reaction mixture was allowed to warm to room temperature, and the THF was evaporated. Water (10 mL) was added to the residue, which was then extracted with CH₂Cl₂ (3 x 30 mL). The combined organic phases were dried over Na₂SO₄ and concentrated. The residue was purified by flash column chromatography (38% EtOAc in hexanes) to give the product (0.407 g, 66%) as a white foam: IR (thin film) 3321, 2225, 1694, 1515 cm⁻¹; ¹H NMR (CDCl₃) δ 1.42 (s, 9H), 1.67-1.81 (m, 1H), 1.82-1.97 (m, 1H), 2.34-2.42 (m, 2H), 4.23 (bs, 1H), 4.97-5.06 (m, 1H), 5.39 (dd, 1H, *J* = 16.3, 1.6 Hz), 6.56 (dd, 1H, *J* = 16.3, 5.3 Hz), 6.77 (bs, 1H), 7.15-7.33 (m, 15H).

### Preparation of Intermediate (CBZ-L-Leu-L-Phe)₁O

CBZ-L-Leu-L-Phe (1.5 g, 3.6 mmol) was dissolved in dry CH₂Cl₂ (25 mL) at room temperature under argon. 1-(3-Dimethylaminopropyl)-3-ethytcarbodiimide hydrochloride (0.697 g, 3.64 mmol) was added. The reaction solution was stirred for 20 hours, then diluted with CH₂Cl₂ (20 mL) and washed with water (2 x 20 mL). The combined organic phases were dried over Na₂SO₄ and concentrated to give the anhydride product as a white semi-solid residue (1.18 g, 80%), which was used immediately in the next step of the reaction without further purification or analysis.

### Preparation of Intermediate 3-[CBZ-L-Leu-L-Phe-DL-(Tr-Gln)]-E-Propenonitrile

3-[BOC-DL-(Tr-*Gln*)]-E-Propenonitrile (0.349 g, 0.704 mmol) was stirred in 2-propanol (9 mL) at room temperature. Perchloric acid (60%, 3.2 mL) was added dropwise. The resulting solution was stirred for 1 hour under an argon balloon, diluted with CH₂Cl₂ (100 mL), and poured into a solution of aqueous 1N NaOH/aqueous saturated NaHCO₃ (40 mL:70 mL). The phases were mixed and separated. The aqueous phase was washed again with CH₂Cl₂ (2 x 100 mL). The combined organic phases were dried over Na₂SO₄ and then concentrated to give the crude amine as a white solid (0.314 g), which was used without further purification. This amine was dissolved in acetone (15 mL) and added to the crude (CBZ-L-Leu-L-Phe)₂O (1.18 g, 1.46 mmol) in a round bottom flask. The reaction solution was stirred at room temperature under an argon balloon. After stirring for 4.5 hours, the solvent was evaporated, and the residue was purified by flash column chromatography (30% EtOAc in hexanes, then 30% acetone in hexanes) to give the product (0.448 g, 81%) as a white foam: IR (thin film) 3298, 2226, 1672, 1519 cm⁻¹; Anal. (C₄₉H₅₁N₅O₅) C, H, N.

### Preparation of Product 3-(CBZ-L-Leu-L-Phe-DL-Gln)-E-Propenonitrile

3-[CBZ-L-Leu-L-Phe-DL-(Tr-*Gln*)]-E-Propenonitrile (0.3 81 g, 0.482 mmol) was dissolved in 1:1 CH₂Cl₂/TFA (14 mL) under argon, giving a bright yellow solution. After stirring for 30 minutes, the solvent was evaporated. CCI₄ (15 mL) was added, and the resulting solution was concentrated (3 times). The residue was triturated with Et₂O (8 mL) to give a white solid, which was collected by filtration. This solid was then stirred in acetonitrile (4 mL), collected by filtration, washed with acetonitrile (4 mL), washed with Et₂O (6 mL), and dried *in vacuo* (0.099 g, 38%): mp = 178-184 °C; IR (KBr) 3401, 3284, 2225, 1689, 1650, 1537 cm⁻¹; ¹H NMR (DMSO-*d*₆) (2 diastereomers) δ 0.69 (d, 3H, *J* = 5.3 Hz), 0.73 (d, 3H, *J* = 5.1 Hz), 0.80 (d, 3 H, *J* = 6.6 Hz), 0.83 (d, 3H, *J*= 6.6 Hz), 1.10-1.20 (m, 3H), 1.26-1.40 (m, 2H), 1.46-1.85 (m, 5H), 1.99-2.09 (m, 4H), 2.76 (dd, 1H, *J* = 13.4, 10.9 Hz), 2.83-2.99 (m, 2H), 3.10 (dd, 1H, *J* = 13.6, 4.3 Hz), 3.85-3.93 (m, 1H), 3.96-4.05 (m, 1H), 4.28-4.52 (m, 4H), 4.90-5.07 (m, 5H), 5.71 (d, 1H, *J* = 16.4 Hz), 6.68 (dd, 1H, *J* = 16.4,4.6 Hz), 6.78 (s, 2H), 6.88 (dd, 1H, *J* = 16.3,4.7 Hz), 7.16-7.37 (m, 22H), 7.41-7.47 (m, 2H), 7.96 (d, 1H, *J* = 8.2 Hz), 8.03-8.10 (m, 2H), 8.38 (d, 1H, *J* = 8.2 Hz); Anal. (C₃₀H₃₇N₅O₅) C, H, N.

### Example 27 - Preparation of Compound 6: N-Ethyl-3-(CBZ-L-Leu-L-Phe-L-Gln)-E-Propenamide

### Preparation of Intermediate N-Ethyl-3-[BOC-L-(Tr-Gln)]-E-Propenamide

Isobutyl chloroformate (0.161 mL, 1.24 mmol) was added to a solution of 3-[BOC-L-(Tr-*Gln*)]-E-propenoic acid (0.639 g, 1.24 mmol) and 4-methylmorpholine (1.36 mL, 12.4 mmol) in CH₂Cl₂ at 0 °C. The resulting solution was stirred for 20 minutes at 0 °C, then ethylamine hydrochloride (0.810 g, 9.93 mmol) was added. The reaction mixture was warmed to 23 °C and was stirred for 24 hours, then was partitioned between water (100 mL) and a 9:1 mixture of CH₂Cl₂ and CH₃OH (2 x 100 mL). The organic layers were dried over Na₂SO₄ and were concentrated. Purification of the residue by flash column chromatography (5% CH₃OH/CH₂Cl₂) provided an oil, which was triturated with EtOAc to afford a white solid. The solid was filtered, washed with EtOAc (2 x 20 mL), and was air-dried to give N-ethyl-3-[BOG-L-(Tr-*Gln*)]-E-propenamide (0.055 g, 8%): mp = 240 °C (dec); IR (thin film) 3255,3085,1715,1665,1612,1529 cm⁻¹; ¹H NMR (CDCl₃) δ 1.15 (t, 3H, *J* = 7.2 Hz), 1.42 (s, 9H), 1.63-1.80 (m, 1H), 1.83-2.05 (m, 1H), 2.34-2.39 (m, 2H), 3.29-3.38 (m, 2H), 4.26 (s, br, 1H), 4.75 (s, br, 1H), 5.43 (s, br, 1H), 5.81 (d, 1H, *J* = 15.4 Hz), 6.65 (dd, 1H, *J* = 15.4,5.9 Hz), 6.85 (s, 1H), 7.19-7.33 (m, 15H); Anal. (C₃₃H₃₇N₃O₄) C, H, N.

### Preparation of Intermediate N-Ethyl-3-[CBZ-L-Leu-L-Phe-L-(Tr-Gln)]-E-Propenamide

N-Ethyl-3-[BOC-L-(Tr-*Gln*)]-E-propenamide (0.040 g, 0.074 mmol) was deprotected and coupled with CBZ-L-Leu-L-Phe (0.030 g, 0.073 mmol) using the procedure described in Example 28 for the preparation of ethyl-2-fluoro-3-[CBZ-L-Leu-L-Phe-L-(Tr-*Gln*)]-E-propenoate to provide N-ethyl-3-[CBZ-L-Leu-L-Phe-L-(Tr-*Gln*)]-E-propenamide (0.043 g, 70%) as a white solid: mp = 190 °C (dec); IR (thin film) 3283, 3067, 1693, 1642, 1535 cm⁻¹; ¹H NMR (CDCl₃) δ 0.83 (d, 3H, *J* = 9.0 Hz), 0.85 (d, 3H, *J* = 9.0 Hz), 1.14 (t, 2H, *J* = 7.3 Hz), 1.21-1.32 (m, 1H), 1.37-1.52 (m, 2H), 1.71 -1.78 (m, 1H), 1.94-2.05 (m, 1H), 2.26 (t, 2H, *J*= 7.3 Hz), 2.91 (dd, 1H, *J* = 13.8,7.6 Hz), 3.16 (dd, 1H, *J* = 13.8, 6.2 Hz), 3.26-3.35 (m, 2H), 3.94-4.01 (m, 1H), 4.53-4.55 (m, 2H), 4.89-4.94 (m, 3H), 5.56-5.65 (m, 2H), 6.51 (d, 1H, *J* = 8.1 Hz), 6.60 (dd, 1H, *J* = 15.1, 4.8 Hz), 6.81 (d, 1H, *J* = 8.4 Hz), 7.02 (s, 1H), 7.10-7.36 (m, 26H); Anal. (C₅₁H₅₇N₅O₆) C, H, N.

### Preparation of Products N-Ethyl-3-(CBZ-L-Leu-L-Phe-L-Gln)-E-Propenamide

Using the procedure described in Example 4 for the preparation of compound 3, N-ethyl-3-[CBZ-L-Leu-L-Phe-L-(Tr-*Gln*)]-E-propenamide was deprotected to produce the product. mp = 230 °C (dec), R_{*f*} = 0.28 (10% MeOH in CH₂Cl₂); IR (KBr) 3404, 3075, 2943, 1692, 1643 cm⁻¹; ¹H NMR (DMSO-*d*₆) δ 0.78 (d, 3H, *J* = 11.5 Hz), 0.80 (d, 3H, *J* = 11.5 Hz), 1.02 (t, 3H, *J* = 7.3 Hz), 1.24-1.29 (m, 2H), 1.32-1.47 (m, 1H), 1.67-1.71 (m, 2H), 2.03-2.08 (m, 2H), 2.77-2.85 (m, 1H), 2.99-3.16 (m, 3H), 3.91-3.98 (m, 1H), 4.29-4.34 (m, 1H), 4.48-4.49 (m, 1H), 4.97 (d, 1H, *J* = 12.5 Hz), 5.04 (d, 1H, *J* = 12.5 Hz), 5.85 (d, 1H, *J* = 15.3 Hz), 6.43 (dd, 1H, *J* = 15.4, 6.4 Hz), 6.75 (s, 1H), 7.20 (bs, 7H), 7.30-7.34 (m, 4H), 7.41 (d, 1H, *J* = 7.8 Hz), 7.90 (d, 1H, *J* = 7.8 Hz), 7.97 (t, 1H, *J* = 5.1 Hz), 8.08 (d, 1H, *J* = 8.1 Hz); Anal. (C₃₂H₄₃N₅O₆) C, H, N.

### Example 28 - Preparation of Compound 8: Ethyl-2-Fluoro-3-(CBZ-L-Leu-L-Phe-L-Gln)-E-Propenoate

### Preparation of Intermediate Ethyl-2-Fluoro-3-[BOC-L-(Tr-Gln)]-E-Propenoate

Sodium bis(trimethylsilyl)amide (0.264 mL of a 1.0 M solution in THF, 0.264 mmol) was added to a solution of triethyl-2-fluoro-2-phosphonoacetate (0.054 mL, 0.266 mmol) in THF (10 mL) at -78 °C, and the resulting solution was stirred for 15 minutes at that temperature. BOC-L-(Tr-Glutaminal) (0.125 g, 0.264 mmol) in THF (10 mL) was added via cannula, and the reaction mixture was stirred for 30 minutes at -78 °C then was partitioned between 0.5 M HCl (100 mL) and a 1:1 mixture of EtOAc and hexanes (2 x 100 mL). The organic layers were dried over Na₂SO₄ and were concentrated. Purification of the residue by flash column chromatography (30% EtOAc in hexanes) provided ethyl-2-fluoro-3-[BOC-L-(Tr-*Gln*)]-E-propenoate (0.094 g, 63%) as a white foam: IR (thin film) 3324, 1724, 1670 cm⁻¹; ¹H NMR (CDCl₃) δ 1.33 (t, 3H, *J* = 7.2 Hz), 1.41 (s, 9H), 1.92-2.05 (m, 2H), 2.39 (t, 2H, *J* = 7.2 Hz), 4.28 (q, 2H, *J* = 7.2 Hz), 5.00 (bs, 2H), 5.74 (dd, 1H, *J* = 19.8, 8.6 Hz), 6.78 (s, 1H), 7.14-7.32 (m, 15H); Anal. (C₃₃H₃₇FN₂O₅) C, H, N.

### Preparation of Intermediate Ethyl-2-Fluoro-3-[CBZ-L-Leu-L-Phe-L-(Tr-Gln)]-E-Propenoate.

A solution of HCl in 1,4-dioxane (4 mL of a 4.0 M solution, 16 mmol) was added to a solution of ethyl-2-fluoro-3-[BOC-L-(Tr-*Gln*)]-E-propenoate (0.310 g, 0.553 mmol) in the same solvent (4 mL) at 23 °C. The reaction mixture was stirred for 4 hours at 23 °C, then was concentrated. The resulting oil was dissolved in CH₂Cl₂, and CBZ-L-Leu-L-Phe (0.228 g, 0.553 mmol), 1-hydroxybenzotriazole hydrate (0.112 g, 0.828 mmol), 4-methylmorpholine (0.182 mL, 1.67 mmol), and 1-(3-dimethylaminopropyl)-3-ethylcarbodiimide hydrochloride (0.159 g, 0.829 mmol) were added sequentially. The reaction mixture was stirred for 12 hours at 23 °C, then was partitioned between water (100 mL) and EtOAc (2 x 100 mL). The organic layers were dried over Na₂SO₄ and were concentrated. Purification of the residue by flash column chromatography (5% CH₃OH/CH₂Cl₂) afforded ethyl-2-fluoro-3-[CBZ-L-Leu-L-Phe-L-(Tr-*Gln*)]-E-propenoate (0.203 g, 43%) as a white foam: IR (thin film) 3394,3066,1724,1647 cm⁻¹; ¹H NMR (CDCl₃) δ 0.84 (d, 3H, *J* = 5.9 Hz), 0.86 (d, 3H, *J* = 6.2 Hz), 1.32 (t, 3H, *J* = 7.0 Hz), 1.37-1.57 (m, 3H), 1.82-1.84 (m, 2H), 2.26-2.29 (m, 2H), 2.97-2.99 (m, 2H), 3.99-4.05 (m, 1H), 4.26 (q, 2H, *J* = 7.0 Hz), 4.46-4.49 (m, 1H), 4.95 (s, 2H), 5.06 (d, 1H, *J* = 6.5 Hz), 5.16-5.21 (m, 1H), 5.54 (dd, 1H, *J* = 19.9,9.7 Hz), 6.55 (d, 1H, *J* = 7.5 Hz), 6.79 (d, 1H, *J* = 7.5 Hz), 6.99 (s, 1H), 7.07-7.42 (m, 25H); Anal. (C₅₁H₅₅FN₄O₇) C, H, N.

### Preparation of Product Ethyl-2-Fluoro-3-(CBZ-L-Leu-L-Phe-L-Gln)-E-Propenoate

Using the procedure described in Example 4 for the preparation of compound 3, ethyl-2-fluoro-3-[CBZ-L-Leu-L-Phe-L-(Tr-*Gln*)]-E-propenoate was deprotected to produce the product. mp = 210-211 °C, R_{*f*} = 0.57 (10% MeOH in CH₂Cl₂); IR (KBr) 3401, 3300, 3072, 2943, 1693, 1648 cm⁻¹; ¹H NMR (DMSO-*d*₆) δ 0.79 (d, 3H, *J* = 10.9 Hz), 0.82 (d, 3H, *J* = 10.9 Hz), 1.27 (t, 3H, *J* = 7.2 Hz), 1.32-1.49 (m, 3H), 1.65-1.80 (m, 2H), 1.99-2.06 (m, 2H), 2.78-2.96 (m, 2H), 3.96-4.01 (m, 1H), 4.25 (q, 2H, *J* = 7.2 Hz), 4.39-4.41 (m, 1H), 4.97-5.07 (m, 3H), 5.65 (dd, 1H, *J* = 21.2, 10.0 Hz), 6.74 (s, 1H), 7.16-7.30 (m, 7H), 7.32-7.34 (m, 4H), 7.44 (d, 1H, *J* = 8.1 Hz), 7.94 (d, 1H, *J* = 8.1 Hz), 8.03 (d, 1H, *J* = 7.8 Hz). Anal. (C₃₂H₄₁FN₄O₇) C, H, N.

### Example 29 - Preparation of Compound 9: Methyl-[2-(CBZ-L-Leu-L-Phe-L-Gln)-E-Vinyl] Sulfone

### Preparation of Intermediate Methyl-(2-[BOC-L-(Tr-Gln)]-E-Vinyl) Sulfone

Sodium bis(trimethylsilyl)amide (1.04 mL of a 1.0 M solution in THF, 1.04 mmol) was added to a solution of methanesulfonylmethyl-phosphinic acid diethyl ether (0.217 g, 0.943 mmol) in THF (30 mL) at -78 °C, and the resulting solution was stirred for 15 minutes at that temperature. BOC-L-(Tr-Glutaminal) (0.446 g, 0.944 mmol) in THF (15 mL) was added via cannula, and the reaction mixture was stirred for 30 minutes at -78 °C then was partitioned between 0.5 M HCl (100 mL) and a 1:1 mixture of EtOAc and hexanes (2 x 100 mL). The organic layers were dried over Na₂SO₄ and were concentrated. Purification of the residue by flash column chromatography (40% hexanes in EtOAc) provided methyl-(2-[BOC-L-(Tr-*Gln*)]-E-vinyl) sulfone (0.359 g, 69%) as a white foam: IR (thin film) 3348, 1688, 1495 cm⁻¹; ¹H NMR (CDCl₃) δ 1.43 (s, 9H), 1.64-1.81 (m, 1H), 1.83-2.01 (m, 1H), 2.40 (t, 2H, *J* = 6.7 Hz), 2.91 (s, 3H), 4.35 (s, br, 1H), 5.01-5.04 (m, 1H), 6.42 (dd, 1H, *J* = 15.0, 1.7 Hz), 6.78 (s, 1H), 6.78 (dd, 1H, *J* = 15.0, 5.0 Hz), 7.18-7.33 (m, 15H); Anal. (C₃₁H₃₆N₂O₅S) C, H, N.

### Preparation of Intermediate Methyl-(2-[CBZ-L-Leu-L-Phe-L-(Tr-Gln)]-E-Vinyl) Sulfone.

Using the procedure described in Example 28 for the preparation of ethyl-2-fluoro-3-[CBZ-L-Leu-L-Phe-L-(Tr-*Gln*)]-E-propenoate, methyl-(2-[BOC-L-(Tr-*Gln*)]-E-vinyl) sulfone (0.359 g, 0.654 mmol) was deprotected and coupled with CBZ-L-Leu-L-Phe (0.270 g, 0.655 mmol) to provide methyl-(2-[CBZ-L-Leu-L-Phe-L-(Tr-*Gln*)]-E-vinyl) sulfone (0.160 g, 29%) as a white foam: IR (thin film) 3296, 3061, 1649, 1529 cm⁻¹; ¹H NMR (CDCl₃) δ 0.84 (d, 3H, *J* = 8.9 Hz), 0.86 (d, 3H, *J* = 8.9 Hz), 1.24-1.36 (m, 2H), 1.42-1.55 (m, 2H), 1.72-1.75 (m, 1H), 1.96-1.99 (m, 1H), 2.23-2.32 (m, 2H), 2.85 (s, 3H), 2.97 (dd, 1H, *J* = 13.8, 7.5 Hz), 3.13 (dd, 1H, *J* = 13.8, 6.1 Hz), 3.92-3.99 (m, 1H), 4.43-4.56 (m, 2H), 4.88 (s, br, 2H), 4.95 (d, 1H, *J* = 5.9 Hz), 6.20 (d, 1H, *J* = 14.9 Hz), 6.47 (d, 1H, *J* = 7.2 Hz), 6.70 (dd, 1H, *J* = 14.9,4.4 Hz), 6.98 (d, 1H, *J* = 8.1 Hz), 7.09-7.38 (m, 25H).

### Preparation of Product - Methyl-[2-(CBZ-L-Leu-L-Phe-L-Gln)-E-Vinyl] Sulfone

Using the procedure described in Example 4 for the preparation of compound 3, methyl-(2-[CBZ-L-Leu-L-Phe-L-(Tr-*Gln*)]-E-vinyl) sulfone was deprotected to produce the product. mp = 220 °C (dec), R_{*f*} = 0.44 (10% MeOH in CH₂Cl₂); IR (KBr) 3413, 3284, 3049, 2951, 1690, 1649 cm⁻¹; ¹H NMR (DMSO-*d*₆) δ 0.79 (d, 3H, *J* = 10.6 Hz), 0.81 (d, 3H, *J* = 10.6 Hz), 1.27-1.38 (m, 2H), 1.40-1.50 (m, 1H), 1.63-1.80 (m, 2H), 2.08 (t, *2*H,J = 7.5 Hz), 2.82-2.89 (m, 1H), 2.96 (s, 3H), 2.98-3.04 (m, 1H), 3.94-3.99 (m, 1H), 4.45-4.53 (m, 2H), 4.98 (d, 1H, *J* = 12.5 Hz), 5.05 (d, 1H, *J* = 12.5 Hz), 6.38 (d, 1H, *J* = 14.9), 6.60 (dd, 1H, *J* = 15.4, 5.1 Hz), 6.78 (s, 1H), 7.17-7.31 (m, 7H), 7.34-7.36 (m, 4H), 7.43 (d, 1H, *J* = 8.1 Hz), 8.01 (d, 1H, *J* = 8.1 Hz), 8.13 (d, 1H, *J* = 8.1 Hz); Ana;l. (C₃₀H₄₀N₄O₇S) C, H, N.

### Example 30 - Preparation of Compound 10: Phenyl-[2-(CBZ-L-Leu-L-Phe-L-Gln)-E-Vinyl] Sulfone

### Preparation of Intermediate Phenyl-(2-[BOC-L-(Tr-Gln)]-E-Vinyl) Sulfone

Sodium bis(trimethylsilyl)amide (1.14 mL of a 1.0 M solution in THF, 1.14 mmol) was added to a solution of benzenesulfonylmethyl-phosphinic acid diethyl ether (0.304 g, 1.04 mmol) in THF (20 mL) at -78 °C, and the resulting solution was stirred for 15 minutes at that temperature. BOC-L-(Tr-Glutaminal) (0.491 g, 1.04 mmol) in THF (10 mL) was added via cannula, and the reaction mixture was stirred for 30 minutes at -78 °C then was partitioned between 0.5 M HCl (100 mL) and a 1:1 mixture ofEtOAc and hexanes (2 x 100 mL). The organic layers were dried over Na₂SO₄ and were concentrated. Purification of the residue by flash column chromatography (gradient elution, 30-40% EtOAc in hexanes) provided phenyl-(2-[BOC-L-(Tr-*Gln*)]-E-vinyl) sulfone (0.540 g, 85%) as a white foam: IR (thin film) 3347, 2250, 1688,1493 cm⁻¹; ¹H NMR (CDCl₃) δ 1.37 (s, 9H), 1.73-1.81 (m, 1H), 1.83-1.94 (m, 1H), 2.38 (t, 2H, *J* = 6.7 Hz), 4.33 (s, br, 1H), 4.88-4.90 (m, 1H), 6.37 (dd, 1H, *J* = 15.3, 1.6 Hz), 6.79-6.86 (m, 2H), 7.17-7.32 (m, 15H), 7.49-7.54 (m, 2H), 7.58-7.63 (m, 1H), 7.83-7.87 (m, 2H); Anal. (C₃₆H₃₈N₂O₅S) C, H, N.

### Preparation of Intermediate Phenyl-(2-[CBZ-L-Leu-L-Phe-L-(Tr-Gln)]-E-Vinyl) Sulfone

Using the procedure described in Example 28 for the preparation of ethyl-2-fluoro-3-[CBZ-L-Leu-L-Phe-L-(Tr-*Gln*)]-E-propenoate, phenyl-(2-[BOC-L-(Tr-*Gln*)]-E-vinyl) sulfone (0.205 g, 0.336 mmol) was deprotected and coupled with CBZ-L-Leu-L-Phe (0.138 g, 0.335 mmol) to provide phenyl-(2-[CBZ-L-Leu-L-Phe-L-(Tr-*Gln*)]-E-vinyl) sulfone (0.100 g, 33%) as a white foam: IR (thin film) 3298, 3061, 1652, 1518 cm⁻¹; ¹H NMR (CDCl₃) δ 0.81 (d, 3H, *J* = 6.9 Hz), 0.83 (d, 3H, *J* = 6.9 Hz), 1.24-1.69 (m, 5H), 1.91 (s, br, 1H), 2.16-2.31 (m, 2H), 2.91 (dd, 1H, *J* = 13.5, 7.5 Hz), 3.05 (dd, 1H, *J* = 13.5, 6.7 Hz), 3.91-3.98 (m, 1H), 4.38-4.45 (m, 1H), 4.54 (s, br, 1H), 4.87 (s, br, 1H), 5.06 (d, 1H, *J* = 6.2 Hz), 6.12 (d, 1H, *J* = 15.3 Hz), 6.57 (d, 1H, *J* = 7.2 Hz), 6.75 (dd, 1H, *J* = 15.3, 4.4 Hz), 6.85 (d, 1H, *J* = 8.4 Hz), 7.05 (d, 1H, *J* = 7.2 Hz), 7.10-7.37 (m, 24H), 7.40-7.62 (m, 3H), 7.79-7.82 (m, 2H); Anal. (C₅₄H₅₆N₄O₇S) C, H, N.

### Preparation of Product - Phenyl-[2-(CBZ-L-Leu-L-Phe-L-Gln)-E-Vinyl] Sulfone

Using the procedure described in Example 4 for the preparation of compound 3, phenyl-(2-[CBZ-L-Leu-L-Phe-L-(Tr-*Gln*)]-E-vinyl) sulfone was deprotected to produce the product. mp = 230 °C (dec), R_{*f*} = 0.40 (10% MeOH in CH₂Cl₂); IR (KBr) 3400,3288, 3062,2960,1685,1644 cm⁻¹; ¹H NMR (DMSO-d₆) δ 0.78 (d, 3H, *J* = 10.6 Hz), 0.81 (d, 3H, *J* = 10.6 Hz), 1.26-1.39 (m, 2H), 1.47-1.59 (m, 1H), 1.61-1.66 (m, 1H), 1.76-1.79 (m, 1H), 2.04 (t, 2H, *J* = 7.0 Hz), 2.77-2.96 (m, 2H), 3.95-4.00 (m, 1H), 4.43-4.45 (m, 2H), 4.96 (d, 1H, *J* = 12.6 Hz), 5.02 (d, 1H, *J* = 12.6 Hz), 6.33 (d, 1H, *J* = 14.9 Hz), 6.74-6.81 (m, 2H), 7.11-7.18 (m, 7H), 7.20-7.38 (m, 4H), 7.42 (d, 1H, *J* = 7.8 Hz), 7.65 (d, 2H, *J* = 7.8 Hz), 7.71 (d, 1H, *J* = 7.5 Hz), 7.82 (d, 2H, *J* = 6.9 Hz), 8.00 (d, 1H, *J* = 7.8 Hz), 8.09 (d, 1H, *J* = 8.1 Hz); Anal. (C₃₅H₄₂N₄O₇S) C, H, N.

### Example 31 - Preparation of Compound 11: Ethyl-2-Fluoro-3-[BOC-L-(Cyanomethyl)-Ala]-E-Propenoate

### Preparation of Intermediate BOC-L-Gln-OMe

To a solution of BOC-L-Gln (20 g, 81 mmol) in 50 mL ofEtOAC and MeOH at 0 °C was added diazomethane in 250 mL of Et₂O with stirring. The resulting yellow solution was stirred at 0 °C for 5 minutes and then warmed up to room temperature and stirred for 20 minutes. Argon gas was then bubbled through the yellow reaction mixture to remove excess diazomethane. The crude product was concentrated and purified by crystallization from methyl-*tert*-butyl ether. Yield 100%. ¹H NMR (CDCl₃) δ 1.45 (s, 9H), 1.96 (m, 1H), 2.21 (m, 1H), 2.36 (m, 2H), 3.76 (s, 3H,), 4.34 (m, 1H], 5.32 (m, 1H), 5.44 (bs, 1H), 6.16 (bs, 1H). Anal. (C₁₁H₂₀N₂O₅) C, H, N.

### Preparation of Intermediate BOC-L-(Cyanomethyl)-Ala-OMe

To a solution of BOC-L-Gln-OMe (10 g, 38 mmol) in 100 mL of pyridine at 0 °C was added 3.5 mL of POCl₃ dropwise. The reaction was warmed to room temperature and stirred overnight. The reaction mixture was diluted with 100 mL EtOAc and washed with 1N HCl (2 x 50 mL). The organics were combined and dried over Na₂SO₄, concentrated to yield the crude product which was purified by flash column chromatography (1:4 EtOAc/hexane) to give the product in 67% yield. ¹H NMR (CDCl₃) δ 1.45 (s, 9H), 2.03 (m, 1H), 2.27 (m, 1H), 2.46 (m, 2H), 3.80 (s, 3H), 4.38 (m, 1H), 5.20 (m, 1H).

### Preparation of Intermediate BOC-L-(Cyanomethyl)-Alaninol

This compound was prepared in 84% yield from BOC-L-(cyanomethyl)-Ala-OMe using the procedure described in Example 2 for the preparation of CBZ-L-(N-Ac-amino)-alaninol. The compound was purified by flash column chromatography (50:50 EtOAc/hexane). ¹H NMR (CDCl₃) δ 1.45 (s, 9H), 1.92 (m, 2H), 2.19 (m, 1H), 2.46 (m, 2H), 3.71 (m, 3H), 4.83 (m, 1H). Anal. (C₁₀H₁₈N₂O₃·0.4 H₂O) C, H, N.

### Preparation of Intermediate BOC-L-(Cyanomethyl)-Alaninal

To a solution of oxalyl chloride (1.63 g, 12.57 mmol) in CH₂Cl₂ (30 mL) at -78 °C was added DMSO dropwise (2.01 g, 25.74 mmol). After the addition, the reaction was stirred for 5 minutes. A solution of BOC-L-(cyanomethyl)-alaninol (2.5 g, 11.7 mmol) in 20 mL was added at -78 °C with stirring. After 20 minutes, the reaction was treated with NEt₃ (8.15 mL, 58.5 mmol) and stirred for another 20 minutes. Water (40 mL) was added at -60 °C, and then the reaction was warmed up to room temperature. The water layer was separated and extracted with EtOAc (2 x 50 mL). The organic layers were combined and dried over MgSO₄, and then concentrated to give 2.1 g crude product which was purified by flash column chromatography using a gradient of 3:7 EtOAc/hexane to 5:5 EtOAc/hexane to give the aldehyde in 60% yield. ¹H NMR (CDCl₃) δ 1.37 (m, 3H), 1.42 (s, 9H), 1.46 (s, 9H), 1.91 (m, 1H), 2.55-2.30 (m, 3H), 4.25 (m, 1H), 5.27 (m, 1H), 9.63 (s,1H).

### Preparation of Intermediate Ethyl-2-Fluoro-3-[BOC-L-(Cyanomethyl)-Ala]-E-Propenoate

A solution of triethyl 2-fluoro-phosphonoacetate (0.31 g, 1.27 mmol) in 4 mL THF was cooled at -78 °C and then n-BuLi (0.56 mL of 2.5 M solution in hexanes, 1.39 mmol) was added. The resulting solution was stirred at -78 °C for 20 minutes, and then a solution of BOC-L-(cyanomethyl)-alaninal (0.124 g, 0.58 mmol) in 2 mL THF was added to the reaction mixture. The reaction was allowed to stir at -78 °C for 1 hour and then warmed up to room temperature and stirred overnight. Aqueous 6 N HCl (10 mL) was added to the reaction, and the organic layer was separated and washed with brine (2 x 10 mL) and concentrated. The crude product was purified by flash column chromatography (30:70 EtOAc/hexane) to give 0.07 g. product (55% yield). ¹H NMR (CDCl₃) δ 2.2-1.8 (m, 2H), 2.45 (m, 2H), 4.33 (m, 2H), 4.77 (m, 1H), 5.01 (m, 1H,), 5.89 (m, 1H). Anal. (C₁₄H₂₁N₂O₄F·0.15 H₂O) C,H,N. MS calcd for C₁₄H₂₁N₂O₄F (M+Na), found 323.

### Production of Product - Ethyl-2-Fluoro-3-[CBZ-L-Leu-L-Phe-L-(Cyanomethyl)-Ala]-E-Propenoate

A solution of ethyl-2-fluoro-3-[BOC-L-(cyanomethyl)-*Ala*]-E-propenoate (0.055 g, 0.18 mmol) in 1 mL CH₂Cl₂ was cooled to 0 °C, and 0.3 mL of TFA was added. The reaction was then warmed to room temperature, stirred for 3 hours, concentrated, and trace amounts of water were removed by toluene azeotrope. This crude product was dissolved in 2 mL DMF and a solution of benzotriazol-1-yloxytris(dimethylamino)phosphonium hexafluorophosphate (BOP) (0.12 g, 027 mmol), CBZ-L-Leu-L-Phe (0.11 g, 0.27 mmol), and Et₃N (0.075 mL, 0.54 mmol) was added at 0 °C, and the reaction was stirred for 4 hours. This reaction was diluted with saturated aqueous NaHCO₃ solution and extracted with EtOAc (3 x 15 mL). The organics layers were combined and dried with MgSO₄ and concentrated. The residue was purified by flash column chromatography using a solvent gradient of 1% MeOH/CH₂Cl₂ to 5% MeOH/CH₂Cl₂ yielding the product in 37% (2-steps). Anal (C₃₂H₃₉N₄O₆F) C, H, N. HRMS calcd for C₃₂H₃₉N₄O₆F+Na 617.2751 (M + Na), found 617.2738.

### Example 32 - Preparation of Compound 20: Diethyl-[2-(CBZ-L-Leu-L-Phe-L-Gln)-E-Vinyl] Phosphonate

### Preparation of Intermediate CBZ-L-(Tr-Gln)

CBZ-L-Gln (28.03 g, 100 mmol) was dissolved in 300 mL of glacial acetic acid. To this solution was added triphenylmethanol (26.83 g, 100 mmol), acetic anhydride (18.87 mL, 200 mmol), and 0.5 mL of sulfuric acid. The reaction was heated to 55 °C, stirring for one hour. After cooling to room temperature the mixture was concentrated under reduced pressure to one-third the original volume. Ice water was added, and the product extracted with EtOAc. The organic layer was washed with water and brine, dried over MgSO₄, and concentrated. The crude product was recrystallized from CH₂Cl₂/hexane, and the resulting crystals washed with Et₂O, yielding 37.27 g (71%) as a white solid: IR (KBr) 3418, 3295, 3059, 3032, 2949, 2515, 1699, 1628, 1539, 1504, 1447, 1418, 1341, 1242,1209, 1061,748, 696 cm⁻¹; ¹H NMR (DMSO-*d*₆) δ 1.71 (m, 1H), 1.88 (m, 1H), 2.38 (m, 2H), 3.97 (m, 1H), 5.04 (s, 2H), 7.14-7.35 (m, 20H), 7.52 (d, 1 *H, J =* 7.7 Hz), 8.60 (s, 1H).

### Preparation of Intermediate CBZ-L-(Tr-Gln)OMe

CBZ-L-(Tr-Gln) (0.26 g, 0.5 mmol) was added to a stirring solution of 0.25 mL of acetyl chloride in 5 mL of MeOH, and stirring was continued at room temperature for 1 hour. The solvent was removed in vacuo, and the residue dissolved in 100 ml CH₂Cl₂. The organic layer was washed with water, saturated NaHCO₃, and brine followed by drying over Na₂SO₄. The crude product was purified on a short flash silica gel column, eluting with 20% EtOAc/hexane. The product (0.23 g, 84%) was obtained as a white solid: IR (KBr) 3405, 3277, 3057, 3034, 2953, 1724, 1643, 1532, 1493, 1447, 1207, 1042, 750, 698 cm⁻¹; ¹H NMR (DMSO-*d*₆) δ 1.16 (t, 1H, *J* = 7.0 Hz), 1.77 (m, 1H), 1.97 (m, 1H), 3.61 (s, 3H), 4.99 (m, 1H), 5.03 (s, 2H), 7.02-7.55 (m, 20H), 7.69 (d, 1H, *J* = 7.7 Hz), 8.59 (s, 1H). Anal. (C₃₃H₃₂N₂O₅) C, H, N.

### Preparation of Intermediate CBZ-L-(Tr-Glutaminol)

CBZ-L-(Tr-Gln)OMe (1.50 g, 2.79 mmol) was dissolved in 20 mL of THF and 10 mL of EtOH. LiCl (0.24 g, 5.6 mmol) was added, and the mixture stirred for 10 minutes until all solids had dissolved. NaBH₄ (0.21 g, 5.6 mmol) was added, and the reaction stirred overnight at room temperature. The solvents were removed in vacuo, the residue taken up in water, and the pH was adjusted to 2-3 with 10% HCl. The product was extracted with EtOAc, and the organic layer was washed with water and brine before drying over MgSO₄. The crude product was purified on a short flash silica gel column, eluting with an increasing gradient of EtOAc/benzene, yielding 1.02 g (72%) of a white glassy solid: IR (KBr) 3408, 3318, 3057, 3032, 2947, 1699, 1674, 1516, 1447, 1240, 1059, 752, 698 cm⁻¹; ¹H NMR (DMSO-*d*₆) δ 1.40 (m, 1H), 1.72 (m, 1H), 2.26 (m, 2H), 3.17-3.50 (m, 3H), 4.64 (t, 1H, *J* = 5.0 Hz), 5.00 (s, 2H), 7.00-7.40 (m, 20H), 6.96 (d, 1H, *J* = 8.5 Hz), 8.54 (s, 1H). Anal. (C₃₂H₃₂N₂O₄) C, H, N.

### Preparation of Intermediate L-(Tr-Glutaminol)

This amino alcohol was prepared from CBZ-L-(Tr-glutaminol) in 98% yield using the procedure described in Example 2 for the preparation of L-(N-Ac-amino)-alaninol. IR (KBr) 3255, 3057, 3016, 2916, 1642, 1527,1491, 1446, 1057, 1036, 750, 700, 636 cm⁻¹; ¹H NMR (DMSO-*d*₆) δ 1.29 (m, 1H), 1.53 (m, 1H), 2.29 (m, 2H), 3.08 (m, 1H), 3.18 (m, 2H), 3.38 (bs, 2H), 4.43 (bs, 1H), 7.14-7.28 (m, 15H), 8.62 (s, 1H). Anal. (C₂₄H₂₆N₂O₂) C, H, N.

### Preparation of Intermediate CBZ-L-Leu-L-Phe-L-(Tr-Glutaminol)

Using the procedure described in Example 1 for the preparation of CBZ-L-Leu-L-Phe-L-methioninol, this derivative was synthesized from CBZ-L-Leu-L-Phe and L-Tr-glutaminol in 62% yield as a white solid: IR (KBr) 3302, 3057, 3032, 2951, 1954, 1885, 1657, 1520, 1238, 1045, 746, 698 cm⁻¹; ¹H NMR (DMSO-*d*₆) δ 0.79 (t, 6H, *J* = 7.0 Hz), 1.30 (m, 2H), 1.44 (m, 2H), 1.75 (m, 1H), 2.22 (m, 2H), 2.82 (m, 1H), 2.97 (m, 1H), 3.14 (m, 1H), 3.25 (m, 1H), 3.63 (m, 1H), 3.95 (m, 1H), 4.48 (m, 1H), 4.65 (t, 1H, J = 5.0 Hz), 4.96 (d, 1H, *J* = 13.0 Hz), 5.02 (d, 1H, *J* = 13.0 Hz), 7.07-7.33 (m, 25H), 7.42 (d, 1H, *J* = 8.0 Hz), 7.66 (d, 1H, *J =* 8.5 Hz), 7.86 (d, 1H, *J* = 8.0 Hz), 8.52 (s, 1H). Anal. (C₄₇H₅₂N₄O₆·0.5 H₂O) C, H, N.

### Preparation of Intermediate CBZ-L-Leu-L-Phe-L-(Tr-Glutaminal)

Using the procedure described in Example 1 for the preparation of CBZ-L-Leu-L-Phe-L-methioninal, this aldehyde was synthesized from CBZ-L-Leu-L-Phe-L-(Tr-glutaminol) in 92% yield as a white glassy solid, which was used immediately. ¹H NMR (DMSO-*d*₆) δ 0.79 (t, 6H, *J* = 7.0 Hz), 1.00-1.98 (m, 5H), 2.27 (m, 2H), 2.84 (m, 1H), 3.02 (m, 1H), 3.98 (m, 2H), 4.58 (m, 1H), 4.99 (s, 2H), 7.14-7.32 (m, 25H), 7.39 (d, 1H, *J* = 8.0 Hz), 7.97 (d, 1H, *J* = 8.5 Hz), 8.38 (d, 1H, *J* = 8.0 Hz), 8.60 (s, 1H), 9.20 (s, 1H).

### Preparation of Intermediate Diethyl-(2-[CBZ-L-Leu-L-Phe-L-(Tr-Gln)]-E-Vinyl) Phosphonate

Tetraethyl methylenediphosphonate (0.21 mL, 0.86 mmol) was dissolved in 10 mL of THF and cooled to 0 °C. Potassium bis(trimethylsilyl)amide (0.5 M in toluene) was added dropwise via syringe, and the reaction stirred at 0 °C for 30 minutes. After cooling the reaction to -30 °C a solution of CBZ-L-Leu-L-Phe-L-(Tr-glutaminol) (0.63 g, 0.82 mmol) in 6 mL of THF was added dropwise. The reaction was allowed to warm slowly to room temperature and stirred overnight. The solvent was removed by evaporation, and the crude product was purified by flash column chromatography eluting with 1% (saturated anhydrous NH₃/MeOH)/ CHCl₃ to afford 0.50 g (68%) of a white crystalline solid: IR (KBr) 3289, 3059, 3032, 2957, 1667, 1532, 1447, 1246, 1026, 968, 748, 698 cm⁻¹; ¹H NMR (DMSO-*d*₆) δ 0.78 (t, 6H, *J* = 7.0 Hz), 1.20 (m, 6H), 1.15-1.78 (m, 5H), 2.25 (m, 2H), 2.85 (m, 1H), 2.97 (m, 1H), 3.86-4.07 (m, 5H), 4.32 (m, 1H), 4.51 (m, 1H), 4.95 (d, 1H, *J* = 13.0 Hz), 5.02 (d, 1H, *J* = 13.0 Hz), 5.52 (t, 1H, *J* = 19.0 Hz), 6.48 (t, 1H, *J* = 19.0 Hz), 7.07-7.32 (m, 25H), 7.41 (d, 1H, *J* = 8.0 Hz), 7.97 (d, 1H, *J* = 8.5 Hz), 8.05 (d, 1H, *J* = 8.0 Hz), 8.59 (s, 1H); MS (M+H) 901, (M-H) 899. Anal. (C₅₂H₆₁N₄O₂P·2.5 H₂O) C, H, N.

### Preparation of Product Diethyl-[2-(CBZ-L-Leu-L-Phe-L-Gln)-E-Vinyl] Phosphonate

The protected amide diethyl-[2-(CBZ-L-Leu-L-Phe-L-Tr-*Gln*)-E-vinyl] phosphonate (0.469 g, 0.52 mmol) was dissolved in 10 mL of CH₂Cl₂. Triisopropylsilane (0.52 mL) was added as a triphenylmethyl cation scavenger. TFA (1.0 mL) was added, and the reaction was stirred overnight at room temperature. The reaction was poured into EtOAc and washed with saturated NaHCO₃ solution. The organic layer was separated and washed with water and brine followed by drying over MgSO₄. The product was purified by flash column chromatography eluting with 2-3% MeOH/CHCl₃ to give in 67% yield of a white solid: IR (KBr) 3291, 3063, 2955, 1647, 1541, 1236, 1026, 968, 746, 698 cm⁻¹; ¹H NMR (DMSO-*d*₆) δ 0.79 (m, 6H), 1.21 (t, *6H,J =* 7.0 Hz), 1.28 (m, 2H), 1.52 (m, 1H), 1.63 (m, 1H), 1.75 (m, 1H), 2.06 (m, 2H), 2.85 (m, 1H), 3.00 (m, 1H), 3.92 (m, 5H), 4.34 (m, 1H), 4.50 (m, 1H), 4.97 (d, 1H, *J* = 13.0 Hz), 5.04 (d, 1H, *J* = 13.0 Hz), 5.54 (t, 1H, *J* = 19.0 Hz), 6.49 (t, 1H, *J* = 19.0 Hz), 6.77 (bs, 1H), 7.15-7.34 (m, 11H), 7.44 (d, 1H, *J* = 8.0 Hz), 8.00 (d, 1H, *J =* 8.5 Hz), 8.03 (d, 1H, *J* = 8.0 Hz); HRMS calcd for C₃₃H₄₅N₄O₈P 659.3210 (M+H), found 659.3223. Anal. (C₃₃H₄₈N₄O₈P) C, H, N.

### Example 33 - Preparation of Compound 29: Ethyl-3-[N-(1-Tr-4-Methoxyindole-2-Carbonyl)-L-(4-Cl-Phe)-L-Gln]-E-Propenoate

### Preparation of Product - Ethyl-3-[N-(1-Tr-4-Methoxyindole-2-Carbonyl)-L-(4-Cl-Phe)-L-Gln]-E-Propenoate

This compound was prepared by the deprotection of ethyl-3-[N-(4-methoxyindole-2-carbonyl)-L-(4-Cl-Phe)-L-(Tr-*Gln*)]-E-propenoate, using the procedure described in Example 32 for the preparation of compound 20, but in the absence of triisopropylsilane. ¹H NMR (DMSO-*d*₆) δ 1.20 (t, 3H, *J* = 7.0 Hz), 1.74 (m, 2H), 2.03 (t, 2H, *J* = 8.0 Hz), 2.94 (m, 2H), 3.89 (s, 3H), 4.11 (q, 2H, *J* = 7.0 Hz), 4.46 (m, 1H), 4.60 (m, 1H), 5.70 (d, 1H, *J* = 15.0 Hz), 6.54 (d, 1H, *J* = 7.8 Hz), 6.70 (dd, 1H, *J* = 15.0, 5.7 Hz), 6.75 (bs, 1H), 6.87 (d, 1H, *J* = 8.5 Hz), 7.06 (m, 5H), 7.31 (m, 18H), 7.72 (bs, 1H), 8.26 (d, 1H, *J* = 8.2 Hz), 8.61 (d, 1H, *J* = 8.1 Hz); HRMS calcd for C₄₇H₄₅N₄O₆Cl+Cs 929.2082 (M+Cs), found 929.2078 Anal. (C₄₇H₄₅N₄O₆Cl·1.0 H₂O) C, H, N.

### Example 34- Preparation of Compound 167: Ethyl-3-[Ethylthiocarbonyl-L-α-(t-Butyl-Gly)-L-Phe-L-Gln]-E-Propenoate.

### Preparation of Intermediate CBZ-L-Phe-L-(Tr-Glutaminol).

Using the procedure described in Example 16 for the preparation of BOC-L-(4-Cl-Phe)-(Tr-glutaminol), CBZ-L-Phe-L-(Tr-glutaminol) was synthesized from CBZ-L-Phe and L-(Tr-glutaminol) in 67% yield as a white glassy solid: IR (KBr) 3304, 3059, 3030, 2936, 1956, 1887, 1809, 1659, 1495, 1446, 1246, 1036, 750, 698 cm⁻¹; ¹H NMR (DMSO-*d*₆) δ 1.47 (m, 1H), 1.72 (m, 1H), 2.26 (m, 2H), 2.75 (m, 1H), 2.94 (m, 1H), 3.18 (m, 1H), 3.26 (m, 1H), 3.66 (m, 1H), 4.21 (m, 1H), 4.66 (m, 1H), 4.90 (m, 2H), 7.15-7.30 (m, 25H), 7.43 (d, 1H, *J =* 8.5 Hz), 7.72 (d, 1H, *J =* 9.0 Hz), 8.49 (s, 1H). Anal. (C₄₁H₄₁N₃O₅•1.0 H₂O) C, H, N.

### Preparation of Intermediate L-Phe-L-(Tr-Glutaminol).

Using the procedure described in Example 2 for the preparation of L-(N-Ac-amino)-alaninol, L-Phe-L-(Tr-Glutaminol) was synthesized from CBZ-L-Phe-L-(Tr-glutaminol) in quantitative yield as a white glassy solid: IR (KBr) 3293, 3061, 3026, 2938, 2361, 1669,1495, 1446, 752, 700 cm⁻¹; ¹H NMR (DMSO-*d*₆) δ 1.46 (m, 1H), 1.78 (m, 1H), 2.28 (m, 2H), 3.10 (m, 2H), 3.21 (m, 1H), 3.25 (m, 1H), 3.62 (m, 1H), 3.86 (t, 1H, *J =* 6.0 Hz), 4.72 (m, 1H), 7.10-7.32 (m, 20H), 8.14 (d, 1H, *J* = 8.0 Hz), 8.53 (s, 1H). MS calcd for C₃₃H₃₅N₃O₃+H 522, found 522. Anal. (C₃₃H₃₅N₃O₃•0.55 CH₂Cl₂) C, H, N.

### Preparation of Intermediate BOC-L-α-(t-Butyl-Gly)-L-Phe-L-(Tr-Glutaminol).

L-Phe-L-(Tr-Glutaminol) (0.65 g, 1.25 mmol) was dissolved in 5 mL of DMF. Diisopropylethylamine (0.44 mL, 2.5 mmol) was added, followed by 0.29 g (1.25 mmol) of BOC-L-α-t-butylglycine. The reaction was cooled to 0°C and HATU [O-(7-azabenztriazol-1-yl)-1,1,3,3-tetramethyluronium hexafluorophosphate] (0.48 g, 1.25 mmol) was added. The reaction mixture was allowed to warm to rt at which time the DMF was removed in vacuo. The residue was dissolved with EtOAc, and the organic phase washed consecutively with 10% aq HCl solution, sat. NaHCO₃ solution, H₂O, and brine. The solvent was dried (MgSO₄) and filtered, and the residue purified by flash silica gel chromatography using a gradient solvent system (0-1.5% MeOH/CHCl₃) to give 0.78 g (85%) of a white amorphous solid; IR (KBr) 3314, 2967, 1657, 1495, 1368, 1246, 1169, 1057, 752, 700 cm⁻¹; ¹H NMR (DMSO-*d*₆) δ 0.78 (s, 9H), 1.37 (s, 10H), 1.72 (m, 1H), 2.23 (m, 2H), 2.80 (m, 1H), 2.92 (m, 1H), 3.08 (m, 1H), 3.21 (m, 1H), 3.60 (m, 1H), 3.83 (d, 1H, *J* = 9.0 Hz), 4.55 (m, 1H), 4.59 (t, 1H, *J* = 5.5 Hz), 6.42 (d, 1H, *J =* 9.0 Hz), 7.14-7.28 (m, 20H), 7.67 (d, 1H, *J =* 8.0 Hz), 7.95 (d, 1H, *J =* 8.0 Hz), 8.45 (s, 1H); Anal. (C₄₄H₅₄N₄O₆•1.0 H₂O) C, H, N.

### Preparation of Intermediate L-α-(t-Butyl-Gly)-L-Phe-L-(Tr-Glutaminol) Hydrochloride Salt.

BOC-L-α-(t-butyl-Gly)-L-Phe-L-(Tr-glutaminol) (0.745 g, 1.01 mmol) was dissolved in 2 mL of CH₂Cl₂ followed by 20 mL of Et₂O. Dry HCl gas was carefully bubbled into the solution until the white solid stopped precipitating. The reaction mixture was concentrated, and 2-3 mL of THF was added which redissolved the white solids. Thin layer chromatography indicated that the reaction went to completion. The THF was removed under vacuum and white solids were washed thoroughly with an excess of Et₂O and dried to yield L-α-(t-butyl-Gly)-L-Phe-L-(Tr-glutaminol) hydrochloride salt in 95% yield. IR(KBr) 3258, 3057, 2967, 1661, 1520, 700 cm⁻¹; ¹H NMR (DMSO-*d*_{*6*}) δ 0.95 (s, 9H), 1.44 (m, 1H), 1.72 (m, 1H), 2.13 (m, 1H), 2.25 (m, 1H), 2.97 (m, 2H), 3.06 (m, 1H), 3.15 (m, 1H), 3.60 (m, 2H), 4.25 (bs, 1H), 4.55 (m, 1H), 7.13-7.27 (m, 20H), 7.89 (d, 1H, *J* = 8.0 Hz), 8.13 (bs, 2H), 8.49 (s, 1H), 8.61 (d, 1H, *J =* 7.7 Hz); Anal. (C₃₉H₄₆N₄O₄•HCl·1.0 H₂O) C, H, N.

### Preparation of Intermediate Ethylthiocarbonyl-L-α-(t-Butyl-Gly)-L-Phe-L-(Tr-Glutaminol).

L-α-(t-Butyl-Gly)-L-Phe-L-(Tr-glutaminol) hydrochloride salt (0.61 g, 0.91 mmol) was dissolved in 9 mL of CH₂Cl₂. Triethylamine (0.26 mL, 1.87 mmol) was added, followed by the addition of 0.097 g (0.91mL) of ethyl chlorothiolformate. After stirring for five minutes at rt, the solvent was removed under reduced pressure, and the residue was purified by column chromatography on silica gel eluting with a gradient solvent system (0-2% MeOH/CHCl₃) to give 0.47 g (71%) of a white amorphous solid: IR(KBr) 3300, 3059, 3026, 2967, 1649, 1493, 1194, 750, 698 cm⁻¹; ¹H NMR (DMSO-*d*₆) 0.83 δ (s, 9H), 1.16 (t, 3H, *J =* 7.0 Hz), 1.42 (m, 1H), 1.69 (m, 1H), 2.23 (m, 2H), 2.75 (q, 2H, *J* = 7.0 Hz), 2.80 (m, 1H), 2.96 (m, 1H), 3.08 (m, 1H), 3.18 (m, 1H), 3.62 (m, 1H), 4.25 (d, 1H, *J* = 9.0 Hz), 4.48 (m, 1H), 5.75 (t, 1H, *J =* 5.0 Hz), 7.10-7.28 (m, 20H), 7.60 (d, 1H, *J =* 8.5 Hz), 7.93 (d, 1H, *J* = 9.0 Hz), 8.09 (d, 1H, *J =* 7.7 Hz), 8.48 (s, 1H); Anal. (C₄₂H₅₀N₄O₅S) C, H, N.

### Preparation of Intermediate Ethylthiocarbonyl-L-α-(t-Butyl-Gly)-L-Phe-L-(Tr-Glutaminal).

Using the general procedure described in Example 1 for the preparation of CBZ-L L-Leu-L-Phe-L-methioninal (sulfoxide), ethylthiocarbonyl-L-α-(t-butyl-Gly)- L-Phe-L-(Tr-glutaminal) was synthesized from ethylthiocarbonyl-L-α-(t-butyl-Gly)-L-Phe-L-(Tr-glutaminol) in quantitative yield and isolated as a white amorphous solid and used without further purification: ¹H NMR (DMSO-*d*₆) δ 0.83 (s, 9H), 1.16 (t, 3H, *J =* 7.0 Hz), 1.55 (m, 1H), 1.86 (m, 1H), 2.26 (m, 2H), 2.74 (q, 2H, *J =* 7.0 Hz), 2.85 (m, 1H), 2.98 (m, 1H), 3.90 (m, 1H), 4.25 (d, 1H, *J =* 9.0 Hz), 4.59 (m, 1H), 7.14-7.28 (m, 20H), 7.93 (d, 1H, *J =* 9.0 Hz), 8.18 (d, 1H, *J =* 7.7 Hz), 8.38 (d, 1H, *J =* 6.6 Hz), 8.52 (s, 1H), 9.13 (s, 1H).

### Preparation of Intermediate Ethyl-3-[Ethylthiocarbonyl-L-α-(t-Butyl-Gly)-L-Phe-L-(Tr-Gln)]-E-Propenoate.

Using the procedure described in Example 1 for the preparation of ethyl-3-[CBZ-L-Leu-L-Phe-L-*Met* (sulfoxide)]-E-propenoate, ethyl-3-[ethylthiocarbonyl-L-α-(t-butyl-Gly)-L-Phe-L-(Tr-*Gln*)]-E-propenoate was synthesized from ethylthiocarbonyl-L-α-(t-butyt-Gly)-L-Phe-L-(Tr-glutaminal) (0.22 g, 0.30 mmol) to give 0.28 g of material contaminated with triphenylphosphine oxide which was used without further purification: white amorphous solid: ¹H NMR (DMSO-*d*₆) δ 0.83 (s, 9H), 1.21 (m, 6H), 1.60 (m, 2H), 2.25 (m, 2H), 2.74 (q, 2H, *J =* 7.0 Hz), 2.82 (m, 1H), 2. 92 (m, 1H), 4.09 (q, 2H, *J =* 7.0 Hz), 4.25 (d, 1H, *J* = 9.0 Hz), 4.34 (m, 1H), 4.52 (m, 1H), 5.53 (d, 1H, *J =* 15.5 Hz), 6.63 (dd, 1H, *J* = 15.5, 5.5 Hz), 7.08-7.28 (m, 20H), 7.93 (d, 1H, *J =* 9.0 Hz), 8.07 (d, 1H, *J =* 7.7 Hz), 8.16 (d, 1H, *J =* 7.7 Hz), 8.51 (s, 1H).

### Preparation of Product - Ethyl-3-[Ethylthiocarbonyl-L-α-(t-Butyl-Gly)-L-Phe-L-Gln]-E-Propenoate.

Ethyl-3-[ethylthiocarbonyl-L-α-(t-butyl-Gly)-L-Phe-L-(Tr-*Gln*)]-E-propenoate, impure with triphenylphosphine oxide (0.28 g), was dissolved in 6 mL of CH₂Cl₂. TFA (0.6 mL) was added, and the reaction stirred at rt for 4 hours. The reaction was poured into an EtOAc/ sat. NaHCO₃ solution and agitated until white solids began to precipitate out of the organic layer. The aqueous layer was separated, and the solids were filtered and washed with EtOAc to give 0.074 g of a white solid (45% yield from the ethylthiocarbonyl-L-α-(t-butyl-Gly)-L-Phe-L-(Tr-glutaminal); 2 steps): IR(KBr) 3302, 2967, 1645, 1541, 1196 cm⁻¹; ¹H NMR (DMSO-*d*₆) δ 0.83 (s, 9H), 1.18 (m, 6H), 1.67 (m, 2H), 2.03 (m, 2H), 2.75 (q, 2H, *J =* 7.0 Hz), 2.86 (m, 1H), 2.93 (m, 1H), 4.10 (q, 2H, *J* = 7.0 Hz), 4.25 (d, 1H, *J* = 9.0 Hz), 4.35 (m, 1H), 4.49 (m, 1H), 5.55 (d, 1H, *J* = 15.5 Hz), 6.64 (dd, 1H, *J* = 15.5, 5.5 Hz), 6.73 (bs), 7.19 (m, 6H), 7.97 (d, 1H, *J =* 8.5 Hz), 8.07 (d, 1H, *J* = 8.0 Hz), 8.15 (d, 1H, *J* = 7.7 Hz); HRMS calcd for C₂₇H₄₀N₄O₆S+Cs 681.1723, found 681.1738. Anal. (C₂₇H₄₀N₄O₆S) C, H, N.

### Example 35 - Preparation of Compound 168: Ethyl-2-Methyl-3-[Ethylthiocarbonyl-L-α-(t-Butyl-Gly)-L-Phe-L-Gln]-E-Propenoate.

### Preparation of Intermediate Ethyl-2-Methyl-3-[Ethylthiocarbonyl-L-α-(t-Butyl-Gly)--Phe-L-(Tr-Gln)]-E-Propenoate.

Using the procedure described in Example 1 for the preparation of ethyl-3-[CBZ-L-Leu-L-Phe-L-*Met* (sulfoxide)]-E-propenoate, ethyl-2-methyl-3-[ethylthiocarbonyl-L-α-(t-butyl-Gly)-L-Phe-L-(Tr-*Gln*)]-E-propenoate was synthesized from ethylthiocarbonyl-L-α-(t-butyl-Gly)-L-Phe-L-(Tr-glutaminal) (0.22 g, 0.30 mmol) and (carbethoxyethylidene)triphenylphosphorane (0.14 g, 0.37 mrnol). The product (0.31 g), a white amorphous solid, contaminated with triphenylphosphine oxide, was isolated after column chromatography and used without further purification: ¹H NMR (DMSO-*d*₆) δ 0.83 (s, 9H), 1.18 (m, 6H), 1.54 (m, 1H), 1.66 (m, 1H), 1.73 (s, 3H), 2.21 (m, 2H), 2.75 (q, *2H, J =* 7.0 Hz), 2.80 (m, 1H), 2.88 (m, 1H), 4.12 (q, *2H, J =* 7.0 Hz), 4.24 (d, 1H, *J* = 9.0 Hz), 4.44 (m, 2H), 6.27 (d, 1H, *J* = 8.3 Hz), 7.13-7.27 (m, 20H), 7.95 (d, 1H, *J* = 9.0 Hz), 8.03 (d, 1H, *J* = 8.0 Hz), 8.09 (d, 1H, *J* = 7.0 Hz), 8.51 (s, 1H).

### Preparation of Product - Ethyl-2-Methyl-3-[Ethylthiocarbonyl-L-α-(t-Butyl-Gly)-L-Phe-L-Gln]-E-Propenoate.

Using the procedure described in Example 34 for the preparation of ethyl-3-[ethylthiocarbonyl-L-α-(t-butyl-Gly)-L-Phe-L-*Gln*]-E-propenoate, ethyl-2-methyl-3-[ethylthiocarbonyl-L-α-(t-butyl-Gly)-L-Phe-L-*Gln*]-E-propenoate was synthesized from ethyl-2-methyl-3-[ethylthiocarbonyl-L-α-(t-butyl-Gly)-L-Phe-L-(Tr-*Gln*)]-E-propenoate and isolated as a white glassy solid after purification by column chromatography on silica gel using a gradient solvent system (0-2% MeOH/CHCl₃) (58% yield; two steps from ethylthiocarbonyl-L-α-(t-butyl-Gly)-L-Phe-L-(Tr-glutaminal): IR (KBr) 3302, 2967, 1647, 1541, 1261, 1202 cm⁻¹; ¹H NMR (DMSO-*d*₆) δ 0.83 (s, 9H), 1.18 (m, 6H), 1.65 (m, 1H), 1.69 (m, 1H), 1.77 (s, 3H), 2.00 (m, 2H), 2.75 (q, 2H, *J =* 7.0 Hz), 2.86 (m, 2H), 4.12 (q, 2H, *J =* 7.0 Hz), 4.24 (d, 1H, *J =* 9.0 Hz), 4.42 (m, 2H), 6.26 (d, 1H, *J =* 8.5 Hz), 6.71 (bs, 1H), 7.15 (m, 6H), 7.96 (d, 1H, *J =* 9.0 Hz), 8.03 (d, 1H, *J =* 7.7 Hz), 8.07 (d, 1H, J = 7.0 Hz); HRMS calcd for C₂₈H₄₂N₄O₆S+Cs 695.1879, found 695.1864. Anal. (C₂₈H₄₂N₄O₆S•0.2 CHCl₃) C, H, N.

### Example 36 - Preparation of Compound 178: Ethyl-3-[Cyclopentylthiocarbonyl-L-(S-Me-Pen)-L-Phe-L-Gln]-E-Propenoate.

### Preparation of Intermediate BOC-L-(S-Me-Pen)-L-Phe-L-(Tr-Glutaminol).

L-Phe-L-(Tr-Glutaminol) (0.64 g, 1.25 mmol) was dissolved in 4 mL of DMF. Diisopropylethylamine (0.43 mL, 2.46 mmol) was added, followed by BOC-S-methyl-L-penicillamine (0.32 g, 1.25 mmol; generated from the BOC-S-methyl-L-penicillamine dicyclohexylammonium salt (Sigma Chemical, St. Louis, MO) and aq HCl / EtOEt extraction and drying by benzene azeotrope). The solution was cooled to 0°C, HATU (O-7-azabenzotriazol-1-yl)-1,1,3,3-tetramethyluronium hexafluorophosphate] (0.468 g, 1.25 mmol) was added, and the reaction mixture was allowed to warm to rt. The DMF was then removed in vacuo, the residue was dissolved with EtOAc, and the organic phase was washed consecutively with 10% HCl solution, sat NaHCO₃, H₂O, and brine. The organic phase was dried over MgSO₄, filtered, and concentrated to give a residue which was purified by column chromatography on silica gel using a gradient solvent system (0-1% MeOH/CHCl₃) to yield 0.76 g (81%) of a white amorphous solid: IR (KBr) 3308, 2937, 1695,1677, 1506, 1493, 1448,1367,1246, 1165, 700 cm⁻¹; ¹H NMR (DMSO-*d*₆) δ 1.07 (s, 3H), 1.19 (s, 3H), 1.37 (s, 9H), 1.66-1.75 (m, 2H), 1.94 (s, 3H), 2.19-2.25 (m, 2H), 2.78-2.83 (m, 1H), 2.95-3.01 (m, 1H), 3.06-3.12 (m, 1H), 3.19-3.23 (m, 1H), 3.62-3.65 (m, 1H), 4.12 (d, 1H, *J =* 3.0 Hz), 4.48-4.55 (m, 1H), 4.59-4.62 (m, 1H), 6.50 (d, 1H, *J* = 9.0 Hz), 7.14-7.28 (m, 20H), 7.62 (d, 1H, *J* = 6.0Hz), 8.21 (d, 1H, *J =* 6.0 Hz), 8.47 (s, 1H). MS calcd for C₄₄H₅₄N₄O₆S+H 767, found 767.

### Preparation of Intermediate L-(S-Me-Pen)-L-Phe-L-(Tr-Glutaminol) Hydrochloride Salt.

To a solution of BOC-L-(S-Me-Pen)-L-Phe-L-(Tr-glutaminol) (0.69 g, 0.91 mmol) in 6 mL of 1,4-dioxane was added 4 mL of 4M HCl/1,4-dioxane. The reaction mixture was stirred at rt for 3 h under an argon atmosphere. At this time the solvent was removed in vacuo to give 0.61 g (97%) of a white solid which was used without further purification: IR (KBr) 3313, 3057, 2926, 1664, 1493, 1448, 750, 700 cm⁻¹; ¹H NMR (DMSO-*d*_{*6*}) δ 1.18 (s, 3H), 1.39 (s, 3H), 1.66-1.78 (m, 2H), 2.01 (s, 3H), 2.06-2.15 (m, 1H), 2.27-2.39 (m, 1H), 2.83-3.08 (m, 2H), 3.14-3.29 (m, 2H), 3.33-3.40 (m, 3H), 3.59-3.68 (m, 1H), 3.84-3.89 (m, 1H), 7.13-7.27 (m, 20H), 7.91 (d, 1H, *J =* 9.0 Hz), 8.15-8.26 (m, 2H), 8.52 (s, 1H), 8.76 (d, 1H, *J =* 6.0 Hz).

### Preparation of Intermediate Cyclopentylthiocarbonyl-L-(S-Me-Pen)-L-Phe-L-(Tr-Glutaminol).

A solution of cyclopentyl chlorothiolformate (0.133 g, 0.81 mmol), prepared as described in Example 37, in 2 mL of CH₂Cl₂ was added dropwise to a solution of L-(S-Me-Pen)-L-Phe-L-(Tr-glutaminol) hydrochloride salt (0.57 g, 0.81 mmol) in 10 mL of CH₂Cl₂. To this solution was added 0.24 mL(1.7 mmol) of Et₃N. The reaction mixture was stirred for 15 min at rt, and the solvent was removed under vacuum. The residue was purified by column chromatography on silica gel chromatography using a gradient solvent system (0-2% MeOH/CHCl₃) to give 0.512 g (80%) of a white amorphous solid: IR (KBr) 3358, 2939, 1649, 1516, 1448,1190, 700 cm⁻¹; ¹H NMR (DMSO-*d*_{*6*}) δ 1.13 (s, 3H), 1.23 (s, 3H), 1.37-1.63 (m, 10H), 1.96 (s, 3H), 1.98-2.01 (m, 1H), 2.16-2.33 (m, 1H), 2.7-2.89 (m, 1H), 3.07-3.23 (m, 2H), 3.24-3.28 (m, 1H), 3.53-3.57 (m, 1H), 3.59-3.66 (m, 1H), 4.37-4.47 (m, 1H), 4.54-4.60 (m, 2H), 7.14-7.28 (m, 20H), 7.55 (d, 1H, *J* = 9.0 Hz), 7.99 (d, 1H, *J =* 9.0 Hz), 8.36 (d, 1H, *J =* 6.0 Hz), 8.49 (s, 1H). MS calcd for C₄₅H₅₄N₄O₅S₂+H 795, found 795.

### Preparation of Intermediates Cyclopentylthiocarbonyl-L-(S-Me-Pen)-L-Phe-L-(Tr-Glutaminal) & Cyclopentylthiocarbonyl-L-[S(O)-Me-Pen]-L-Phe-L-(Tr-Glutaminal).

Cyclopentylthiocarbonyl-L-(S-Me-Pen)-L-Phe-L-(Tr-glutaminol) (0.46 g, 0.58 mmol) was dissolved in 10 mL of anh DMSO. o-Iodoxybenzoic acid (0.48 g, 1.73 mmol) was added, and the reaction mixture was stirred at rt for 3 h. The DMSO was removed under high vacuum. The residue was twice diluted with CH₂Cl₂ and the solvent was evaporated to remove any residual DMSO. The residue was diluted with EtOAc, and triturated to form a white solid which was filtered off. The filtrate was washed with an aq 10% Na₂S₂O₃/10% NaHCO₃ solution, water and brine and dried over MgSO₄. Filtration and concentration gave 0.40 g (87%) of a white glassy solid which was used without further purification. The product was shown to be a mixture of the sulfide and sulfoxide by NMR analysis. ¹H NMR (DMSO-*d*₆) (mixture of sulfide and sulfoxide) δ 1.12 (s), 1.24 (s), 1.32 (s), 1.45-1.66 (m), 1.95-2.13 (m), 2.29 (s), 2.40 (s), 2.53 (s), 2.82-2.87 (m), 2.99-3.23 (m), 3.52-3.57 (m), 3.95-4.03 (m), 4.55-4.83 (m), 7.14-7.28 (m), 7.89-8.06 (m), 8.41-8.58 (m), 9.15 (s), 9.18 (s).

### Preparation of Intermediates Ethyl-3-[Cyclopentylthiocarbonyl-L-(S-Me-Pen)-L-Phe-L-(Tr-Gln)]-E-Propenoate & Ethyl-3-(Cyclopentylthiocarbonyl-L-[S(O)-Me-Pen]-L-Phe-L-[Tr-Gln])-E-Propenoate.

The mixture of cyclopentylthiocarbonyl-L-(S-Me-Pen)-L-Phe-L-(Tr-glutaminal) and cyclopentylthiocarbonyl-L-[S(O)-Me-Pen]-L-Phe-L-(Tr-glutaminal) (0.40 g, approximately 0.51 mmol) was dissolved in 10 mL of anh THF. To this solution was added (carbethoxymethylene) triphenylphosphorane (0.21 g, 0.61 mmol), and the reaction mixture was stirred overnight at rt. The solvent was removed in vacuo, and the residue was purified by column chromatography on silica gel using a gradient solvent system (0-2% MeOH/CHCl₃) to give 0.184 g of the sulfide product and 0.132 g sulfoxide product (contaminated with triphenylphosphine oxide):
Ethyl-3-[cyclopentylthiocarbonyl-L-(S-Me-Pen)-L-Phe-L-(Tr-*Gln*)]-E-propenoate: ¹H NMR (DMSO-*d*₆) δ 1.14 (s, 3H), 1.21 (t, 3H, J = 6.0 Hz), 1.24 (s, 3H), 1.46-1.68 (m, 10H), 1.96 (s, 3H), 2.25-2.31 (m, 2H), 2.78-2.85 (m, 1H), 2.96-3.00 (m, 1H), 3.54-3.72 (m, 1H), 4.05-4.13 (m, 2H), 4.32-4.47 (m, 1H), 4.49-4.55 (m, 1H), 4.56-4.59 (m, 1H), 5.57 (d, 1H, *J* = 15.0 Hz), 6.64 (dd, 1H, *J* =15.0, 3.0 Hz), 7.13-7.26 (m, 20H), 7.99-8.04 (m, 2H), 8.45 (d, 1H, *J* = 9.0 Hz), 8.55 (s, 1H). Ethyl-3-(cyclopentylthiocarbonyl-L-[S(O)-Me-Pen]-L-Phe-L-[Tr-*Gln*])-E-propenoate: ¹H NMR (DMSO-*d*₆) (mixture of diastereomers): δ 1.11-1.15 (m), 1.19-1.23 (m), 1.35-1.66 (m), 1.98-2.00 (m), 2.18-2.35 (m), 2.41 (s), 2.64-2.83 (m), 2.89-3.02 (m), 3.51-3.56 (m), 4.11 (q, J = 6.0 Hz), 4.34-4.40 (m), 4.48-4.59 (m), 4.63-4.66 (m), 5.51-5.57 (m), 6.61-6.68 (m), 7.13-7.28 (m), 8.12-8.24 (m), 8.42-8.53 (m), 8.55-8.57 (m).

### Preparation of Product - Ethyl-3-[Cyclopentylthiocarbonyl-L-(S-Me-Pen)-L-Phe-L-Gln]-E-Propenoate

Ethyl-3-[cyclopentylthiocarbonyl-L-(S-Me-Pen)-L-Phe-L-(Tr-*Gln*)]-E-propenoate (0.184 g) was dissolved in 10 mL CH₂Cl₂. To this solution was added I mL of trifluoroacetic acid, and the reaction mixture was stirred at rt overnight. The solvent was removed under vacuum and the residue was purified by column chromatography on silica gel using a gradient solvent system (0-2% MeOH/CHCl₃) to give 0.044 g (24%; 3 steps from cyclopentylthiocarbonyl-L-(S-Me-Pen)-L-Phe-L-(Tr-glutaminol)) as a white amorphous solid: IR (KBr) 3296, 2984, 1787, 1655, 1560, 1541, 1280, 1194 cm⁻¹; ¹H NMR (DMSO-*d*₆) δ 1.14 (s, 3H), 1.21 (t, 3H, *J =* 6.0 Hz), 1.25 (s, 3H), 1.40-1.70 (m, 10H), 2.02 (s, 3H), 2.05-2.24 (m, 2H), 2.79-2.86 (m, 1H), 2.93-3.00 (m, 1H), 3.43-3.55 (m, 1H), 4.09 (q, 2H, *J* = 6.0 Hz), 4.31-4.36 (m, 1H), 4.43-4.50 (m, 1H), 4.56 (d, 1H, *J* = 6.0 Hz), 5.58 (d, 1H, *J =* 15.0 Hz), 6.65 (dd, 1H, *J =* 15.0, 6.0 Hz), 6.75 (bs, 1H), 7.15-7.21 (m, 6H), 7.99-8.06 (m, 2H), 8.45 (d, 1H, *J* = 6.0 Hz). HRMS calcd for C₃₀H₄₄N₄O₆S₂+Cs 753.1757, found 753.1737. Anal. (C₃₀H₄₄N₄O₆S₂) C, H, N, S.

### Example 37 - Preparation of Compound 173: Ethyl-3-[Cyclopentylthiocarbonyl-L-(S-Ph-Cys)-L-Phe-L-Gln]-E-Propenoate.

### Preparation of Intermediate Cyclopentyl Chlorothiolformate.

Cyclopentanethiol (10.7 mL, 0.1 mol) was dissolved in 200 mL of CH₂Cl₂. Triphosgene (11.13 g, 37.5 mmol) was added and the reaction mixture was cooled to 0 °C. Et₃N (14.1 mL, 0.1 mol) was added dropwise, and the reaction was allowed to warm to room temperature over a period of one hour. The solvent was carefully removed under reduced pressure at 20 °C due to the volatility of the product. The resulting residue was taken up in Et₂O, and the solids were filtered and washed with more Et₂O. The solvent was again carefully removed under reduced pressure, and the was product purified by distillation (85% yield): colorless liquid (bp 70-74 °C; 1 tort): IR(neat) 1756, 830 cm⁻¹; ¹H NMR (benzene-*d*₆) δ 1.01-1.23 (m, 6H), 1.49-1.60 (m, 2H), 3.20-3.29 (m, 1H).

### Preparation of Intermediate BOC-L-(S-Ph-Cys).

To a suspension of 19.73 g (0.1 mol) L-(S-Ph-Cys) (purchased from Davos Chemical Corp., Englewood Cliffs, NJ) in 72 mL of *tert*-butanol was added a solution of NaOH (4.1 g, 0.1025 mol) in 100 mL H₂O. Once the suspension became a clear solution di-*tert*-butyl dicarbonate (22.92 g, 0.105 mol) was added. The clear solution became a slurry and was allowed to stir at rt overnight. At this time the turbid solution was washed twice with pet. ether. The organic layer was washed 3 times with a sat NaHCO₃ solution and the aqueous layers were combined. The aqueous layer was then carefully acidified to pH 2-3 with a sat KHSO₄ solution and extracted with a large excess of Et₂O. The organic phase was dried over Na₂SO₄, filtered and concentrated under vacuum to give 27.4 g (92%) of BOC-L-(S-Ph-Cys) as white solid. Any residual H₂O and/or *tert*-butanol was removed by benzene azeotrope before using the material. ¹H NMR (DMSO-*d*_{*6*}) δ 1.36 (s, 9H), 3.10 (dd, 1H, *J =* 13.6, 9.6 Hz), 3.34 (dd, 1H, *J =* 13.6, 4.4 Hz), 4.01 (m, 1H), 7.20 (m, 2H), 7.34 (m, 3H), 12.82 (bs, 1H).

### Preparation of Intermediate BOC-L-(S-Ph-Cys)-L-Phe-L-(Tr-glutaminol).

BOC-L-(S-Ph-Cys) (0.45 g, 1.5 mmol) was dissolved in 2 mL of DMF and 2 mL of CH₂Cl₂. To this solution was added N-hydroxysuccinimide (0.17 g, 1.5 mmol), followed by dicyclohexylcarbodiimide (0.31 g, 1.5 mmol). The reaction was stirred at rt for 2 h. The mixture was then filtered into a separate flask containing L-Phe-L-(Tr-glutaminol) (0.78 g, 1.5 mmol) dissolved in 4 mL of DMF and 2 mL of CH₂Cl₂. The reaction mixture was stirred overnight and the solvent was removed in vacuo. The residue was purified by column chromatography on silica gel using a gradient solvent system (0-2% MeOH/CHCl₃) to give 1.06 g (88%) of a white amorphous solid: IR (KBr) 3304, 3061, 2972, 2928, 1645, 1516, 1493, 1367, 1248, 1165, 1024, 742, 698 cm⁻¹; ¹H NMR (DMSO-*d*₆) δ 1.35 (s, 9H), 1.35-1.43 (m, 1H), 1.70-1.74 (m, 1H), 2.20-2.33 (m, 2H), 2.82-2.92 (m, 1H), 2.93-3.10 (m, 1H), 3.11-3.23 (m, 2H), 3.24-3.32 (m, 2H), 3.58-3.68 (m, 1H), 3.80-3.98 (m, 1H), 4.58-4.64 (m, 1H), 4.65-4.77 (m, 1H), 7.14-7.30 (m, 26H), 7.75 (d, 1H, *J =* 6.0 Hz), 7.83 (d, 1H, *J =* 6.0 Hz), 8.51 (s, 1H). MS calcd for C₄₇H₅₂N₄O₆S+H 801, found 801.

### Preparation of Intermediate L-(S-Ph-Cys)-L-Phe-L-(Tr-glutaminol) Hydrochloride Salt.

Using the procedure described in Example 36 for the preparation of L-(S-Me-Pen)-L-Phe-L-(Tr-glutaminol) hydrochloride salt, L-(S-Ph-Cys)-L-Phe-L-(Tr-glutaminol) hydrochloride salt was synthesized from BOC-L-(S-Ph-Cys)-L-Phe-L-(Tr-glutaminol) to give 0.182 g of white solid which was used without further purification: IR (KBr) 3325, 3057, 2949, 1685, 1655, 1560, 1493, 1448, 746, 700 cm⁻¹; ¹H NMR (DMSO-*d*₆) δ 1.41-1.67 (m, 1H), 1.69-1.81 (m, 1H), 2.26-2.44 (m, 2H), 2.86-2.97 (m, 1H), 2.98-3.23 (m, 1H), 3.25-3.43 (m, 4H), 3.60-3.84 (m, 2H), 4.02-4.20 (m, 1H), 4.44-4.60 (m, 1H), 7.08-7.48 (m, 25H), 7.87 (d, 1H, *J* = 6.0 Hz), 8.46 (bs, 3H), 8.55 (s, 1H), 8.87 (d, 1H, *J =* 6.0 Hz).

### Preparation of Intermediate Cyclopentylthiocarbonyl-L-(S-Ph-Cys)-L-Phe-L-(Tr-Glutaminol).

Using the procedure described in Example 36 for the preparation of cyclopentylthiocarbonyl-L-(S-Me-Pen)-L-Phe-L-(Tr-glutaminol), cyclopentylthiocarbonyl-L-(S-Ph-Cys)-L-Phe-L-(Tr-glutaminol) was synthesized from L-(S-Ph-Cys)-L-Phe-L-(Tr-glutaminol) hydrochloride salt in 75% yield: white amorphous solid: IR (KBr) 3288, 3059, 2960, 1637, 1494,1448, 1205, 746, 700 cm⁻¹; ¹H NMR (DMSO-*d*₆) δ 1.42-1.98 (m, 10H), 1.99-2.26 (m, 1H), 2.48-2.50 (m, 1H), 2.96-2.98 (m, 1H), 3.01-3.19 (m, 1H), 3.19-3.55 (m, 6H), 3.64-3.85 (m, 1H), 4.36-4.40 (m, 1H), 4.46-4.58 (m, 1H), 7.14-7.30 (m, 25H), 7.68 (d, 1H, *J =* 6.0 Hz), 8.01 (d, 1H, *J =* 6.0 Hz), 8.41 (d, 1H, *J* = 6.0 Hz), 8.52 (s, 1H). MS calcd for C₄₈H₅₂N₄O₅S₂+H 829, found 829.

### Preparation of Intermediate Cyclopentylthiocarbonyl-L-(S-Ph-Cys)-L-Phe-L-(Tr-Glutaminal).

Using the procedure described in Example 36 for the preparation of cyclopentylthiocarbonyl-L-(S-Me-Pen)-L-Phe-L-(Tr-glutaminal) and cyclopentylthiocarbonyl-L-[S(O)-Me-Pen]-L-Phe-L-(Tr-glutaminal), cyclopentylthiocarbonyl-L-(S-Ph-Cys)-L-Phe-L-(Tr-glutaminal) was synthesized from cyclopentylthiocarbonyl-L-(S-Ph-Cys)-L-Phe-L-(Tr-glutaminol) in 98% yield: white amorphous solid used without further purification: ¹H NMR (DMSO-*d*₆) δ 1.45-1.70 (m, 8H), 2.02-2.28 (m, 3H), 2.35-2.51 (m, 1H), 2.95-3.02 (m, 2H), 3.04-3.22 (m, 1H), 3.24-3.36 (m, 1H), 3.56-3.59 (m, 1H), 4.02-4.08 (m, 1H), 4.47-4.59 (m, 1H), 4.60-4.80 (m, 1H), 7.20-7.36 (m, 25H), 8.22 (d, 1H, J = 6.0 Hz), 8.43-8.48 (m, 2H), 8.65 (s, 1H), 9.27 (s, 1H).

### Preparation of Intermediate Ethyl-3-[Cyclopentylthiocarbonyl-L-(S-Ph-Cys)-L-Phe-L-(Tr-Gln)]-E-Propenoate.

Using the procedure described in Example 1 for the preparation of ethyl-3-[CBZ-L-Leu-L-Phe-L-*Met* (sulfoxide)]-E-propenoate, ethyl-3-[cyclopentylthiocarbonyl-L-(S-Ph-Cys)-L-Phe-L-(Tr-*Gln*)]-E-propenoate was synthesized from cyclopentylthiocarbonyl-L-(S-Ph-Cys)-L-Phe-L-(Tr-glutaminal) to give 0.26 g of material contaminated with triphenylphosphine oxide (after column chromatography) which was used without further purification: ¹H NMR (DMSO-*d*₆) δ 1.19 (t, 3H, J = 6.0 Hz), 1.47-1.59 (m, 10H), 1.93-2.23 (m, 1H), 2.25-2.34 (m, 1H), 2.83-2.93 (m, 1H), 2.95-3.16 (m, 1H), 3.19-3.29 (m, 2H), 3.51-3.56 (m, 1H), 4.09 (q, 2H, *J* = 6.0 Hz), 4.35-4.44 (m, 2H), 4.46-4.48 (m, 1H), 5.64 (d, 1H, *J* = 15.0 Hz), 6.68 (dd, 1H, *J* = 15.0, 3.0 Hz), 7.13-7.29 (m, 25H), 8.07 (d, 1H, *J =* 6.0 Hz), 8.13 (d, 1H, *J* = 6.0 Hz), 8.42 (d, 1H, *J =* 6.0 Hz), 8.58 (s, 1H).

### Preparation of Product - Ethyl-3-[Cyclopentylthiocarbonyl-L-(S-Ph-Cys)-L-Phe-L-Gln]-E-Propenoate.

Using the procedure described in Example 34 for the preparation of ethyl-3-[ethylthiocarbonyl-L-α-(t-butyl-Gly)-L-Phe-L-*Gln*]-E-propenoate, ethyl-3-[cyclopentylthiocarbonyl-L-(S-Ph-Cys)-L-Phe-L-*Gln*]-E-propenoate was synthesized from ethyl-3-[cyclopentylthiocarbonyl-L-(S-Ph-Cys)-L-Phe-L-(Tr-*Gln*)]-E-propenoate in 35% yield (2 steps from cyclopentylthiocarbonyl-L-(S-Ph-Cys)-L-Phe-L-(Tr-glutaminal)): white amorphous solid: IR (KBr) 3294, 1712, 1655, 1633, 1545, 1203, 738, 700 cm⁻¹; ¹H NMR (DMSO-*d*₆) δ 1.19 (t, 3H, *J* = 6.0 Hz), 1.56-1.76 (m, 10H), 1.98-2.08 (m, 2H), 2.84-2.99 (m, 2H), 3.17-3.39 (m, 2H), 3.51-3.76 (m, 1H), 4.08 (q, 2H, J = 6.0 Hz), 4.39-4.45 (m, 3H), 5.64 (d, 1H, *J =* 15.0 Hz), 6.69 (dd, 1H, *J =* 15.0, 3.0 Hz), 6.77 (bs, 1H), 7.18-7.32 (m, 11H), 8.08 (d, 1H, *J =* 6.0 Hz), 8.18 (d, 1H, *J* = 6.0 Hz), 8.43 (d, 1H, *J* = 6.0 Hz). HRMS calcd for C₃₃H₄₂N₄O₆S₂+Cs 787.1600, found 787.1618. Anal. (C₃₃H₄₂N₄O₆S₂) C, H, N, S.

### Example 38 - Preparation of Compound 174: Ethyl-3-[Cyclopentylthiocarbonyl-L-α-(t-Butyl-Gly)-L-(4-Me-Phe)-L-Gln]-E-Propenoate.

### Preparation of Intermediate Fmoc-L-(4-Me-Phe)-L-(Tr-Glutaminol).

Using the procedure described in Example 1 for the preparation of CBZ-L-Leu-L-Phe-L-methioninol, this derivative was synthesized from Fmoc-L-4-Me-Phe (purchased from Neosystems Laboratories, Strasbourg, France) and L-(Tr-glutaminol) in 85% yield and isolated as a white solid. IR (KBr) 3316, 3283, 3024, 2946, 1694, 1667, 1448, 1256, 1041, 760, 700 cm⁻¹; ¹H NMR (DMSO-*d*₆) δ 1.56 (m, 1H), 1.77 (m, 1H), 2.22 (s, 3H), 2.26 (m, 2H), 2.74 (m, 1H), 2.90 (m, 1H), 3.17 (m, 1H), 3.69 (m, 1H), 4.03-4.23 (m, 4H), 7.03-7.54 (m, 21H), 7.39 (t, 2H, *J =* 7.4 Hz), 7.50 (d, 1H, *J =* 8.5 Hz), 7.59 (d, 1H, *J =* 7.4 Hz), 7.60 (d, 1H, *J =* 7.7 Hz), 7.70 (d, 1H, *J =* 8.8 Hz), 7.87 (d, 2H, *J =* 7.4 Hz), 8.45 (s, 1H); MS calcd for C₄₉H₄₇N₃O₅+Cs 890, found 890.

### Preparation of Intermediate L-(4-Me-Phe)-L-(Tr-Glutaminol).

To a solution of Fmoc-L-(4-Me-Phe)-L-(Tr-glutaminol) (3.25 g, 4.29 mmol) in anh DMF (10 mL) was added piperidine (0.51 mL, 5.15 mmol). The solution was stirred and monitored by TLC. Upon consumption of the starting material, the reaction mixture was concentrated to a residue and then subjected to column chromatography on silica gel (5% MeOH/CH₂Cl₂) to afford the product as white solid in 87% yield. IR (KBr) 3326, 3054, 3030, 2953, 2872, 1651,1516, 1491, 1447, 1036, 700 cm⁻¹; ¹H NMR (DMSO-*d*₆) δ 1.47 (m, 1H), 1.75 (m, 3H), 2.13 (m, 1H), 2.23 (s, 3H), 2.57 (dd, 1H, *J =* 13.2, 8.1 Hz), 2.88 (dd, 1H, *J* = 13.6, 4.8 Hz), 3.20 (m, 1H), 3.30 (m, 1H), 3.66 (m, 1H), 4.64 (t, 1H, *J* = 5.5 Hz), 7.07 (m, 4H), 7.10-7.28 (m, 15H), 7.62 (d, 1H, *J =* 8.8 Hz), 8.54 (s, 1H); MS calcd for C₃₄H₃₇N₃O₃+Na 558, found 558.

### Preparation of Intermediate Cyclopentylthiocarbonyl-L-α-(t-Butyl-Gly).

A stirred suspension of L-α-(t-butyl-Gly) (0.656 g, 5.0 mmol) in 18 mL CH₂Cl₂, and diisopropylethylamine (3.5 mL, 20 mmol) was cooled to 0 °C. To this mixture chlorotrimethylsilane (0.83 mL, 6.5 mmol) was added dropwise. The slurry was allowed to warm to rt, and the mixture was stirred for about 2 h. At this time the mixture was recooled to 0 °C, and cyclopentyl chlorothiolformate (0.823 g, 5.0 mmol) was added dropwise. The slurry became a pale yellow solution after stirring at rt for approximately 5 h. The solution was concentrated, redissolved in an excess of EtOAc and washed with H₂O, 10% aq KHSO₄, H₂O and brine. The organic phase was dried over MgSO₄, filtered and concentrated to give cyclopentylthiocarbonyl-L-α-(t-butyl-Gly) as a yellow oil in nearly quantitative yield which was azeotroped with benzene to remove any residual water before being used in the next step. IR (film) 3324, 2965, 2920, 2872, 1726, 1642, 1518, 1202 cm⁻¹; ¹H NMR (CDCl₃) δ 1.03 (s, 9H), 1.48-1.73 (m, 6H), 2.10 (m, 2H), 3.72 (m, 1H), 4.46 (m, 1H), 5.79 (m, 1H); MS calcd for C₁₂H₂₁NO₃S+Na 282, found 282.

### Preparation of Intermediate Cyclopentylthiocarbonyl-L-α-(t-Butyl-Gly)-L-(4-Me-Phe)-L-(Tr-Glutaminol).

This preparation was carried out following the procedure ofL. A. Carpino, *J*. *Am*. *Chem*. *Soc*. **1993**, *115*, 4397. Cyclopentylthiocarbonyl-L-α-(t-butyl-Gly) (0.325 g, 1.25 mmol) was dissolved in 8.0 mL of DMF. Diisopropylethylamine (0.45 mL, 2.5 mmol) was added, followed by 0.67 g (1.25 mmol) of N-Me-L-(4-Me-Phe)-L-(Tr-glutaminol). The reaction was cooled to 0°C and O-(7-azabenzotriazol-1-yl)-1,1,3,3-tetramethyluronium hexafluorophosphate (HATU) (0.476 g, 1.25 mmol) was added. The reaction mixture was allowed to warm to rt whereupon the DMF was removed in vacuo. The residue was dissolved with EtOAc, and the organic phase washed consecutively with 1N HCl, a sat NaHCO₃ solution, H₂O, and brine. The solvent was dried over MgSO₄, filtered, and concentrated to give a residue which was subjected to column chromatography on silica gel (gradient; 2-5% MeOH/CHCl₃) to give 0.95 g (98%) of a white amorphous solid: IR(KBr) 3302, 2957, 2876, 1669, 1645, 1537, 1447, 1196, 700 cm⁻¹; ¹H NMR (CDCl₃) δ 0.88 (s, 9H), 1.48-1.70 (m, 9H), 1.85 (m, 1H), 2.04 (m, 2H), 2.28 (s, 3H), 2.32 (m, 2H), 2.92 (m, 2H), 3.25 (dd, 1H, *J* = 8.1, 3.5 Hz), 3.30 (dd, 1H, *J* = 10.9, 3.7 Hz), 3.66 (m, 1H), 3.72 (m, 1H), 4.14 (m, 1H), 4.47 (m, 1H), 6.04 (d, 1H, *J* = 7.7 Hz), 6.52 (d, 1H, *J* = 7.7 Hz), 6.60 (d, 1H, *J* = 7.0 Hz), 7.05 (m, 5H), 7.24 (m, 15H). MS calcd for C₄₆H₅₆N₄O₅S+Na 799, found 799.

### Preparation of Intermediate Cyclopentylthiocarbonyl-L-α-(t-Butyl-Gly)-L-(4-Me-Phe)-L-(Tr-Glutantinal).

Using the general procedure described in Example 1 for the preparation of CBZ-L-Leu-L-Phe-L-methioninal (sulfoxide), cyclopentylthiocarbonyl-L-α-(t-butyl-Gly)-L-(4-Me-Phe)-L-(Tr-glutaminal) was synthesized from cyclopentylthiocarbonyl-L-α-(t-butyl-Gly)-L-(4-Me-Phe)-L-(Tr-glutaminol) in quantitative yield and isolated as a white amorphous solid and used without further purification: IR(film) 3302, 3061, 3030, 2961, 2870, 1730, 1644, 1514, 1493, 1196, 911, 733, 700 cm⁻¹; ¹H NMR (CDCl₃) δ 0.90 (s, 9H), 1.46-1.68 (m, 8H), 1.86 (m, 1H), 2.00-2.24 (m, 2H), 2.28 (s, 3H), 2.31 (m, 1H), 2.96 (m, 2H), 3.58 (m, 1H), 4.05 (m, 1H), 4.14 (m, 1H), 4.52 (m, 1H), 5.88 (m, 1H), 6.28 (m, 1H), 6.90 (m, 1H), 7.07 (m, 5H), 7.25 (m, 15H), 9.30 (s, 1H); MS calcd for C₄₆H₅₄N₄O₅S•CH₃OH (methyl-hemiacetal)+Na 829, found 829.

### Preparation of Intermediate Ethyl-3-[Cyclopentylthiocarbonyl-L-α-(t-Butyl-Gly)-L-(4-Me-Phe)-L-(Tr-Gln)]-E-Propenoate.

Using the procedure described in Example 1 for the preparation of ethyl-3-[CBZ-L-Leu-L-Phe-L-*Met* (sulfoxide)]-E-propenoate, ethyl-3-[cyclopentylthiocarbonyl-L-α-(t-butyl-Gly)-L-(4-Me-Phe)-L-(Tr-*Gln*)]-E-propenoate was synthesized from cyclopentylthiocarbonyl-L-α-(t-butyl-Gly)-L-(4-Me-Phe)-L-(Tr-glutaminal) (0.468 g, 0.627 mmol) to give 0.52 g of material contaminated with triphenylphosphine oxide after column chromatography on silica gel (gradient: 1-2.5% MeOH/CH₂Cl₂), which was used without further purification: white amorphous solid: IR(film) 3302, 3061, 2967, 2868, 1721, 1642,1514, 1491, 1370, 1192, 1036, 911, 731, 700 cm⁻¹; ¹H NMR (CDCl₃) δ 0.72 (s, 9H), 1.29 (t, 3H, *J =* 7.0 Hz), 1.46-1.68 (m, 6H), 1.86-2.05 (m, 4H), 2.29 (s, 3H), 2.32 (m, 2H), 2.91 (m, 2H), 3.00 (m, 1H), 3.62 (m, 1H), 4.07 (m, 1H), 4.17 (q, 2H, *J =* 7.2 Hz), 4.43 (m, 2H), 5.61 (dd, 1H, *J =* 15.8, 1.5 Hz), 5.95 (m, 1H), 6.34 (m, 1H), 6.57 (m, 1H), 6.64 (dd, 1H, *J* = 15.8, 5.5 Hz), 7.03 (m, 5H), 7.24 (m, 15H). MS calcd for C₅₀H₆₀N₄O₆S+Na 867, found 867.

### Preparation of Product - Ethy1-3-[Cyclopentylthiocarbonyl-L-α-(t-Butyl-Gly)-L-(4-Me-Phe)-L-Gln]-E-Propenoate.

Using the procedure described in Example 34 for the preparation of ethyl-3-[ethylthiocarbonyl-L-α-(t-butyl-Gly)-L-Phe-L-*Gln*]-E-propenoate, ethyl-3-[cyclopentylthiocarbonyl-L-α-(t-butyl-Gly)-L-(4-Me-Phe)-L-*Gln*]-E-propenoate was synthesized from ethyl-3-[cyclopentylthiocarbonyl-L-α-(t-butyl-Gly)-L-(4-Me-Phe)-L-(Tr-*Gln*)]-E-propenoate and isolated as a white solid after purification by column chromatography on silica gel using a gradient solvent system (1-5% MeOH/CH₂Cl₂) (57% yield; two steps from cyclopentylthiocarbonyl-L-α-(t-butyl-Gly)-L-(4-Me-Phe)-L-(Tr-glutaminal): IR (KBr) 3318, 2973, 2951, 2868, 1715, 1651, 1539, 1371, 1192 cm⁻¹; ¹H NMR (DMSO-*d*₆) δ 0.83 (s, 9H), 1.21 (t, 3H, *J* = 7.2 Hz), 1.41-1.72 (m, 8H), 2.02 (m, 4H), 2.22 (s, 3H), 2.81 (m, 2H), 3.54 (m, 1H), 4.10 (q, 2H, *J =* 7.0 Hz), 4.24 (d, 1H, *J =* 9.3 Hz), 4.36 (m, 1H), 4.43 (m, 1H), 5.56 (dd, 1H, *J =* 15.7, 1.4 Hz), 6.65 (dd, 1H, *J =* 15.7, 5.5 Hz), 6.73 (s, 1H), 7.03 (m, 4H), 7.13 (s, 1H), 7.86 (d, 1H, *J* = 9.3 Hz), 8.04 (d, 1H, *J =* 8.4 Hz), 8.12 (d, 1H, *J =* 7.8 Hz); HRMS calcd for C₃₁H₄₆N₄O₆S+Cs 735.2192, found 735.2180. Anal. (C₃₁H₄₆N₄O₆S) C, H, N, S.

### Example 39 - Preparation of Compound 175: Ethyl-2-Methyl-3-[Cyclopentylthiocarbonyl-L-α-(t-Butyl-Gly)-L-(4-Me-Phe)-L-Gln]-E-Propenoate.

### Preparation of Intermediate Ethyl-2-Methyl-3-[Cyclopentylthiocarbonyl-L-α-(t-Bntyl-Gly)-L-(4-Me-Phe)-L-(Tr-Gln)]-E-Propenoate.

Using the procedure described in Example 1 for the preparation of ethyl-3-[CBZ-L-Leu-L-Phe-L-*Met* (sulfoxide)]-E-propenoate, ethyl-2-methyl-3-[cyclopentylthiocarbonyl-L-α-(t-butyl-Gly)-L-(4-Me-Phe)-L-(Tr-*Gln*)]-E-propenoate was synthesized from cyclopentylthiocarbonyl-L-α-(t-butyl-Gly)-L-(4-Me-Phe)-L-(Tr-glutaminal) (0.466 g, 0.60 mmol) and (carbethoxyethylidene)triphenylphosphorane (0.24 g, 0.66 mmol) to give 0.487 g of material contaminated with triphenylphosphine oxide after column chromatography on silica gel (gradient: 1-2.5% MeOH/CH₂Cl₂) which was used without further purification. white amorphous solid: IR(film) 3302, 3063, 2967, 2870, 1711, 1642, 1516, 1491, 1250, 1194, 911, 731, 698 cm⁻¹; ¹H NMR (CDCl₃) δ 0.88 (s, 9H), 1.31 (t, 3H, *J* = 7.2 Hz), 1.50-1.77 (m, 6H), 1.81 (m, 2H), 1.82 (s, 3H), 2.06 (m, 2H), 2.28 (s, 3H), 2.31 (m, 2H), 2.93 (m, 2H), 3.64 (m, 1H), 4.04 (m, 1H), 4.20 (q, 2H, *J* = 7.0 Hz), 4.40 (m, 1H), 4.58 (m, 1H), 5.90 (m, 1H), 6.30 (m, 3H), 7.01 (m, 5H), 7.24 (m, 15H). MS calcd for C₅₁H₆₂N₄O₆S+Na 881, found 881.

### Preparation of Product - Ethyl-2-Methyl-3-[Cyclopentylthiocarbonyl-L-α-(t-Butyl-Gly)-L-(4-Me-Phe)-L-Gln]-E-Propenoate.

Using the procedure described in Example 34 for the preparation of ethyl-3-[ethylthiocarbonyl-L-α-(t-butyl-Gly)-L-Phe-L-*Gln*]-E-propenoate, ethyl-2-methyl-3-[cyclopentylthiocarbonyl-L-α-(t-butyl-Gly)-L-(4-Me-Phe)-L-*Gln*]-E-propenoate was synthesized from ethyl-2-methyl-3-[cyclopentylthiocarbonyl-L-α-(t-butyl-Gly)-L-(4-Me-Phe)-L-(Tr-*Gln*)]-E-propenoate and isolated as a white solid after purification by column chromatography on silica gel using a gradient solvent system (1-5% MeOH/CH₂Cl₂) (55% yield; two steps from cyclopentylthiocarbonyl-L-α-(t-butyl-Gly)-L-(4-Me-Phe)-L-(Tr-glutaminal): IR (KBr) 3324, 2963, 2870, 1707, 1647, 1550, 1516, 1257, 1196 cm⁻¹; ¹H NMR (DMSO-*d*_{*6*}) δ 0.83 (s, 9H), 1.22 (t, 3H, *J* = 7.2 Hz), 1.41-1.73 (m, 8H), 1.77 (m, 3H), 2.00 (m, 4H), 2.20 (s, 3H), 2.78 (m, 2H), 3.55 (m, 1H), 4.12 (q, 2H, *J* = 7.0 Hz), 4.23 (d, 1H, *J =* 9.0 Hz), 4.35 (m, 1H), 4.48 (m, 1H), 6.29 (dd, 1H, *J =* 9.3, 1.2 Hz), 6.72 (s, 1H), 6.99 (m, 4H), 7.13 (s, 1H), 7.86 (d, 1H, *J =* 9.0 Hz), 8.03 (m, 2H); HRMS calcd for C₃₂H₄₈N₄O₆S+Cs 749.2349, found 749.2336. Anal. (C₃₂H₄₈N₄O₆S) C, H, N, S.

### Example 40 - Preparation of Compound 176: Ethyl-3-[Cyclopentylthiocarbonyl-L-α-(t-Butyl-Gly)-L-(4-F-Phe)-L-Gln]-E-Propenoate.

### Preparation of Intermediate Cyclopentylthiocarbonyl-L-α-(t-Butyl-Gly)-L-(4-F-Phe)-L-(Tr-Glutaminol).

This intermediate was prepared as a white solid in 75% yield from cyclopentylthiocarbonyl-L-α-(t-butyl-Gly) and the free base of L-(4-F-Phe)-L-(Tr-glutaminol)·HCl using the procedure described to prepare cyclopentylthiocarbonyl-L-α-(t-butyl-Gly)-L-(4-Me-Phe)-L-(Tr-glutaminol). IR(KBr) 3299, 3063, 2969, 2870, 1651, 1510, 1447, 1225, 1192, 766, 700 cm⁻¹; ¹H NMR (CDCl₃) δ 0.88 (s, 9H), 1.50-1.76 (m, 9H), 1.85 (m, 1H), 2.05 (m, 2H), 2.36 (m, 2H), 2.50 (m, 1H), 2.92 (m, 2H), 3.32 (m, 2H), 3.66 (m, 1H), 3.73 (m, 1H), 4.17 (m, 1H), 4.69 (m, 1H), 6.09 (d, 1H, *J* = 7.0 Hz), 6.74 (m, 1H), 6.91 (m, 2H), 7.05 (m, 2H), 7.24 (m, 15H). MS calcd for C₄₆H₅₃N₄O₅SF+Na 803, found 803.

### Preparation of Intermediate Cyclopentylthiocarbonyl-L-α-(t-Butyl-Gly)-L-(4-F-Phe)-L-(Tr-Glutaminal).

Using the general procedure described in Example 1 for the preparation CBZ-L-Leu-L-Phe-L-methioninal (sulfoxide), cyclopentylthiocarbonyl-L-α-(t-butyl-Gly)-L-(4-F-Phe)-L-(Tr-glutaminal) was synthesized from cyclopentylthiocarbonyl-L-α-(t-butyl-Gly)-L-(4-F-Phe)-L-(Tr-glutaminol) in quantitative yield and isolated as a white amorphous solid and used without further purification: IR(film) 3302, 3061, 3030, 2961, 2866, 1732, 1644, 1510,1447, 1225, 1196, 911, 733, 700 cm⁻¹; ¹H NMR (CDCl₃) δ 0.90 (s, 9H), 1.48-1.67 (m, 8H), 1.85 (m, 1H), 2.00-2.28 (m, 2H), 2.36 (m, 2H), 2.90 (dd, 1H, *J* = 14.9, 6.1 Hz), 3.03 (dd, 1H *J* = 14.5, 6.8 Hz), 3.64 (m, 1H), 4.07 (m, 1H), 4.18 (m, 1H), 4.53 (m, 1H), 5.92 (m, 1H), 6.31 (m, 1H), 6.92 (m, 2H), 7.10 (m, 3H), 7-23 (m, 15H), 9.31 (s, 1H); MS calcd for C₄₅H₅₃N₄O₅SF•CH₃OH (methyl-hemiacetal)+Na 833, found 833.

### Preparation of Intermediate Ethyl-3-[Cyclopentylthiocarbonyl-L-α-(t-Butyl-Gly)-L-(4-F-Phe)-L-(Tr-Gln)]-E-Propenoate.

Using the procedure described in Example 1 for the preparation of ethyl-3-[CBZ-L-Leu-L-Phe-L-*Met* (sulfoxide)]-E-propenoate, ethyl-3-[cyclopentylthiocarbonyl-L-α-(t-butyl-Gly)-L-(4-F-Phe)-L-(Tr-*Gln*)]-E-propenoate was synthesized from cyclopentylthiocarbonyl-L-α-(t-butyl-Gly)-L-(4-F-Phe)-L-(Tr-glutaminal) (0.343 g, 0.44 mmol) to give 0.377 g of material contaminated with triphenylphosphine oxide after column chromatography on silica gel (gradient: 1-2.5% MeOH/CH₂Cl₂) which was used without further purification: white amorphous solid: IR(KBr) 3314, 3285, 2969, 2936, 1723, 1651, 1510, 1447, 1370, 1190, 1038, 700 cm⁻¹; ¹H NMR (CDCl₃) δ 0.88 (s, 9H), 1.28 (t, 3H,*J* = 7.0 Hz), 1.48-1.78 (m, 8H), 1.83-2.15 (m, 4H), 2.32 (m, 2H), 2.85 (m, 1H), 3.00 (m, 1H), 3.61 (m, 1H), 4.16 (q, 2H, *J =* 7.0 Hz), 4.39 (m, 2H), 5.54 (d, 1H, *J* = 15.4 Hz), 6.17 (m, 1H), 6.63 (dd, 1H, *J* = 15.4, 4.0 Hz), 6.91 (m, 2H), 7.01 (m, 2H), 7.28 (m, 15H), 7.45 (m, 1H), 7.54 (m, 1H), 7.63 (m, 1H). MS calcd for C₄₉H₅₇N₄O₆SF+Na 871, found 871.

### Preparation of Product - Ethyl-3-[Cyclopentylthiocarbonyl-L-α-(t-Butyl-Gly)-L-(4-F-Phe)-L-Gln]-E-Propenoate.

Using the procedure described in Example 34 for the preparation of ethyl-3-[ethylthiocarbonyl-L-α-(t-butyl-Gly)-L-Phe-L-*Gln*]-E-propenoate, ethyl-3-[cyclopentylthiocarbonyl-L-α-(t-butyl-Gly)-L-(4-F-Phe)-L-*Gln*]-E-propenoate was synthesized from ethyl-3-[cyclopentylthiocarbonyl-L-α-(t-butyl-Gly)-L-(4-F-Phe)-L-(Tr-*Gln*)]-E-propenoate and isolated as a white solid after purification by column chromatography on silica gel using a gradient solvent system (1-5% MeOH/CH₂Cl₂) (56% yield; two steps from cyclopentylthiocarbonyl-L-α-(t-butyl-Gly)-L-(4-F-Phe)-L-(Tr-glutaminal): IR (KBr) 3310, 2961, 2868, 1713, 1649, 1512, 1192 cm⁻¹; ¹H NMR (DMSO-*d*₆) δ 0.83 (s, 9H), 1.21 (t, 3H, *J* = 7.2 Hz), 1.40-1.69 (m, 8H), 2.01 (m, 4H), 2.80 (dd, 1H, *J* = 14.0, 8.1 Hz), 2.90 (dd, 1H, *J* = 13.2, 7.0 Hz), 3.54 (quin, 1H, *J* = 7.2 Hz), 4.09 (q, *2H, J =* 6.9 Hz), 4.28 (d, 1H, *J =* 9.6 Hz), 4.38 (m, 1H), 4.47 (m, 1H), 5.48 (dd, 1H, *J* = 15.6, 1.3 Hz), 6.64 (dd, 1H, *J* = 15.6, 5.3 Hz), 6.74 (bs, 1H), 7.00 (t, 2H, J = 8.8 Hz), 7.13 (bs, 1H), 7.20 (d, 1H, J = 8.5 Hz), 7.22 (d, 1H, J = 8.5 Hz), 7.88 (d, 1H, J = 9.2 Hz), 8.08 (d, 1H, *J* = 8.1 Hz), 8.18 (d, 1H, *J* = 7.7 Hz); HRMS calcd for C₃₀H₄₃N₄O₆SF+Cs 739.1942, found 739.1954. Anal. (C₃₀H₄₃N₄O₆SF) C, H, N, S.

### Example 41 - Preparation of Compound 177: Ethyl-2-Methyl-3-[Cyclopentylthiocarbonyl-L-α-(t-Butyl-Gly)-L-(4-F-Phe)-L-Gln]-E-Propenoate.

### Preparation of Intermediate Ethyl-2-Methyl-3-[Cyclopentylthiocarbonyl-L-α-(t-Butyl-Gly)-L-(4-F-Phe)-L-(Tr-Gln)]-E-Propenoate.

Using the procedure described in Example 1 for the preparation of ethyl-3-[CBZ-L-Leu-L-Phe-L-*Met* (sulfoxide)]-E-propenoate, ethyl-2-methyl-3-[cyclopentylthiocarbonyl-L-α-(t-butyl-Gly)-L-(4-F-Phe)-L-(Tr-*Gln*)]-E-propenoate was synthesized from cyclopentylthiocarbonyl-L-α-(t-butyl-Gly)-L-(4-F-Phe)-L-(Tr-glutaminal) (0.297 g, 0.38 mmol) and (carbethoxyethylidene)triphenylphosphorane (0.152 g, 0.42 mmol) to give 0.377 g of material contaminated with triphenylphosphine oxide after column chromatography on silica gel (gradient: 1-2.5% MeOH/CH₂Cl₂) which was used without further purification. white amorphous solid: IR(film) 3356, 3291, 3063, 2973, 2951, 1711, 1651, 1510, 1447, 1256, 1190, 752, 700 cm⁻¹; ¹H NMR (CDCl₃) δ 0.90 (s, 9H), 1.31 (t, 3H, *J* = 7.0 Hz), 1.51-1.83 (m, 11H, 2.17 (m, 2H), 2.28 (m, 2H), 2.75-3.02 (m, 2H), 3.66 (m, 1H), 4.16 (m, 3H), 4.45 (m, 1H), 4.60 (m, 1H), 6.30 (m, 2H), 6.58 (m, 1H), 6.78 (m, 1H), 6.88 (m, 2H), 6.98 (m, 3H), 7.20 (m, 15H). MS calcd for C₅₀H₅₉N₄O₆SF+Na 885, found 885.

### Preparation of Product - Ethyl-2-Methyl-3-[Cyclopentylthiocarbonyl-L-α-(t-Butyl-Gly)-L-(4-F-Phe)-L-Gln]-E-Propenoate.

Using the procedure described in Example 34 for the preparation of ethyl-3-[ethylthiocarbonyl-L-α-(t-butyl-Gly)-L-Phe-L-*Gln*]-E-propenoate, ethyl-2-methyl-3-[cyclopentylthiocarbonyl-L-α-(t-butyl-Gly)-L-(4-F-Phe)-L-*Gln*]-E-propenoate was synthesized from ethyl-2-methyl-3-[cyclopentylthiocarbonyl-L-α-(t-butyl-Gly)-L-(4-F-Phe)-L-(Tr-*Gln*)]-E-propenoate and isolated as a white solid after purification by column chromatography on silica gel using a gradient solvent system (1-5% MeOH/CH₂Cl₂) (55% yield; two steps from cyclopentylthiocarbonyl-L-α-(t-butyl-Gly)-L-(4-F-Phe)-L-(Tr-glutaminal): IR (KBr) 3326, 2951, 2868, 1713, 1645, 1553, 1510, 1260, 1194 cm⁻¹; ¹H NMR (DMSO-*d*₆) δ 0.83 (s, 9H), 1.22 (t, 3H, *J =* 7.0 Hz), 1.41-1.75 (m, 8H), 1.77 (m, 3H), 1.92 (m, 4H), 2.77 (dd, 1H, *J =* 13.8, 8.3 Hz), 2.85 ((dd, 1H, *J =* 13.6, 7.0 Hz), 3.55 (quin, 1H, *J =* 7.0 Hz), 4.12 (q, 2H, *J* = 7.1 Hz), 4.22 (d, 1H, *J =* 9.2 Hz), 4.38 (m, 1H), 4.45 (m, 1H), 6.24 (dd, 1H, *J =* 9.2, 1.5 Hz), 6.72 (bs, 1H), 6.96 (t, 2H, *J =* 8.8 Hz), 7.87 (d, 1H, *J =* 8.8 Hz), 8.03 (d, 1H, *J* = 8.1 Hz), 8.11 (d, 1H, *J =* 7.7 Hz); HRMS calcd for C₃₁H₄₅N₄O₆SF+Cs 753.2098, found 753.2084. Anal. (C₃₁H₄₅N₄O₆SF) C, H, N, S.

### Example 42 - Preparation of Compound 179: Ethyl-3-(Cyclopentylthiocarbony]-L-[S(O)-Me-Pen]-L-Phe-L-Gln)-E-Propenoate.

### Preparation of Product Ethyl-3-(Cyclopentylthiocarbonyl-L-[S(O)-Me-Pen]-L-Phe-L-Gln)-E-Propenoate

Using the procedure described in Example 36 for the preparation of ethyl-3-[cyclopentylthiocarbonyl-L-(S-Me-Pen)-L-Phe-L-*Gln*]-E-propenoate, ethyl-3-(cyclopentylthiocarbonyl-L-[S(O)-Me-Pen]-L-Phe-L-*Gln*)-E-propenoate was synthesized from ethyl-3-(cyclopentylthiocarbonyl-L-[S(O)-Me-Pen]-L-Phe-L-[Tr-*Gln*])-E-propenoate in 40% yield (3 steps from cyclopentylthiocarbonyl-L-(S-Me-Pen)-L-Phe-L-(Tr-glutaminol)): white amorphous solid: IR (KBr) 3302, 1662, 1541, 1458, 1205, 1138, 1028 cm⁻¹; ¹H NMR (DMSO-*d*_{*6*}) (mixture of diastereomers) δ 1.03 (s), 1.12 (s), 1.21 (t, 3H, *J =* 6.0 Hz), 1.42-1.76 (m), 2.0-2.21 (m), 2.34 (s), 2.42 (s), 2.80-2.87 (m), 2.93-3.11 (m), 3.47-3.60 (m), 4.10 (q, *J* = 6.0 Hz), 4.35-4.40 (m), 4.44-4.52 (m), 4.64 (d, *J* = 6.0 Hz), 5.58-5.62 (m), 6.60-6.70 (m), 6.75 (bs), 7.14-7.21 (m), 8.16-8.22 (m), 8.41 (d, *J =* 9.0 Hz), 8.54 (d, *J =* 9.0 Hz). HRMS, calcd for C₃₀H₄₄N₄O₇S₂+Cs 769.1706, found 769.1727.

### BIOCHEMICAL AND BIOLOGICAL EVALUATION

### Inhibition of Rhinovirus Protease

Stock solutions (50 mM, in DMSO) of various compounds were prepared; dilutions were in the same solvent. Recombinant Rhinovirus 3C proteases from serotypes 14, 16, 2 or 89 were prepared by the following standard chromatographic procedures: (1) ion exchange using Q Sepharose Fast Flow from Pharmacia; (2) affinity chromatography using Affi-Gel Blue from Biorad; and (3) sizing using Sephadex G-100 from Pharmacia. Assays contained 2% DMSO, 50 mM tris pH 7.6, 1 mM EDTA, a compound at the indicated concentrations, approximately 1µM substrate, and 50-100 nM protease. For Kᵢ determinations, the compound and the enzyme were preincubated for 10 minutes at 30 °C prior to addition of the substrate (substrate start). The k_{obs/I} values were obtained from reactions initiated by addition of enzyme rather than substrate. RVP activity is measured in the fluorescence resonance energy transfer assay. The substrate was (N-terminal) DABCYL-(Gly-Arg-Ala-Val-Phe-Gln-Gly-Pro-Val-Gyl)-EDANS. In the uncleaved peptide, the EDANS fluorescence was quenched by the proximal DABCYL moiety. When the peptide was cleaved, the quenching was relieved, and activity was measured as an increase in fluorescence signal. Data was analyzed using standard non linear fitting programs (Enzfit), and are shown Table 1.

**TABLE I**

| COMPOUND # | RVP | INHIB | k_{obs/I} (M-1 sec-1) |
|---|---|---|---|
| **1** | | 77 (50) | ND |
| **2** | | 6.6µM(Kᵢ) | ND |
| **3** | | 81(0.1) | 37,000 |
| | (16) | | 6,500 |
| | (89) | | 3,400 |
| | (2) | | 1,900 |
| **4** | | 49(0.5) | 790 |
| **5** | | 7.1µM(Kᵢ) | 221 |
| **6** | | 32µM(Kᵢ) | 350 |
| **7** | | 9.5µM(Kᵢ) | 2,400 |
| | (16) | 42(1) | ND |
| **8** | | 36µM(Kᵢ) | 61 |
| **9** | | 20(1) | 160 |
| **10** | | 55(5) | 270 |
| **11** | | 28µM(Kᵢ) | 20,000 |
| **12** | | 4.3µM(Kᵢ) | 2,200 |
| **13** | | 6.5µM(Kᵢ) | 54,000 |
| | (16) | | 9,000 |
| | (2) | | 2,400 |
| | (89) | | 5,500 |
| **14** | | NI | ND |
| **15** | | 55(50) | 27 |
| **16** | | 40(0.25) | 3,500 |
| **17** | | 1.25µM(Kᵢ) | 6,100 |
| **18** | | 15.3µM(Kᵢ) | 7,700 |
| **19** | | 35µM(Kᵢ) | 7,900 |
| **20** | | NI | ND |
| **21** | | 9.9µM(Kᵢ) | 2,100 |
| **22** | | 4.3µM(Kᵢ) | 1,300 |
| **23** | | 177µM(Kᵢ) | 120 |
| **24** | | ND | 500,000 |
| **25** | | 5.5µM(Kᵢ) | 3,700 |
| **26** | | 52(0.1) | 5,400 |
| **27** | | 20µM(Kᵢ) | 3,000 |
| **28** | | 57µM(Kᵢ) | 4,000 |
| **29** | | ND | ND |
| **30** | | 373µM(Kᵢ) | 430 |
| **31** | | 25(10) | 21 |
| **32** | | ND | 280 |
| **33** | | 24(10) | 33 |
| **34** | | 10(10) | 34 |
| **35** | | 16.5µM(Kᵢ) | 46,388 |
| | (2) | ND | 2,357 |
| | (16) | ND | 9,177 |
| **36** | | 15µM(Kᵢ) | 12,000 |
| **37** | | 18.8µM(Kᵢ) | 5,900 |
| **38** | | >50µM(Kᵢ) | 400 |
| **39** | | ND | 1,200 |
| **40** | | ND | 250 |
| **41** | | ND | 8,464 |
| **42** | | ND | 150,000 |
| **43** | | ND | 4,500 |
| **44** | | 12.6µM(Kᵢ) | 21,000 |
| **45** | | NI | ND |
| **46** | | ND | 120,000 |
| **49** | | ND | 460,000 |
| **51** | | ND | 310,000 |
| **52** | | ND | 15,000 |
| **53** | | ND | 11,320 |
| **56** | | 15µM(Kᵢ) | 5,624 |
| **59** | | 2.0µM(Kᵢ) | 200 |
| **60** | | 5.0µM(Kᵢ) | 575 |
| **61** | | ND | 125,940 |
| | (2) | ND | 14,000 |
| | (16) | ND | 25,000 |
| **62** | | ND | 600,000 |
| | (2) | ND | 600,000 |
| | (16) | ND | 300,000 |
| **65** | | 2.9µM(Kᵢ) | ND |
| **66** | | ND | 400,000 |
| **67** | | ND | 9,600 |
| **68** | | 15µM(Kᵢ) | 750 |
| **70** | | ND | 39,000 |
| **71** | | ND | 20,650 |
| **73** | | ND | 20,000 |
| | (2) | ND | 1,750 |
| | (16) | ND | 4,500 |
| **74** | | 2.4µM(Kᵢ) | |
| **75** | | ND | |
| **76** | | 30µM(Kᵢ) | ND |
| **77** | | 4.8µM(Kᵢ) | ND |
| **78** | | 7.0µM(Kᵢ) | |
| **79** | | ND | 13,900 |
| **80** | | ND | 200,000 |
| **81** | | ND | 124,000 |
| **82** | | 26µM(Kᵢ) | 7,300 |
| **83** | | 8.0µM(Kᵢ) | ND |
| **84** | | ND | 18,650 |
| **85** | | 3.0µM(Kᵢ) | 6,500 |
| **86** | | 4.0µM(Kᵢ) | 12,000 |
| **87** | | 6.0µM(Kᵢ) | 5,430 |
| **88** | | >30µM(Kᵢ) | 8,960 |
| **89** | | 5µM(Kᵢ) | 53,360 |
| | (16) | ND | 2,800 |
| **90** | | ND | 10,918 |
| | (16) | ND | 3,600 |
| **91** | | 10µM(Kᵢ) | 5,427 |
| **92** | | ND | 445 |
| **93** | | 30µM(Kᵢ) | 3,444 |
| **94** | | 1.5µM(Kᵢ) | 5,800 |
| **95** | | ND | <1000 |
| **96** | | ND | 300 |
| **97** | | ND | 12,900 |
| **98** | | ND | 91 |
| **99** | | 10 (50) | ND |
| **100** | | ND | 1,200 |
| **101** | | ND | 11,288 |
| **102** | | 12µM(Kᵢ) | 3,845 |
| **103** | | ND | 29,200 |
| | (2) | ND | 1,106 |
| | (16) | ND | 3,354 |
| **104** | | 2.5µM(Kᵢ) | 8,000 |
| | (16) | 1.5µM(Kᵢ) | ND |
| **105** | | ND | 1,200 |
| **106** | | 2.0µM(Kᵢ) | 280,000 |
| | (2) | ND | 28,400 |
| | (16) | ND | 75,000 |
| **107** | | 13.5µM(Kᵢ) | 3,655 |
| **108** | | ND | 4,694 |
| **109** | | ND | 1,348 |
| **110** | | ND | 9,072 |
| **111** | | 5.0µM(Kᵢ) | 2,065 |
| **112** | | 13µM(Kᵢ) | 6,800 |
| **113** | | ND | 8,877 |
| **114** | | ≥1.0µM(Kᵢ) | 82,320 |
| | (2) | ND | 1,971 |
| **115** | | 11µM(Kᵢ) | 4,485 |
| **116** | | ND | 23,670 |
| **117** | | ND | 18,760 |
| **118** | | 39µM(Kᵢ) | 1,448 |
| **119** | | 5.0µM(Kᵢ) | 69,800 |
| **120** | | 6.0µM(Kᵢ) | 91,300 |
| | (2) | ND | 8,900 |
| | (16) | ND | 20,034 |
| **121** | | 12µM(Kⱼ) | 238 |
| **122** | | ND | 1,252 |
| **123** | | ND | 890 |
| **124** | | ND | 1,000 |
| **125** | | ND | >500,000 |
| **126** | | ND | 29,000 |
| **127** | | ND | 28,347 |
| **128** | | ND | 22,691 |
| **129** | | ND | 230,000 |
| **130** | | 30-40nM(Kᵢ) | ND |
| **131** | | NI | NI |
| **132** | | 10µM(Kᵢ) | 10,800 |
| **133** | | ND | 9,600 |
| **134** | | ND | 1,769 |
| **135** | | ND | 16,270 |
| | (2) | ND | 671 |
| | (16) | ND | 3,465 |
| **136** | | ND | 4,210 |
| **137** | | ND | 2,344 |
| | (2) | ND | 643 |
| | (16) | ND | 1,157 |
| **138** | | 20µM(Kᵢ) | 1,769 |
| **139** | | ND | 43,140 |
| | (2) | ND | 691 |
| | (16) | ND | 1,259 |
| **140** | | ND | 7,122 |
| **141** | | ND | 2,309 |
| **142** | | ND | 2,929 |
| **143** | | ND | 2,963 |
| **144** | | ND | ND |
| **145** | | 10-20µM(Kᵢ) | ND |
| **146** | | ND | 62,500 |
| | (2) | ND | 7,790 |
| | (16) | ND | 16,900 |
| **147** | | ND | 18,600 |
| | (2) | ND | 1,000 |
| | (16) | ND | 4,290 |
| **148** | | 1.0µM(Kᵢ) | 57,000 |
| | (2) | ND | 8,300 |
| | (16) | ND | 14,800 |
| **149** | | ND | 39,940 |
| | (2) | ND | 2;840 |
| | (16) | ND | 7,700 |
| **150** | | ND | 573 |
| **151** | | >4.8µM(Kᵢ) | 39,750 |
| **152** | | 3.2µM(Kᵢ) | 38,900 |
| **153** | | 1.4µM(Kᵢ) | 141,200 |
| | (2) | ND | 13,350 |
| | (16) | ND | 30,650 |
| **154** | | 1.1µM(Kᵢ) | 78,900 |
| | (2) | ND | 5,400 |
| | (16) | ND | 13,900 |
| **155** | | 4.2mM(Kᵢ) | 59,425 |
| | (2) | ND | 1,390 |
| | (16) | ND | 5,250 |
| **157** | | 6.0µM(Kᵢ) | 161,500 |
| | (2) | ND | 9,700 |
| | (16) | ND | 30,800 |
| **158** | | 17µM(Kᵢ) | 22,600 |
| | (2) | ND | 2,200 |
| | (16) | ND | 6,400 |
| **159** | | 0.5µM(Kᵢ) | 35,000 |
| | (2) | ND | 2,500 |
| | (16) | ND | 6,500 |
| **160** | | ND | 312,000 |
| | (2) | ND | 26,710 |
| | (16) | ND | 50,000 |
| **161** | | ND | 1,086,000 |
| | (2) | ND | 200,000 |
| | (16) | ND | 126,000 |
| **162** | | ND | 800,000 |
| | (2) | ND | 150,000 |
| | (16) | ND | 80,000 |
| **163** | | 3.6µM(Kᵢ) | 9,800 |
| **164** | | ND | 155,500 |
| **165** | | ND | 97,000 |
| | (2) | ND | 5,600 |
| | (16) | ND | 20,200 |
| **166** | | ND | 40,900 |
| | (2) | ND | 3,500 |
| | (16) | ND | 7,700 |
| **167** | | ND | 165,400 |
| | (2) | ND | 10,700 |
| | (16) | ND | 42,100 |
| **168** | | ND | 37,800 |
| **169** | | ND | 800 |
| **170** | | ND | 85,300 |
| | (2) | ND | 8,400 |
| | (16) | ND | 30,000 |
| **171** | | ND | 21,200 |
| | (2) | ND | 830 |
| | (16) | ND | 3,250 |
| **172** | | ND | 31,700 |
| | (2) | ND | 2,000 |
| | (16) | ND | 6,000 |
| **173** | | ND | 1,000,000 |
| | (2) | ND | 113,000 |
| | (16) | ND | 185,000 |
| **174** | | ND | 800,000 |
| **175** | | ND | 124,000 |
| **176** | | 0.48µM(Kᵢ) | 240,000 |
| **177** | | ND | 80,300 |
| **178** | | ND | 286,300 |
| **179** | | 0.36µM(Kᵢ) | 300,000 |
| **180** | | 0.42µM(Kᵢ) | 300,000 |
| **181** | | ND | 1,000,000 |
| **182** | | ND | 114,360 |
| **183** | | 0.55µM(Kᵢ) | 500,000 |
| | (16) | ND | 60,000 |
| **184** | | ND | 59,900 |
| **185** | | ND | 600,000 |
| **186** | | ND | 950,000 |
| **187** | | NI | ND |
| **188** | | 0.16µM(Kᵢ) | 580,000 |
| **189** | | ND | 386,000 |
| **190** | | ND | 29,230 |

In the above table, all data is for RVP serotype-14 unless otherwise noted in parentheses. All strains of human rhinovirus (HRV) were purchased from American Type Culture Collection (ATCC), except for serotype 14, which was produced from the infectious cDNA clone constructed and supplied to us by Dr. Roland Rueckert at the Institute for Molecular Virology, University of Wisconsin, Madison, Wisconsin. The column designated INHIB represents the percent inhibition, with the concentration of the compound in µM indicated in parentheses, unless Kᵢ was assigned as designated by (Kᵢ), at 10 minute preincubation with 50 nM RVP prior to addition of substrate was used. The data in the column designated k_{obs/l} was measured from progress curves in enzyme start experiments. The designation NI indicates that no inhibition was obtained when 10 µM of a compound was used. The designation ND indicates that a value was not determined for that compound.

### Antirhinoviral HI-HeLa Cell Culture Assay

In the Cell Protection Assay, the ability of compounds to protect cells against HRV infection was measured by the XTT dye reduction method. This method is described in Weislow, O.S., R. Kiser, D.L. Fine, J. Bader, R.H. Shoemaker, and M.R. Boyd, *J. Natl*. *Cancer Inst*. 1989, 81, 577-586, which is incorporated herein by reference.

HI-HeLa cells were infected with HRV-14 at a multiplicity of infection (m.o.i.) of 0.13 (virus particles/cell) or mock-infected with medium only. Infected or mock-infected cells were resuspended at 8 x 10⁵ cells per mL and incubated with appropriate concentrations of compounds of formulas I and II. Two days later, XTT/PMS was added to test plates and the amount of formazan produced was quantified spectrophotometrically at 450/650 nm. The EC₅₀ was calculated as the concentration of compound that increased the percentage of formazan production in compound-treated, virus-infected cells to 50% of that produced by compound-free mock-infected cells. The 50% cytotoxic dose (CC₅₀) was calculated as the concentration of compound that decreased the percentage of formazan produced in compound-treated, mock-infected cells to 50% of that produced in compound-free, mock-infected cells. The therapeutic index (TI) was calculated by dividing the CC₅₀ by the EC₅₀.

All strains of human rhinovirus (HRV) for use in this assay were purchased from American Type Culture Collection (ATCC), except for HRV serotype-14, which was produced from the infectious cDNA clone, constructed and supplied to us by Dr. Roland Rueckert at the Institute for Molecular Virology, University of Wisconsin, Madison, Wisconsin. HRV stocks were propagated, and antiviral assays were performed in HI-HeLa cells (ATCC). Cells were grown in Minimal Essential Medium, available from Life Technologies, with 10% fetal bovine serum.

The compounds were tested against control compounds WIN 51711, WIN 52084, and WIN 54954, all obtained from Sterling-Winthrop Pharmaceuticals, and control compound Pirodavir, obtained from Janssen Pharmaceuticals.

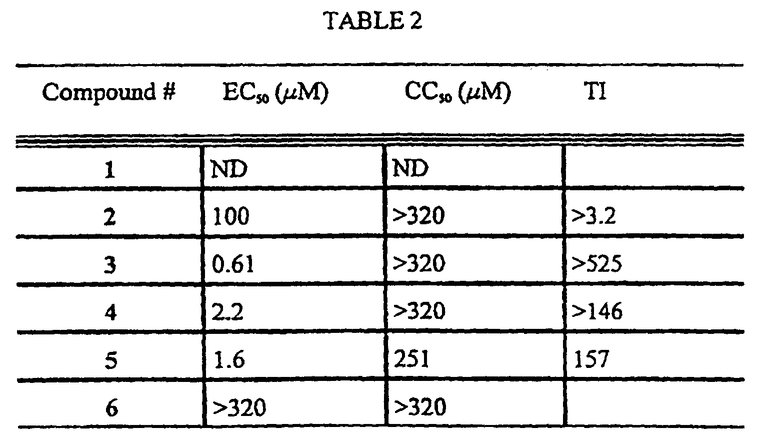

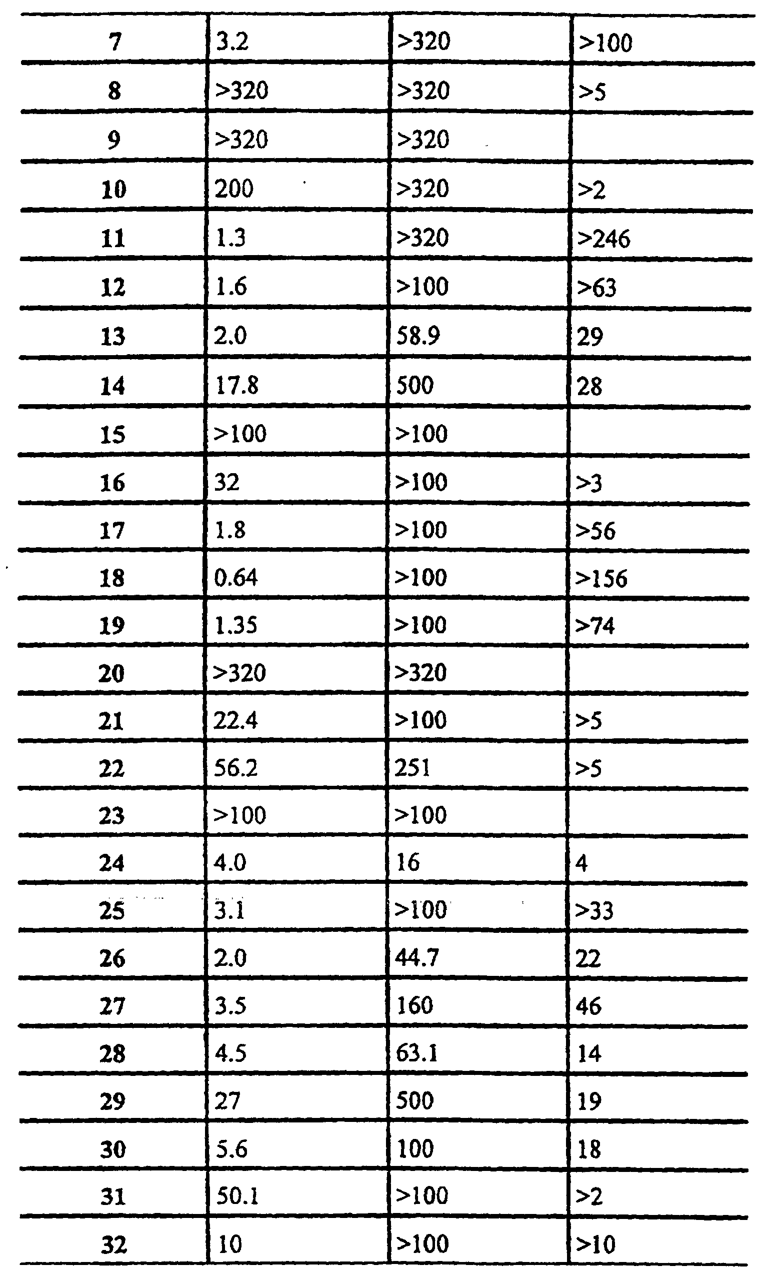

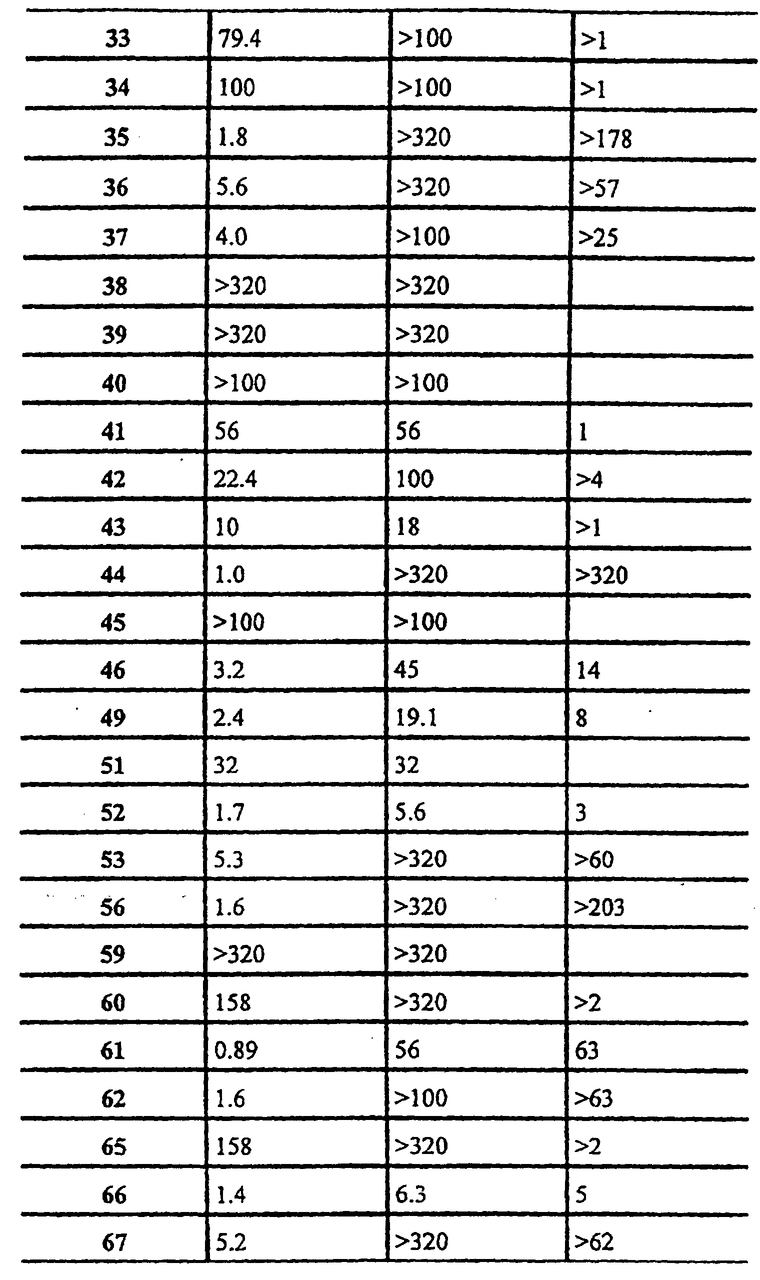

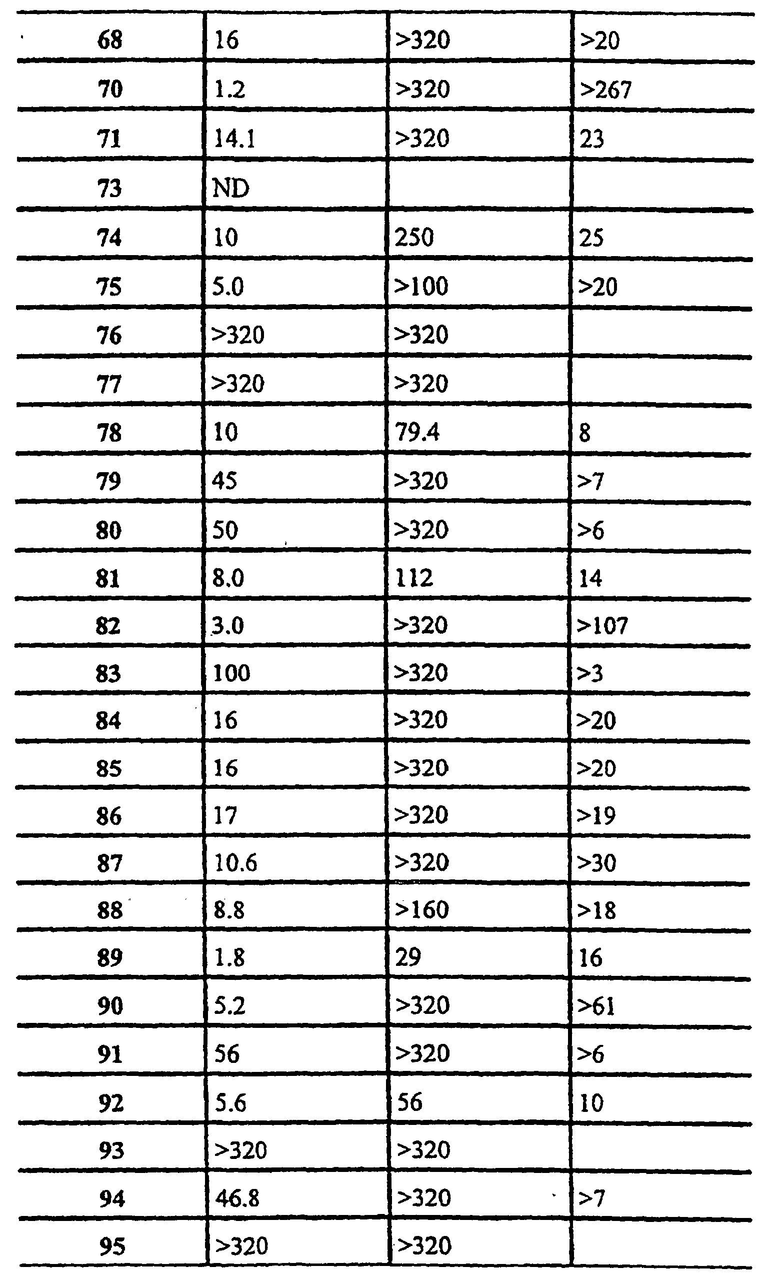

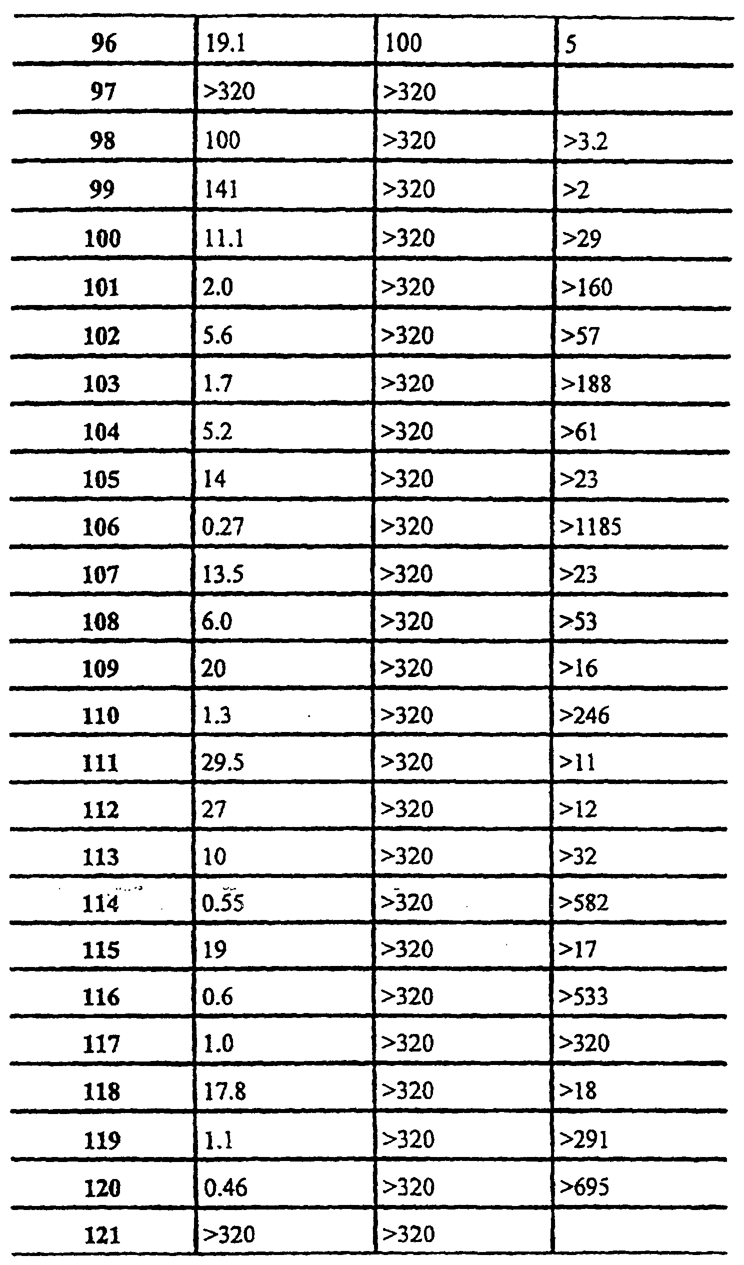

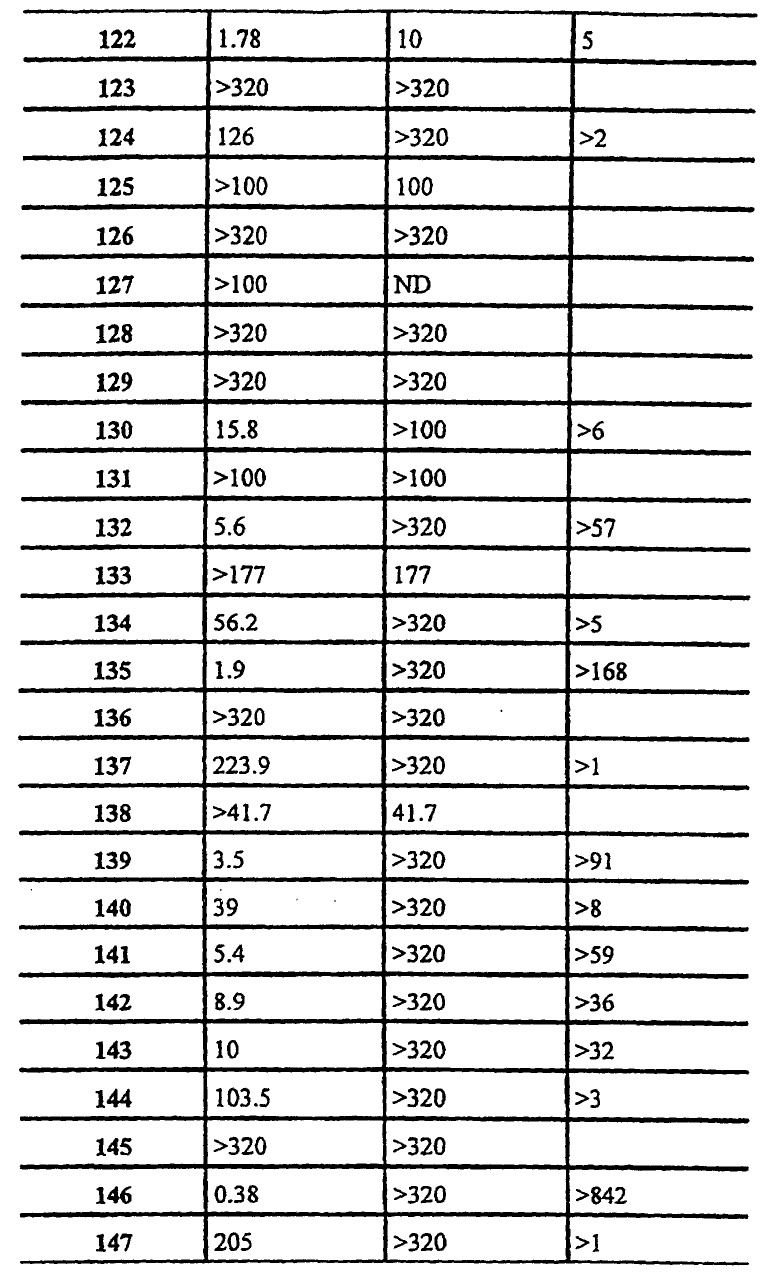

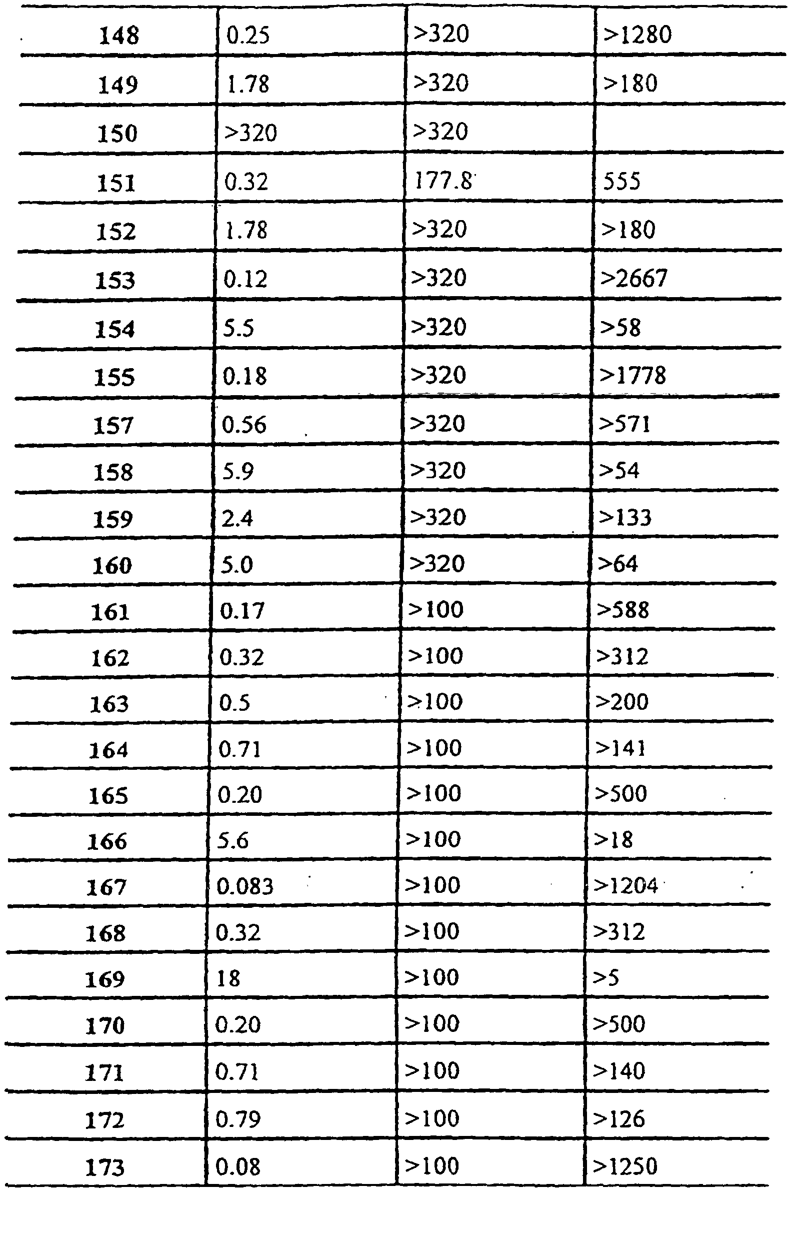

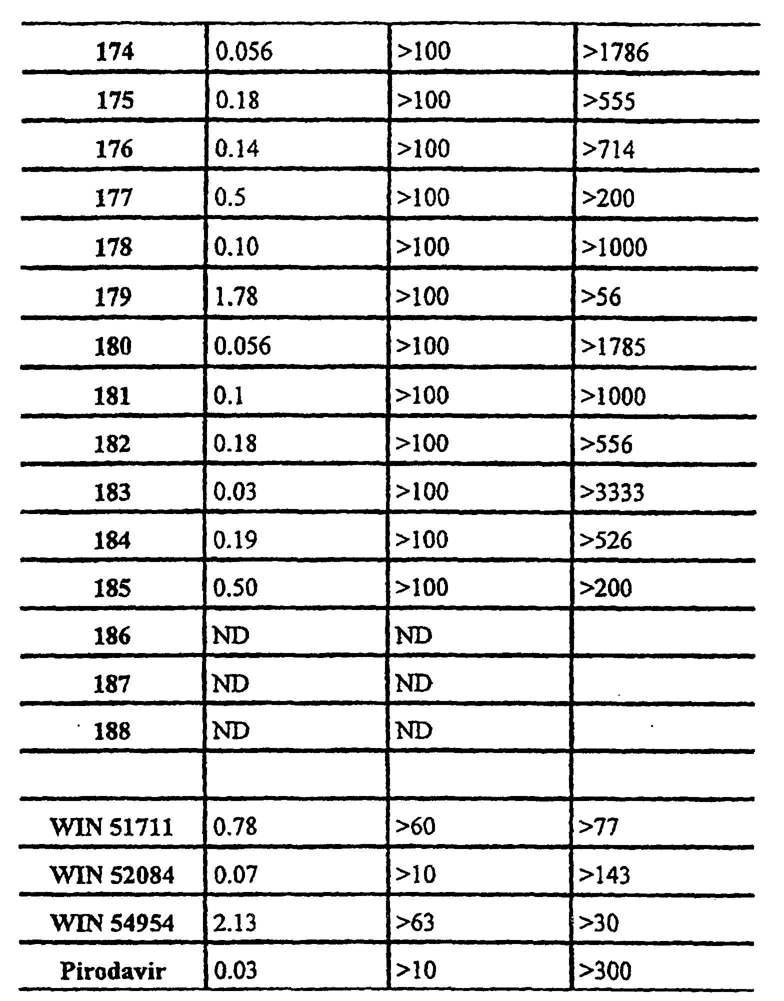

### Normal Human Bronchial Epithelial Cell Assay

Normal human bronchial cells were obtained from cadavers and cultured. The cells were plated at 2 x 10⁴ per well in a 96 well plate. They were allowed to adhere and grow for 24 hours in 200 µL of serum-free bronchial/tracheal epithelial cell growth medium at 37 °C with 5% CO₂. Human Rhinovirus-serotype 10 (HRV-10) was purchased from American Type Culture Collection (ATCC). To start the assay, the supernatant was removed, and HRV-10 at an m.o.i. of 10 (virus particles/cell) was added to each well along with the appropriate amount of compound of formula I or II. The plate was then incubated at 34 °C. After 3 hours the supernatant was removed, and 200 µL of media was added along with the same concentration of compound as used in the beginning of the assay. The plates were incubated for 3-4 days at 34 °C. To determine the amount of cell growth, an MTT assay (0.5 mgs/mL), as described in Mosmann, T.J. *J*. *Immunol. Methods* 1983, *65,* 55-63, which is incorporated herein by reference, was performed on the cells, and the plate was read at an optical density of 540 nm. The results of the assay are set forth in Table 3. The compounds were tested against control compound Pirodavir, obtained from Janssen Pharmaceuticals. The EC₅₀ was measured as described above for the HI-HeLa Cell Culture Assay.

**TABLE 3**

| Compound # | ED₅₀(µM) |
|---|---|
| **3** | 0.04 |
| **4** | 0.15 |
| **5** | 0.001 |
| **11** | 0.0007 |
| **12** | 0.004 |
| **13** | 0.0004 |
| **27** | 0.07 |
| **85** | 0.005 |
| **pirodavir** | 0.0075 |

### Anticoxsackieviral HI-HeLa Cell Culture Assay

The ability of compounds to protect calls against CVB-3 infection was measured by the XTT dye reduction method, which is described in Weislow, O.S., R.Kiser, D.L. Fine, J.Bader, R.H. Shoemaker, and M.R. Boyd, 1989, J. Natl. Cancer Inst. 81:577-586, which is incorporated herein by reference. Specifically, HI-HeLa cells were infected with CVB-3 at a multiplicity of infection (m.o.i.) of 0.08 or mock-infected with medium only. Infected or mock-infected cells were resuspended at 8 x 10⁵ cells per mL and incubated with appropriate concentrations of compound. One day later, XTT/PMS was added to test plates and the amount of formazan produced was quantified spectrophotometrically at 450/650 nm. The EC₅₀ was calculated as the concentration of compound that increased the percentage of formazan production in compound-treated, virus-infected cells to 50% of that produced by compound free, mock-infected cells. The 50% cytotoxic dose (CC₅₀) was calculated as the concentration of drug that decreased the percentage of formazan produced in compound treated, mock-infected cells to 50% of that produced in compound-free, mock-infected cells. The therapeutic index (TI) was calculated by dividing the CC₅₀ by the EC₅₀.

The Coxsackie strain B-3 (CVB-3) was purchased from American Type Culture Collection (ATCC). Virus stocks were propagated and antiviral assays were performed in Hi-HeLa cells (ATCC). Cells were grown in Minimal Essential Medium with 10% fetal bovine serum.

The compounds were tested against control compound WIN 54954, obtained from Sterling Winthrop Pharmaceuticals, and control compound Pirodavir, obtained from Janssen Pharmaceuticals.

**TABLE 4**

| Compound # | EC₅₀ (µM) | CC₅₀ (µM) | TI |
|---|---|---|---|
| **3** | 39.8 | >320 | >8 |
| **11** | 8.9 | >320 | >35 |
| **13** | >100 | >100 | |
| **21** | 158 | >320 | >2 |
| **23** | >100 | >100 | |
| **24** | 10 | 10 | 1 |
| **27** | 20 | 102.7 | >5 |
| **37** | 17.8 | >100 | >5.6 |
| **41** | >100 | >100 | |
| **WIN 54954** | >100 | >100 | |
| **Pirodavir** | >100 | >100 | |

## Claims

1. Compound of the formula (I): 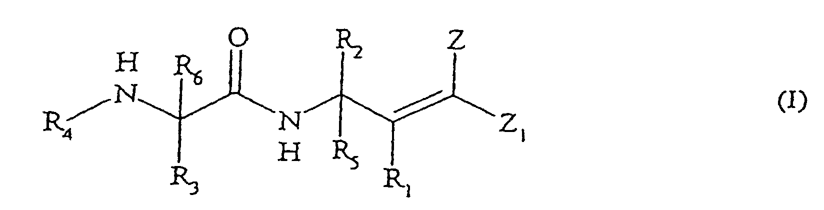 wherein
R₁ is H, F, an alkyl group, SH, an O-alkyl group, or an S-alkyl group;
R₂ and R₅ are independently selected from H, 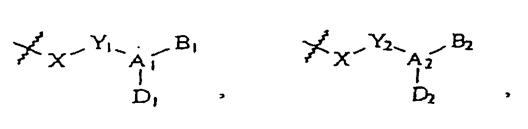 or an alkyl group, wherein said alkyl group is different from 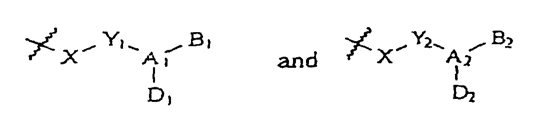 with the proviso that at least one of R₂ or R₅ must be 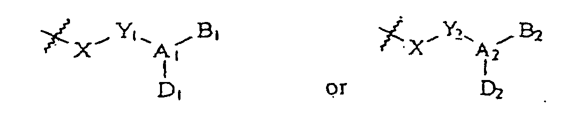 and wherein, when R₂ or R₅ is 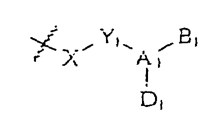 X is -CH or =CF and Y₁ is =CH or =CF
or X and Y₁ together with Q' form a three-membered ring in which Q' is -C(R₁₀)(R₁₁)- or -O-, X is -CH- or -CF-, and Y₁ is -CH-, -CF-, or -C(alkyl)-, where R₁₀ and R₁₁ independently are H, a halogen, or an alkyl group, or, together with the carbon atom to which they are attached, form a cycloalkyl group or a heterocycloalkyl group,
or X is -CH₂-, -CF₂-, -CHF-, or -S-,
and
Y₁ is -O-, -S-, -NR₁₂-, -C(R₁₃)(R₁₄)-, -C(O)-, -C(S)-, or -C(CR₁₃R₁₄)- wherein R₁₂ is H or alkyl, and R₁₃ and R₁₄ independently are H, F, or an alkyl group, or, together with the atom to which they are bonded, form a cycloalkyl group or a heterocycloalkyl group;
and A₁ is C, CH, CF, S, P, Se, N, NR₁₅, S(O), Se(O), P-OR₁₅, or P-NR₁₅R₁₆ wherein R₁₅ and R₁₆ independently are an alkyl group, a cycloalkyl group, a heterocycloalkyl group, an aryl group, or a heteroaryl group, or, together with the atom to which they are bonded, form a heterocycloalkyl group;
and D₁ is a moiety with a lone pair of electrons capable of forming a hydrogen bond;
and B₁ is H, F, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈ wherein R₁₇, R₁₈, and R₁₉ independently are H, an alkyl group, a cycloalkyl group, a heterocycloallcyl group, an aryl group, a heteroaryl group, or an acyl group, or, wherein any two of R₁₇, R₁₈, and R₁₉, together with the atom(s) to which they are bonded, form a heterocycioalkyl group;
and with the provisos that when D₁ is the moiety ≡N with a lone pair of electrons capable of forming a hydrogen bond, B₁ does not exist; and when A₁ is an sp³ carbon, B₁ is not -NR₁₇R₁₈ when D₁ is the moiety -NR₂₅R₂₆ with a lone pair of electrons capable of forming a hydrogen bond, wherein R₂₅ and R₂₆ are independently H, an alkyl group, a cycloalkyl group, a heterocycloalkyl group, an aryl group, or a heteroaryl group;
and wherein D₁-A₁-B₁ optionally forms a nitro group where A₁ is N;
and wherein, when R₂ or R₅ is 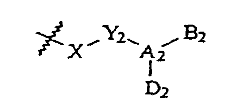 X is =CH or =CF and Y₂ is =C, =CH or =CF,
or X and Y₂ together with Q' form a three-membered ring in which Q' is -C(R₁₀)(R₁₁)- or -O-, X is -CH- or -CF-, and Y₂ is -CH-, -CF-, or -C(alkyl)-, where R₁₀ and R₁₁ independently are H, a halogen, or an alkyl group, or, together with the carbon atom to which they are attached, form a cycloalkyl group or a heterocycloalkyl group,
or X is -CH₂-, -CF₂-, -CHF-, or -S-,
and
Y₂ is -O-, -S-, -N(R'₁₂)-, -C(R'₁₃)(R'₁₄)-, -C(O)-, -C(S)-, or -C(CR'₁₃R'₁₄)- wherein R'₁₂ is H, an alkyl group, a cycloalkyl group, a heterocycloalkyl group, an aryl group, a heteroaryl group, -OR'₁₃, -NR'₁₃R'₁₄, -C(O)-R'₁₃, -SO₂R'₁₃, or -C(S)R'₁₃, and R'₁₃ and R'₁₄, independently are H, F, or an alkyl group, a cycloalkyl group, a heterocycloalkyl group, an aryl group, or a heteroaryl group or, together with the atom to which they are attached, form a cycloalkyl group or a heterocycloalkyl group;
and wherein any combination of Y₂, A₂, B₂, and D₂ forms a cycloalkyl group, a heterocycloalkyl group, an aryl group, or a heteroaryl group;
and A₂ is C, CH, CF, S, P, Se, N, NR₁₅, S(O), Se(O), P-OR₁₅, or P-NR₁₅R₁₆ wherein R₁₅ and R₁₆ independently are an alkyl group, a cycloalkyl group, a heterocycloalkyl group, an aryl group, or a heteroaryl group or, together with the atom to which they are bonded, form a heterocycloalkyl group;
and D₂ is a moiety with a lone pair of electrons capable of forming a hydrogen bond;
and B₂ is H, F, an alkyl group, a cycloalkyl group, a heterocycloalkyl group, an aryl group, a heteroaryl group, -OR₁₇, -SR₁₇, -NR₁₇R₁₈, -NR₁₉NR₁₇R₁₈, or -NR₁₇OR₁₈ wherein R₁₇, R₁₈, and R₁₉ independently are H, an alkyl group, a cycloalkyl group, a heterocycloalkyl group, an aryl group, a heteroaryl group, or an acyl group, or, wherein any two of R₁₇, R₁₈, and R₁₉, together with the atom(s) to which they are bonded, form a heterocycloalkyl group;
R₃ and R₆ are independently H, F, or an alkyl group;
R₄ is H, OH, an alkyl group, an oxo group, a cycloalkyl group, a heterocycloalkyl group, an aryl group, a heteroaryl group, an acyl group, a sulfonyl group, a mercapto group, an alkylthio group, an alkoxy group, a carboxy group, an amino group, an alkylamino group, a dialkylamino group, a carbamoyl group, an arylthio group or a heteroacrylthio group;
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR_{21,}R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃, or -C(S)NR₂₁NR₂₂R₂₃, wherein R₂₁, R₂₂, R₂₃, and R₂₄ are independently H, an alkyl group, a cycloalkyl group, a heterocycloalkyl group, an aryl group, a heteroaryl group, or acyl group, or a thioacyl group, or wherein any two of R₂₁, R₂₂, R₂₃, and R₂₄, together with the atom(s) to which they are bonded, form a heterocycloalkyl group;
or Z₁, as defined above, together with R₁, as defined above, and the atoms to which Z₁ and R₁ are bonded, form a cycloalkyl or heterocycloalkyl group,
or Z and Z₁, both as defined above, together with the atoms to which they are bonded, form a cycloalkyl or heterocycloalkyl group;
or a pharmaceutically acceptable salt, or solvate thereof;
wherein the terms alkyl group, cycloalkyl group, heterocycloalkylgroup, aryl group, heteroaryl group, acyl group, sulfonyl group, alkylthio group, alkoxy group, alkylamino group, dialkylamino goup, arylthio group and heteroarylthio group have the following meanings:
alkyl group: methyl, ethyl, propyl, isopropyl, butyl, isobutyl, t-butyl, ethenyl, pentenyl, butenyl, propenyl, ethynyl, butynyl, propynyl, pentynyl and hexynyl, unsubstituted or substituted by one substituent;
cycloalkyl group: non aromatic, monovalent monocyclic, bicyclic or tricyclic radical containing 3, 4, 5, 6, 7, 8, 9, 10, 11, 12, 13 or 14 carbon ring atoms, which may be saturated or unsaturated, unsubstituted or substituted by one substituent;
heterocycloalkyl group: non aromatic, monovalent, monocyclic, bicyclic or tricyclic radical, which is saturated or unsaturated, containing3, 4, 5, 6, 7, 8, 9, 10, 11, 12, 13, 14, 15, 16, 17 or 18 ring atoms which include 1, 2, 3, 4 or 5 heteroatoms selected from nitrogen, oxygen and sulphur, wherein the radical is unsubstituted or substituted by one substituent;
aryl group: aromatic , monovalent, monocyclic, bicyclic or tricyclic radical containing 6, 10, 14 or 18 carbon ring atoms, which may be unsubstituted or substituted by one substituent;
heteroaryl group: aromatic, monovalent, monocyclic, bicyclic or tricyclic radical containing 4, 5, 6, 7, 8, 9, 10, 11, 12, 13, 14, 15, 16, 17 or 18 ring atoms including 1, 2, 3, 4 or 5 heteroatoms selected from nitrogen, oxygen and sulphur, which may be unsubstituted or substituted by one substituent;
acyl group: -C(O)-R radical wherein R is a substituent;
sulfonyl group: -SO₂R radical,wherein R is a substituent;
alkylthio group: -SR radical, wherein R is an alkyl group;
alkoxy group: OR radical, wherein R is an alkyl group;
alkylamino group: -NHR radical, wherein R is an alkyl group;
dialkylamino group: -NRₐR_{b} radical, wherein Rₐ and R_{b} independently are an alkyl group;
arylthio group: -SR_{c} radical, wherein R_{c} is an aryl group;
heteroarylthio group: -SR_{d} radical, wherein R_{d} is a heteroaryl group;
substituent: hydroxyl group, oxo group, alkyl group, acyl group, sulfonyl group, mercapto group, alkylthio group, alkoxy goup, cycloalkyl group, heterocycloalkyl group, aryl group, heteroaryl group, carboxy group, amino group, alkylamino group, dialkylamino group, carbomoyl group, aryloxygroup, heteroaryloxy group, arylthio group, heteroarylthio group, wherein aryloxy group is the radical -OR_{c} wherein R_{c} is an aryl group and wherein heteroaryloxy groupis the radical-OR_{d} wherein R_{d} is a heteroaryl group.

2. A compound of claim 1 wherein R₁ is H or F, or a pharmaceutically acceptable salt, or solvate thereof.

3. A compound of claim 1 wherein R₄ is an acyl group or a sulfonyl group, or a pharmaceutically acceptable salt, or solvate thereof.

4. A compound of claim 1, wherein at least one of R₂ or R₅ is 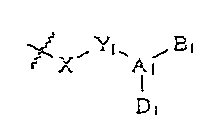 or a pharmaceutically acceptable salt, or solvate thereof.

5. A compound according to claim 4, wherein D₁ is -OR₂₅, =O, =S, ≡N, =NR₂₅, or -NR₂₅R₂₆, wherein R₂₅ and R₂₆ are independently H, an alkyl group, a cycloalkyl group, a heterocycloalkyl group, an aryl group, or a heteroaryl group, or, together with the nitrogen atom to which they are bonded, form a heterocycloalkyl group; or a pharmaceutically acceptable salt, or solvate thereof.

6. A compound according to claim 5 wherein D₁ is =O; or a pharmaceutically acceptable salt, or solvate thereof.

7. A compound according to claim 4, wherein A₁ is C, CH, S, or S(O); or a pharmaceutically acceptable salt, or solvate thereof.

8. A compound according to claim 7 wherein A₁ is C; or a pharmaceutically acceptable salt, or solvate thereof

9. A compound according to claim 4 wherein B₁ is NR₁₇R₁₈,
wherein R₁₇ and R₁₈ are independently H, an alkyl group, a cycloalkyl group, a heterocycloalkyl group, an aryl group, a heteroaryl group, or an acyl group, or
wherein R₁₇ and R₁₈, together with the atom(s) to which they are bonded, form a heterocycloalkyl group;
or a pharmaceutically acceptable salt, or solvate thereof.

10. A compound according to claim 1, wherein at least one of R₂ or R₅ is 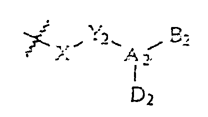 or a pharmaceutically acceptable salt, or solvate thereof.

11. A compound according to claim 10, wherein D₂ is -OR₂₅, =O, =S, ≡N, =NR₂₅, or -NR₂₅R₂₆, wherein R₂₅ and R₂₆ are independently H, an alkyl group, a cycloalkyl group, a heterocycloalkyl group, an aryl group, or a heteroaryl group, or, together with the atom to which they are bonded, form a heterocycloalkyl group; or a pharmaceutically acceptable salt, or solvate thereof.

12. A compound according to claim 11, wherein D₂ is =O; or a pharmaceutically acceptable salt, or solvate thereof.

13. A compound according to claim 10, wherein A₂ is C, CH, S, or S(O); or a pharmaceutically acceptable salt, or solvate thereof.

14. A compound according to claim 13, wherein A₂ is C; or a pharmaceutically acceptable salt, or solvate thereof.

15. A compound according to claim 10 wherein B₂ is NR₁₇R₁₈,
wherein R₁₇ and R₁₈ are independently H, an alkyl group, a cycloalkyl group, a heterocycloalkyl group, an aryl group, a heteroaryl group, or an acyl group, or
wherein R₁₇ and R₁₈, together with the atom(s) to which they are bonded, form a heterocycloallcyl group;
or a pharmaceutically acceptable salt, or solvate thereof.

16. A compound according to claim 1, wherein A₁ is C, CH, S, or S(O) and wherein A₂ is C, CH, S, or S(O); or a pharmaceutically acceptable salt, or solvate thereof.

17. A compound according to claim 1 wherein Z and Z₁ are independently H, an aryl group, or a heteroaryl group, -C(O)R₂₁, -CO₂R₂₁, -CN, -C(O)NR_{21,}R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃, or -C(S)NR₂₁NR₂₂R₂₃;
wherein R₂₁, R₂₂, and R₂₃ are independently H, an alkyl group, a cycloalkyl group, a heterocycloalkyl group, an aryl group, a heteroaryl group, an acyl group, or a thioacyl group, or wherein any two of R₂₁, R₂₂, and R₂₃, together with the atom(s) to which they are bonded, form a heterocycloalkyl group,
or Z and Z₁, together with the atoms to which they are bonded, form a heterocycloalkyl group;
or a pharmaceutically acceptable salt, or solvate thereof.

18. A compound according to formula (I) of the formula II; wherein the definitions for the substituents are as defined in claim 1 : 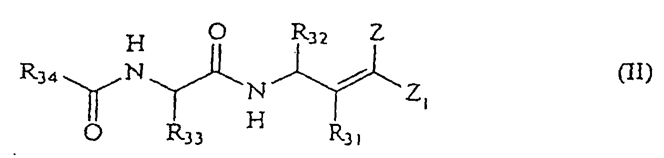 and wherein further
R₃₁ is H, F or an alkyl group;
R₃₂ is selected from one of the following moieties: 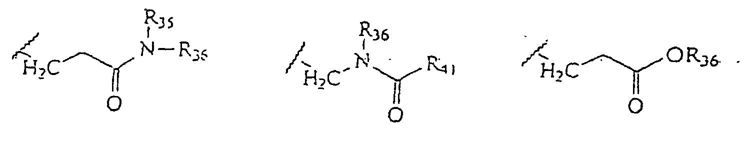 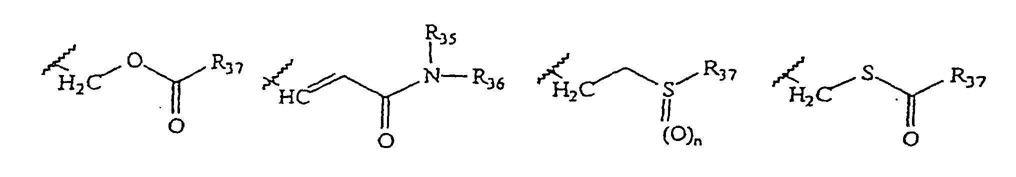 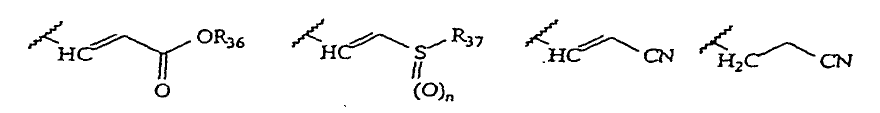 wherein
R₃₅ is H, an alkyl group, an aryl group, -OR₃₈, or -NR₃₈R₃₉, and
R₃₆ is H or an alkyl group,
or R₃₅ and R₃₆, together with the atom(s) to which they are attached, form a heterocycloalkyl group or a heteroaryl group;
R₄₁ is H, an alkyl group, an aryl group, -OR₃₈, -SR₃₉, -NR₃₈R₃₉, -NR₄₀NR₃₈R₃₉, or -NR₃₈OR₃₉, or R₄₁ and R₃₆, together with the atom to which they are attached, form a heterocycloalkyl group, and
R₃₇ is an alkyl group, an aryl group, or -NR₃₈R₃₉;
wherein R₃₈, R₃₉, and R₄₀ independently are H, an alkyl group, a cycloalkyl group, a heterocycloalkyl group, an aryl group, a heteroaryl group, or an acyl group, or, wherein any two of R₃₈, R₃₉, and R₄₀, together with the atom(s) to which they are bonded, form a heterocycloalkyl group,
n is 0, 1 or 2;
R₃₃ is H or an alkyl group;
R₃₄ is an alkyl group, a cycloalkyl group, a heterocycloalkyl group, an aryl group, a heteroaryl group, an O-alkyl, an O-cycloalkyl group, an O-heterocycloalkyl group, an O-aryl group, an O-heteroaryl group, an S-alkyl group, an NH-alkyl group, an NH-aryl group, an N,N-dialkyl group, or an N,N-diaryl group; and
Z and Z₁ are independently H, F, an alkyl group, a cycloalkyl group, a heterocycloalkyl group, an aryl group, a heteroaryl group, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂,-PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃, or -C(S)NR₂₁NR₂₂R₂₃,
wherein R₂₁, R₂₂, R₂₃, and R₂₄ are independently H, an alkyl group, a cycloalkyl group, a heterocycloalkyl group, an aryl group, a heteroaryl group, an acyl group, or a thioacyl group, or wherein any two cf R₂₁, R₂₂, R₂₃, and R₂₄, together with the atom(s) to which they are bonded, form a heterocycloalkyl group,
or Z and Z₁, together with the atoms to which they are bonded, form a heterocycloalkyl group;
or a pharmaceutically acceptable salt, or solvate thereof.

19. A compound according to claim 18 wherein Z and Z₁ are independently H, an aryl group, or a heteroaryl group, -C(O)R₂₁, -CO₂R₂₁, -CN, -C(O)NR_{21,}R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃, or -C(S)NR₂₁NR₂₂R₂₃;
wherein R₂₁, R₂₂, and R₂₃ are independently H, an alkyl group, a cycloalkyl group, a heterocycloalkyl group, an aryl group, a heteroaryl group, or an acyl group, or
wherein any two of R₂₁, R₂₂, and R₂₃, together with the atom(s) to which they are bonded, form a heterocycloalkyl group,
or Z and Z₁, together with the atoms to which they are bonded, form a heterocycloalkyl group;
or a pharmaceutically acceptable salt, or solvate thereof.

20. A compound according to claim 18 wherein R₃₂ is selected from one of the following moieties: 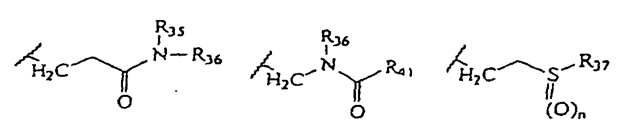 wherein
R₃₅ is H, an alkyl group, an aryl group, -OR₃₈, -SR₃₉, -NR₃₈R₃₉, -NR₄₀NR₃₈R₃₉, or -NR₃₈OR₃₉, and
R₃₆ is H or an alkyl group,
or R₃₅ and R₃₆, together with the atom to which they are attached, form a heterocycloalnyl group or a heteroaryl group;
R₃₇ is an alkyl group, an aryl group, or -NR₃₈R₃₉;
wherein R₃₈, R₃₉, and R₄₀ independently are H, an alkyl group, a cycloalkyl group, a heterocycloalkyl group, an aryl group, a heteroaryl group, or an acyl group, or, wherein any two of R₃₈, R₃₉, and R₄₀, together with the atom(s) to which they are bonded, form a heterocycloalkyl group,
n is 0, 1 or 2;
or a pharmaceutically acceptable salt, or solvate thereof.

21. A compound according to formula (I) of the formula III: 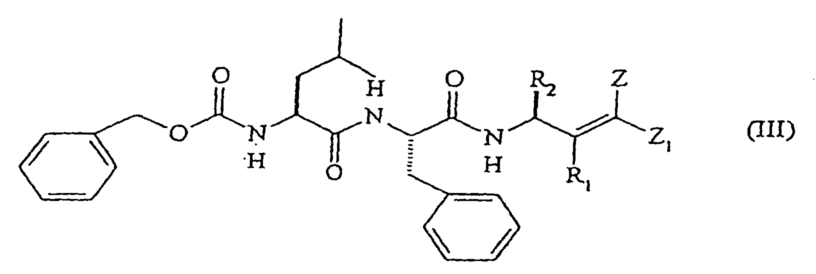 wherein
R₂ is CH₂CH₂C(O)NHCPh₃, R₁ is H, Z is H, and Z₁ is CO₂CH₂CH₃,
R₂ is CH₂CH₂C(O)NH₂, R₁ is H, Z is H, and Z₁ is CO₂CH₂CH₃,
R₂ is CH₂NHC(O)CH₃; R₁ is H, Z is H, and Z₁ is CO₂CH₂CH₃,
R₁ is H, Z is H, and Z₁ is CO₂CH₂CH₃, and R₂ is 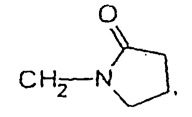 R₂ is CH₂CH₂C(O)NH₂, R₁ is H, Z is CO₂CH₃, and Z₁ is H,
R₂ is CH₂CH₂C(O)NH₂, R₁ is H, Z is H, and Z₁ is CO₂CH₃,
R₂ is CH₂CH₂S(O)CH₃, R₁ is H, Z is H, and Z₁ is CO₂CH₂CH₃,
R₂ is CH₂CH₂C(O)NH₂, R₁ is H, Z is H, and Z₁ is C(O)CH₃,
R₂ is CH₂CH₂C(O)NH₂, R₁ is H, Z is H, and Z₁ is CN,
R₂ is CH₂NHC(O)NH₂, R₁ is H, Z is H, and Z₁ is CO₂CH₂CH₃,
R₂ is CH₂CH₂C(O)NH₂, R₁ is H, Z is H, and Z₁ is CO₂CH(CH₃)₂,
R₂ is CH₂CH₂C(O)NH₂, R₁ is H, Z is H, and Z₁ is 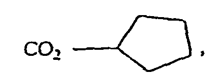 R₂ is CH₂CH₂C(O)NH₂, R₁ is H, Z is H, and Z₁ is 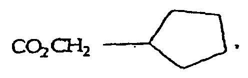 R₂ is CH₂CH₂C(O)NH₂, R₁ is H, Z is H, and Z₁ is 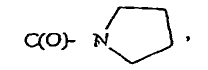 R₂ is CH₂CH₂C(O)NH₂, R₁ is H, Z is H, and Z₁ is C(O)N(CH₃)₂,
R₂ is CH₂CH₂C(O)NH₂; R₁ is H, Z is H, and Z₁ is C(O)Ph,
R₂ is CH₂CH₂C(O)NH₂; R₁ is H, Z is H, and Z₁ is 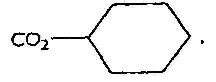 R₂ is CH₂CH₂C(O)NH₂, R₁ is H, Z is H, and Z₁ is 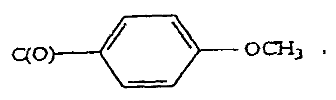 R₂ is CH₂CH₂C(O)NH₂, R₁ is H, Z is H, and Z₁ is 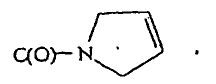 R₂ is CH₂CH₂C(O)NH₂, R₁ is H, Z is H, and Z₁ is 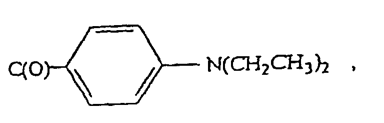 R₂ is CH₂CH₂C(O)NH₂, R₁ is H, Z is H, and Z₁ is CH₂Cl, or
R₂ is CH₂CH₂C(O)NH₂, R₁ is H, Z is H, and Z₁ is 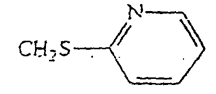 or a pharmaceutically acceptable salt, or solvate thereof.

22. A compound according to formula (I) of the formula IV: 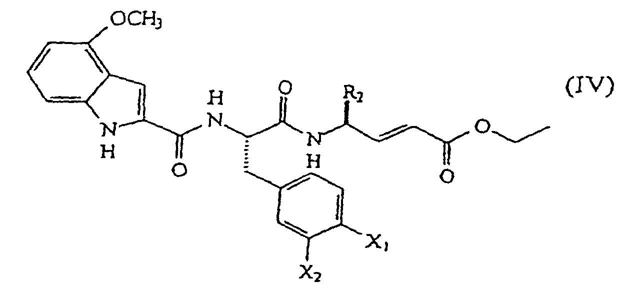 wherein
R₂ is CH₂CH₂C(O)NH₂, X₁ is H, F, or Cl, and X₂ is H, F, or Cl; or a pharmaceutically acceptable salt, or solvate thereof.

23. A compound according to claim 22 wherein X₁ is Cl and X₂ is H; X₁ is F and X₂ is H; or X₁ is H and X₂ is F; or a pharmaceutically acceptable salt, or solvate thereof.

24. A compound according to formula (I) of the formula V: 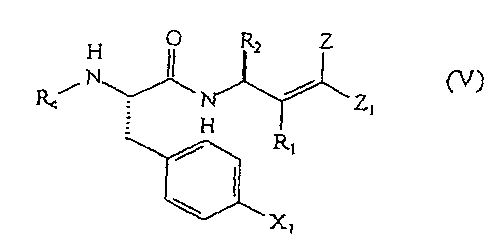 wherein:
R₄ is PhCH₂OC(O), X₁ is H, R₂ is CH₂CH₂C(O)NH₂, R₁ is H, Z is H, and Z₁ is CO₂CH₂CH₃; or
R₄ is CH₃CH₂CH₂SO₂, X₁ is H, R₂ is CH₂CH₂C(O)NH₂, R₁ is H, Z is H, and Z₁ is CO₂CH₂CH₃; or
R₄ is PhCH₂SO₂, X₁ is H, R₂ is CH₂CH₂C(O)NH₂, R₁ is H, Z is H, and Z₁ is CO₂CH₂CH₃; or
R₄ is CH₃CH₂SO₂, X₁ is H, R₂ is CH₂CH₂C(O)NH₂, R₁ is H, Z is H, and Z₁ is CO₂CH₂CH₃; or
R₄ is PhSO₂, X₁ is H, R₁ is CH₂CH₂C(O)NH₂, R₁ is H, Z is H, and Z₁ is CO₂CH₂CH₃;
or a pharmaceutically acceptable salt, or solvate thereof.

25. A compound according to formula (I) of the formula VI or VII: 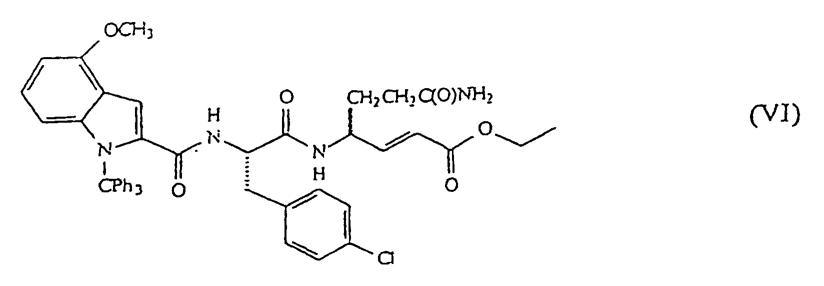 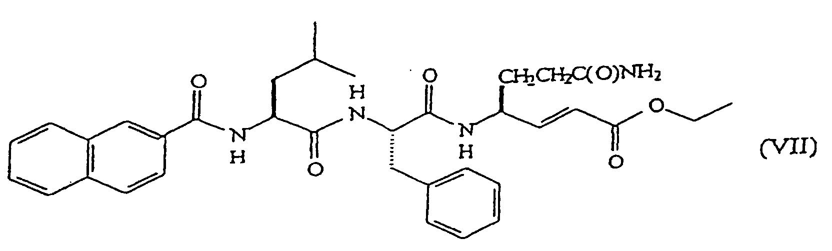 or a pharmaceutically acceptable salt, or solvate thereof.

26. A compound according to formula (I) of the formula VIII: 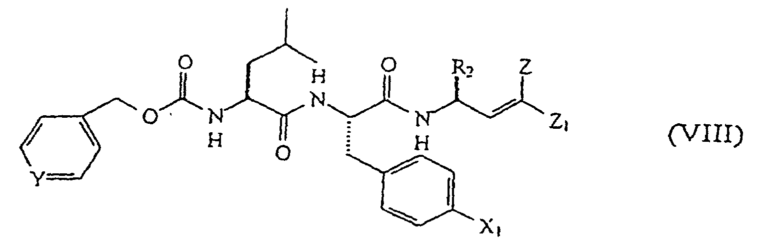 wherein
X₁ is F, R₂ is CH₂CH₂C(O)NH₂, Y is CH, Z is H, and Z₁ is CO₂CH₂CH₃; or
X₁ is H, R₂ is CH₂CH₂C(O)NH₂, Y is N, Z is H, and Z₁ is CO₂CH₂CH₃; or
X₁ is H, R₂ is CH₂CH₂C(O)NH₂, Y is CH, Z is H, and Z₁ is C(O)N(CH₃)OCH₃;
or a pharmaceutically acceptable salt, or solvate thereof.

27. A compound according to formula (I) of the formula III: 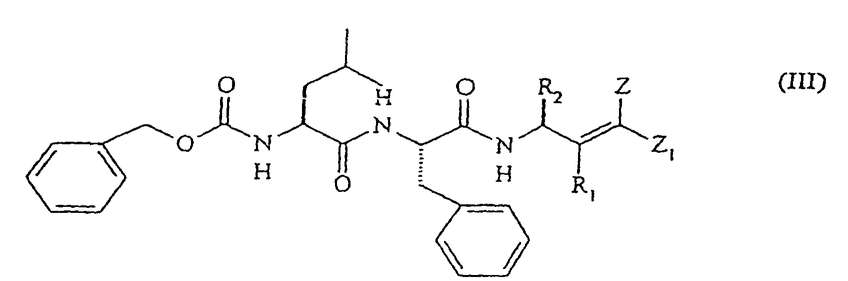 wherein R₂ is CH₂CH₂C(O)NH₂, R₁ is H, Z is CH₃ and Z₁ is CO₂CH₂CH₃,
R₂ is CH₂CH₂C(O)NH₂, R₁ is H, and Z and Z₁ together form 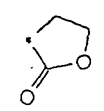 or wherein R₂ is CH₂CH₂C(O)NH₂, R₁ is H, Z is H, and Z₁ is selected from: 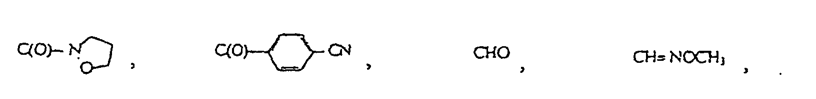 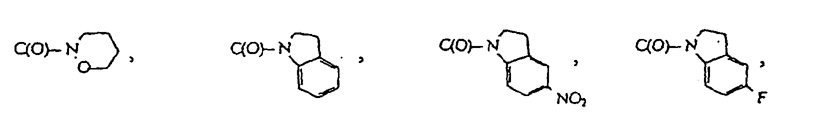 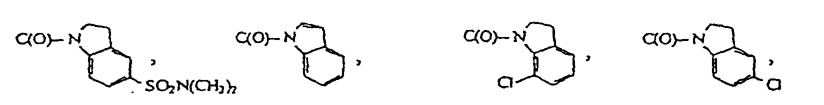 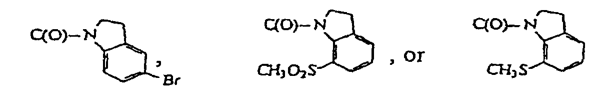 or a pharmaceutically acceptable salt, or solvate thereof.

28. A compound according to formula (I) of the formula XIV: 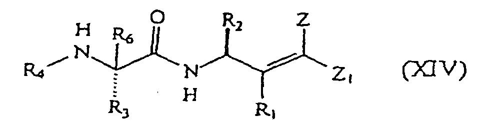 wherein R₆ is H, R₁ is H, R₃ is CH₂CH₂C(O)NH₂, Z is H, Z₁ is CO₂CH₂CH_{3,} and
R₃ is CH₂Ph and R₄ is 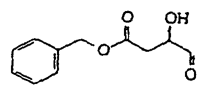 R₃ is 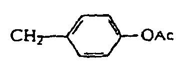 and R₄ is 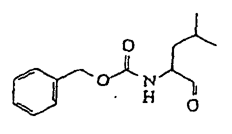 R₂ is CH₂Ph and R₄ is 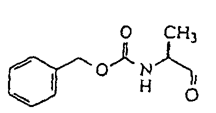 R₃ is CH₂Ph and R₄ is 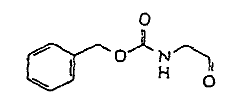 R₃ is 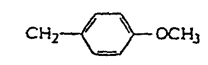 and R₄ is 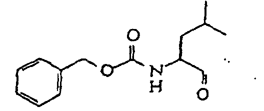 R₃ is CH₂Ph and R₄ is 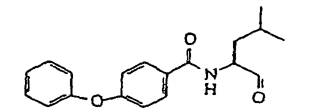 R₃ is 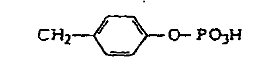 and R₄ is 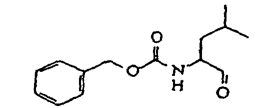 R₃ is CH₂Ph and R₄ is 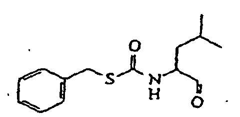 R₃ is CH₂Ph and R₄ is 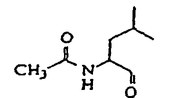 R₃ is CH₂CH₃ and R₄ is 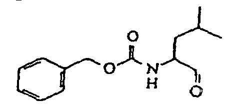 R₃ is CH₃ and R₄ is 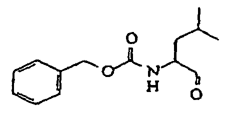 R₃ is CH₂Ph and R₄ is 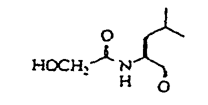 R₃ is 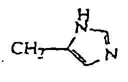 and R₄ is 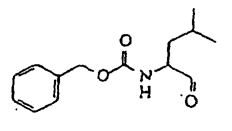 R₃ is 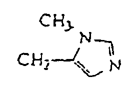 and R₄ is 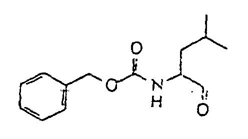 R₃ is 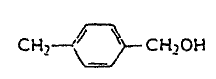 and R₄ is 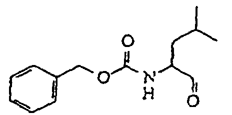 R₃ is CH₂Ph and R₄ is 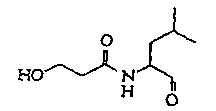 R₃ is CH₂Ph and R₄ is 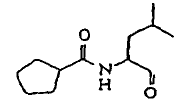 R₃ is CH₂Ph and R₄ is 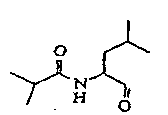 R₃ is CH₂Ph and R₄ is 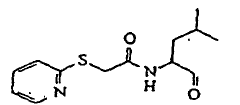 R₃ is CH₂Ph and R₄ is 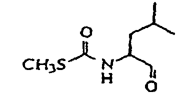 or R₃ is CH₂Ph and R₄ is 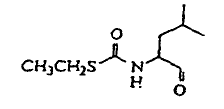 or a phaxmaceutically acceptable salt or solvate thereof.

29. A compound according to formula (I) of the formula XIV: 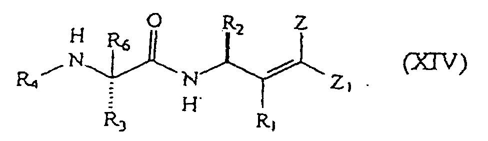 wherein R₆ is H, R₁ is H, R₃ is CH₂Ph, R₂ is CH₂CH₂C(O)NH₂, Z and Z₁ together form and R₄ is 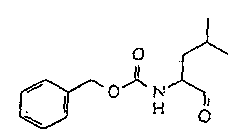 or a pharmaceutically acceptable salt or solvate thereof.

30. A compound according to formula (I) of the formula XIV: 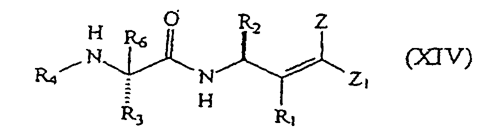 wherein R₆ is H, R₁ is H, R₂ is CH₂CH₂C(O)NH₂, R₄ is 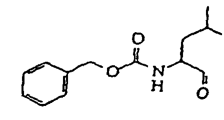 and
R₃ is 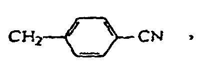 Z is H and Z₁ is CO₂CH₂CH₃
R₃ is CH(OH)CH₃, Z is H and Z₁ is CO₂CH₂CH₃
R₃ is 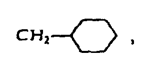 Z is H and Z₁ is CO₂CH₂CH₃
R₃ is CH₂Ph, Z is H and Z₁ is C(O)N(OH)CH₃
R₃ is 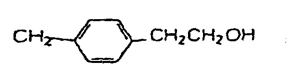 Z is H and Z₁ is CO₂CH₂CH₃
R₃ is 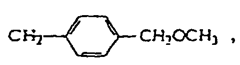 Z is H and Z₁ is CO₂CH₂CH₃
R₃ is CH₂CH(CH₃)₂, Z is H and Z₁ is CO₂CH₂CH₃
R₃ is CH₂SCH₃, Z is H and Z₁ is CO₂CH₂CH₃
R₃ is CH₂SCH₂CH₃, Z is H and Z₁ is CO₂CH₂CH₃ or a pharmaceutically acceptable salt, or solvate thereof.

31. A compound according to formula (I) of the formula IX: 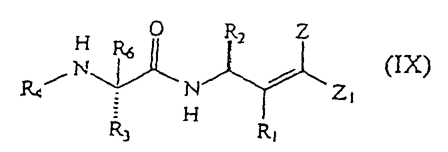 wherein R₆ is H, R₁ is H, R₂ is CH₂CH₂C(O)NH₂, Z is H, and
Z₁ is CO₂CH₂CH₃, R₃ is CH₂Ph, and R₄ is 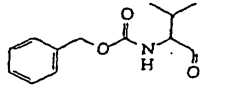 Z₁ is CO₂CH₂CH₃, R₃ is CH₂Ph, and R₄ is 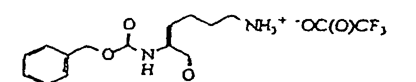 Z₁ is CO₂CH₂CH₃, R₃ is CH₂Ph, and R₄ is 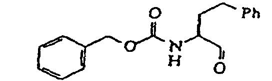 Z₁ is CO₂CH₂CH₃, R₃ is CH₂Ph, and R₄ is 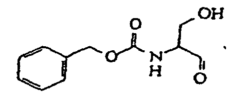 Z₁ is CO₂CH₂CH₃, R₃ is CH₂Ph, and R₄ is 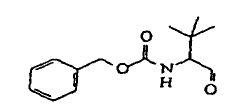 Z₁ is CO₂CH₂CH₃, R₃ is CH₂Ph, and R₄ is 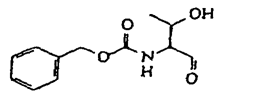 Z₁ is CO₂CH₂CH₃, R₃ is CH₂Ph, and R₄ is 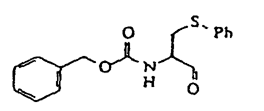 Z₁ is CO₂CH₂CH₃; R₃ is CH₂Ph, and R₄ is 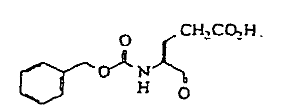 or Z₁ is CO₂CH₂CH₃, R₃ is 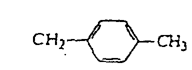 and R₄ is 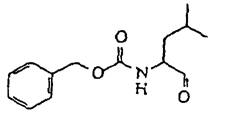 or a pharmaceutically acceptable salt or solvate thereof.

32. A compound according to formula (I) of the formula IX: 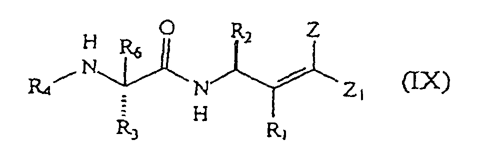 wherein R₆ is H, R₃ is CH₂Ph, R₂ is CH₂CH₂C(O)NH₂, and
R₁ is H, Z is H, Z₁ is CO₂CH₂CH₃, and R₄ is 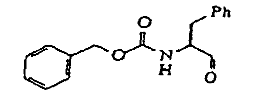 R₁ is H, Z is H, Z₁ is CO₂CH₂CH₃, and R₄ is 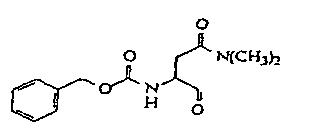 R₁ is H, Z is H, Z₁ is CO₂CH₂CH₃, and R₄ is 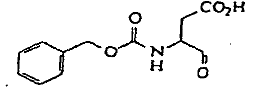 R₁ is H, Z is H, Z₁ is 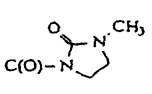 and R₄ is 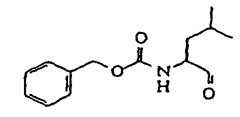 R₁ is H, Z is H, Z₁ is CO₂CH₂CH₃, and R₄ is 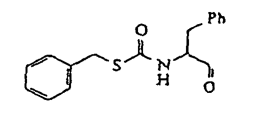 R₁ is H, Z is H, Z₁ is CO₂CH₂CH₃, and R₄ is 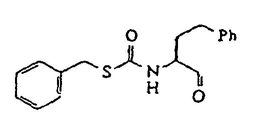 R₁ is H, Z is H, Z₁ is CO₂CH₂C(CH₃)₃, and R₄ is 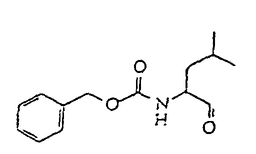 R₁ is H, Z and Z₁ together form 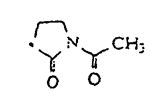 and R₄ is 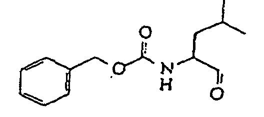 R₁ is H, Z is H, Z₁ is CO₂CH₂CH₃, and R₄ is 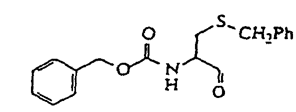 R₁ is H, Z is H, Z₁ is CO₂CH₂CH₃, and R₄ is 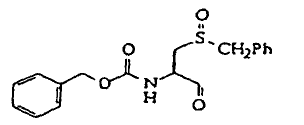 R₁ is H, Z is H, Z₁ is CO₂CH₂CH₃, and R₄ is 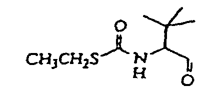 R₁ is H, Z is CH₃, Z₁ is CO₂CH₂CH₃, and R₄ is 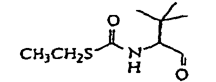 R₁ is H, Z and Z₁ together form 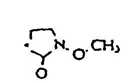 and R₄ is 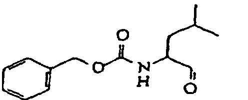 R₁ is H, Z is H, Z₁ is CO₂CH₂CH₃, and R₄ is 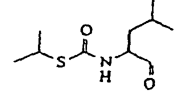 R₁ is H, Z is CH₃, Z₁ is CO₂CH₂CH₃, and R₄ is 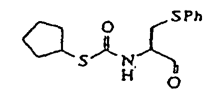 R₁ is H, Z is H, Z₁ is CO₂CH₂CH₃, and R₄ is 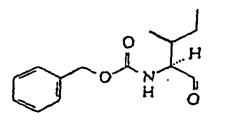 or R₁ is H, Z is H, Z₁ is CO₂CH₂CH₃, and R₄ is 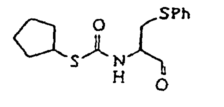 or a pharmaceutically acceptable salt or solvate thereof.

33. A compound according to formula (I) of the formula IX: 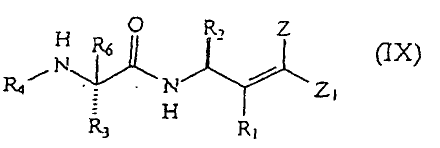 wherein R₆ is H, R₂ is CH₂CH₂C(O)NH₂, R₁ is H, and
Z is H, Z₁ is CO₂CH₂CH₃, R₃ is 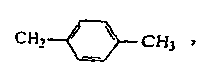 and R₄ is 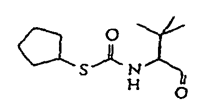 Z is CH₃, Z₁ is CO₂CH₂CH₃, R₃ is 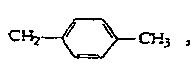 and R₄ is 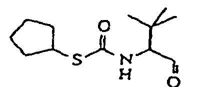 Z is H, Z₁ is CO₂CH₂CH₃, R₃ is 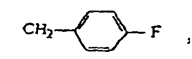 and R₄ is 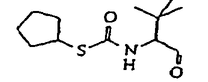 Z is CH₃, Z₁ is CO₂CH₂CH₃, R₃ is 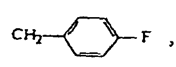 and R₄ is 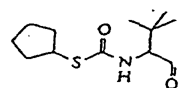 Z is H, Z₁ is CO₂CH₂CH₃, R₃ is CH₂Ph, and R₄ is 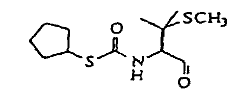 Z is H, Z₁ is CO₂CH₂CH₃, R₃ is CH₂Ph, and R₄ is 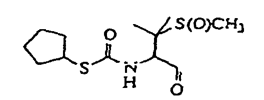 Z is H, Z₁ is CO₂CH₂CH₃, R₃ is CH₂Ph, and R₄ is 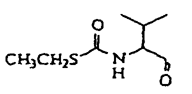 Z and Z₁ together form 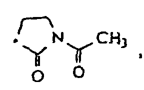 R₃ is 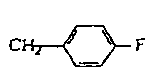 and R₄ is 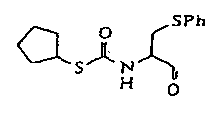 Z is H, Z₁ is CO₂CH₂CH₃, R₃ is CH₂Pb, and R₄ is 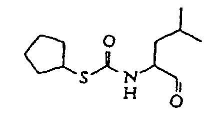 Z is H, Z₁ is CO₂CH₂CH₃, R₃ is 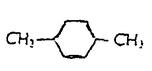 and R₄ is 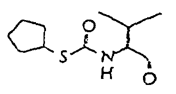 Z is H, Z₁ is CO₂CH₂CH₃, R₃ is 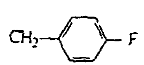 and R₄ is 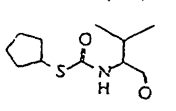 or Z is H, Z₁ is CO₂CH₂CH₃, R₃ is 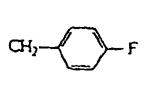 and R₄ is 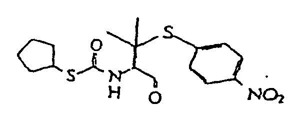 or a pharmaceutically acceptable salt or solvate thereof.

34. A pharmaceutical composition comprising:
(a) a therapeutically effective amount of a compound as defined in any of the preceeding claims or a pharmaceutically acceptable salt, or solvate thereof; and
(b) a pharmaceutically acceptable carrier, diluent, vehicle, or excipient.

35. Use of a compound as defined in any of claims 1 to 33 or a pharmaceutically acceptable salt or solvate thereof for the manufacture of a medicament for the treatment of a disease condition mediated by picornaviral a protease activity in a mammal.

36. A method of inhibiting the activity of a picornaviral 3C protease that comprises contacting the picornaviral 3C protease for the purpose of said inhibiting with an effective amount of a compound as defined in any of claims 1 to 33 or a pharmaceutically acceptable salt, or solvate thereof.

37. A method of inhibiting the activity of a rhinoviral protease that comprises contacting the rhinoviral protease for the purpose of said inhibiting with an effective amount of a compound as defined in any of the claims 1 to 33 or a pharmaceutically acceptable salt or solvate thereof.

38. A method of making a compound according to claim 1, comprising converting a compound of formula Q 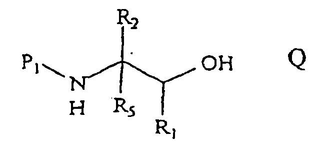 wherein R₁, R₂ and R₅ are as defined in claim 1, and P₁ is a protective group, or a salt or solvate thereof, to a compound of formula I, as defined in claim 1, or a pharmaceutically acceptable salt or solvate thereof

39. A method according to claim 38, wherein P₁ is benzyloxy carbonyl or t-butoxycarbonyl.

40. A method a making a compound according to claim 1, comprising converting a compound of the formula B: 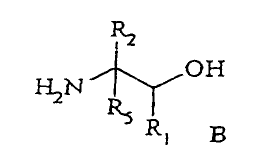 wherein R₁, R₂ and R₅ are as defined in claim 1, or a salt or solvate thereof, to a compound of formula I, as defined in claim 1, or a pharmaceutically acceptable salt or solvate thereof.

41. A method of making a compound according to claim 1, comprising converting a compound of formula O, 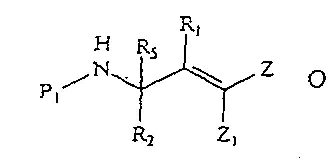 wherein R₁, R₂, R₅, Z and Z₁ are as defined in claim 1 and P₁ is a protective group, or a salt or solvate thereof, to a compound of formula I, as defined in claim 1, or a pharmaceutically acceptable salt or solvate thereof.

42. A method according to claim 41, wherein P₁ is benzyloxy carbonyl or t-butoxycarbonyl.

43. A method of preparing a compound according to claim 1, comprising converting a compound of formula P: 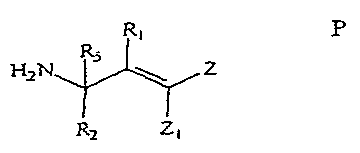 wherein R₁, R₂, R₅, Z and Z₁ are as defined in claim 1, or a salt or solvate thereof, to a compound of formula I, as defined in claim 1, or a pharmaceutically acceptable salt or solvate thereof.

44. Compounds selected from the group consisting of:
Compounds 46-66 and 78 having the formula IX: 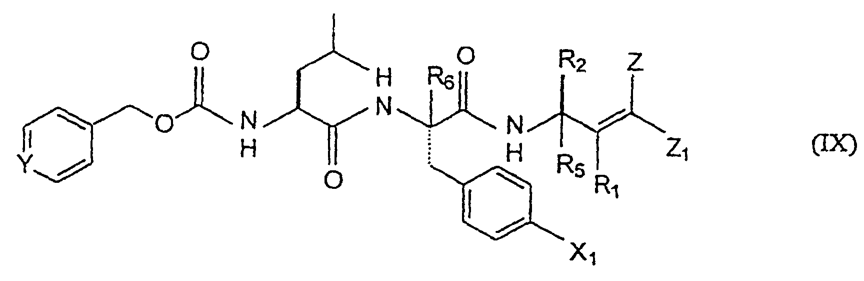
46. R₁ is H; R₂ is CH₂CH₂C(O)NH₂; R₅, R₆, and X₁ are H; Y is CH; Z is H; and Z₁ is 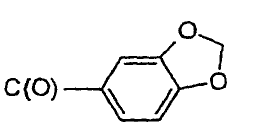
47. R₁ is H; R₂ is CH₂CH₂C(O)NH₂; R₅, R₆ and X₁ are H; Y is CH; Z is H; and Z₁ is 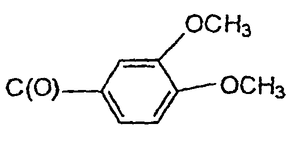
48. R₁ is H; R₂ is CH₂CH₂C(O)NH₂; R₅, R₆, and X₁ are H; Y is CH; Z is H; and Z₁ is 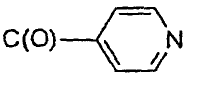
49. R₁ is H; R₂ is CH₂CH₂C(O)NH₂; R₅, R₆, and X₁ are H; Y is CH; Z is H; and Z₁ is 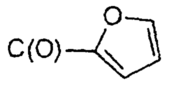
50. R₁ is H; R₂ is CH₂CH₂C(O)NH₂; R₅, R₆, and X₁ are H; Y is CH; Z is H; and Z₁ is 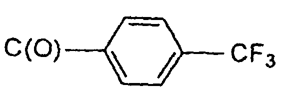
51. R₁ is H; R₂ is CH₂CH₂C(O)NH₂; R₅, R₆, and X₁ are H; Y is CH; Z is H; and Z₁ is 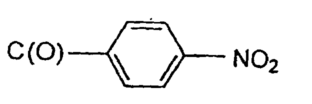
52. R₁ is H; R₂ is CH₂CH₂C(O)NH₂; R₅, R₆, and X₁ are H; Y is CH; Z is H; and Z₁ is C(O)tBu
53. R₁ is H; R₂ is CH₂CH₂C(O)NH₂; R₅ and R₆ are H; X₁ is OH; Y is CH; Z is H; and Z₁ is CO₂CH₂CH₃
54. R₁ is H; R₂ is CH₂CH₂C(O)NH₂; R₅, R₆, and X₁ are H; Y is CH; Z is H; and Z₁ is C(O)C(O)CH₃
55. R₁ is H; R₂ is CH₂CH₂C(O)NH₂; R₅, R₆, and X₁ are H; Y is CH; Z is H; and Z₁ is C(O)C(O)N(CH₃)₂
56. R₁ is H; R₂ is CH₂OC(O)NH₂; R₅, R₆, and X₁ are H; Y is CH; Z is H; and Z₁ is CO₂CH₂CH₃
57. R₁ is H; R₂ is CH₂CH₂C(O)NH₂; R₅, R₆, and X, are H; Y is CH; Z is H; and Z and Z₁ together form 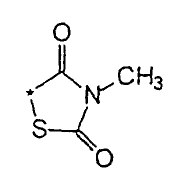 where the S is preferably trans to the R₁ group
58. R₁ is H; R₂ is CH₂CH₂C(O)NH₂; R₅, R₆, and X₁ are H; Y is CH; and Z and Z₁ together form 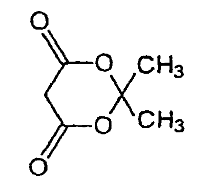
59. R₁ is H; R₂ is CH₂CH₂C(O)NH₂; R₅, R₆, and X₁ are H; Y is CH; Z is H; and Z₁ is C(O)NHPh
60. R₁ is H; R₂ is CH₂CH₂C(O)NH₂; R₅, R₆, and X, are H; Y is CH; Z is H; and Z₁ is C(O)N(CH₃)Ph
61. R₁ is H; R₂ is CH₂CH₂C(O)NH₂; R₅, R₆, and X, are H; Y is CH; Z is H; and Z₁ is 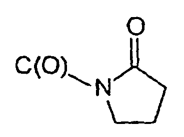
62. R₁ is H; R₂ is CH₂CH₂C(O)NH₂; R₅, R₆, and X₁ are H; Y is CH; Z is H; Z₁ is 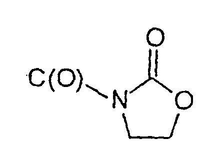
63. R₁, R₅, R₆, X₁, and Z are H; Y is CH; R₂ is CH₂CH₂C(O)NH₂; and Z₁ is 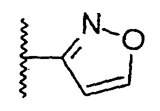
64. R₁, R₅, R₆, X₁, and Z are H; Y is CH; R₂ is CH₂CH₂C(O)NH₂; and Z₁ is 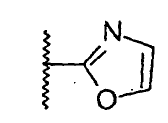
65. R₁, R₅, R₆, X₁, and Z are H; Y is CH; R₂ is CH₂CH₂C(O)NH₂; and Z₁ is 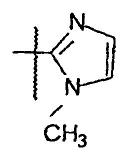
66. R₁, R₅, R₆, X₁, and Z are H; Y is CH; R₂ is CH₂CH₂C(O)NH₂; and Z₁ is 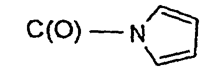
78. R₁, R₅, R₆ and X₁ are H; Y is CH; R₂ is CH₂CH₂C(O)NH₂; Z is CH₂Cl; and Z₁ is H;
Compounds 67-69 having the formula X: 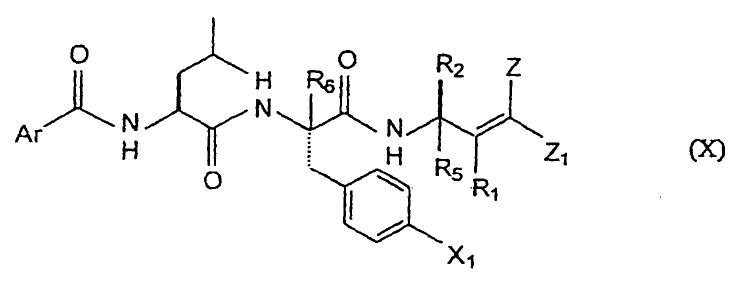
67. R₁, R₅, R₆, X₁, and Z are H; R₂ is CH₂CH₂C(O)NH₂; Z₁ is CO₂CH₂CH₃; and Ar is Ph
68. R₁, R₅, R₆, X₁, and Z are H; R₂ is CH₂CH₂C(O)NH₂; Z₁ is CO₂CH₃; and Ar is 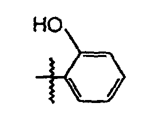
69. R₁, R₅, R₆, X₁, and Z are H; R₂ is CH₂CH₂C(O)NH₂; Z₁ is CO₂CH₂CH₃; and Ar is 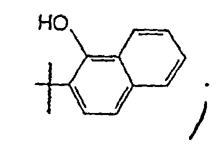 Compounds 70-73 having the formula XI: 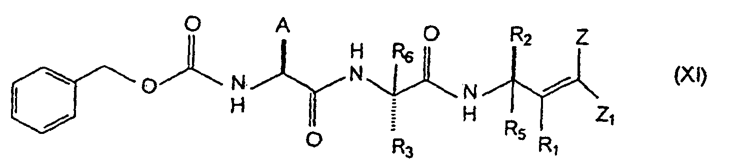
70. R₁, R₅, R₆, and Z are H; R₂ is CH₂CH₂C(O)NH₂; R₃ is CH₂Ph; Z₁ is CO₂CH₂CH₃; and A is 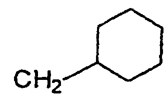
71. R₁, R₅, R₆, and Z are H; R₂ is CH₂CH₂C(O)NH₂; R₃ is CH₂Ph; Z₁ is CO₂CH₂CH₃; and A is Ph
72. R₁, R₅, R₆, and Z are H; R₂ is CH₂CH₂C(O)NH₂; A is CH₂CH(CH₃)₂; Z₁ is CO₂CH₂CH₃; and R₃ is 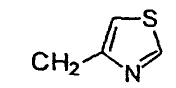
73. R₁, R₅, R₆, and Z are H; R₂ is CH₂CH₂C(O)NH₂; A is CH₂CH(CH₃)₂; Z₁ is CO₂CH₂CH₃; and R₃ is 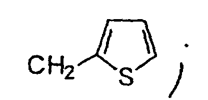 Compounds 1, 6, 8-10, 15, 20, 23, 38-40, 76, and 77 having the formula XII: 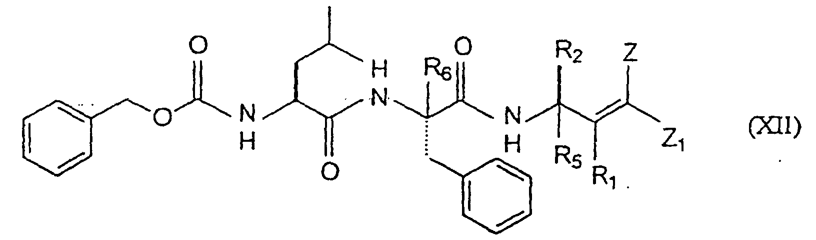
1. R₁ is H; R₂ is CH₂CH₂CN; R₅ is H; R₆ is H; Z is F; and Z₁ is CO₂CH₂CH₃
6. R₁ is H; R₂ is CH₂CH₂C(O)NH₂; R₅ is H; R₆ is H; Z is H; and Z₁ is C(O)NHCH₂CH₃
8. R₁ is H; R₂ is CH₂CH₂C(O)NH₂; R₅ is H; R₆ is H; Z is F; and Z₁ is CO₂CH₂CH₃
9. R₁ is H; R₂ is CH₂CH₂C(O)NH₂; R₅ is H; R₆ is H; Z is H; and Z₁ is SO₂CH₃
10. R₁ is H; R₂ is CH₂CH₂C(O)NH₂; R₅ is H; R₆ is H; Z is H; and Z₁ is SO₂Ph
15. R₁ is H; R₂ is CH₂CH₂C(O)NH₂; R₅ is H; R₆ is H; Z is H; and Z₁ is CO₂H
20. R₁ is H; R₂ is CH₂CH₂C(O)NH₂; R₅ is H; R₆ is H; Z is H; and Z₁ is PO(OCH₂CH₃)₂
23. R₁ is H; R₂ is CH₂CH₂C(O)NH₂; R₅ is H; R₆ is H; Z is H; and Z₁ is 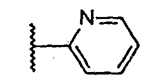
38. R₁ is H; R₂ is CH₂CH₂C(O)NH₂; R₅ is H; R₆ is H; Z is H; and Z₁ is 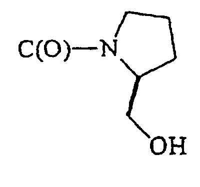
39. R₁ is H; R₂ is CH₂CH₂C(O)NH₂; R₅ is H; R₆ is H; Z is H; and Z₁ is 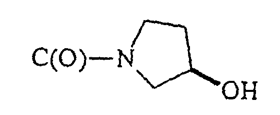
40. R₁ is H; R₂ is CH₂CH₂C(O)NH₂; R₅ is H; R₆ is H; Z is H; and Z₁ is 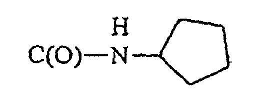
76. R₁ is H; R₂ is CH₂CH₂C(O)NH₂; R₅ is H; R₆ is H; Z is H; and Z₁ is CH₂OAc
77. R₁ is H; R₂ is CH₂CH₂C(O)NH₂; R₅ is H; R₆ is H; Z is H; and Z₁ is 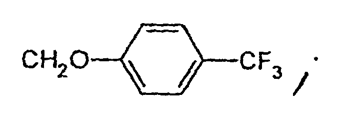
Compound 45 having the formula XIII:
45. 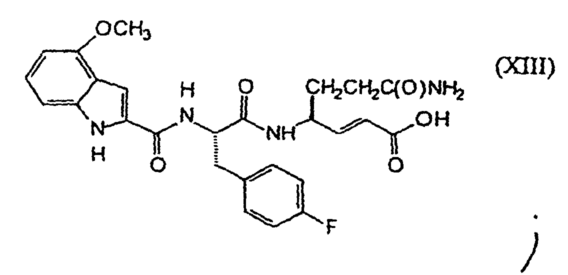 Compounds 79-97, also having the formula III: 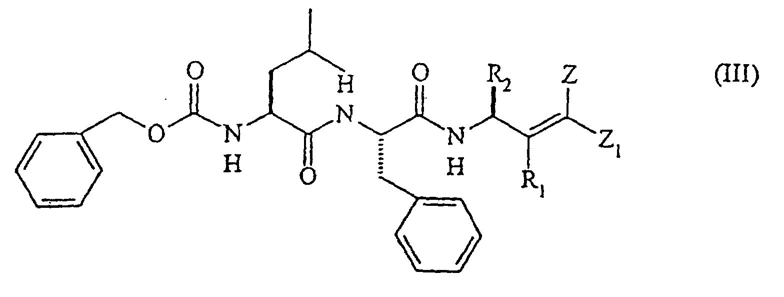
82. R₂ is CH₂CH₂C(O)NH₂, R₁ is H, Z is CH₃ and Z₁ is CO₂CH₂CH₃,
90. R₂ is CH₂CH₂C(O)NH₂, R₁ is H, and Z and Z₁ together form 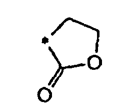 where C=O is preferably cis to the R₁ group
or wherein R₂ is CH₂CH₂C(O)NH₂, R₁ is H, Z is H, and Z₁ is selected from: 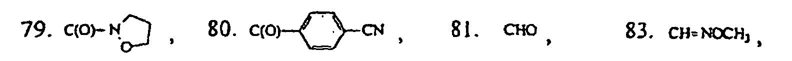 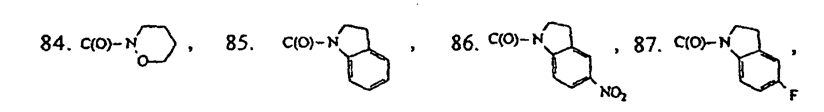 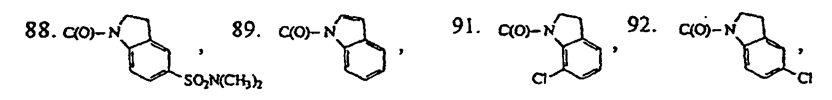 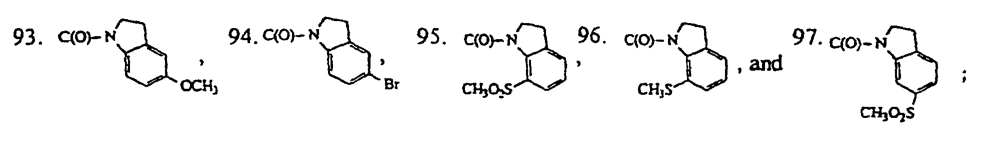 Compounds 98-121 having formula XIV: 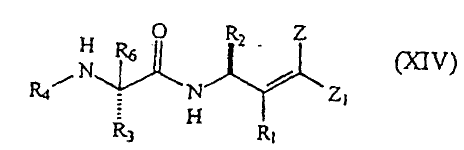 wherein R₆ is H, R₁ is H, R₂ is CH₂CH₂C(O)NH₂, Z is H, Z₁ is CO₂CH₂CH_{3,} and
98. R₃ is CH₂Ph and R₄ is 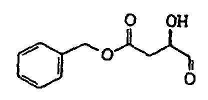
99. R₃ is H and R₄ is 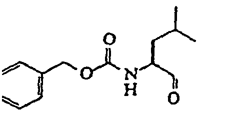
100. R₃ is 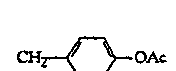 and R₄ is 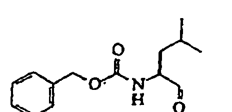
101. R₃ is CH₂Ph and R₄ is 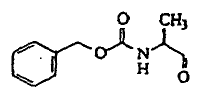
102. R₃ is CH₂Ph and R₄ is 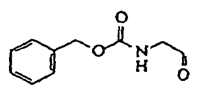
103. R₃ is 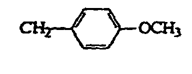 and R₄ is 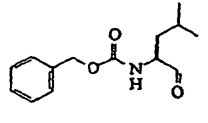
104. R₃ is CH₂Ph and R₄ is 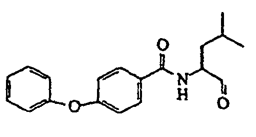
105. R₃ is 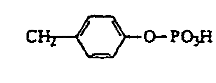 and R₄ is 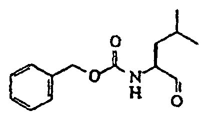
106. R₃ is CH₂Ph and R₄ is 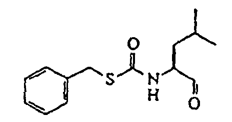
107. R₃ is CH₂Ph and R₄ is 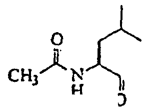
108. R₃ is CH₂CH₃ and R₄ is 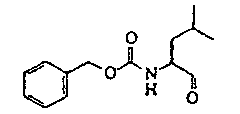
109. R₃ is CH₃ and R₄ is 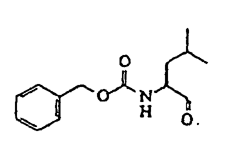
110. R₃ is CH₂Ph and R₄ is 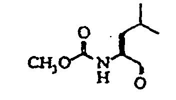
111. R₃ is CH₂PH and R₄ is 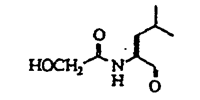
112. R₃ is 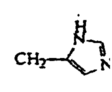 and R₄ is 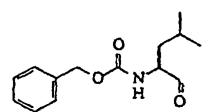
113. R₃ is 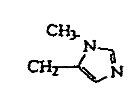 and R₄ is 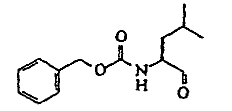
114. R₃ is 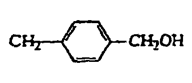 and R₄ is 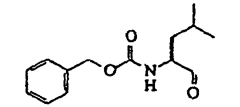
115. R₃ is CH₂Ph and R₄ is 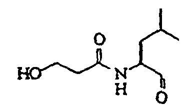
116. R₃ is CH₂Ph and R₄ is 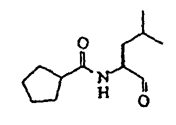
117. R₃ is CH₂Ph and R₄ is 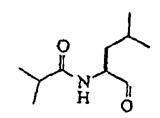
118. R₃ is CH₂Ph and R₄ is 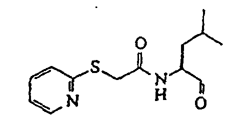
119. R₃ is CH₂Ph and R₄ is 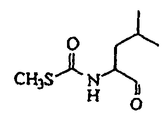
120. R₃ is CH₂Ph and R₄ is 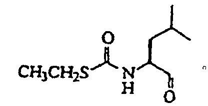
121. R₃ is CH₂CH₂CO₂H and R₄ is 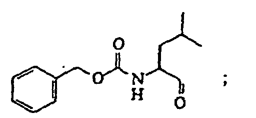 Compounds 122-130, also having the formula XIV: 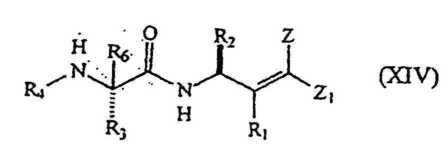 wherein R₆ is H, R₁ is H, R₃ is CH₂Ph and
122. R₂ is CH₂OC(O)NHC(O)CH₂Cl, Z is H, Z₁ is CO₂CH₂CH₃ and R₄ is 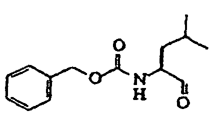
123. R₂ is CH₂CH₂C(O)NH₂, Z is H, Z₁ is CO₂CH₂CH₃ and R₄ is 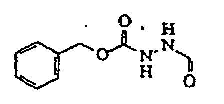
124. R₂ is CH₂CH₂C(O)NH₂, Z is H, Z₁ is 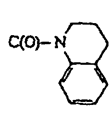 and R₄ is 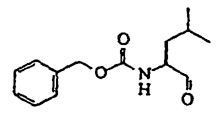
125. R₂ is CH₂CH₂C(O)NH₂, Z is H, Z₁ is NO₂, and R₄ is 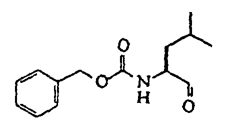
126. R₂ is CH₂CH₂C(O)NH₂, Z is H, Z₁ is 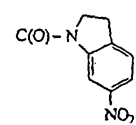 and R₄ is 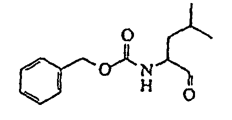
127. R₂ is CH₂CH₂C(O)NH₂, Z is H, Z₁ is 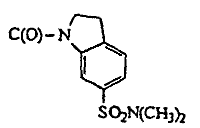 and R₄ is 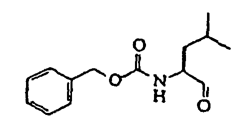
128. R₂ is CH₂CH₂C(O)NH₂, Z is H, Z₁ is 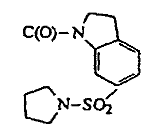 and R₄ is 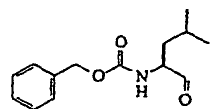
129. R₂ is CH₂CH₂C(O)NH₂, Z is H, Z₁ is CO₂CH₂CH₃, and R₄ is 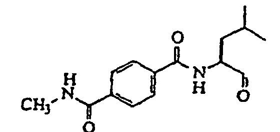
130. R₂ is CH₂CH₂C(O)NH₂, Z and Z₁ together form 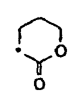 and R₄ is 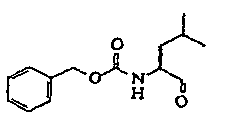 where C=O is preferably cis to the R₁ group;
Compounds 131-145, also having the formula XIV: 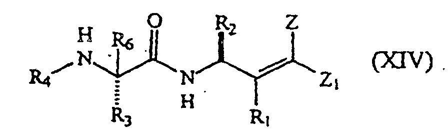 wherein R₆ is H, R₁ is H, R₂ is CH₂CH₂C(O)NH₂, R₄ is 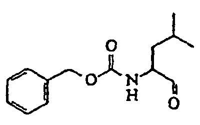 and
131. R₃ is CH₂Ph, Z is H and Z₁ is 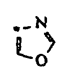
132. R₃ is 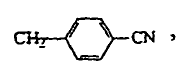 Z is H and Z₁ is CO₂CH₂CH₃
133. R₃ is 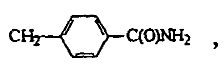 Z is H and Z₁ is CO₂CH₂CH₃
134. R₃ is CH(OH)CH₃, Z is H and Z₁ is CO₂CH₂CH₃
135. R₃ is 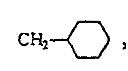 Z is H and Z₁ is CO₂CH₂CH₃
136. R₃ is 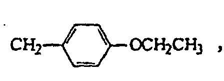 Z is H and Z₁ is CO₂CH₂CH₃
137. R₃ is CH₂CH₂CH₃, Z is H and Z₁ is CO₂CH₂CH₃
138. R₃ is CH₂Ph, Z is H and Z₁ is C(O)N(OH)CH₃
139. R₃ is 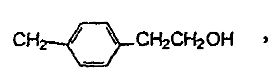 Z is H and Z₁ is CO₂CH₂CH₃
140. R₃ is 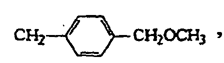 Z is H and Z₁ is CO₂CH₂CH₃
141. R₃ is CH₂CH(CH₃)₂, Z is H and Z₁ is CO₂CH₂CH₃
142. R₃ is CH₂SCH₃, Z is H and Z₁ is CO₂CH₂CH₃
143. R₃ is CH₂SCH₂CH₃, Z is H, and Z₁ is CO₂CH₂CH₃
144. R₃ is CH₂Ph, Z is CH₃, and Z₁ is CO₂H,
145. R₃ is CH₂Ph, Z is H, and Z₁ is 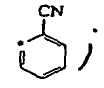 Compounds 146-155, also having the formula XIV: 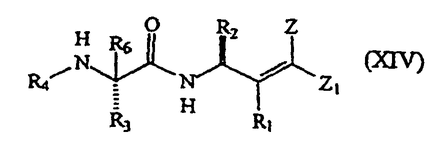 wherein R₆ is H, R₁ is H, R₂ is CH₂CH₂C(O)NH₂, Z is H, and
146. Z₁ is CO₂CH₂CH₃, R₃ is CH₂Ph, and R₄ is 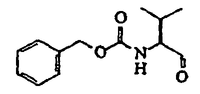
147. Z₁ is CO₂CH₂CH₃, R₃ is CH₂Ph, and R₄ is 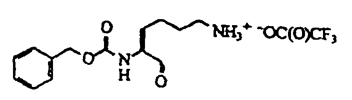
148. Z₁ is CO₂CH₂CH₃, R₃ is CH₂Ph, and R₄ is 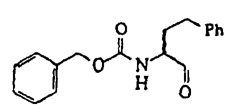
149. Z₁ is CO₂CH₂CH₃, R₃ is CH₂Ph, and R₄ is 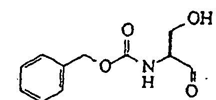
150. Z₁ is 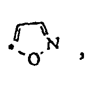 R₃ is CH₂Ph, and R₄ is 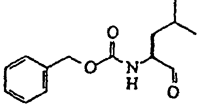
151. Z₁ is CO₂CH₂CH₃, R₃ is CH₂Ph, and R₄ is 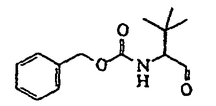
152. Z₁ is CO₂CH₂CH₃, R₃ is CH₂Ph, and R₄ is 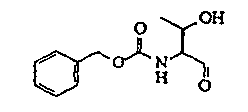
153. Z₁ is CO₂CH₂CH₃, R₃ is CH₂Ph, and R₄ is 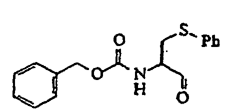
154. Z₁ is CO₂CH₂CH₃, R₃ is CH₂Ph, and R₄ is 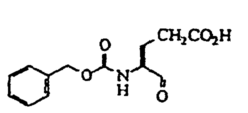
155. Z₁ is CO₂CH₂CH₃, R₃ is 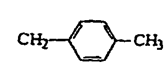 and R₄ is 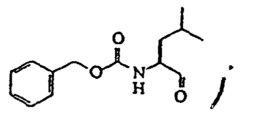 Compounds 156-173, also having formula XIV: 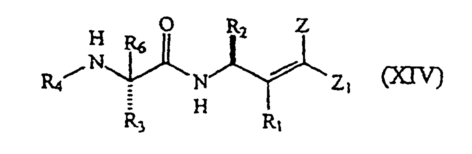 wherein R₆ is H, R₃ is CH₂Ph, R₂ is CH₂CH₂C(O)NH₂, and
156. R₁ is OH, Z is H, Z₁ is CO₂CH₂CH₃, and R₄ is 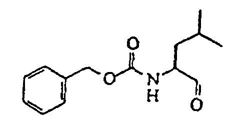
157. R₁ is H, Z is H, Z₁ is CO₂CH₂CH₃, and R₄ is 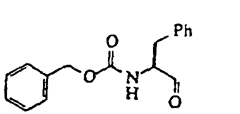
158. R₁ is H, Z is H, Z₁ is CO₂CH₂CH₃, and R₄ is 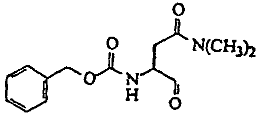
159. R₁ is H, Z is H, Z₁ is CO₂CH₂CH₃, and R₄ is 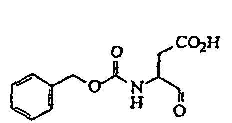
160. R₁ is H, Z is H, Z₁ is 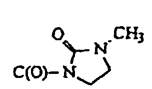 and R₄ is 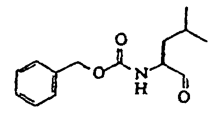
161. R₁ is H, Z is H, Z₁ is CO₂CH₂CH₃, and R₄ is 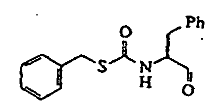
162. R₁ is H, Z is H, Z₁ is CO₂CH₂CH₃, and R₄ is 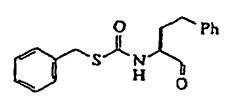
163. R₁ is H, Z is H, Z₁ is CO₂CH₂C(CH₃)₃, and R₄ is 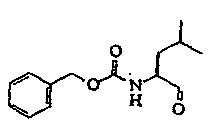
164. R₁ is H, Z and Z₁ together form 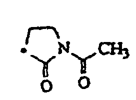 and R₄ is 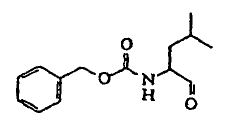 where C=O is preferably cis to the R₁ group
165. R₁ is H, Z is H, Z₁ is CO₂CH₂CH₃, and R₄ is 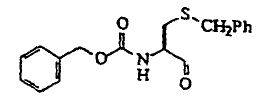
166. R₁ is H, Z is H, Z₁ is CO₂CH₂CH₃, and R₄ is 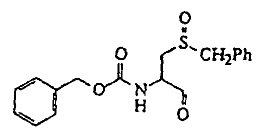
167. R₁ is H, Z is H, Z₁ is CO₂CH₂CH₃, and R₄ is 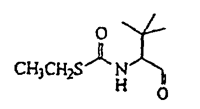
168. R₁ is H, Z is CH₃, Z₁ is CO₂CH₂CH₃, and R₄ is 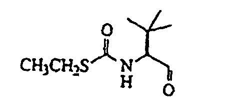
169. R₁ is H, Z and Z₁ together form 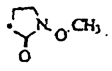 and R₄ is 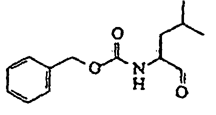 where C=O is preferably cis to R₁
170. R₁ is H, Z is H, Z₁ is CO₂CH₂CH₃, and R₄ is 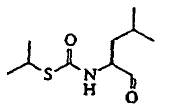
171. R₁ is H, Z is CH₃, Z₁ is CO₂CH₂CH₃, and R₄ is 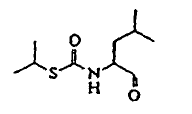
172. R₁ is H, Z is H, Z₁ is CO₂CH₂CH₃, and R₄ is 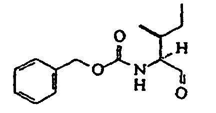
173. R₁ is H, Z is H, Z₁ is CO₂CH₂CH₃, and R₄ is 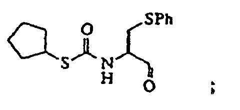 Compounds 174-188, also having the formula XIV: 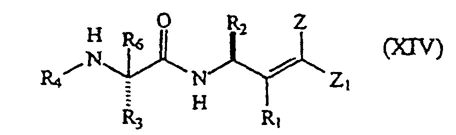 wherein R₆ is H, R₂ is CH₂CH₂C(O)NH₂, R₁ is H, and
174. Z is H, Z₁ is CO₂CH₂CH₃, R₃ is 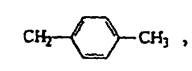 and R₄ is 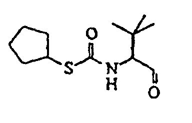
175. Z is CH₃, Z₁ is CO₂CH₂CH₃, R₃ is 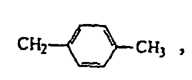 and R₄ is 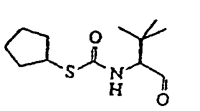
176. Z is H, Z₁ is CO₂CH₂CH₃, R₃ is 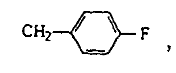 and R₄ is 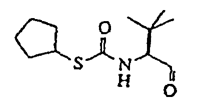
177. Z is CH₃, Z₁ is CO₂CH₂CH₃, R₃ is 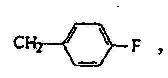 and R₄ is 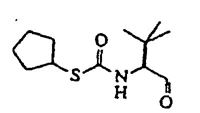
178. Z is H, Z₁ is CO₂CH₂CH₃, R₃ is CH₂Ph, and R₄ is 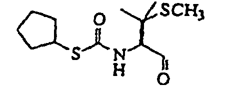
179. Z is H, Z₁ is CO₂CH₂CH₃, R₃ is CH₂Ph, and R₄ is 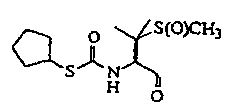
180. Z is H, Z₁ is CO₂CH₂CH₃, R₃ is CH₂Ph, and R₄ is 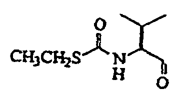
181. Z and Z₁ together form 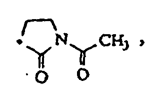 R₃ is 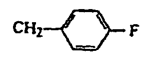 and R₄ is 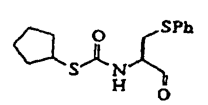 where C=O is preferably cis to the R₁ group
182. Z is H, Z₁ is CO₂CH₂CH₃, R₃ is CH₂Ph, and R₄ is 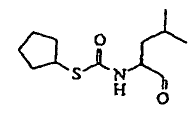
183. Z is H, Z₁ is CO₂CH₂CH₃, R₃ is 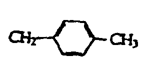 and R₄ is 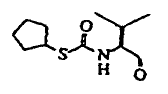
184. Z is H, Z₁ is CO₂CH₂CH₃, R₃ is 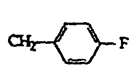 and R₄ is 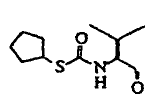
185. Z is H, Z₁ is CO₂CH₂CH₃, R₃ is 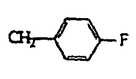 and R₄ is 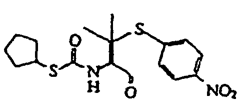
186. Z is H, Z₁ is CO₂CH₂Ph, R₃ is 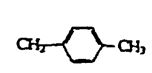 and R₄ is 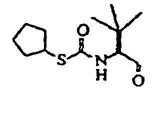
187. Z is CH₃, Z₁ is CO₂CH₂CH₃, R₃ is CH₂Ph and R₄ 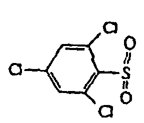 is
188. Z is H, Z₁ is CO₂CH₂CH₂OCH₃, R₃ is 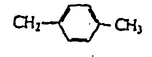 and R₄ is 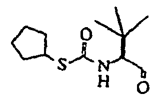
189. R₃ is 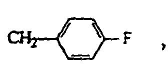 R₄ is 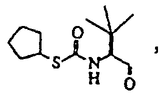 and Z and Z₁ together form 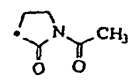 where C=O is preferably cis to the R₁ group
190. Z is H, Z₁ is CO₂CH₂CH₃, R₃ is 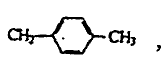 and R₄ is 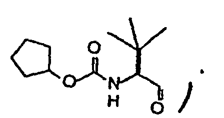 and compounds of formula (III) below 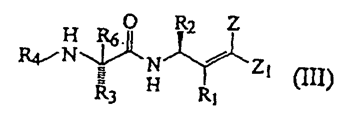 wherein R₆ is H, R₁ is H, R₃ is CH₂Ph, R₂ is CH₂CH₂C(O)NH₂, Z is H, Z₁ is CO₂CH₂CH₃, and R₄ is selected from the following: 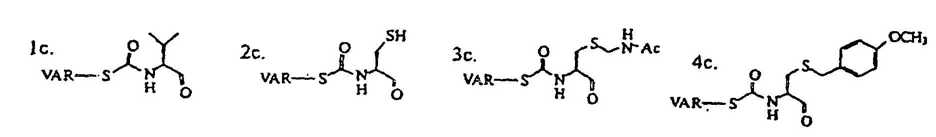 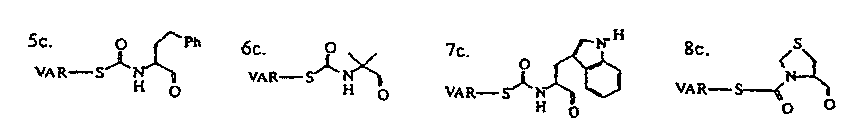 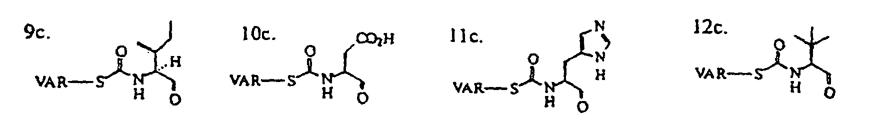 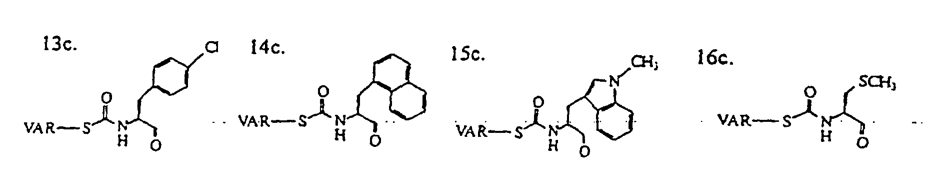 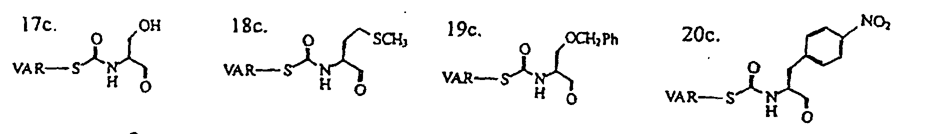 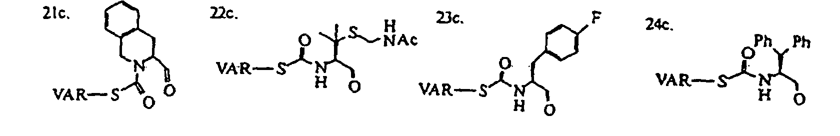 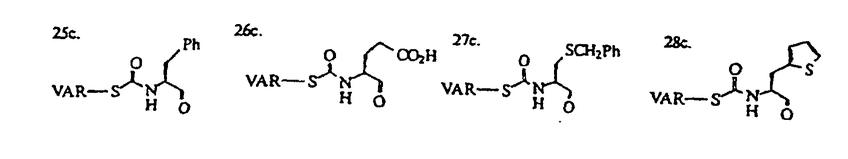 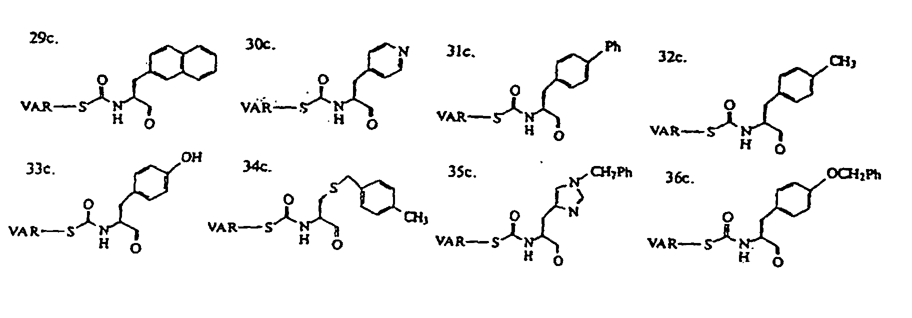 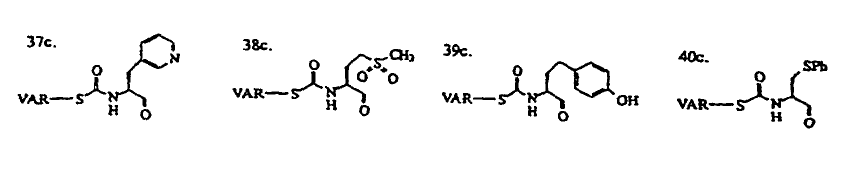 wherein VAR is selected from -CH₂CH₃, -CH(CH₃)₂, -CH₂CH(CH₃)₂, -CH₂-Ph,

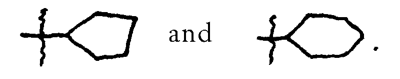

## Patentansprüche

1. Verbindung der Formel (I): 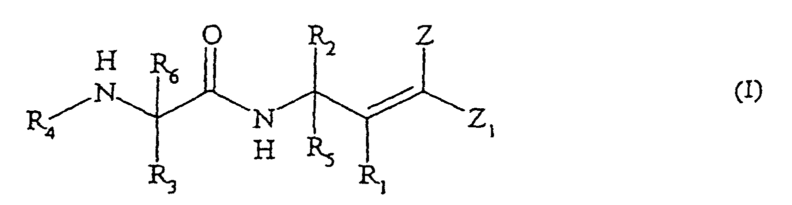 worin
R₁ H, F, eine Alkylgruppe, SH, eine O-Alkylgruppe oder eine S-Alkylgruppe ist;
R₂ und R₅ unabhängig voneinander ausgewählt sind aus H, 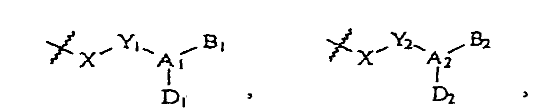 oder einer Alkylgruppe, wobei die Alkylgruppe von 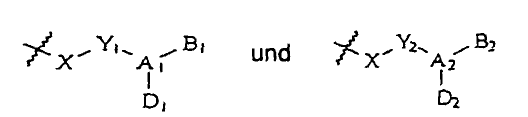 verschieden ist, mit der Maßgabe, dass wenigstens eines von R₂ oder R₅ 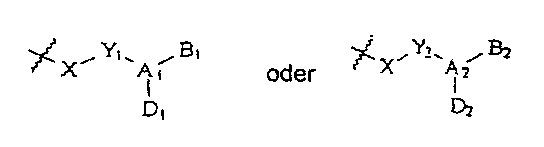 sein muss, und worin, wenn R₂ oder R₅ 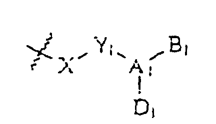 ist,
X =CH oder =CF ist und Y₁ =CH oder =CF ist
oder X und Y₁ zusammen mit Q' einen dreigliedrigen Ring bilden, in welchem Q' -C(R₁₀)(R₁₁)- oder -O- ist, X -CH- oder -CF- ist und Y₁ -CH-, -CF- oder -C(Alkyl)- ist, wobei R₁₀ und R₁₁ unabhängig voneinander H, ein Halogen oder eine Alkylgruppe sind oder zusammen mit dem Kohlenstoffatom, an das sie gebunden sind, eine Cycloalkylgruppe oder eine Heterocycloalkylgruppe bilden,
oder X -CH₂-, -CF₂-, -CHF- oder -S- ist,
und
Y₁ -O-, -S-, -NR₁₂-, -C(R₁₃)(R₁₄)-, -C(O)-. -C(S)- oder -C(CR₁₃R₁₄)- ist, worin R₁₂ H oder Alkyl ist und R₁₃ und R₁₄ unabhängig voneinander H, F oder eine Alkylgruppe sind oder zusammen mit dem Atom, an das sie gebunden sind, eine Cycloalkylgruppe oder eine Heterocycloalkylgruppe bilden;
und A, C, CH, CF, S, P, Se, N, NR₁₅, S(O), Se(O), P-OR₁₅ oder P-NR₁₅R₁₆ ist, worin R₁₅ und R₁₆ unabhängig voneinander eine Alkylgruppe, eine Cycloalkylgruppe, eine Heterocycloalkyigruppe, eine Arylgruppe oder eine Heteroarylgruppe sind oder zusammen mit dem Atom, an das sie gebunden sind, eine Heterocycloalkylgruppe bilden;
und D₁ eine Komponente mit einem einsamen Elektronenpaar ist, die eine Wasserstoffbindung bilden kann;
und B₁ H, F, eine Alkylgruppe, eine Cycloalkylgruppe, eine Heterocycloalkylgruppe, eine Arylgruppe, eine Heteroarylgruppe, -OR₁₇, -SR₁₇, -NR₁₇R₁₈, -NR₁₉NR₁₇R₁₈ oder -NR₁₇OR₁₈ ist,
worin R₁₇, R₁₈ und R₁₉ unabhängig voneinander H, eine Alkylgruppe, eine Cycloalkylgruppe, eine Heterocycloalkylgruppe, eine Arylgruppe, eine Heteroarylgruppe oder eine Acylgruppe sind oder worin beliebige zwei von R₁₇, R₁₈ und R₁₉ zusammen mit dem (den) Atom(en), an das (die) sie gebunden sind, eine Heterocycloalkylgruppe bilden,
und mit den Maßgaben, dass, wenn D₁ die Komponente ≡N mit einem einsamen Elektronenpaar ist, die eine Wasserstoffbindung bilden kann, B₁ nicht existiert; und wenn A₁ ein sp³-Kohlenstoff ist, B₁ nicht -NR₁₇R₁₈ ist, wenn D₁ die Komponente -NR₂₅R₂₆ mit einem einsamen Elektronenpaar ist, die eine Wasserstoffbindung bilden kann, worin R₂₅ und R₂₆ unabhängig voneinander H, eine Alkylgruppe, eine Cycloalkylgruppe, eine Heterocycloalkylgruppe, eine Arylgruppe oder eine Heteroarylgruppe sind;
und worin D₁-A₁-B₁ gegebenenfalls eine Nitrogruppe bildet, wobei A₁ N ist;
und worin, wenn R₂ oder R₅ 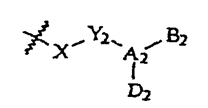 ist,
X =CH oder =CF ist und Y₂ =C, =CH oder =CF ist,
oder X und Y₂ zusammen mit Q' einen dreigliedrigen Ring bilden,
in welchem Q' -C(R₁₀)(R₁₁)- oder -O- ist, X -CH- oder -CF- ist und Y₂ -CH-, -CF- oder -C(Alkyl)- ist, wobei R₁₀ und R₁₁ unabhängig voneinander H, ein Halogen oder eine Alkylgruppe sind oder zusammen mit dem Kohlenstoffatom, an das sie gebunden sind, eine Cycloalkylgruppe oder eine Heterocycloalkylgruppe bilden,
oder X -CH₂-, -CF₂-, -CHF- oder -S- ist,
und
Y₂ -O-, -S-, -N(R'₁₂)-, -C(R'₁₃)(R'₁₄)-, -C(O)-, -C(S)- oder -C(CR'₁₃R'₁₄)- ist, worin R'₁₂ H, eine Alkylgruppe, eine Cycloalkylgruppe, eine Heterocycloalkylgruppe, eine Arylgruppe, eine Heteroarylgruppe, -OR'₁₃, -NR'₁₃R'₁₄, -C(O)-R'₁₃, -SO₂R'₁₃ oder -C(S)R'₁₃ ist und R'₁₃ und R'₁₄ unabhängig voneinander H, F oder eine Alkylgruppe, eine Cycloalkylgruppe, eine Heterocycloalkylgruppe, eine Arylgruppe oder eine Heteroarylgruppe sind oder zusammen mit dem Atom, an das sie gebunden sind, eine Cycloalkylgruppe oder eine Heterocycloalkylgruppe bilden,
und worin eine beliebige Kombination von Y₂, A₂, B₂ und D₂ eine Cycloalkylgruppe, eine Heterocycloalkylgruppe, eine Arylgruppe oder eine Heteroarylgruppe bildet;
und A₂ C, CH, CF, S, P, Se, N, NR₁₅, S(O), Se(O), P-OR₁₅ oder P-NR₁₅R₁₆ ist, worin R₁₅ und R₁₆ unabhängig voneinander eine Alkylgruppe, eine Cycloalkylgruppe, eine Heterocycloalkylgruppe, eine Arylgruppe oder eine Heteroarylgruppe sind oder zusammen mit dem Atom, an das sie gebunden sind, eine Heterocycloalkylgruppe bilden;
und D₂ eine Komponente mit einem einsamen Elektronenpaar ist, die eine Wasserstoffbindung bilden kann;
und B₂ H, F, eine Alkylgruppe, eine Cycloalkylgruppe, eine Heterocycloalkylgruppe, eine Arylgruppe, eine Heteroarylgruppe, -OR₁₇, -SR₁₇, -NR₁₇R₁₈, -NR₁₉NR₁₇R₁₈, oder -NR₁₇OR₁₈ ist,
worin R₁₇, R₁₈ und R₁₉ unabhängig voneinander H, eine Alkylgruppe, eine Cycloalkylgruppe, eine Heterocycloalkylgruppe, eine Arylgruppe, eine Heteroarylgruppe oder eine Acylgruppe sind oder worin beliebige zwei von R₁₇, R₁₈ und R₁₉ zusammen mit dem (den) Atom(en), an das (die) sie gebunden sind, eine Heterocycloalkylgruppe bilden;
R₃ und R₆ unabhängig voneinander H, F oder eine Alkylgruppe sind;
R₄ H, OH, eine Alkylgruppe, eine Oxogruppe, eine Cycloalkylgruppe, eine Heterocycloalkylgruppe, eine Arylgruppe, eine Heteroarylgruppe, eine Acylgruppe, eine Sulfonylgruppe, eine Mercaptogruppe, eine Alkylthiogruppe, eine Alkoxygruppe, eine Carboxygruppe, eine Aminogruppe, eine Alkylaminogruppe, eine Dialkylaminogruppe, eine Carbamoylgruppe, eine Arylthiogruppe oder eine Heteroarylthiogruppe ist;
Z und Z₁ unabhängig voneinander H, F, eine Alkylgruppe, eine Cycloalkylgruppe, eine Heterocycloalkylgruppe, eine Arylgruppe, eine Heteroarylgruppe, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃ oder -C(S)NR₂₁NR₂₂R₂₃ sind,
worin R₂₁, R₂₂, R₂₃ und R₂₄ unabhängig voneinander H, eine Alkylgruppe, eine Cycloalkylgruppe, eine Heterocycloalkylgruppe, eine Arylgruppe, eine Heteroarylgruppe oder eine Acylgruppe oder eine Thioacylgruppe sind oder worin beliebige zwei von R₂₁, R₂₂, R₂₃ und R₂₄ zusammen mit dem (den) Atom(en), an das (die) sie gebunden sind, eine Heterocycloalkylgruppe bilden;
oder Z₁, wie vorstehend definiert, zusammen mit R₁, wie vorstehend definiert, und den Atomen, an die Z₁ und R₁ gebunden sind, eine Cycloalkyl- oder Heterocycloalkylgruppe bilden,
oder Z und Z₁, beide wie vorstehend definiert, zusammen mit den Atomen,
an die sie gebunden sind, eine Cycloalkyl- oder Heterocycloalkylgruppe bilden; oder ein pharmazeutisch annehmbares Salz oder Solvat davon;
worin die Begriffe Alkylgruppe, Cycloalkylgruppe, Heterocycloalkylgruppe, Arylgruppe, Heteroarylgruppe, Acylgruppe, Sulfonylgruppe, Alkylthiogruppe, Alkoxygruppe, Alkylaminogruppe, Dialkylaminogruppe, Arylthiogruppe und Heteroarylthiogruppe die folgenden Bedeutungen haben:
Alkylgruppe: Methyl, Ethyl, Propyl, Isopropyl, Butyl, Isobutyl, t-Butyl, Ethenyl, Pentenyl, Butenyl, Propenyl, Ethinyl, Butinyl, Propinyl, Pentinyl und Hexinyl, unsubstituiert oder durch einen Substituenten substituiert;
Cycloalkylgruppe: nicht-aromatischer, einwertiger, monocyclischer, bicyclischer oder tricyclischer Rest, der 3, 4, 5, 6, 7, 8, 9,10, 11, 12, 13 oder 14 Kohlenstoffringatome enthält, welcher bzw. welche gesättigt oder ungesättigt, unsubstituiert oder durch einen Substituenten substituiert sein kann bzw. sein können;
Heterocycloalkylgruppe: nicht-aromatischer, einwertiger, monocyclischer, bicyclischer oder tricyclischer Rest, welcher gesättigt oder ungesättigt ist, enthaltend 3, 4, 5, 6, 7, 8, 9, 10, 11, 12, 13, 14, 15, 16, 17 oder 18 Ringatome, welche 1, 2, 3, 4 oder 5 Heteroatome, ausgewählt aus Stickstoff, Sauerstoff und Schwefel, einschließen, wobei der Rest unsubstituiert oder durch einen Substituenten substituiert ist;
Arylgruppe: aromatischer, einwertiger, monocyclischer, bicyclischer oder tricyclischer Rest, der 6, 10, 14 oder 18 Kohlenstoffringatome enthält, welcher bzw. welche unsubstituiert oder durch einen Substituenten substituiert sein kann bzw. sein können;
Heteroarylgruppe: aromatischer, einwertiger, monocyclischer, bicyclischer oder tricyclischer Rest, der 4, 5, 6, 7, 8, 9,10, 11, 12, 13, 14, 15, 16, 17 oder 18 Ringatome, einschließlich 1, 2, 3, 4 oder 5 Heteroatome, ausgewählt aus Stickstoff, Sauerstoff und Schwefel, enthält, welcher bzw. welche unsubstituiert oder durch einen Substituenten substituiert sein kann bzw. sein können;
Acylgruppe: -C(O)-R -Rest, worin R ein Substituent ist;
Sulfonylgruppe: -SO₂R -Rest, worin R ein Substituent ist;
Alkylthiogruppe: -SR -Rest, worin R eine Alkylgruppe ist;
Alkoxygruppe: OR -Rest, worin R eine Alkylgruppe ist;
Alkylaminogruppe: -NHR -Rest, worin R eine Alkylgruppe ist;
Dialkylaminogruppe: -NRₐR_{b} -Rest, worin Rₐ und R_{b} unabhängig voneinander eine Alkylgruppe sind;
Arylthiogruppe: -SR_{c} -Rest, worin R_{c} eine Arylgruppe ist;
Heteroarylthiogruppe: -SR_{d} -Rest, worin R_{d} eine Heteroarylgruppe ist;
Substituent: Hydroxylgruppe, Oxogruppe, Alkylgruppe, Acylgruppe, Sulfonylgruppe, Mercaptogruppe, Alkylthiogruppe, Alkoxygruppe, Cycloalkylgruppe, Heterocycloalkylgruppe, Arylgruppe, Heteroarylgruppe, Carboxygruppe, Aminogruppe, Alkylaminogruppe, Dialkylaminogruppe, Carbamoylgruppe, Aryloxygruppe, Heteroaryloxygruppe, Arylthiogruppe, Heteroarylthiogruppe, worin Aryloxygruppe der Rest -OR_{c} ist, worin R_{c} eine Arylgruppe ist, und worin Heteroaryloxygruppe der Rest -OR_{d} ist, worin R_{d} eine Heteroarylgruppe ist.

2. Verbindung nach Anspruch 1, worin R₁ H oder F ist, oder ein pharmazeutisch annehmbares Salz oder Solvat davon.

3. Verbindung nach Anspruch 1, worin R₄ eine Acylgruppe oder eine Sulfonylgruppe ist, oder ein pharmazeutisch annehmbares Salz oder Solvat davon.

4. Verbindung nach Anspruch 1, worin wenigstens eines von R₂ oder R₅ 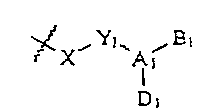 ist, oder ein pharmazeutisch annehmbares Salz oder Solvat davon.

5. Verbindung nach Anspruch 4, worin D₁ -OR₂₅, =O, =S, ≡N, =NR₂₅ oder -NR₂₅R₂₆ ist, worin R₂₅ und R₂₆ unabhängig voneinander H, eine Alkylgruppe, eine Cycloalkylgruppe, eine Heterocycloalkylgruppe, eine Arylgruppe oder eine Heteroarylgruppe sind oder zusammen mit dem Stickstoffatom, an das sie gebunden sind, eine Heterocycloalkylgruppe bilden; oder ein pharmazeutisch annehmbares Salz oder Solvat davon.

6. Verbindung nach Anspruch 5, worin D₁ =O ist; oder ein pharmazeutisch annehmbares Salz oder Solvat davon.

7. Verbindung nach Anspruch 4, worin A₁ C, CH, S oder S(O) ist; oder ein pharmazeutisch annehmbares Salz oder Solvat davon.

8. Verbindung nach Anspruch 7, worin A₁ C ist; oder ein pharmazeutisch annehmbares Salz oder Solvat davon.

9. Verbindung nach Anspruch 4, worin B₁ NR₁₇R₁₈ ist, worin R₁₇ und R₁₈ unabhängig voneinander H, eine Alkylgruppe, eine Cycloalkylgruppe, eine Heterocycloalkylgruppe, eine Arylgruppe, eine Heteroarylgruppe oder eine Acylgruppe sind oder worin R₁₇ und R₁₈ zusammen mit dem (den) Atom(en), an das (die) sie gebunden sind, eine Heterocycloalkylgruppe bilden;
oder ein pharmazeutisch annehmbares Salz oder Solvat davon.

10. Verbindung nach Anspruch 1, worin wenigstens eines von R₂ oder R₅ 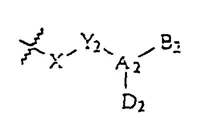 ist, oder ein pharmazeutisch annehmbares Salz oder Solvat davon.

11. Verbindung nach Anspruch 10, worin D₂ -OR_{25,} =O, =S, ≡N, =NR₂₅ oder -NR₂₅R₂₆ ist, worin R₂₅ und R₂₆ unabhängig voneinander H, eine Alkylgruppe, eine Cycloalkylgruppe, eine Heterocycloalkylgruppe, eine Arylgruppe oder eine Heteroarylgruppe sind, oder zusammen mit dem Atom, an das sie gebunden sind, eine Heterocycloalkylgruppe bilden; oder ein pharmazeutisch annehmbares Salz oder Solvat davon.

12. Verbindung nach Anspruch 11, worin D₂ =O ist; oder ein pharmazeutisch annehmbares Salz oder Solvat davon.

13. Verbindung nach Anspruch 10, worin A₂ C, CH, S oder S(O) ist; oder ein pharmazeutisch annehmbares Salz oder Solvat davon.

14. Verbindung nach Anspruch 13, worin A₂ C ist; oder ein pharmazeutisch annehmbares Salz oder Solvat davon.

15. Verbindung nach Anspruch 10, worin B₂ NR₁₇R₁₈ ist, worin R₁₇ und R₁₈ unabhängig voneinander H, eine Alkylgruppe, eine Cycloalkylgruppe, eine Heterocycloalkylgruppe, eine Arylgruppe, eine Heteroarylgruppe oder eine Acylgruppe sind, oder worin R₁₇ und R₁₈ zusammen mit dem (den) Atom(en), an das (die) sie gebunden sind, eine Heterocycloalkylgruppe bilden; oder ein pharmazeutisch annehmbares Salz oder Solvat davon.

16. Verbindung nach Anspruch 1, worin A₁ C, CH, S oder S(O) ist und worin A₂ C, CH, S oder S(O) ist; oder ein pharmazeutisch annehmbares Salz oder Solvat davon.

17. Verbindung nach Anspruch 1, worin Z und Z₁ unabhängig voneinander H, eine Arylgruppe oder eine Heteroarylgruppe, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃ oder -C(S)NR₂₁NR₂₂R₂₃ sind;
worin R₂₁, R₂₂ und R₂₃ unabhängig voneinander H, eine Alkylgruppe, eine Cycloalkylgruppe, eine Heterocycloalkylgruppe, eine Arylgruppe, eine Heteroarylgruppe, eine Acylgruppe oder eine Thioacylgruppe sind, oder worin beliebige zwei von R₂₁, R₂₂ und R₂₃ zusammen mit dem (den) Atom(en), an das (die) sie gebunden sind, eine Heterocycloalkylgruppe bilden,
oder Z und Z₁, zusammen mit den Atomen, an die sie gebunden sind, eine Heterocycloalkylgruppe bilden;
oder ein pharmazeutisch annehmbares Salz oder Solvat davon.

18. Verbindung gemäß Formel (I) mit der Formel II, worin die Definitionen für die Substituenten wie in Anspruch 1 definiert sind: 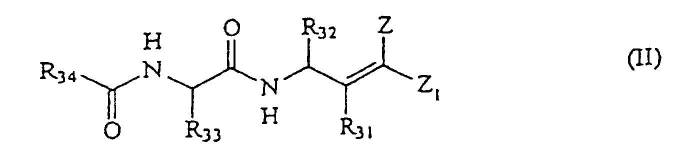 und worin außerdem
R₃₁ H, F oder eine Alkylgruppe ist;
R₃₂ ausgewählt ist aus einer der folgenden Komponenten: 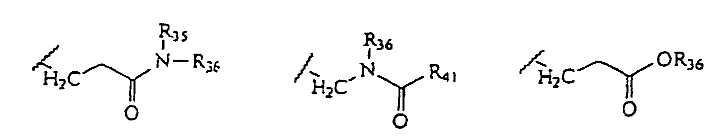 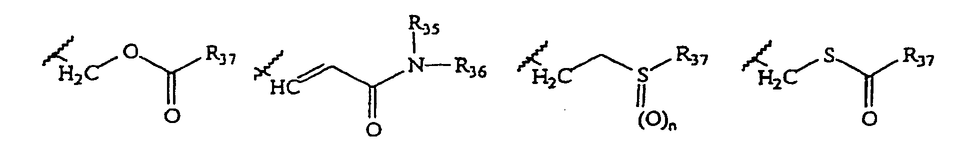 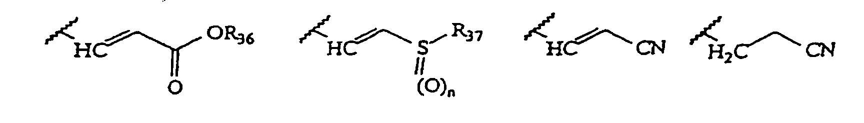 worin
R₃₅ H, eine Alkylgruppe, eine Arylgruppe, -OR₃₈ oder -NR₃₈R₃₉ ist und
R₃₆ H oder eine Alkylgruppe ist,
oder R₃₅ und R₃₆ zusammen mit dem (den) Atom(en), an das (die) sie gebunden sind, eine Heterocycloalkylgruppe oder eine Heteroarylgruppe bilden;
R₄₁ H, eine Alkylgruppe, eine Arylgruppe, -OR₃₈, -SR₃₉, -NR₃₈R₃₉, -NR₄₀NR₃₈R₃₉ oder -NR₃₈OR₃₉ ist oder R₄₁ und R₃₆, zusammen mit dem Atom, an das sie gebunden sind, eine Heterocycloalkylgruppe bilden, und R₃₇ eine Alkylgruppe, eine Arylgruppe oder -NR₃₈R₃₉ ist;
worin R₃₈, R₃₉ und R₄₀ unabhängig voneinander H, eine Alkylgruppe, eine Cycloalkylgruppe, eine Heterocycloalkylgruppe, eine Arylgruppe, eine Heteroarylgruppe oder eine Acylgruppe sind oder worin beliebige zwei von R₃₈, R₃₉ und R₄₀ zusammen mit dem (den) Atom(en), an das (die) sie gebunden sind, eine Heterocycloalkylgruppe bilden,
n 0, 1 oder 2 ist;
R₃₃ H oder eine Alkylgruppe ist;
R₃₄ eine Alkylgruppe, eine Cycloalkylgruppe, eine Heterocycloalkylgruppe, eine Arylgruppe, eine Heteroarylgruppe, ein O-Alkyl, eine O-Cycloalkylgruppe, eine O-Heterocycloalkylgruppe, eine O-Arylgruppe, eine O-Heteroarylgruppe, eine S-Alkylgruppe, eine NH-Alkylgruppe, eine NH-Arylgruppe, eine N,N-Dialkylgruppe oder eine N,N-Diarylgruppe ist; und
Z und Z₁ unabhängig voneinander H, F, eine Alkylgruppe, eine Cycloalkylgruppe, eine Heterocycloalkylgruppe, eine Arylgruppe, eine Heteroarylgruppe, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃ oder -C(S)NR₂₁NR₂₂R₂₃ sind;
worin R₂₁, R₂₂, R₂₃ und R₂₄ unabhängig voneinander H, eine Alkylgruppe, eine Cycloalkylgruppe, eine Heterocycloalkylgruppe, eine Arylgruppe, eine Heteroarylgruppe, eine Acylgruppe oder eine Thioacylgruppe sind, oder worin beliebige zwei von R₂₁, R₂₂, R₂₃ und R₂₄ zusammen mit dem (den) Atom(en), an das (die) sie gebunden sind, eine Heterocycloalkylgruppe, bilden,
oder Z und Z₁ zusammen mit den Atomen, an die sie gebunden sind, eine Heterocycloalkylgruppe bilden;
oder ein pharmazeutisch annehmbares Salz oder Solvat davon.

19. Verbindung nach Anspruch 18, worin Z und Z₁ unabhängig voneinander H, eine Arylgruppe oder eine Heteroarylgruppe, -C(O)R₂₁, -CO₂R₂₁, -CN, -C(O)NR₂₁R₂₂, -C(O)NR₂₁OR₂₂, -C(S)R₂₁, -C(S)NR₂₁R₂₂. -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃ oder -C(S)NR₂₁NR₂₂R₂₃ sind;
worin R₂₁, R₂₂ und R₂₃ unabhängig voneinander H, eine Alkylgruppe, eine Cycloalkylgruppe, eine Heterocycloalkylgruppe, eine Arylgruppe, eine Heteroarylgruppe oder eine Acylgruppe sind, oder worin beliebige zwei von R₂₁, R₂₂ und R₂₃ zusammen mit dem (den) Atom(en), an das (die) sie gebunden sind, eine Heterocycloalkylgruppe bilden,
oder Z und Z₁ zusammen mit den Atomen, an die sie gebunden sind, eine Heterocycloalkylgruppe bilden;
oder ein pharmazeutisch annehmbares Salz oder Solvat davon.

20. Verbindung nach Anspruch 18, worin R₃₂ ausgewählt ist aus einer der folgenden Komponenten: 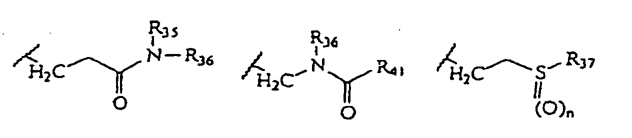 worin
R₃₅ H, eine Alkylgruppe, eine Arylgruppe, -OR₃₈, -SR₃₉, -NR₃₈R₃₉, -NR₄₀NR₃₈R₃₉ oder -NR₃₈OR₃₉ ist, und
R₃₆ H oder eine Alkylgruppe ist,
oder R₃₅ und R₃₆ zusammen mit dem Atom, an das sie gebunden sind, eine Heterocycloalkylgruppe oder eine Heteroarylgruppe bilden;
R₃₇ eine Alkylgruppe, eine Arylgruppe oder -NR₃₈R₃₉ ist;
worin R₃₈, R₃₉ und R₄₀ unabhängig voneinander H, eine Alkylgruppe, eine Cycloalkylgruppe, eine Heterocycloalkylgruppe, eine Arylgruppe, eine Heteroarylgruppe oder eine Acylgruppe sind oder worin beliebige zwei von R₃₈, R₃₉ und R₄₀ zusammen mit dem (den) Atom(en), an das (die) sie gebunden sind, eine Heterocycloalkylgruppe bilden,
n 0, 1 oder 2 ist;
oder ein pharmazeutisch annehmbares Salz oder Solvat davon.

21. Verbindung gemäß Formel (I) mit der Formel III: 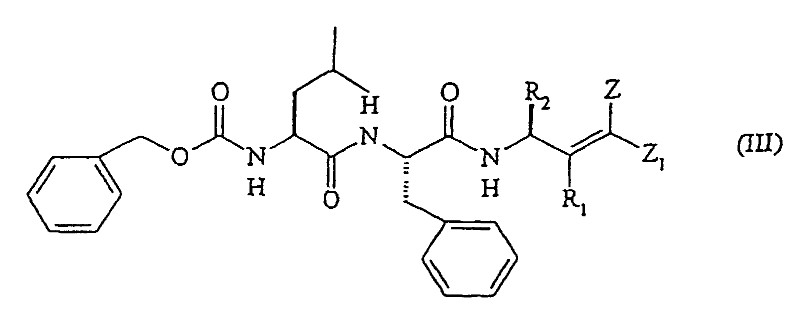 worin
R₂ CH₂CH₂C(O)NHCPh₃ ist, R₁ H ist, Z H ist und Z₁ CO₂CH₂CH₃ ist,
R₂ CH₂CH₂C(O)NH₂ ist, R₁ H ist, Z H ist und Z₁ CO₂CH₂CH₃ ist,
R₂ CH₂NHC(O)CH₃ ist, R₁ H ist, Z H ist und Z₁ CO₂CH₂CH₃ ist,
R₁ H ist, Z H ist und Z₁ CO₂CH₂CH₃ ist, und R₂ ist 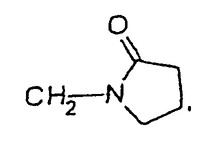 R₂ CH₂CH₂C(O)NH₂ ist, R₁ H ist, Z CO₂CH₃ ist und Z₁ H ist,
R₂ CH₂CH₂C(O)NH₂ ist, R₁ H ist, Z H ist und Z₁ CO₂CH₃ ist,
R₂ CH₂CH₂S(O)CH₃ ist, R₁ H ist, Z H ist und Z₁ CO₂CH₂CH₃ ist,
R₂ CH₂CH₂C(O)NH₂ ist, R₁ H ist, Z H ist und Z₁ C(O)CH₃ ist,
R₂ CH₂CH₂C(O)NH₂ ist, R₁ H ist, Z H ist und Z₁ CN ist,
R₂ CH₂NHC(O)NH₂ ist, R₁ H ist, Z H ist und Z₁ CO₂CH₂CH₃ ist,
R₂ CH₂CH₂C(O)NH₂ ist, R₁ H ist, Z H ist und Z₁ CO₂CH(CH₃)₂ ist,
R₂ CH₂CH₂C(O)NH₂ ist, R₁ H ist, Z H ist und Z₁ ist 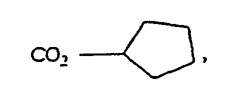 R₂ CH₂CH₂C(O)NH₂ ist, R₁ H ist, Z H ist und Z₁ ist 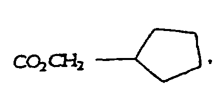 R₂ CH₂CH₂C(O)NH₂ ist, R₁ H ist, Z H ist und Z₁ ist 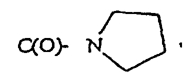 R₂ CH₂CH₂C(O)NH₂ ist, R₁ H ist, Z H ist und Z₁ C(O)N(CH₃)₂ ist,
R₂ CH₂CH₂C(O)NH₂ ist, R₁ H ist, Z H ist und Z₁ C(O)Ph ist,
R₂ CH₂CH₂C(O)NH₂ ist, R₁ H ist, Z H ist und Z₁ ist 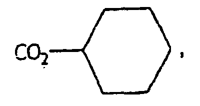 R₂ CH₂CH₂C(O)NH₂ ist, R₁ H ist, Z H ist und Z₁ ist 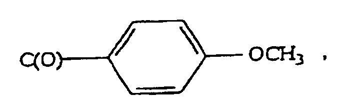 R₂ CH₂CH₂C(O)NH₂ ist, R₁ H ist, Z H ist und Z₁ ist 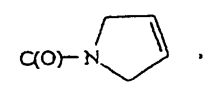 R₂ CH₂CH₂C(O)NH₂ ist, R₁ H ist, Z H ist und Z₁ ist 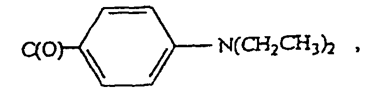 R₂ CH₂CH₂C(O)NH₂ ist, R₁ H ist, Z H ist und Z₁ CH₂Cl ist, oder
R₂ CH₂CH₂C(O)NH₂ ist, R₁ H ist, Z H ist und Z₁ ist 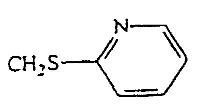 oder ein pharmazeutisch annehmbares Salz oder Solvat davon.

22. Verbindung gemäß Formel (I) mit der Formel IV: 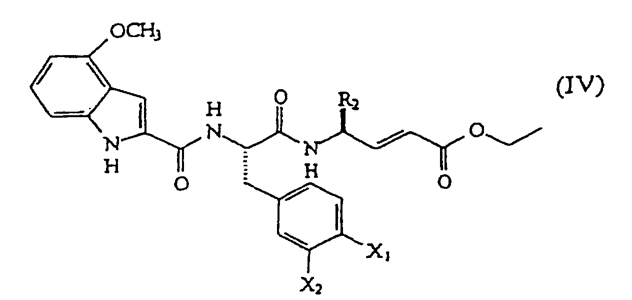 worin
R₂ CH₂CH₂C(O)NH₂ ist, X₁ H, F oder CI ist, und X₂ H, F oder CI ist; oder ein pharmazeutisch annehmbares Salz oder Solvat davon.

23. Verbindung nach Anspruch 22, worin X₁ CI ist und X₂ H ist; X₁ F ist und X₂ H ist; oder X₁ H ist und X₂ F ist; oder ein pharmazeutisch annehmbares Salz oder Solvat davon.

24. Verbindung gemäß Formel (I) mit der Formel V: 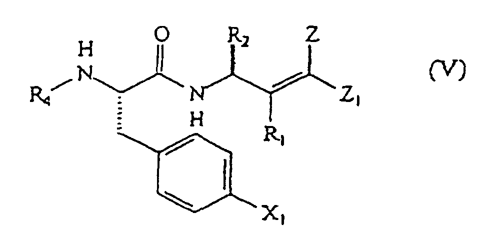 worin:
R₄ PhCH₂OC(O) ist, X₁ H ist, R₂ CH₂CH₂C(O)NH₂ ist, R₁ H ist, Z H ist und Z₁ CO₂CH₂CH₃ ist; oder
R₄ CH₃CH₂CH₂SO₂ ist, X₁ H ist, R₂ CH₂CH₂C(O)NH₂ ist, R₁ H ist, Z H ist und Z₁ CO₂CH₂CH₃ ist; oder
R₄ PhCH₂SO₂ ist, X₁ H ist, R₂ CH₂CH₂C(O)NH₂ ist, R₁ H ist, Z H ist und Z₁ CO₂CH₂CH₃ ist; oder
R₄ CH₃CH₂SO₂ ist, X₁ H ist, R₂ CH₂CH₂C(O)NH₂ ist, R₁ H ist, Z H ist und Z₁ CO₂CH₂CH₃ ist; oder
R₄ PhSO₂ ist, X₁ H ist, R₂ CH₂CH₂C(O)NH₂ ist, R₁ H ist, Z H ist und Z₁ CO₂CH₂CH₃ ist;
oder ein pharmazeutisch annehmbares Salz oder Solvat davon.

25. Verbindung gemäß Formel (I) mit der Formel VI oder VII: 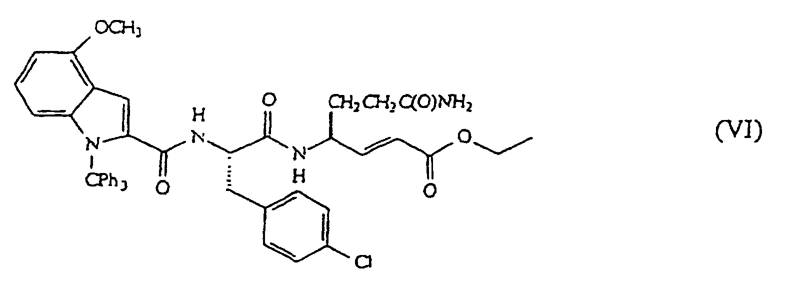 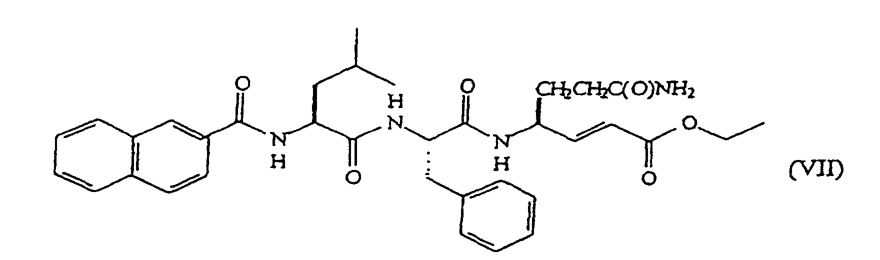 oder ein pharmazeutisch annehmbares Salz oder Solvat davon.

26. Verbindung gemäß Formel (I) mit der Formel VIII: 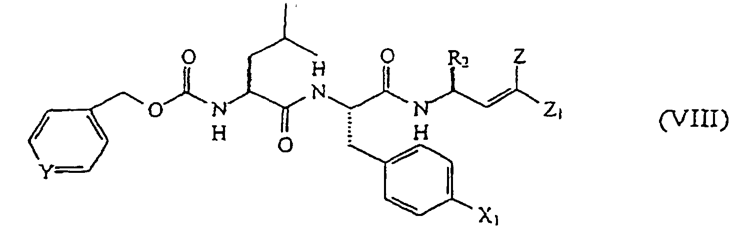 worin
X₁ F ist, R₂ CH₂CH₂C(O)NH₂ ist, Y CH ist, Z H ist und Z₁ CO₂CH₂CH₃ ist; oder
X₁ H ist, R₂ CH₂CH₂C(O)NH₂ ist, Y N ist, Z H ist und Z₁ CO₂CH₂CH₃ ist; oder
X₁ H ist, R₂ CH₂CH₂C(O)NH₂ ist, Y CH ist, Z H ist und Z₁ C(O)N(CH₃)OCH₃ ist;
oder ein pharmazeutisch annehmbares Salz oder Solvat davon.

27. Verbindung gemäß Formel (I) mit der Formel III: 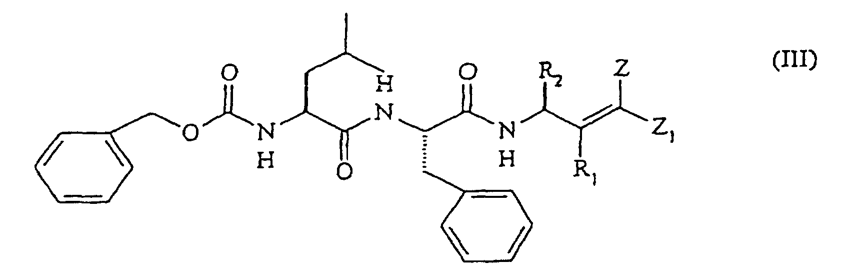 worin R₂ CH₂CH₂C(O)NH₂ ist, R₁ H ist, Z CH₃ ist und Z₁ CO₂CH₂CH₃ ist,
R₂ CH₂CH₂C(O)NH₂ ist, R₁ H ist und Z und Z₁ zusammen 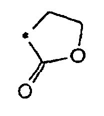 bilden,
oder worin R₂ CH₂CH₂C(O)NH₂ ist, R₁ H ist, Z H ist und Z₁ ausgewählt ist aus: 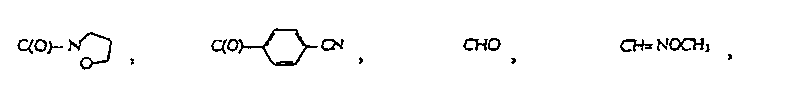 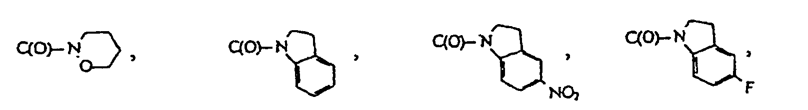 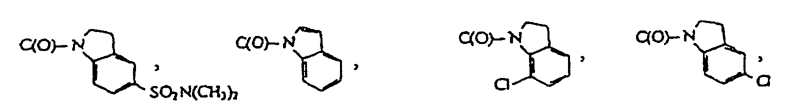 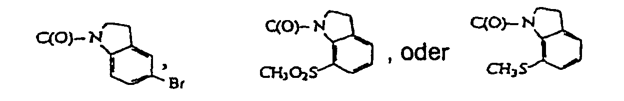 oder ein pharmazeutisch annehmbares Salz oder Solvat davon.

28. Verbindung gemäß Formel (I) mit der Formel XIV: 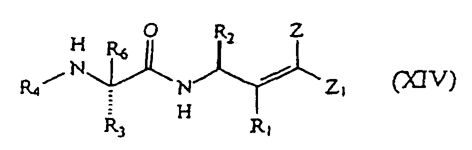 worin R₆ H ist, R₁ H ist, R₂ CH₂CH₂C(O)NH₂ ist, Z H ist, Z₁ CO₂CH₂CH₃ ist und R₃ CH₂Ph ist und R₄ 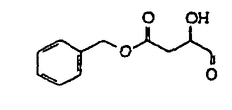 ist R₃ 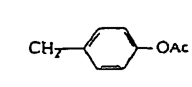 ist und R₄ 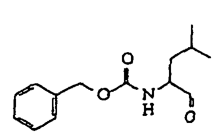 ist
R₃ CH₂Ph ist und R₄ 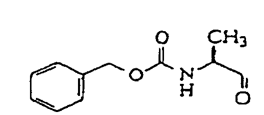 ist
R₃ CH₂Ph ist und R₄ 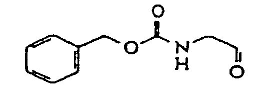 ist
R₃ 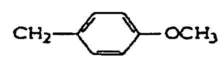 ist und R₄ 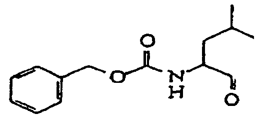 ist
R₃ CH₂Ph ist und R₄ 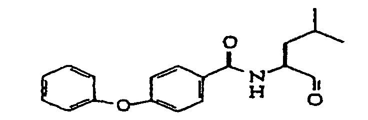 ist
R₃ 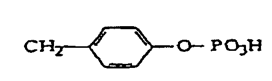 ist und R₄ 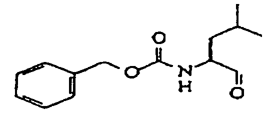 ist
R₃ CH₂Ph ist und R₄ 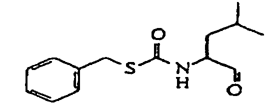 ist
R₃ CH₂Ph ist und R₄ 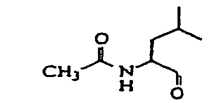 ist
R₃ CH₂CH₃ ist und R₄ 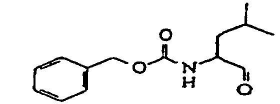 ist
R₃ CH₃ ist und R₄ 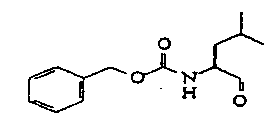 ist
R₃ CH₂Ph ist und R₄ 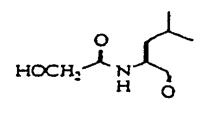 ist
R₃ 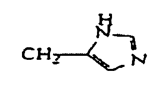 ist und R₄ 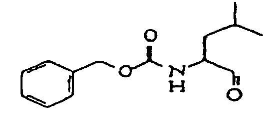 ist R₃ 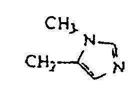 ist und R₄ 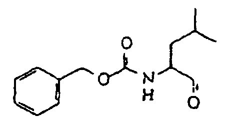 ist
R₃ 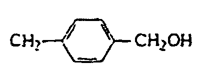 ist und R₄ 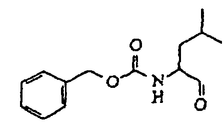 ist
R₃ CH₂Ph ist und R₄ 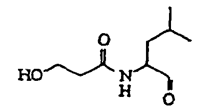 ist
R₃ CH₂Ph ist und R₄ 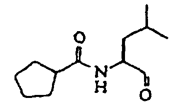 ist
R₃ CH₂Ph ist und R₄ 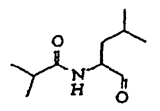 ist
R₃ CH₂Ph ist und R₄ 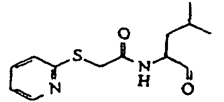 ist
R₃ CH₂Ph ist und R₄ 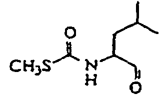 ist
oder R₃ CH₂Ph ist und R₄ 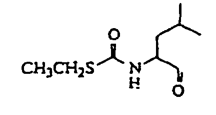 ist
oder ein pharmazeutisch annehmbares Salz oder Solvat davon.

29. Verbindung gemäß Formel (I) mit der Formel XIV: 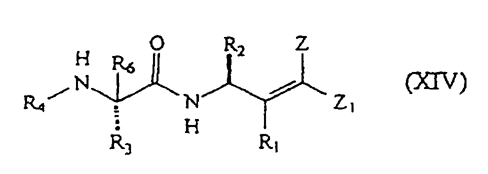 worin R₆ H ist, R₁ H ist, R₃ CH₂Ph ist, R₂ CH₂CH₂C(O)NH₂ ist, Z und Z₁ zusammen 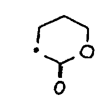 bilden
und R₄ 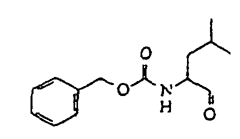 ist
oder ein pharmazeutisch annehmbares Salz oder Solvat davon.

30. Verbindung gemäß Formel (I) mit der Formel XIV: 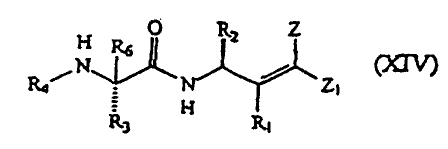 worin R₆ H ist, R₁ H ist, R₂ CH₂CH₂C(O)NH₂ ist, R₄ 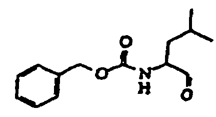 ist und
R₃ ist 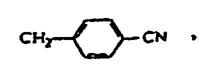 Z ist H und Z₁ ist CO₂CH₂CH₃
R₃ ist CH(OH)CH₃, Z ist H und Z₁ ist CO₂CH₂CH₃
R₃ ist 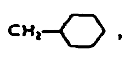 Z ist H und Z₁ ist CO₂CH₂CH₃
R₃ ist CH₂Ph, Z ist H und Z₁ ist C(O)N(OH)CH₃
R₃ ist 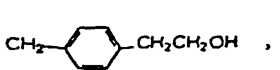 Z ist H und Z₁ ist CO₂CH₂CH₃
R₃ ist 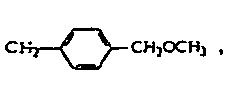 Z ist H und Z₁ ist CO₂CH₂CH₃ R₃ ist CH₂CH(CH₃)₂, Z ist H und Z₁ ist CO₂CH₂CH₃
R₃ ist CH₂SCH₃, Z ist H und Z₁ ist CO₂CH₂CH₃
oder R₃ ist CH₂SCH₂CH₃, Z ist H und Z₁ ist CO₂CH₂CH₃
oder ein pharmazeutisch annehmbares Salz oder Solvat davon.

31. Verbindung gemäß Formel (I) mit der Formel IX: 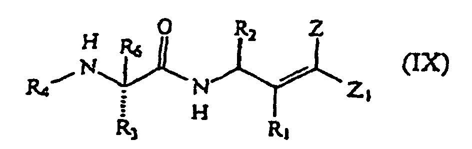 worin R₆ H ist, R₁ H ist, R₂ CH₂CH₂C(O)NH₂ ist, Z H ist und Z₁ ist CO₂CH₂CH₃, R₃ ist CH₂Ph und R₄ ist 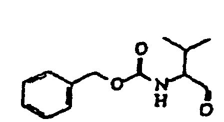 Z₁ ist CO₂CH₂CH₃, R₃ ist CH₂Ph und R₄ ist 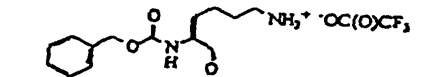 Z₁ ist CO₂CH₂CH₃, R₃ ist CH₂Ph und R₄ ist 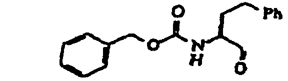 Z₁ ist CO₂CH₂CH₃, R₃ ist CH₂Ph und R₄ ist 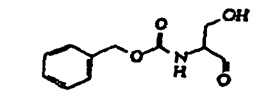 Z₁ ist CO₂CH₂CH₃, R₃ ist CH₂Ph und R₄ ist 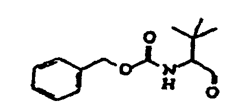 Z₁ ist CO₂CH₂CH₃, R₃ ist CH₂Ph und R₄ ist 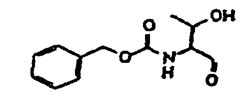 Z₁ ist CO₂CH₂CH₃, R₃ ist CH₂Ph und R₄ ist 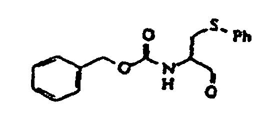 Z₁ ist CO₂CH₂CH₃, R₃ ist CH₂Ph und R₄ ist 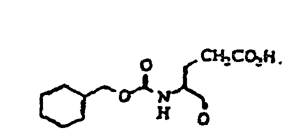 oder Z₁ ist CO₂CH₂CH₃, R₃ ist 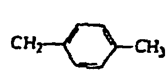 und R₄ ist 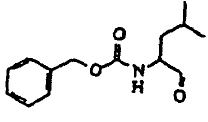 oder ein pharmazeutisch annehmbares Salz oder Solvat davon.

32. Verbindung gemäß Formel (I) mit der Formel IX: 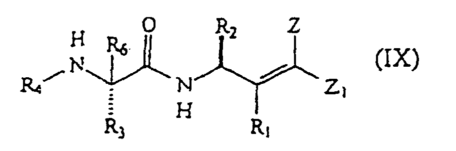 worin R₆ H ist, R₃ CH₂Ph ist, R₂ CH₂CH₂C(O)NH₂ ist und
R₁ H ist, Z H ist, Z₁ CO₂CH₂CH₃ ist und R₄ 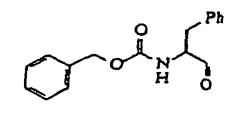 ist
R₁ H ist, Z H ist, Z₁ CO₂CH₂CH₃ ist und R₄ 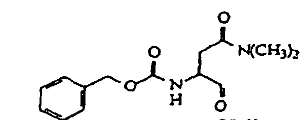 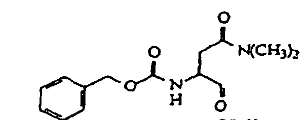 ist
R₁ H ist, Z H ist, Z₁ CO₂CH₂CH₃ ist und R₄ 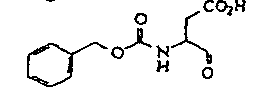 ist
R₁ H ist, Z H ist, Z₁ 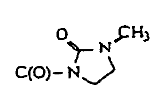 ist und R₄ 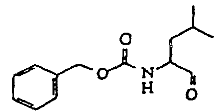 ist
R₁ H ist, Z H ist, Z₁ CO₂CH₂CH₃ ist und R₄ 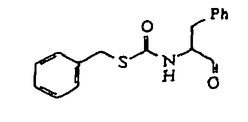 ist
R₁ H ist, Z H ist, Z₁ CO₂CH₂CH₃ ist und R₄ 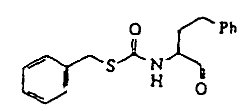 ist
R₁ H ist, Z H ist, Z₁ CO₂CH₂C(CH₃)₃ ist und R₄ 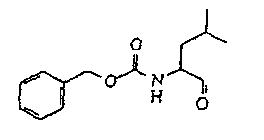 ist
R₁ H ist, Z und Z₁ zusammen 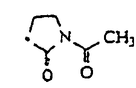 bilden und R₄ 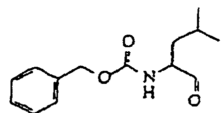 ist
R₁ H ist, Z H ist, Z₁ CO₂CH₂CH₃ ist und R₄ 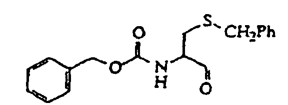 ist
R₁ H ist, Z H ist, Z₁ CO₂CH₂CH₃ ist und R₄ 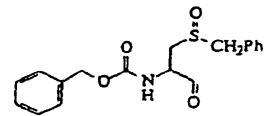 ist
R₁ H ist, Z H ist, Z₁ CO₂CH₂CH₃ ist und R₄ 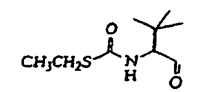 ist
R₁ H ist, Z CH₃ ist, Z₁ CO₂CH₂CH₃ ist und R₄ 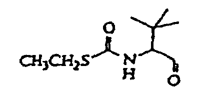 ist
R₁ H ist, Z und Z₁ zusammen 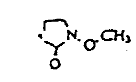 bilden und R₄ 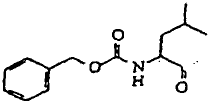 ist
R₁ H ist, Z H ist, Z₁ CO₂CH₂CH₃ ist und R₄ 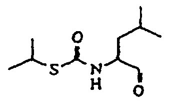 ist
R₁ H ist, Z CH₃ ist, Z₁ CO₂CH₂CH₃ ist und R₄ 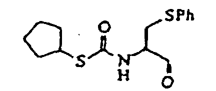 ist
R₁ H ist, Z H ist, Z₁ CO₂CH₂CH₃ ist und R₄ 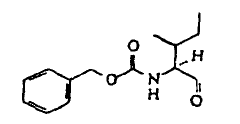 ist
oder R₁ H ist, Z H ist, Z₁ CO₂CH₂CH₃ ist und R₄ 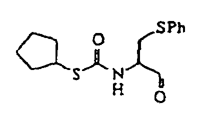 ist
oder ein pharmazeutisch annehmbares Salz oder Solvat davon.

33. Verbindung gemäß Formel (I) mit der Formel IX: 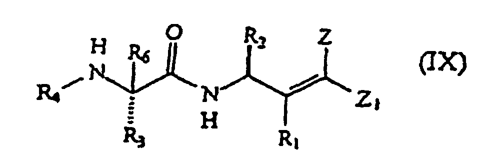 worin R₆ H ist, R₂ CH₂CH₂C(O)NH₂ ist, R₁ H ist und Z ist H, Z₁ ist CO₂CH₂CH₃, R₃ ist 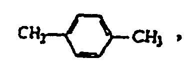 und R₄ ist 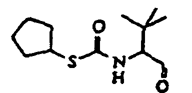 Z ist CH₃, Z₁ ist CO₂CH₂CH₃, R₃ ist 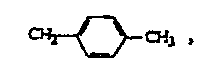 und R₄ ist 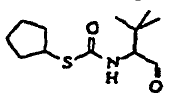 Z ist H, Z₁ ist CO₂CH₂CH₃, R₃ ist 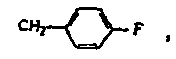 und R₄ ist 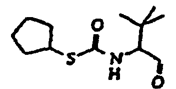 Z ist CH₃, Z₁ ist CO₂CH₂CH₃, R₃ ist 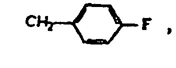 und R₄ ist 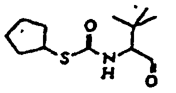 Z ist H, Z₁ ist CO₂CH₂CH₃, R₃ ist CH₂Ph und R₄ ist 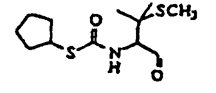 Z ist H, Z₁ ist CO₂CH₂CH₃, R₃ ist CH₂Ph und R₄ ist 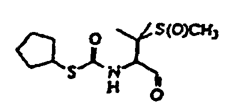 Z ist H, Z₁ ist CO₂CH₂CH₃, R₃ ist CH₂Ph und R₄ ist 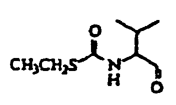 Z und Z₁ bilden zusammen 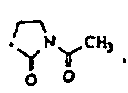 R₃ ist 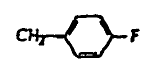 und R₄ ist 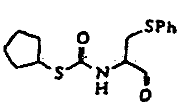 Z ist H, Z₁ ist CO₂CH₂CH₃, R₃ ist CH₂Ph und R₄ ist 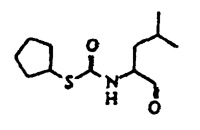 Z ist H, Z₁ ist CO₂CH₂CH₃, R₃ ist 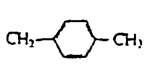 und R₄ ist 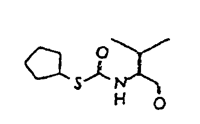 Z ist H, Z₁ ist CO₂CH₂CH₃, R₃ ist 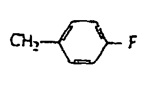 und R₄ ist 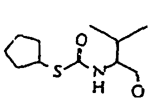 oder Z ist H, Z₁ ist CO₂CH₂CH₃, R₃ ist 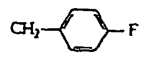 und R₄ ist 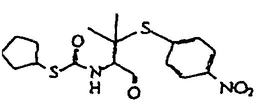 oder ein pharmazeutisch annehmbares Salz oder Solvat davon.

34. Pharmazeutische Zusammensetzung, umfassend:
(a) eine therapeutisch wirksame Menge einer Verbindung wie in einem der vorangehenden Ansprüche definiert oder eines pharmazeutisch annehmbaren Salzes oder Solvats davon; und
(b) einen pharmazeutisch annehmbaren Träger, ein pharmazeutisch annehmbares Verdünnungsmittel, Vehikel oder Excipiens.

35. Verwendung einer Verbindung wie in einem der Ansprüche 1 bis 33 definiert oder eines pharmazeutisch annehmbaren Salzes oder Solvats davon zur Herstellung eines Medikaments für die Behandlung eines durch picomavirale Proteaseaktivität vermittelten Krankheitszustands bei einem Säuger.

36. Verfahren zum Hemmen der Aktivität einer picornaviralen 3C-Protease, welches das Inkontaktbringen der picomaviralen 3C-Protease zum Zweck des Hemmens mit einer wirksamen Menge einer Verbindung wie in einem der Ansprüche 1 bis 33 definiert oder eines pharmazeutisch annehmbaren Salzes oder Solvats davon umfasst.

37. Verfahren zum Hemmen der Aktivität einer rhinoviralen Protease, welches das Inkontaktbringen der rhinoviralen Protease zum Zweck des Hemmens mit einer wirksamen Menge einer Verbindung wie in einem der Ansprüche 1 bis 33 definiert oder eines pharmazeutisch annehmbaren Salzes oder Solvats davon umfasst.

38. Verfahren zum Herstellen einer Verbindung nach Anspruch 1, umfassend das Umwandeln einer Verbindung der Formel Q 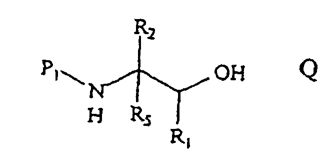 worin R₁, R₂ und R₅ wie in Anspruch 1 definiert sind und P₁ eine Schutzgruppe ist, oder eines Salzes oder Solvats davon, in eine Verbindung der Formel I, wie in Anspruch 1 definiert, oder ein pharmazeutisch annehmbares Salz oder Solvat davon.

39. Verfahren nach Anspruch 38, worin P₁ Benzyloxycarbonyl oder t-Butoxycarbonyl ist.

40. Verfahren zum Herstellen einer Verbindung nach Anspruch 1, umfassend das Umwandeln einer Verbindung der Formel B: 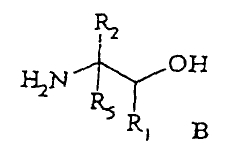 worin R₁, R₂ und R₅ wie in Anspruch 1 definiert sind, oder eines Salzes oder Solvats davon, in eine Verbindung der Formel I, wie in Anspruch 1 definiert, oder ein pharmazeutisch annehmbares Salz oder Solvat davon.

41. Verfahren zum Herstellen einer Verbindung nach Anspruch 1, umfassend das Umwandeln einer Verbindung der Formel O: 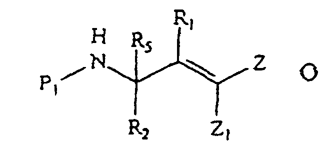 worin R₁, R₂, R₅, Z und Z₁ wie in Anspruch 1 definiert sind und P₁ eine Schutzgruppe ist, oder eines Salzes oder Solvats davon, in eine Verbindung der Formel I, wie in Anspruch 1 definiert, oder ein pharmazeutisch annehmbares Salz oder Solvat davon.

42. Verfahren nach Anspruch 41, worin P₁ Benzyloxycarbonyl oder t-Butoxycarbonyl ist.

43. Verfahren zum Herstellen einer Verbindung nach Anspruch 1, umfassend das Umwandeln einer Verbindung der Formel P: 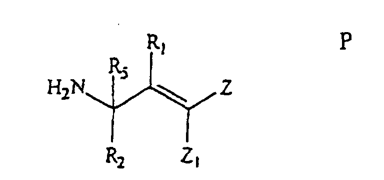 worin R₁, R₂, R₅, Z und Z₁ wie in Anspruch 1 definiert sind, oder eines Salzes oder Solvats davon, in eine Verbindung der Formel I, wie in Anspruch 1 definiert, oder ein pharmazeutisch annehmbares Salz oder Solvat davon.

44. Verbindungen ausgewählt aus der Gruppe bestehend aus:
Verbindungen 46 - 66 und 78 mit der Formel IX: 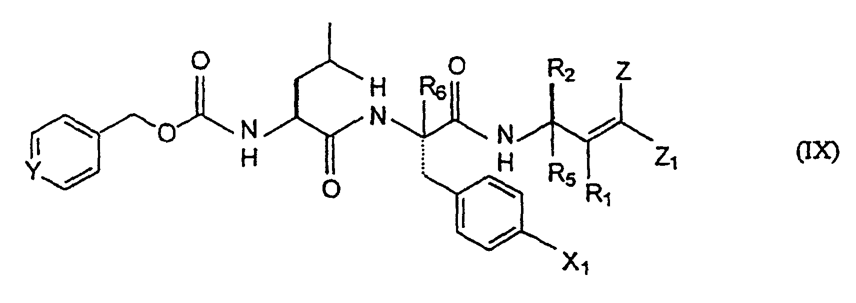
46. R₁ ist H; R₂ ist CH₂CH₂C(O)NH₂; R₅, R₆ und X₁ sind H; Y ist CH; Z ist H; und Z₁ ist 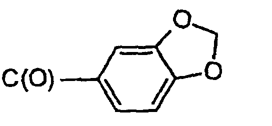
47. R₁ ist H; R₂ ist CH₂CH₂C(O)NH₂; R₅, R₆ und X₁ sind H; Y ist CH; Z ist H; und Z₁ ist 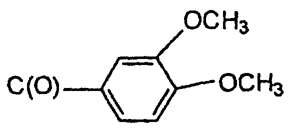
48. R₁ ist H; R₂ ist CH₂CH₂C(O)NH₂; R₅, R₆ und X₁ sind H; Y ist CH; Z ist H; und Z₁ist 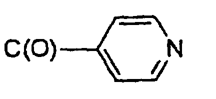
49. R₁ ist H; R₂ ist CH₂CH₂C(O)NH₂; R₅, R₆ und X₁ sind H; Y ist CH; Z ist H; und Z₁ ist 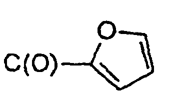
50. R₁ ist H; R₂ ist CH₂CH₂C(O)NH₂; R₅, R₆ und X₁ sind H; Y ist CH; Z ist H; und Z₁ ist 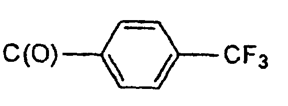
51. R₁ ist H; R₂ ist CH₂CH₂C(O)NH₂; R₅, R₆ und X₁ sind H; Y ist CH; Z ist H; und Z₁ is 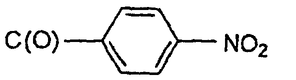
52. R₁ ist H; R₂ ist CH₂CH₂C(O)NH₂; R₅, R₆ und X₁ sind H; Y ist CH; Z ist H; und Z₁ ist C(O)tBu
53. R₁ ist H; R₂ ist CH₂CH₂C(O)NH₂; R₅ und R₆ sind H; X₁ ist OH; Y ist CH; Z ist H; und Z₁ ist CO₂CH₂CH₃
54. R₁ ist H; R₂ ist CH₂CH₂C(O)NH₂; R_{5,} R₆ und X₁ sind H; Y ist CH; Z ist H; und Z₁ ist C(O)C(O)CH₃
55. R₁ ist H; R₂ ist CH₂CH₂C(O)NH₂; R_{5,} R₆ und X₁ sind H; Y ist CH; Z ist H; und Z₁ ist C(O)C(O)N(CH₃)₂
56. R₁ ist H; R₂ ist CH₂OC(O)NH₂; R_{5,} R₆ und X₁ sind H; Y ist CH; Z ist H; und Z₁ ist CO₂CH₂CH₃
57. R₁ ist H; R₂ ist CH₂CH₂C(O)NH₂; R₅, R₆ und X₁ sind H; Y ist CH; Z ist H; und Z und Z₁ bilden zusammen 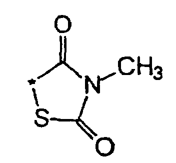 wobei das S vorzugsweise trans zu der R₁-Gruppe angeordnet ist.
58. R₁ ist H; R₂ ist CH₂CH₂C(O)NH₂; R₅, R₆ und X₁ sind H; Y ist CH; und Z und Z₁ bilden zusammen 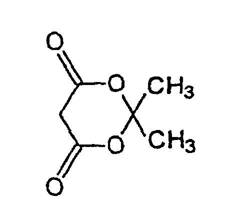
59. R₁ ist H; R₂ ist CH₂CH₂C(O)NH₂; R₅, R₆ und X₁ sind H; Y ist CH; Z ist H; und Z₁ ist C(O)NHPh
60. R₁ ist H; R₂ ist CH₂CH₂C(O)NH₂; R₅, R₆ und X₁ sind H; Y ist CH; Z ist H; und Z₁ ist C(O)N(CH₃)Ph
61. R₁ ist H; R₂ ist CH₂CH₂C(O)NH₂; R₅, R₆ und X₁ sind H; Y ist CH; Z ist H; und Z₁ ist 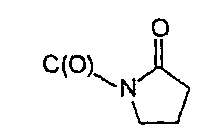
62. R₁ ist H; R₂ ist CH₂CH₂C(O)NH₂; R₅, R₆ und X₁ sind H; Y ist CH; Z ist H; und Z₁ ist 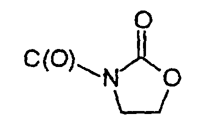
63. R₁, R₅, R₆, X₁ und Z sind H; Y ist CH; R₂ ist CH₂CH₂C(O)NH₂; und Z₁ ist 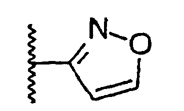
64. R₁, R₅, R₆, X₁ und Z sind H; Y ist CH; R₂ ist CH₂CH₂C(O)NH₂; und Z₁ ist 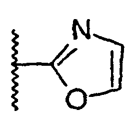
65. R₁, R_{5.} R₆, X₁ und Z sind H; Y ist CH; R₂ ist CH₂CH₂C(O)NH₂; und Z₁ ist 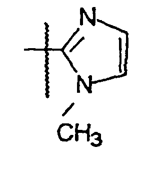
66. R₁, R₅, R₆, X₁ und Z sind H; Y ist CH; R₂ ist CH₂CH₂C(O)NH₂; und Z₁ ist 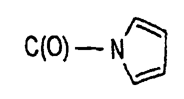
78. R₁, R₅, R₆ und X₁ sind H; Y ist CH; R₂ ist CH₂CH₂C(O)NH₂; Z ist CH₂Cl; und Z₁ ist H;
Verbindungen 67 - 69 mit der Formel X: 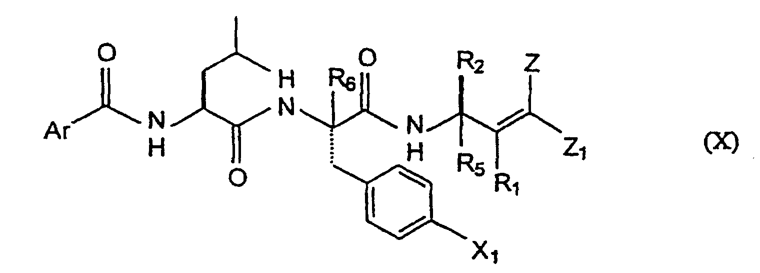
67. R₁, R₅, R₆, X₁ und Z sind H; R₂ ist CH₂CH₂C(O)NH₂; Z₁ ist CO₂CH₂CH₃; und Ar ist Ph
68. R₁, R₅, R₆, X₁ und Z sind H; R₂ ist CH₂CH₂C(O)NH₂; Z₁ ist CO₂CH₃; und Ar ist 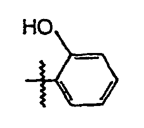
69. R₁, R₅, R₆, X₁ und Z sind H; R₂ ist CH₂CH₂C(O)NH₂; Z₁ ist CO₂CH₂CH₃; und Ar ist 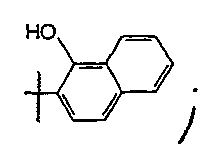
Verbindungen 70 - 73 mit der Formel XI: 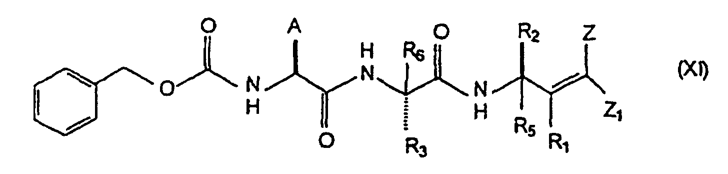
70. R₁, R₅, R₆ und Z sind H; R₂ ist CH₂CH₂C(O)NH₂; R₃ ist CH₂Ph; Z₁ ist CO₂CH₂CH₃; und A ist 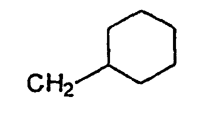
71. R₁, R₅, R₆ und Z sind H; R₂ ist CH₂CH₂C(O)NH₂; R₃ ist CH₂Ph; Z₁ ist CO₂CH₂CH₃; und A ist Ph
72. R₁, R₅, R₆ und Z sind H; R₂ ist CH₂CH₂C(O)NH₂; A ist CH₂CH(CH₃)₂; Z₁ ist CO₂CH₂CH₃; und R₃ ist 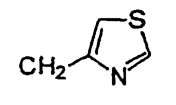
73. R₁, R₅. R₆ und Z sind H; R₂ ist CH₂CH₂C(O)NH₂; A ist CH₂CH(CH₃)₂; Z₁ ist CO₂CH₂CH₃; und R₃ ist 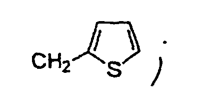
Verbindungen 1, 6, 8 - 10, 15, 20, 23, 38 - 40, 76 und 77 mit der Formel XII: 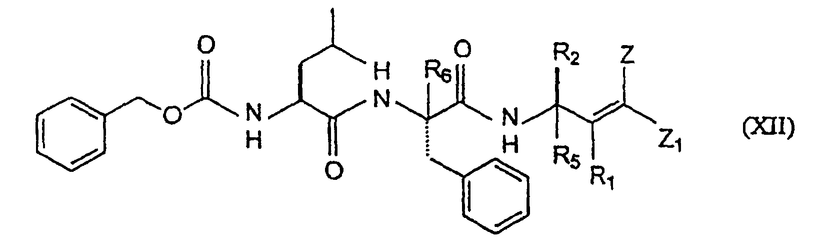
1. R₁ ist H; R₂ ist CH₂CH₂CN; R₅ ist H; R₆ ist H; Z ist F; und Z₁ ist CO₂CH₂CH₃
6. R₁ ist H; R₂ ist CH₂CH₂C(O)NH₂; R₅ ist H; R₆ ist H; Z ist H; und Z₁ ist C(O)NHCH₂CH₃
8. R₁ ist H; R₂ ist CH₂CH₂C(O)NH₂; R₅ ist H; R₆ ist H; Z ist F; und Z₁ ist CO₂CH₂CH₃
9. R₁ ist H; R₂ ist CH₂CH₂C(O)NH₂; R₅ ist H; R₆ ist H; Z ist H; und Z₁ ist SO₂CH₃
10. R₁ ist H; R₂ ist CH₂CH₂C(O)NH₂; R₅ ist H; R₆ ist H; Z ist H; und Z₁ ist SO₂Ph
15. R₁ ist H; R₂ ist CH₂CH₂C(O)NH₂; R₅ ist H; R₆ ist H; Z ist H; und Z₁ ist CO₂H
20. R₁ ist H; R₂ ist CH₂CH₂C(O)NH₂; R₅ ist H; R₆ ist H; Z ist H; und Z₁ ist PO(OCH₂CH₃)₂
23. R₁ ist H; R₂ ist CH₂CH₂C(O)NH₂; R₅ ist H; R₆ ist H; Z ist H; und Z₁ ist 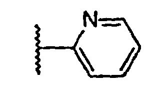
38. R₁ ist H; R₂ ist CH₂CH₂C(O)NH₂; R₅ ist H; R₆ ist H; Z ist H; und Z₁ ist 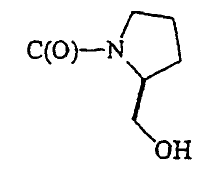
39. R₁ ist H; R₂ ist CH₂CH₂C(O)NH₂; R₅ ist H; R₆ ist H; Z ist H; und Z₁ ist 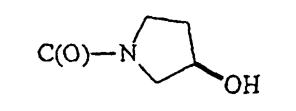
40. R₁ ist H; R₂ ist CH₂CH₂C(O)NH₂; R₅ ist H; R₆ ist H; Z ist H; und Z₁ ist 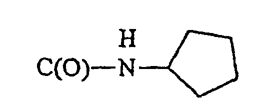
76. R₁ ist H; R₂ ist CH₂CH₂C(O)NH₂; R₅ ist H; R₆ ist H; Z ist H; und Z₁ ist CH₂OAc
77. R₁ ist H; R₂ ist CH₂CH₂C(O)NH₂; R₅ ist H; R₆ ist H; Z ist H; und Z₁ ist 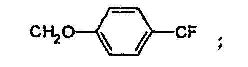
Verbindung 45 mit der Formel XIII:
45. 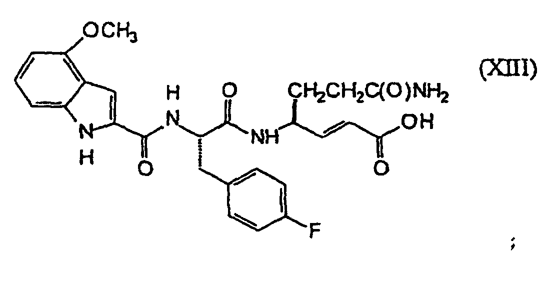
Verbindungen 79 - 97, ebenfalls mit der Formel III: 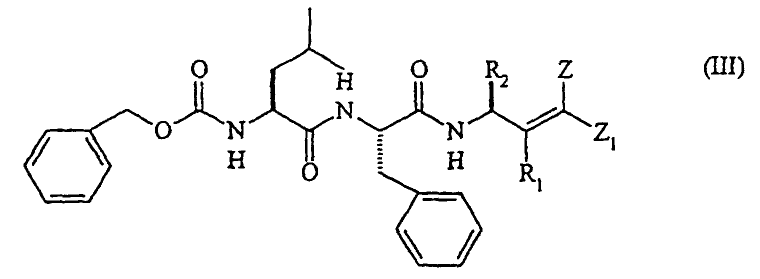
82. R₂ ist CH₂CH₂C(O)NH₂, R₁ ist H, Z ist CH₃ und Z₁ ist CO₂CH₂CH₃,
90. R₂ ist CH₂CH₂C(O)NH₂, R₁ ist H und Z und Z₁ bilden zusammen 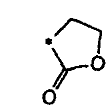 wobei C=O vorzugsweise cis zu der R₁-Gruppe angeordnet ist
oder worin R₂ CH₂CH₂C(O)NH₂ ist, R₁ H ist, Z H ist und Z₁ ausgewählt ist aus: 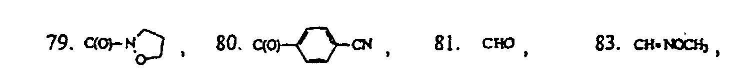 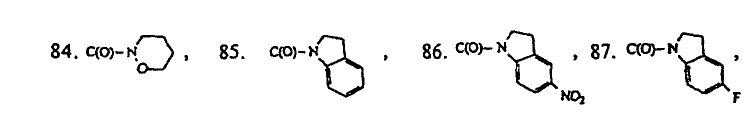 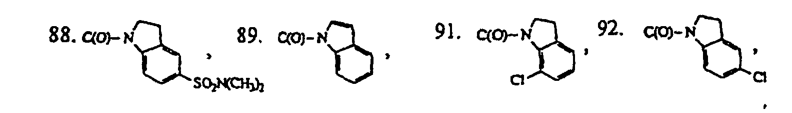 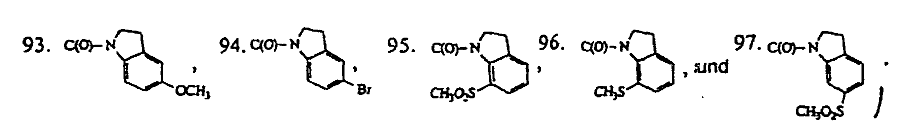
Verbindungen 98 - 121 mit der Formel XIV: 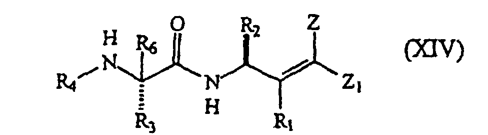 worin R₆ H ist, R₁ H ist, R₂ CH₂CH₂C(O)NH₂ ist, Z H ist, Z₁ CO₂CH₂CH₃ ist und
98. R₃ ist CH₂Ph und R₄ ist 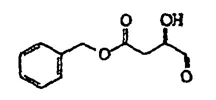
99. R₃ ist H und R₄ ist 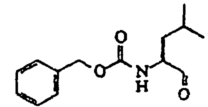
100. R₃ ist 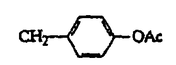 und R₄ ist 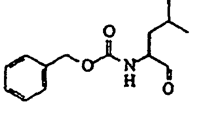
101. R₃ ist CH₂Ph und R₄ ist 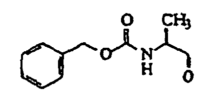
102. R₃ ist CH₂Ph und R₄ ist 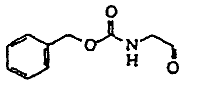
103. R₃ ist 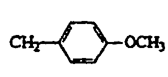 und R₄ ist 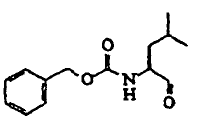
104. R₃ ist CH₂Ph und R₄ ist 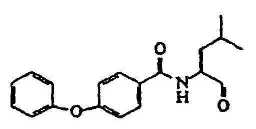
105. R₃ ist 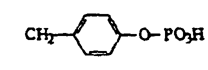 und R₄ ist 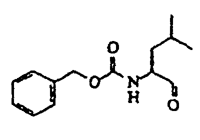
106. R₃ ist CH₂Ph und R₄ ist 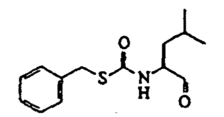
107. R₃ ist CH₂Ph und R₄ ist 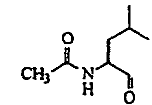
108. R₃ ist CH₂CH₃ und R₄ ist 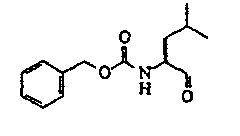
109. R₃ ist CH₃ und R₄ ist 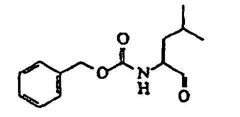
110. R₃ ist CH₂Ph und R₄ ist 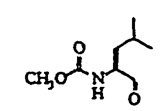
111. R₃ ist CH₂Ph und R₄ ist 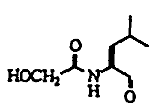
112. R₃ ist 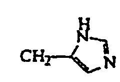 und R₄ ist 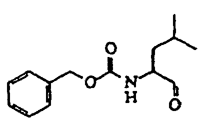
113. R₃ ist 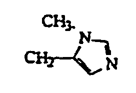 und R₄ ist 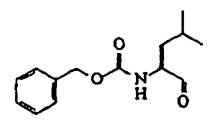
114. R₃ ist 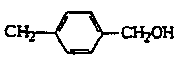 und R₄ ist 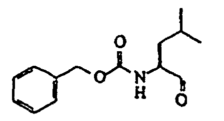
115. R₃ ist CH₂Ph und R₄ ist 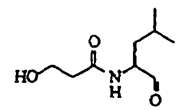
116. R₃ ist CH₂Ph und R₄ ist 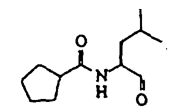
117. R₃ ist CH₂Ph und R₄ ist 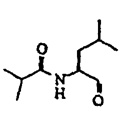
118. R₃ ist CH₂Ph und R₄ ist 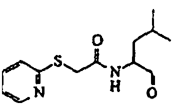
119. R₃ ist CH₂Ph und R₄ ist 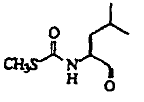
120. R₃ ist CH₂Ph und R₄ ist 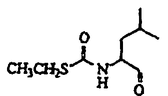
121. R₃ ist CH₂CH₂CO₂H und R₄ ist 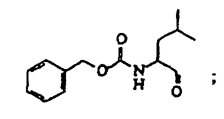
Verbindungen 122 - 130, ebenfalls mit der Formel XIV: 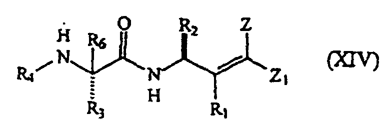 worin R₆ H ist, R₁ H ist, R₃ CH₂Ph ist und
122. R₂ ist CH₂OC(O)NHC(O)CH₂Cl, Z ist H, Z₁ ist CO₂CH₂CH₃ und R₄ ist 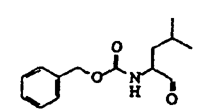
123. R₂ ist CH₂CH₂C(O)NH₂, Z ist H, Z₁ ist CO₂CH₂CH₃ und R₄ ist 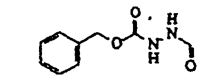
124. R₂ ist CH₂CH₂C(O)NH₂, Z ist H, Z₁ ist 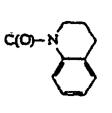 und R₄ ist 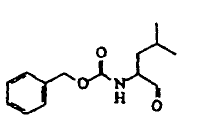
125. R₂ ist CH₂CH₂C(O)NH₂, Z ist H, Z₁ ist NO₂ und R₄ ist 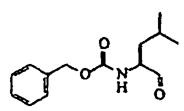
126. R₂ ist CH₂CH₂C(O)NH₂, Z ist H, Z₁ ist 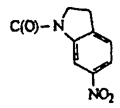 und R₄ ist 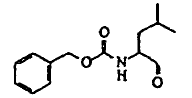
127. R₂ ist CH₂CH₂C(O)NH₂, Z ist H, Z₁ ist 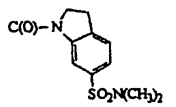 und R₄ ist 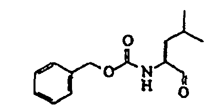
128. R₂ ist CH₂CH₂C(O)NH₂, Z ist H, Z₁ ist 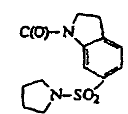 und R₄ ist 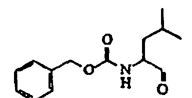
129. R₂ ist CH₂CH₂C(O)NH₂, Z ist H, Z₁ ist CO₂CH₂CH₃ und R₄ ist 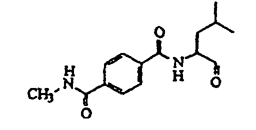
130. R₂ ist CH₂CH₂C(O)NH₂, Z und Z₁ bilden zusammen 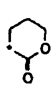 und R₄ ist 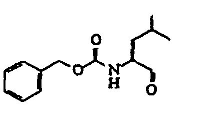 wobei C=O vorzugsweise cis zu der R₁-Gruppe angeordnet ist;
Verbindungen 131 - 145, ebenfalls mit der Formel XIV: 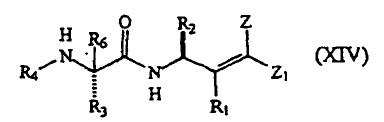 worin R₆ H ist, R₁ H ist, R₂ CH₂CH₂C(O)NH₂ ist, R₄ 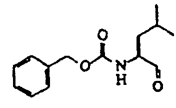 ist und
131. R₃ ist CH₂Ph, Z ist H und Z₁ ist 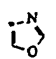
132. R₃ ist 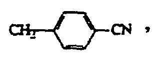 Z ist H und Z₁ ist CO₂CH₂CH₃
133. R₃ ist 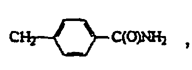 Z ist H und Z₁ ist CO₂CH₂CH₃
134. R₃ ist CH(OH)CH₃, Z ist H und Z₁ ist CO₂CH₂CH₃
135. R₃ ist 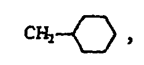 Z ist H und Z₁ ist CO₂CH₂CH₃
136. R₃ ist 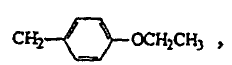 Z ist H und Z₁ ist CO₂CH₂CH₃
137. R₃ ist CH₂CH₂CH₃, Z ist H und Z₁ ist CO₂CH₂CH₃
138. R₃ ist CH₂Ph, Z ist H und Z₁ ist C(O)N(OH)CH₃
139. R₃ ist 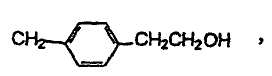 Z ist H und Z₁ ist CO₂CH₂CH₃
140. R₃ ist 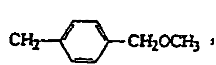 Z ist H und Z₁ ist CO₂CH₂CH₃
141. R₃ ist CH₂CH(CH₃)₂, Z ist H und Z₁ ist CO₂CH₂CH₃
142. R₃ ist CH₂SCH₃, Z ist H und Z₁ ist CO₂CH₂CH₃
143. R₃ ist CH₂SCH₂CH₃, Z ist H und Z₁ ist CO₂CH₂CH₃
144. R₃ ist CH₂Ph, Z ist CH₃ und Z₁ ist CO₂H
145. R₃ ist CH₂Ph, Z ist H und Z₁ ist 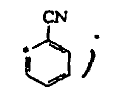
Verbindungen 146 - 155, ebenfalls mit der Formel XIV: 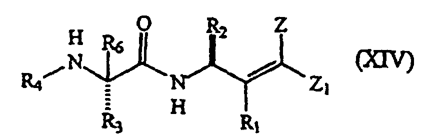 worin R₆ H ist, R₁ H ist, R₂ CH₂CH₂C(O)NH₂ ist, Z H ist und
146. Z₁ ist CO₂CH₂CH₃, R₃ ist CH₂Ph und R₄ ist 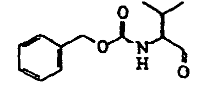
147. Z₁ ist CO₂CH₂CH₃, R₃ ist CH₂Ph und R₄ ist 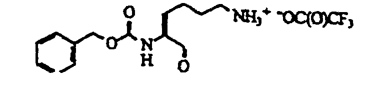
148. Z₁ ist CO₂CH₂CH₃, R₃ ist CH₂Ph und R₄ ist 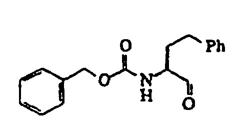
149. Z₁ ist CO₂CH₂CH₃, R₃ ist CH₂Ph und R₄ ist 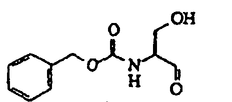
150. Z₁ ist 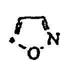 R₃ ist CH₂Ph und R₄ ist 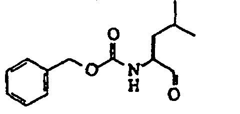
151. Z₁ ist CO₂CH₂CH₃, R₃ ist CH₂Ph und R₄ ist 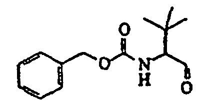
152. Z₁ ist CO₂CH₂CH₃, R₃ ist CH₂Ph und R₄ ist 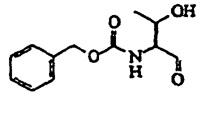
153. Z₁ ist CO₂CH₂CH₃, R₃ ist CH₂Ph und R₄ ist 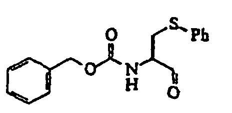
154. Z₁ ist CO₂CH₂CH₃, R₃ ist CH₂Ph und R₄ ist 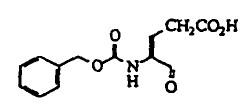
155. Z₁ ist CO₂CH₂CH₃, R₃ ist 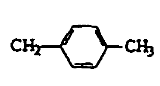 und R₄ ist 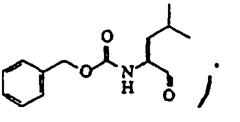
Verbindungen 156- 173, ebenfalls mit der Formel XIV: 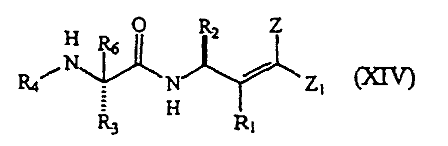 worin R₆ H ist, R₃ CH₂Ph ist, R₂ CH₂CH₂C(O)NH₂ ist, und
156. R₁ ist OH, Z ist H, Z₁ ist CO₂CH₂CH₃ und R₄ ist 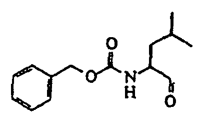
157. R₁ ist H, Z ist H, Z₁ ist CO₂CH₂CH₃ und R₄ ist 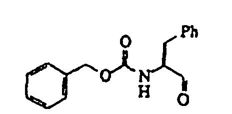
158. R₁ ist H, Z ist H, Z₁ ist CO₂CH₂CH₃ und R₄ ist 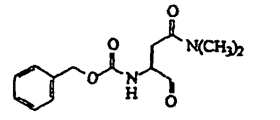
159. R₁ ist H, Z ist H, Z₁ ist CO₂CH₂CH₃ und R₄ ist 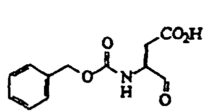
160. R₁ ist H, Z ist H, Z₁ ist 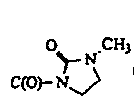 und R₄ ist 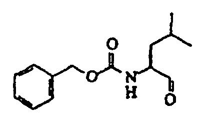
161. R₁ ist H, Z ist H, Z₁ ist CO₂CH₂CH₃ und R₄ ist 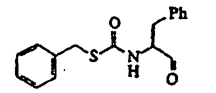
162. R₁ ist H, Z ist H, Z₁ ist CO₂CH₂CH₃ und R₄ ist 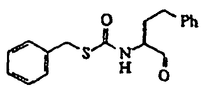
163. R₁ ist H, Z ist H, Z₁ ist CO₂CH₂C(CH₃)₃ und R₄ ist 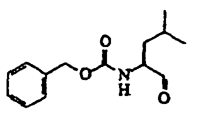
164. R₁ ist H, Z und Z₁ bilden zusammen 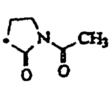 und R₄ ist
wobei C=O vorzugsweise cis zu der R₁-Gruppe angeordnet ist 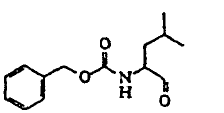
165. R₁ ist H, Z ist H, Z₁ ist CO₂CH₂CH₃ und R₄ ist 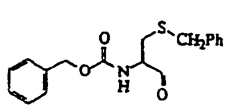
166. R₁ ist H, Z ist H, Z₁ ist CO₂CH₂CH₃ und R₄ ist 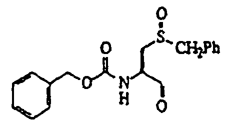
167. R₁ ist H, Z ist H, Z₁ ist CO₂CH₂CH₃ und R₄ ist 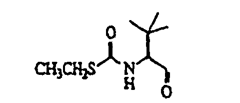
168. R₁ ist H, Z ist CH₃, Z₁ ist CO₂CH₂CH₃ und R₄ ist 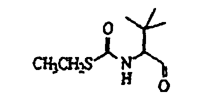
169. R₁ ist H, Z und Z₁ bilden zusammen 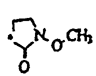 und R₄ ist 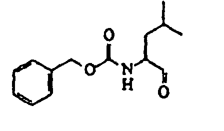 wobei C=O vorzugsweise cis zu R₁ angeordnet ist
170. R₁ ist H, Z ist H, Z₁ ist CO₂CH₂CH₃ und R₄ ist 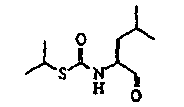
171. R₁ ist H, Z ist CH₃, Z₁ ist CO₂CH₂CH₃ und R₄ ist 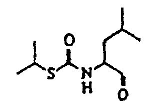
172. R₁ ist H, Z ist H, Z₁ ist CO₂CH₂CH₃ und R₄ ist 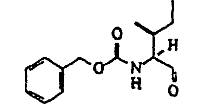
173. R₁ ist H, Z ist H, Z₁ ist CO₂CH₂CH₃ und R₄ ist 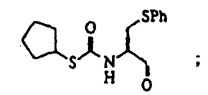
Verbindungen 174 - 188, ebenfalls mit der Formel XIV: 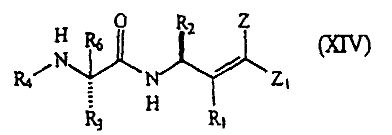 worin R₆ H ist, R₂ CH₂CH₂C(O)NH₂ ist, R₁ H ist und
174. Z ist H, Z₁ ist CO₂CH₂CH₃, R₃ ist 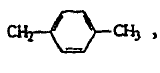 und R₄ ist 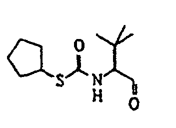
175. Z ist CH₃, Z₁ ist CO₂CH₂CH₃, R₃ ist 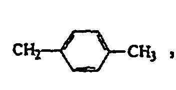 und R₄ ist 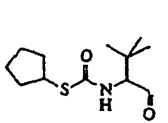
176. Z ist H, Z₁ ist CO₂CH₂CH₃, R₃ ist 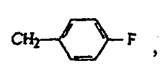 und R₄ ist 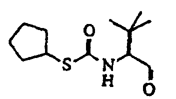
177. Z ist CH₃, Z₁ ist CO₂CH₂CH₃, R₃ ist 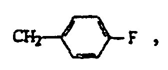 und R₄ ist 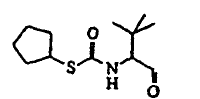
178. Z ist H, Z₁ ist CO₂CH₂CH₃, R₃ ist CH₂Ph und R₄ ist 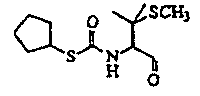
179. Z ist H, Z₁ ist CO₂CH₂CH₃, R₃ ist CH₂Ph und R₄ ist 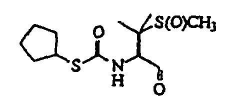
180. Z ist H, Z₁ ist CO₂CH₂CH₃, R₃ ist CH₂Ph und R₄ ist 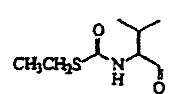
181. Z und Z₁ bilden zusammen 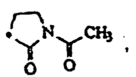 R₃ ist 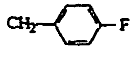 und R₄ ist 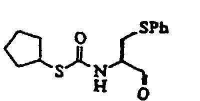 wobei C=O vorzugsweise cis zu der R₁-Gruppe angeordnet ist
182. Z ist H, Z₁ ist CO₂CH₂CH₃, R₃ ist CH₂Ph und R₄ ist 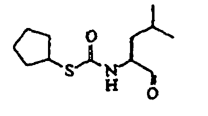
183. Z ist H, Z₁ ist CO₂CH₂CH₃, R₃ ist 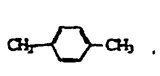 und R₄ ist 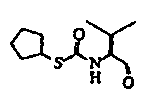
184. Z ist H, Z₁ ist CO₂CH₂CH₃, R₃ ist 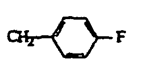 und R₄ ist 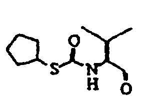
185. Z ist H, Z₁ ist CO₂CH₂CH₃, R₃ ist 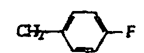 und R₄ ist 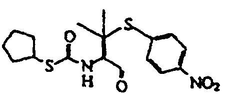
186. Z ist H, Z₁ ist CO₂CH₂Ph, R₃ ist 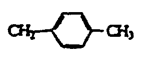 und R₄ ist 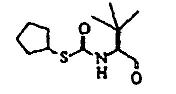
187. Z ist CH₃, Z₁ ist CO₂CH₂CH₃, R₃ ist CH₂Ph und R₄ ist 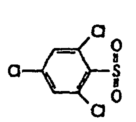
188. Z ist H, Z₁ ist CO₂CH₂CH₂OCH₃, R₃ ist 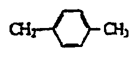 und R₄ ist 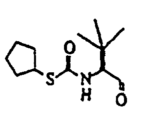
189. R₃ ist 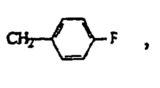 R₄ ist 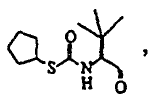 und Z und Z₁ bilden zusammen 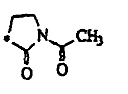 wobei C=O vorzugsweise cis zu der R₁-Gruppe angeordnet ist
190. Z ist H, Z₁ ist CO₂CH₂CH₃, R₃ ist 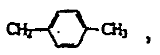 und R₄ ist 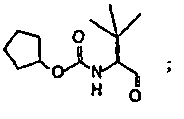 und Verbindungen der nachstehenden Formel (III): 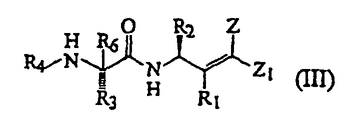 worin R₆ H ist, R₁ H ist, R₃ CH₂Ph ist, R₂ CH₂CH₂C(O)NH₂ ist, Z H ist, Z₁ CO₂CH₂CH₃ ist, und R₄ ausgewählt ist aus dem Folgenden: 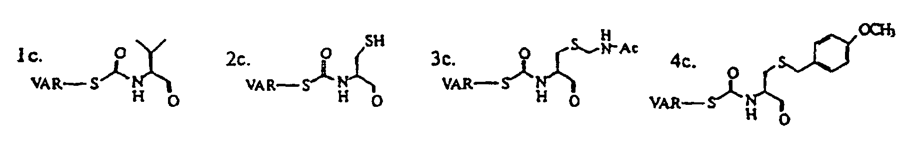 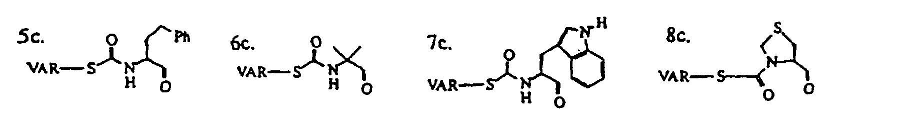 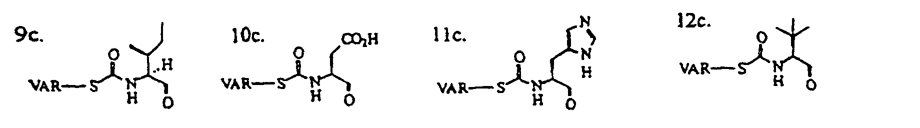 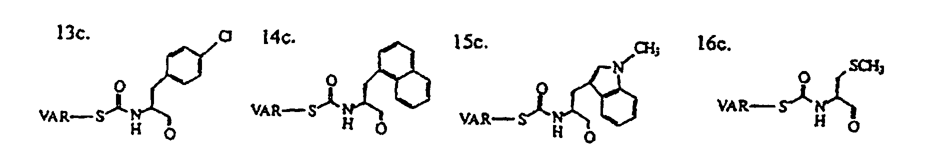 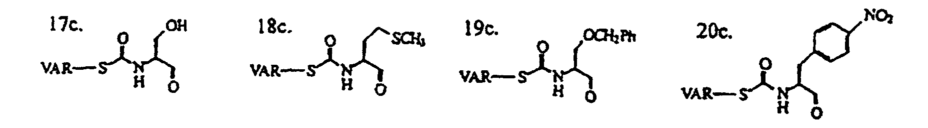 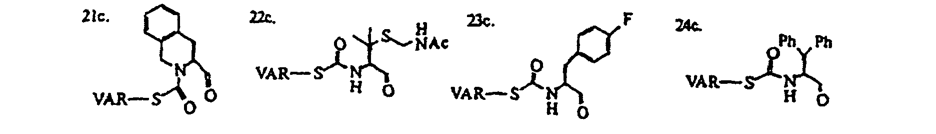 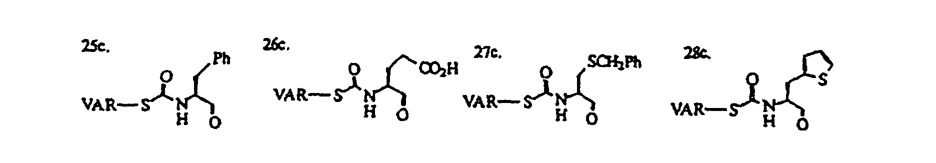 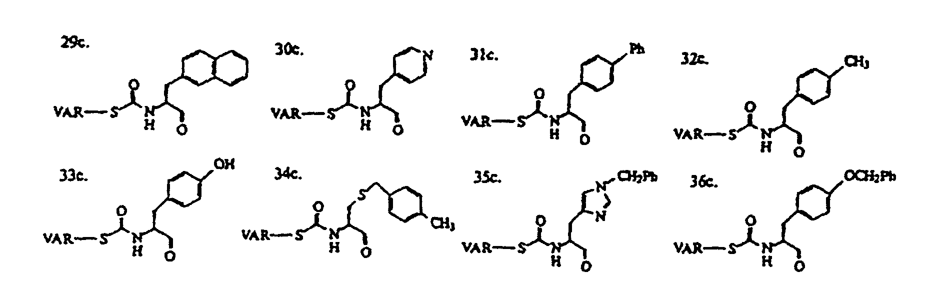 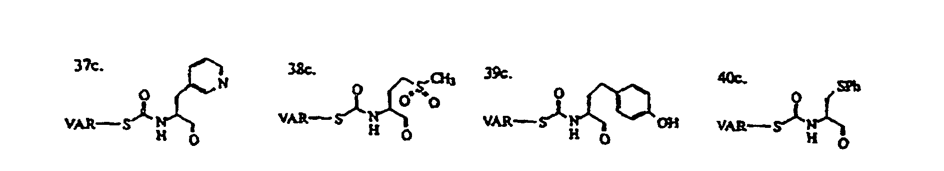 worin VAR ausgewählt ist aus -CH₂CH₃, -CH(CH₃)₂, -CH₂CH(CH₃)₂, -CH₂-Ph,

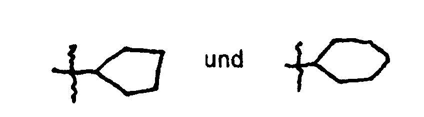

## Revendications

1. Composé de formule (I) 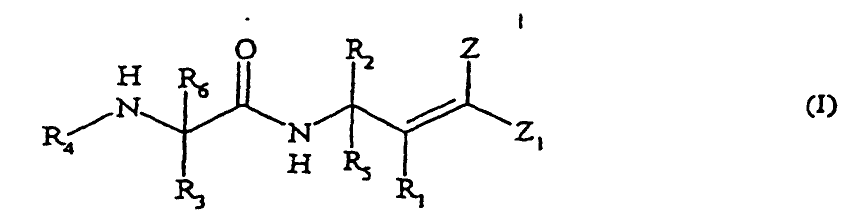 où,
R₁ est H, F, un groupe alkyle, SH, un groupe O-alkyle ou un groupe S-alkyle ;
R₂ et R₅ sont choisis, de façon indépendante, parmi H, 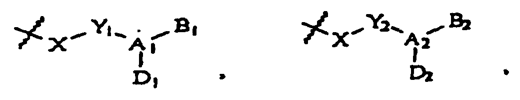 ou un groupe alkyle, ledit groupe alkyle étant différent de 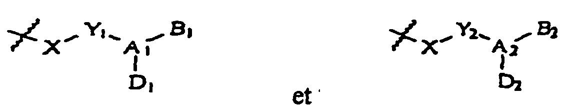 sous réserve qu'au moins un parmi R₂ ou R₅ soit obligatoirement 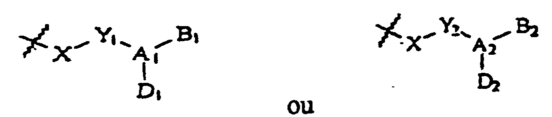 et où, si R₂ ou R₅ est 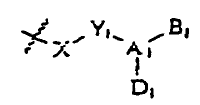 X est =CH ou =CF et Y, est =CH ou =CF
ou X et Y₁ forment ensemble avec Q' un cycle à trois membres
dans lequel Q' est -C(R₁₀)(R₁₁)- ou -O-, X est -CH- ou -CF- et Y, est -CH-, -CF- ou -C(alkyle)-, où R₁₀ et R₁₁ sont, de façon indépendante, H, un halogène, ou un groupe alkyle, ou forment, avec l'atome de carbone auquel ils sont attachés, un groupe cycloalkyle ou un groupe hétérocycloalkyle,
ou X est -CH₂-, -CF₂-, -CHF-, ou -S-,
et
Y₁ est -O-, -S-, -NR₁₂-, -C(R₁₃)(R₁₄)-, -C(O)-, -C(S)-, ou -C(CR₁₃R₁₄)-
où R₁₂ est H ou alkyle et R₁₃ et R₁₄ sont, de façon indépendante, H, F, ou un groupe alkyle, ou forment, avec l'atome auquel ils sont liés, un groupe cycloalkyle ou un groupe hétérocycloalkyle ;
et A₁ est C, CH, CF, S, P, Se, N, NR₁₅, S(O), Se(O), P-OR₁₅ ou P-NR₁₅R₁₆
où R₁₅ et R₁₆ sont, de façon indépendante, un groupe alkyle, un groupe cycloalkyle, un groupe hétérocycloalkyle, un groupe aryle, ou un groupe hétéroaryle, ou forment, avec l'atome auquel ils sont liés, un groupe hétérocycloalkyle ;
et D₁ est un fragment avec un doublet libre d'électrons capable de former une liaison hydrogène ;
et B₁ est H, F, un groupe alkyle, un groupe cycloalkyle, un groupe hétérocycloalkyle, un groupe aryle, un groupe hétéroaryle, -OR₁₇, -SR₁₇, -NR₁₇R₁₈, -NR₁₉NR₁₇R₁₈ ou -NR₁₇OR₁₈,
où R₁₇, R₁₈ et R₁₉ sont, de façon indépendante, H, un groupe alkyle, un groupe cycloalkyle, un groupe hétérocycloalkyle, un groupe aryle, un groupe hétéroaryle, ou un groupe acyle, ou bien où deux quelconques parmi R₁₇, R₁₈ et R₁₉, avec le ou les atomes auxquels ils sont liés, forment un groupe hétérocycloalkyle ;
et sous réserves que, si D₁ est un fragment ≡N avec un doublet libre d'électrons capable de former une liaison hydrogène, B₁ n'existe pas ; et si A₁ est un carbone sp³, B₁ n'est pas -NR₁₇R₁₈ si D₁ est le fragment -NR₂₅R₂₆ avec un doublet libre d'électrons capable de former une liaison hydrogène, où R₂₅ et R₂₆ sont, de façon indépendante, H, un groupe alkyle, un groupe cycloalkyle, un groupe hétérocycloalkyle, un groupe aryle, ou un groupe hétéroaryle ; et où D₁-A₁-B₁ forme de façon optionnelle un groupe nitro où A₁ est N ;
et où, si R₂ ou R₅ est 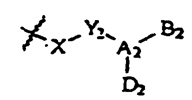 X est =CH ou =CF et Y₂ est =C, =CH ou =CF
ou X et Y₂ forment ensemble avec Q' un cycle à trois membres
dans lequel Q' est -C(R₁₀)(R₁₁)- ou -O-, X est -CH- ou -CF- et Y₂ est -CH-, -CF- ou -C(alkyle)-, où R₁₀ et R₁₁ sont, de façon indépendante, H, un halogène, ou un groupe alkyle, ou forment, avec l'atome de carbone auquel ils sont attachés, un groupe cycloalkyle ou un groupe hétérocycloalkyle,
ou X est -CH₂-, -CF₂-, -CHF-, ou -S-,
et
Y₂ est -O-, -S-, -N(R'₁₂)-, -C(R'₁₃)(R'₁₄)-, -C(O)-, -C(S)-, ou -C(CR'₁₃R'₁₄)-
où R'₁₂ est H, un groupe alkyle, un groupe cycloalkyle, un groupe hétérocycloalkyle, un groupe aryle, un groupe hétéroaryle, -OR'₁₃-, -NR'₁₃R'₁₄-, -C(O)-R'₁₃, -SO₂R'₁₃ ou -C(S)R'₁₃ et R'₁₃ et R'₁₄ sont, de façon indépendante, H, F, ou un groupe alkyle, un groupe cycloalkyle, un groupe hétérocycloalkyle, un groupe aryle ou un groupe hétéroaryle ou forment, avec l'atome auquel ils sont liés, un groupe cycloalkyle ou un groupe hétérocycloalkyle ;
et où toute combinaison de Y₂, A₂, B₂ et D₂ forme un groupe cycloalkyle, un groupe hétérocycloalkyle, un groupe aryle ou un groupe hétéroaryle ;
et A₂ est C, CH, CF, S, P, Se, N, NR₁₅, S(O), Se(O), P-OR₁₅ ou P-NR₁₅R₁₆
où R₁₅ et R₁₆ sont, de façon indépendante, un groupe alkyle, un groupe cycloalkyle, un groupe hétérocycloalkyle, un groupe aryle, ou un groupe hétéroaryle, ou forment, avec l'atome auquel ils sont liés, un groupe hétérocycloalkyle ;
et D₂ est un fragment avec un doublet libre d'électrons capable de former une liaison hydrogène ;
et B₂ est H, F, un groupe alkyle, un groupe cycloalkyle, un groupe hétérocycloalkyle, un groupe aryle, un groupe hétéroaryle, -OR₁₇, -SR₁₇, -NR₁₇R₁₈, -NR₁₉NR₁₇R₁₈ ou -NR₁₇OR₁₈,
où R₁₇, R₁₈ et R₁₉ sont, de façon indépendante, H, un groupe alkyle, un groupe cycloalkyle, un groupe hétérocycloalkyle, un groupe aryle, un groupe hétéroaryle, ou un groupe acyle, ou bien où deux quelconques parmi R₁₇, R₁₈ et R₁₉, avec le ou les atomes auxquels ils sont liés, forment un groupe hétérocycloalkyle ;
R₃ et R₆ sont, de façon indépendante, H, F ou un groupe alkyle ;
R₄ est H, OH, un groupe alkyle, un groupe oxo, un groupe cycloalkyle, un groupe hétérocycloalkyle, un groupe aryle, un groupe hétéroaryle, un groupe acyle, un groupe sulfonyle, un groupe mercapto, un groupe alkylthio, un groupe alcoxy, un groupe carboxy, un groupe amino, un groupe alkylamino, un groupe dialkylamino, un groupe carbamoyle, un groupe arylthio ou un groupe hétéroarylthio ;
Z et Z₁ sont, de façon indépendante, H, F, un groupe alkyle, un groupe cycloalkyle, un groupe hétérocycloalkyle, un groupe aryle, un groupe hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃P₂₄), -C(O)NR₂₁NR₂₂R₂₃ ou -C(S)NR₂₁NP₂₂R₂₃,
où R₂₁, R₂₂, R₂₃ et R₂₄ sont, de façon indépendante, H, un groupe alkyle, un groupe cycloalkyle, un groupe hétérocycloalkyle, un groupe aryle, un groupe hétéroaryle, ou un groupe acyle, ou un groupe thioacyle, ou bien où deux quelconques parmi R₂₁, R₂₂, R₂₃ et R₂₄ avec le ou les atomes auxquels ils sont liés, forment un groupe hétérocycloalkyle ;
ou Z₁, tel que défini ci-dessus, avec R₁, tel que défini ci-dessus, et les atomes auxquels Z₁ et R₁ sont liés, forment un groupe cycloalkyle ou hétérocycloalkyle,
ou Z et Z₁, tous deux tels que définis ci-dessus, forment avec les atomes auxquels ils sont liés un groupe cycloalkyle ou un groupe hétérocycloalkyle ;
ou un sel ou solvate de celui-ci pharmaceutiquement acceptable ;
où les termes groupe alkyle, groupe cycloalkyle, groupe hétérocycloalkyle, groupe aryle, groupe hétéroaryle, groupe acyle, groupe sulfonyle, groupe alkylthio , groupe alcoxy, groupe alkylamino, groupe dialkylamino, groupe arylthio et groupe hétéroarylthio ont les significations suivantes :
groupe alkyle : méthyle, éthyle, propyle, isopropyle, butyle, isobutyle, t-butyle, éthényle, pentényle, butényle, propényle, éthynyle, butynyle, propynyle, pentynyle et hexynyle, non-substitués ou substitués par un substituant ;
groupe cycloalkyle : radical non-aromatique, monovalent, monocyclique, bicyclique ou tricyclique contenant 3, 4, 5, 6, 7, 8, 9, 10, 11, 12, 13 ou 14 atomes de carbone cycliques, pouvant être saturé ou non-saturé, non-substitué ou substitué par un substituant ;
groupe hétérocycloalkyle : radical non-aromatique, monovalent, monocyclique, bicyclique ou tricyclique, saturé ou insaturé, contenant 3, 4, 5, 6, 7, 8, 9, 10, 11, 12, 13, 14, 15, 16, 17 ou 18 atomes cycliques, qui incluent 1, 2, 3, 4 ou 5 hétéroatomes choisis parmi l'azote, l'oxygène ou le soufre, le radical étant non-substitué ou substitué par un substituant ;
groupe aryle : radical aromatique, monovalent, monocyclique, bicyclique ou tricyclique, contenant 6, 10, 14, ou 18 atomes de carbone cycliques, pouvant être non-substitué ou substitué par un substituant ;
groupe hétéroaryle : radical aromatique, monovalent, monocyclique, bicyclique ou tricyclique, contenant 4, 5, 6, 7, 8, 9, 10, 11, 12, 13, 14, 15, 16, 17 ou 18 atomes cycliques incluant 1, 2, 3, 4 ou 5 hétéroatomes choisis parmi l'azote, l'oxygène ou le soufre, pouvant être non-substitué ou substitué par un substituant ;
groupe acyle : radical -C(O)R où R est un substituant ;
groupe sulfonyle : radical -SO₂R, où R est un substituant ;
groupe arylthio : radical -SR où R est un groupe alkyle ;
groupe alcoxy : radical OR où R est un groupe alkyle ;
groupe alkylamino : radical -NHR où R est un groupe alkyle ;
groupe dialkylamino : radical -NRₐR_{b} où Rₐ et R_{b} sont de façon indépendante un groupe alkyle ;
groupe arylthio : radical -SR_{c} où R_{c} est un groupe aryle ;
groupe hétéroarylthio : radical -SR_{d} où R_{d} est un groupe hétéroaryle ; substituant : groupe hydroxyle, groupe oxo, groupe alkyle, groupe acyle, groupe sulfonyle, groupe mercapto, groupe alkylthio, groupe alcoxy, groupe cycloalkyle, groupe hétérocycloalkyle, groupe aryle, groupe hétéroaryle, groupe carboxy, groupe amino, groupe alkylamino, groupe dialkylamino, groupe carbamoyle, groupe aryloxy, groupe hétéroaryloxy, groupe arylthio, groupe hétéroarylthio, où le groupe aryloxy est le radical -OR_{c} où R_{c} est un groupe aryle et où le groupe hétéroaryloxy est le radical -OR_{d} où R_{d} est un groupe hétéroaryle.

2. Composé selon la revendication 1, où R₁ est H ou F, ou un sel ou solvate de celui-ci pharmaceutiquement acceptable.

3. Composé selon la revendication 1, où R₄ est un groupe acyle ou un groupe sulfonyle, ou un sel ou solvate de celui-ci pharmaceutiquement acceptable.

4. Composé selon la revendication 1, où au moins un des R₂ ou R₅ est 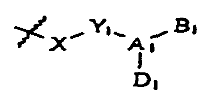 ou sel ou solvate de celui-ci pharmaceutiquement acceptable.

5. Composé selon la revendication 4, où D₁ est -OR₂₅, =O, =S, ≡N, =NR₂₅ ou -NR₂₅R₂₆, où R₂₅ et R₂₆ sont, de façon indépendante, H, un groupe alkyle, un groupe cycloalkyle, un groupe hétérocycloalkyle, un groupe aryle ou un groupe hétéroaryle, ou, avec l'atome d'azote auxquels ils sont liés, forment un groupe hétérocycloalkyle, ou un sel ou solvate de celui-ci pharmaceutiquement acceptable.

6. Composé selon la revendication 5, où D₁ est =O; ou un sel ou solvate de celui-ci pharmaceutiquement acceptable.

7. Composé selon la revendication 4, où A₁ est C, CH, S ou S(O) ; ou un sel ou solvate de celui-ci pharmaceutiquement acceptable.

8. Composé selon la revendication 7, où A₁ est C ; ou un sel ou solvate de celui-ci pharmaceutiquement acceptable.

9. Composé selon la revendication 4, où B₁ est NR₁₇R₁₈ où R₁₇ et R₁₈ sont, de façon indépendante, H, un groupe alkyle, un groupe cycloalkyle, un groupe hétérocycloalkyle, un groupe aryle, un groupe hétéroaryle, ou un groupe acyle ou bien où R₁₇ et R₁₈ avec le ou les atomes auxquels ils sont liés, forment un groupe hétérocycloalkyle ;
ou un sel ou solvate de celui-ci pharmaceutiquement acceptable.

10. Composé selon la revendication 1, où au moins un des R₂ ou R₅ est 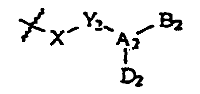 ou un sel ou solvate de celui-ci pharmaceutiquement acceptable.

11. Composé selon la revendication 10, où D₂ est -OR₂₅, =O, =S, ≡N, =NR₂₅ ou -NR₂₅R₂₆, où R₂₅ et R₂₆ sont, de façon indépendante, H, un groupe alkyle, un groupe cycloalkyle, un groupe hétérocycloalkyle, un groupe aryle ou un groupe hétéroaryle, ou, avec l'atome auxquels ils sont liés, forment un groupe hétérocycloalkyle, ou un sel ou solvate de celui-ci pharmaceutiquement acceptable.

12. Composé selon la revendication 11, où D₂ est =O ; ou un sel ou solvate de celui-ci pharmaceutiquement acceptable:

13. Composé selon la revendication 10, où A₂ est C, CH, S ou S(O) ; ou un sel ou solvate de celui-ci pharmaceutiquement acceptable.

14. Composé selon la revendication 13, où A₂ est C ; ou un sel ou solvate de celui-ci pharmaceutiquement acceptable.

15. Composé selon la revendication 10, où B₂ est NR₁₇R₁₈ où R₁₇ et R₁₈ sont, de façon indépendante, H, un groupe alkyle, un groupe cycloalkyle, un groupe hétérocycloalkyle, un groupe aryle, un groupe hétéroaryle, ou un groupe acyle ou bien où R₁₇ et R₁₈ avec le ou les atomes auxquels ils sont liés, forment un groupe hétérocycloalkyle ;
ou un sel ou solvate de celui-ci pharmaceutiquement acceptable.

16. Composé selon la revendication 1, où A₁ est C, CH, S ou S(O) et où A₂ est C, CH, S ou S(O) ; ou un sel ou solvate de celui-ci pharmaceutiquement acceptable.

17. Composé selon la revendication 1, où Z et Z₁ sont, de façon indépendante, H, un groupe aryle, un groupe hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃ OU -C(S)NR₂₁NR₂₂R₂₃ ;
où R₂₁, R₂₂ et R₂₃ sont, de façon indépendante, H, un groupe alkyle, un groupe cycloalkyle, un groupe hétérocycloalkyle, un groupe aryle, un groupe hétéroaryle, un groupe acyle, ou un groupe thioacyle, ou bien où deux quelconques parmi R₂₁, R₂₂ et R₂₃ avec le ou les atomes auxquels ils sont liés, forment un groupe hétérocycloalkyle ;
ou bien Z et Z₁ forment avec les atomes auxquels ils sont liés un groupe hétérocycloalkyle ;
ou un sel ou solvate de celui-ci pharmaceutiquement acceptable.

18. Composé selon la formule (I) ayant la formule II, où les substituants sont tels que définis dans la revendication 1 : 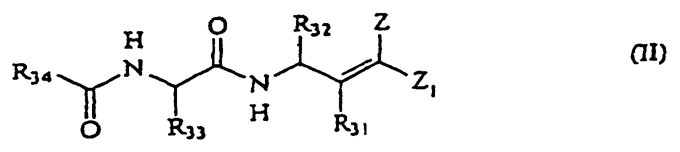 et où, de plus, R₃₁ est H, F ou un groupe alkyle ;
R₃₂ est choisi parmi les fragments suivants : 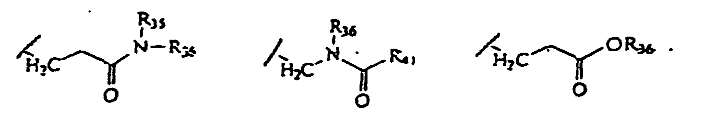 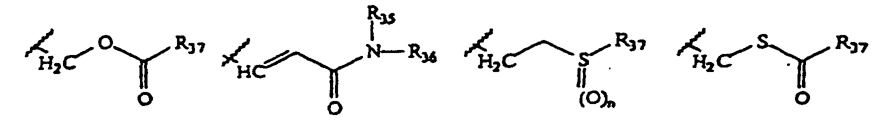 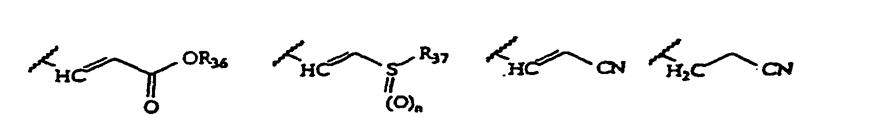 où
R₃₅ est H, un groupe alkyle, un groupe aryle, -OR₃₈ ou -NR₃₈R₃₉, et R₃₆ est H ou un groupe alkyle,
ou bien R₃₅ et R₃₆, avec le ou les atomes auxquels ils sont liés, forment un groupe hétérocycloalkyle ou un groupe hétéroaryle ;
R₄₁ est H, un groupe alkyle, un groupe aryle, -OR₃₈, -SR₃₉, -NR₃₈R₃₉, -NR₄₀NR₃₈R₃₉, ou -NR₃₈OR₃₉, ou bien R₄₁ et R₃₆, avec l'atome auquel ils sont liés, forment un groupe hétérocycloalkyle, et R₃₇ est un groupe alkyle, un groupe aryle ou -NR₃₈R₃₉ ;
où R₃₈, R₃₉ et R₄₀ sont, de façon indépendante, H, un groupe alkyle, un groupe cycloalkyle, un groupe hétérocycloalkyle, un groupe aryle, un groupe hétéroaryle ou un groupe acyle ou bien où deux quelconques parmi R₃₈, R₃₉ et R₄₀, avec le ou les atomes auxquels ils sont liés, forment un groupe hétérocycloalkyle,
n est égal à 0, 1 ou 2 ;
R₃₃ est H ou un groupe alkyle ;
R₃₄ est un groupe alkyle, un groupe cycloalkyle, un groupe hétérocycloalkyle, un groupe aryle, un groupe hétéroaryle, un groupe O-alkyle, un groupe O-cycloalkyle, un groupe O-hétérocycloalkyle, un groupe O-aryle, un groupe O-hétéroaryle, un groupe S-alkyle, un groupe NH-alkyle, un groupe NH-aryle, un groupe N,N-dialkyle ou un groupe N,N-diaryle ; et
Z et Z₁ sont, de façon indépendante, H, F, un groupe alkyle, un groupe cycloalkyle, un groupe hétérocycloalkyle, un groupe aryle, un groupe hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -PO(OR₂₁)₂, -PO(R₂₁)(R₂₂), -PO(NR₂₁R₂₂)(OR₂₃), -PO(NR₂₁R₂₂)(NR₂₃R₂₄), -C(O)NR₂₁NR₂₂R₂₃ ou -C(S)NR₂₁NR₂₂R₂₃,
où R₂₁, R₂₂, R₂₃ et R₂₄ sont, de façon indépendante, H, un groupe alkyle, un groupe cycloalkyle, un groupe hétérocycloalkyle, un groupe aryle, un groupe hétéroaryle, un groupe acyle, ou un groupe thioacyle, ou bien où deux quelconques parmi R₂₁, R₂₂, R₂₃ et R₂₄, avec le ou les atomes auxquels ils sont liés, forment un groupe hétérocycloalkyle ; ou Z et Z₁, avec les atomes auxquels ils sont liés, forment un groupe hétérocycloalkyle ; ou un sel ou solvate de celui-ci pharmaceutiquement acceptable.

19. Composé selon la revendication 18, où Z et Z₁ sont, de façon indépendante, H, un groupe aryle, un groupe hétéroaryle, -C(O)R₂₁, -CO₂R₂₁, -CN, -C(O)NR₂₁R₂₂, -C(O)NR₂₁OR₂₂, -C(S)R₂₁, -C(S)NR₂₁R₂₂, -NO₂, -SOR₂₁, -SO₂R₂₁, -SO₂NR₂₁R₂₂, -SO(NR₂₁)(OR₂₂), -SONR₂₁, -SO₃R₂₁, -C(O)NR₂₁NR₂₂R₂₃ ou -C(S)NR₂₁NR₂₂R₂₃ ;
où R₂₁, R₂₂ et R₂₃ sont, de façon indépendante, H, un groupe alkyle, un groupe cycloalkyle, un groupe hétérocycloalkyle, un groupe aryle, un groupe hétéroaryle ou un groupe acyle, ou bien où deux quelconques parmi R₂₁, R₂₂ et R₂₃, avec le ou les atomes auxquels ils sont liés, forment un groupe hétérocycloalkyle ; ou bien Z et Z₁ forment avec les atomes auxquels ils sont liés un groupe hétérocycloalkyle ; ou un sel ou solvate de celui-ci pharmaceutiquement acceptable.

20. Composé selon la revendication 18, où R₃₂ est choisi parmi l'un des fragments suivants : 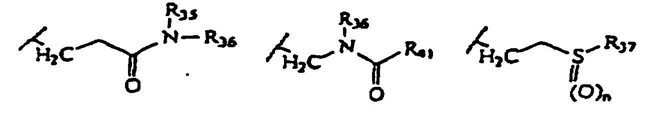 où
R₃₅ est H, un groupe alkyle, un groupe aryle, -OR₃₈, -SR₃₉, -NR₃₈R₃₉, -NR₄₀NR₃₈R₃₉, OU -NR₃₈OR₃₉, et
R₃₆ est H ou un groupe alkyle,
ou bien R₃₅ et R₃₆, avec l'atome auquel ils sont liés, forment un groupe hétérocycloalkyle ou un groupe hétéroaryle ;
R₃₇ est un groupe alkyle, un groupe aryle ou -NR₃₈R₃₉ ;
où R₃₈, R₃₉ et R₄₀ sont, de façon indépendante, H, un groupe alkyle, un groupe cycloalkyle, un groupe hétérocycloalkyle, un groupe aryle, un groupe hétéroaryle ou un groupe acyle ou bien où deux quelconques parmi R₃₈, R₃₉ et R₄₀, avec le ou les atomes auxquels ils sont liés, forment un groupe hétérocycloalkyle,
n est égal à 0, 1 ou 2 ;
ou un sel ou solvate de celui-ci pharmaceutiquement acceptable.

21. Composé selon la formule (I) ayant la formule III : 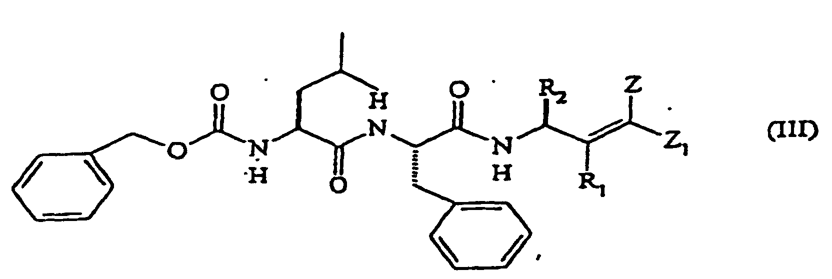 où
R₂ est CH₂CH₂C(O)NHCPh₃, R₁ est H, Z est H, et Z₁ est CO₂CH₂CH₃,
R₂ est CH₂CH₂C(O)NH₂, R₁ est H, Z est H, et Z₁ est CO₂CH₂CH₃,
R₂ est CH₂NHC(O)CH₃, R₁ est H, Z est H, et Z₁ est CO₂CH₂CH₃,
R₁ est H, Z est H, et Z₁ est CO₂CH₂CH₃, et R₂ est 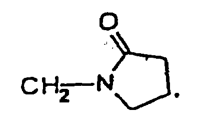 R₂ est CH₂CH₂C(O)NH₂, R₁ est H, Z est CO₂CH₃ et Z₁ est H,
R₂ est CH₂CH₂C(O)NH₂, R₁ est H, Z est H, et Z₁ est CO₂CH₃,
R₂ est CH₂CH₂S(O)CH₃, R₁ est H, Z est H, et Z₁ est CO₂CH₂CH₃,
R₂ est CH₂CH₂C(O)NH₂, R₁ est H, Z est H, et Z₁ est C(O)CH₃,
R₂ est CH₂CH₂C(O)NH₂, R₁ est H, Z est H, et Z₁ est CN,
R₂ est CH₂NHC(O)NH₂, R₁ est H, Z est H, et Z₁ est CO₂CH₂CH₃,
R₂ est CH₂CH₂C(O)NH₂, R₁ est H, Z est H, et Z₁ est CO₂CH(CH₃)₂,
R₂ est CH₂CH₂C(O)NH₂, R₁ est H, Z est H, et Z₁ est 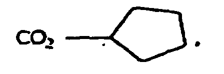 R₂ est CH₂CH₂C(O)NH₂, R₁ est H, Z est H, et Z₁ est 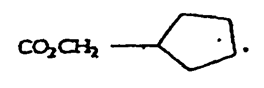 R₂ est CH₂CH₂C(O)NH₂, R₁ est H, Z est H, et Z₁ est 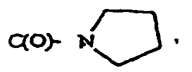 R₂ est CH₂CH₂C(O)NH₂, R₁ est H, Z est H, et Z₁ est C(O)N(CH₃)₂,
R₂ est CH₂CH₂C(O)NH₂, R₁ est H, Z est H, et Z₁ est C(O)Ph,
R₂ est CH₂CH₂C(O)NH₂, R₁ est H, Z est H, et Z₁ est 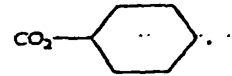 R₂ est CH₂CH₂C(O)NH₂, R₁ est H, Z est H, et Z₁ est 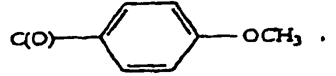 R₂ est CH₂CH₂C(O)NH₂, R₁ est H, Z est H, et Z₁ est 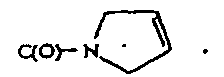 R₂ est CH₂CH₂C(O)NH₂, R₁ est H, Z est H, et Z₁ est 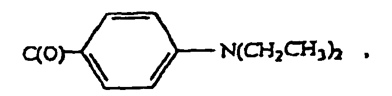 R₂ est CH₂CH₂C(O)NH₂, R₁ est H, Z est H, et Z₁ est CH₂Cl, ou
R₂ est CH₂CH₂C(O)NH₂, R₁ est H, Z est H, et Z₁ est 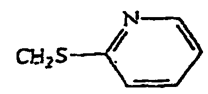 ou un sel ou solvate de celui-ci pharmaceutiquement acceptable.

22. Composé selon la formule (I) ayant la formule IV : 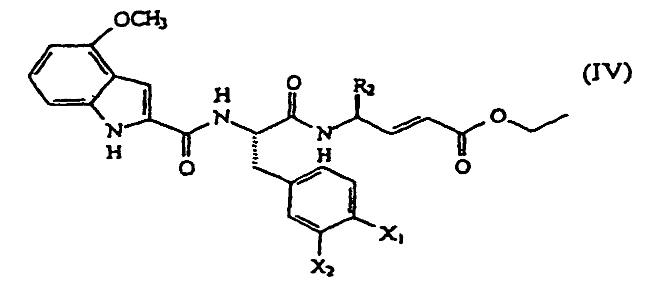 R₂ est CH₂CH₂C(O)NH₂, X₁ est H, F ou Cl et X₂ est H, F ou Cl ; ou un sel ou solvate de celui-ci pharmaceutiquement acceptable.

23. Composé selon la revendication 22, où X₁ est Cl et X₂ est H ; X₁ est F et X₂ est H ; ou X₁ est H et X₂ est F ; ou un sel ou solvate de celui-ci pharmaceutiquement acceptable.

24. Composé selon la formule (I) ayant la formule V : 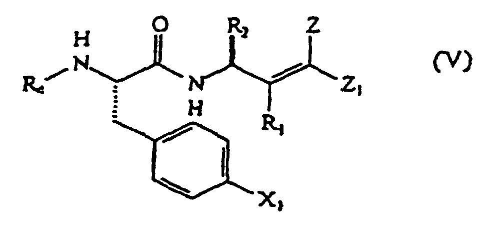 où
R₄ est PhCH₂OC(O), X₁ est H, R₂ est CH₂CH₂C(O)NH₂, R₁ est H, Z est H et Z₁ est CO₂CH₂CH₃ ; ou
R₄ est CH₃CH₂CH₂SO₂, X₁ est H, R₂ est CH₂CH₂C(O)NH₂, R₁ est H, Z est H et Z₁ est CO₂CH₂CH₃ ; ou
R₄ est PhCH₂SO₂, X, est H, R₂ est CH₂CH₂C(O)NH₂, R₁ est H, Z est H et Z₁ est CO₂CH₂CH₃ ; ou
R₄ est CH₃CH₂SO₂, X₁ est H, R₂ est CH₂CH₂C(O)NH₂, R₁ est H, Z est H et Z₁ est CO₂CH₂CH₃ ; ou
R₄ est PhSO₂, X₁ est H, R₂ est CH₂CH₂C(O)NH₂, R₁ est H, Z est H et Z₁ est CO₂CH₂CH₃ ;
ou un sel ou solvate de celui-ci pharmaceutiquement acceptable.

25. Composé selon la formule (I) ayant la formule VI ou VII : 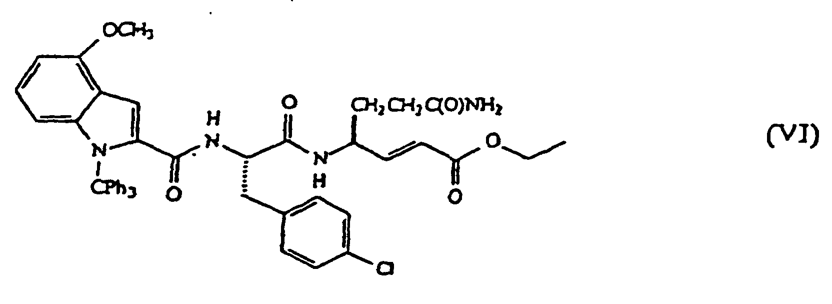 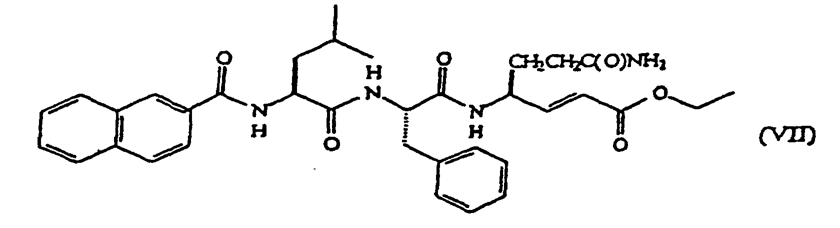 ou un sel ou solvate de celui-ci pharmaceutiquement acceptable.

26. Composé selon la formule (I) ayant la formule VIII : 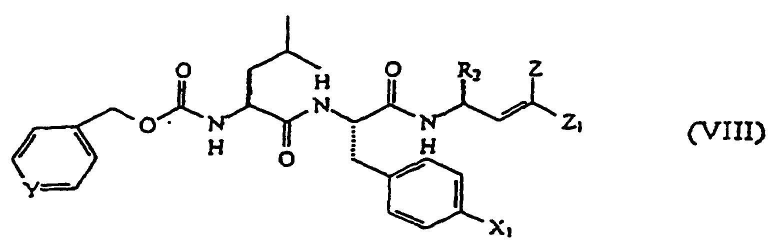 où
X₁ est F, R₂ est CH₂CH₂C(O)NH₂, Y est CH, Z est H et Z₁ est CO₂CH₂CH₃ ; ou
X₁ est H, R₂ est CH₂CH₂C(O)NH₂, Y est N, Z est H et Z₁ est CO₂CH₂CH₃ ; ou X₁ est H, R₂ est CH₂CH₂C(O)NH₂, Y est CH, Z est H et Z₁ est C(O)N(CH₃)OCH₃ ;
ou un sel ou solvate de celui-ci pharmaceutiquement acceptable.

27. Composé selon la formule (I) ayant la formule III : 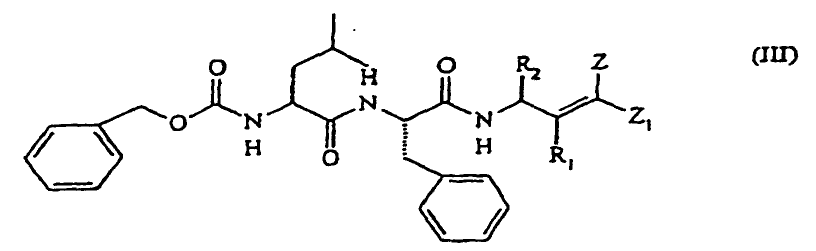 où
R₂ est CH₂CH₂C(O)NH₂, R₁ est H, Z est CH₃, et Z₁ est CO₂CH₂CH₃,
R₂ est CH₂CH₂C(O)NH₂, R₁ est H, et Z et Z₁ forment ensemble 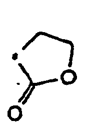 ou bien où R₂ est CH₂CH₂C(O)NH₂, R₁ est H, Z est H, et Z₁ est choisi parmi 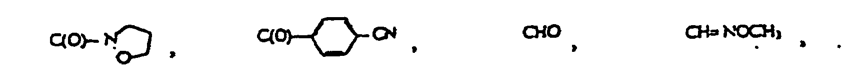 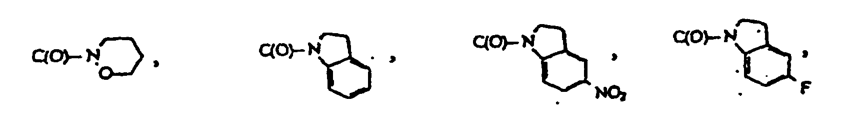 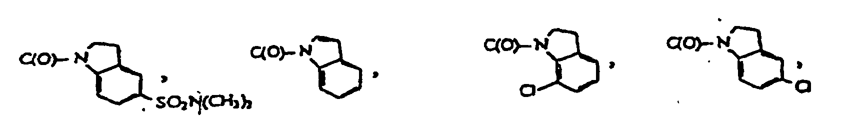 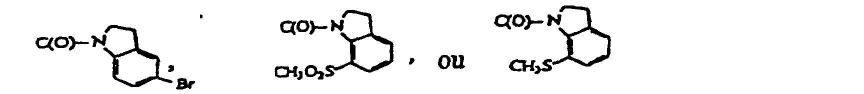 ou un sel ou solvate de celui-ci pharmaceutiquement acceptable.

28. Composé selon la formule (I) ayant la formule XIV : 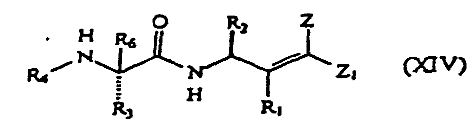 où
R₆ est H, R₁ est H, R₂ est CH₂CH₂C(O)NH₂, Z est H, Z₁ est CO₂CH₂CH₃ et R₃ est CH₂Ph et R₄ est 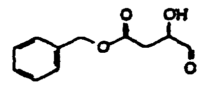 R₃ est 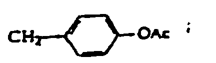 et R₄ est 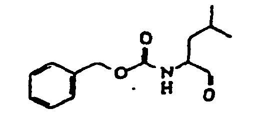 R₃ est CH₂Ph et R₄ est
R₃ est CH₂Ph et R₄ est 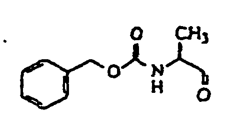 R₃ est 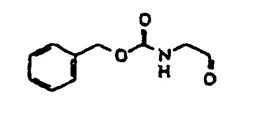 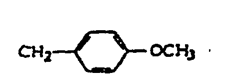 et R₄ est 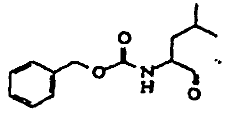 R₃ est CH₂Ph et R₄ est 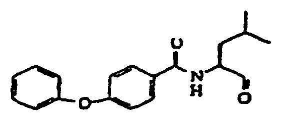 R₃ est 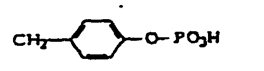 et R₄ est 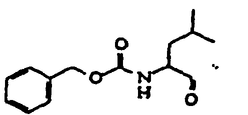 R₃ est CH₂Ph et R₄ est 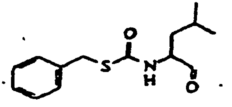 R₃ est CH₂Ph et R₄ est 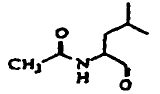 R₃ est CH₂CH₃ et R₄ est 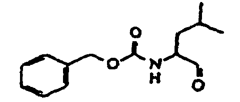 R₃ est CH₃ et R₄ est 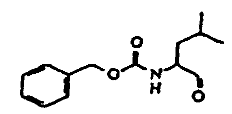 R₃ est CH₂Ph et R₄ est 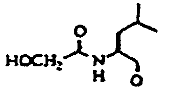 R₃ est 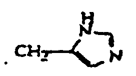 et R₄ est 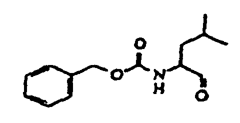 R₃ est 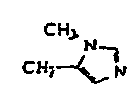 et R₄ est 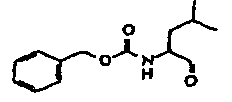 R₃ est 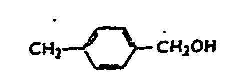 et R₄ est 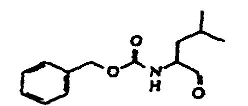 R₃ est CH₂Ph et R₄ est 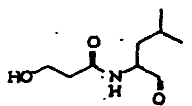 R₃ est CH₂Ph et R₄ est 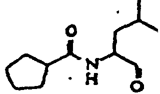 R₃ est CH₂Ph et R₄ est 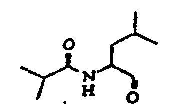 R₃ est CH₂Ph et R₄ est 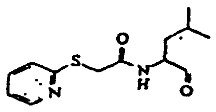 R₃ est CH₂Ph et R₄ est 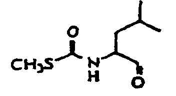 ou R₃ est CH₂Ph et R₄ est 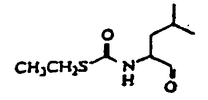 ou un sel ou solvate de celui-ci pharmaceutiquement acceptable.

29. Composé selon la formule (I) ayant la formule XIV : 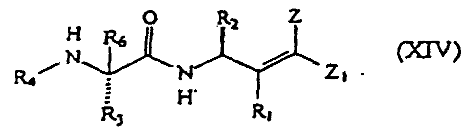 où R₆ est H, R₁ est H, R₃ est CH₂Ph, R₂ est CH₂CH₂C(O)NH₂, Z et Z₁ forment ensemble 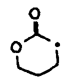 et R₄ est 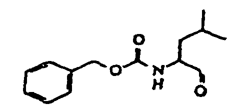 ou un sel ou solvate de celui-ci pharmaceutiquement acceptable.

30. Composé selon la formule (I) ayant la formule XIV : 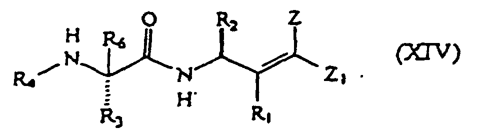 où R₆ est H, R₁ est H, R₂ est CH₂CH₂C(O)NH₂, R₄ est 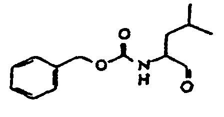 et R₃ est 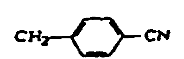 Z est H et Z₁ est CO₂CH₂CH₃
R₃ est CH(OH)CH₃, Z est H et Z₁ est CO₂CH₂CH₃
R₃ est 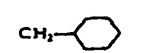 Z est H et Z₁ est CO₂CH₂CH₃
R₃ est CH₂Ph, Z est H et Z₁ est C(O)N(OH)CH₃
R₃ est 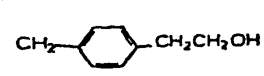 Z est H et Z₁ est CO₂CH₂CH₃
R₃ est 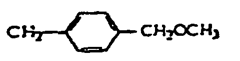 Z est H et Z₁ est CO₂CH₂CH₃
R₃ est CH₂CH(CH₃)₂, Z est H et Z₁ est CO₂CH₂CH₃
R₃ est CH₂SCH₃, Z est H et Z₁ est CO₂CH₂CH₃
ou R₃ est CH₂SCH₂CH₃, Z est H et Z₁ est CO₂CH₂CH₃
ou un sel ou solvate de celui-ci pharmaceutiquement acceptable.

31. Composé selon la formule (I) ayant la formule IX : 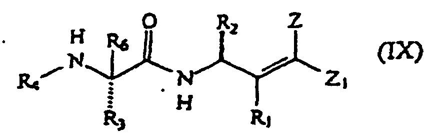 où R₆ est H, R₁ est H, R₂ est CH₂CH₂C(O)NH₂, Z est H et
Z₁ est CO₂CH₂CH₃, R₃ est CH₂Ph et R₄ est 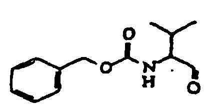 Z₁ est CO₂CH₂CH₃, R₃ est CH₂Ph et R₄ est 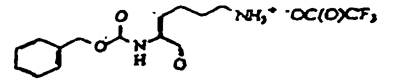 Z₁ est CO₂CH₂CH₃, R₃ est CH₂Ph et R₄ est
Z₁ est CO₂CH₂CH₃, R₃ est CH₂Ph et R₄ est 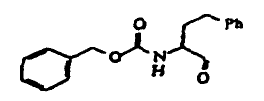 Z₁ est CO₂CH₂CH₃, R₃ est CH₂Ph et R₄ est 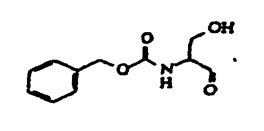 Z₁ est CO₂CH₂CH₃, R₃ est CH₂Ph et R₄ est 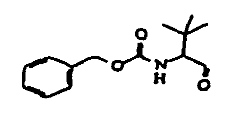 Z₁ est CO₂CH₂CH₃, R₃ est CH₂Ph et R₄ est 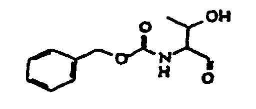 Z₁ est CO₂CH₂CH₃, R₃ est CH₂Ph et R₄ est 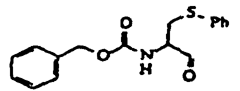 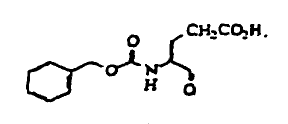 Z₁ est CO₂CH₂CH₃, R₃ est 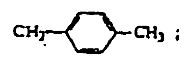 et R₄ est 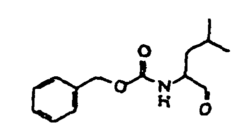 ou un sel ou solvate de celui-ci pharmaceutiquement acceptable.

32. Composé selon la formule (I) ayant la formule IX : 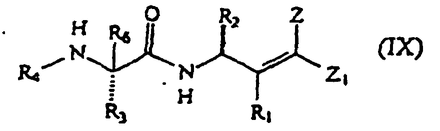 où R₆ est H, R₃ est CH₂Ph, R₂ est CH₂CH₂C(O)NH₂ et
R₁ est H, Z est H, Z₁ est CO₂CH₂CH₃, et R₄ est 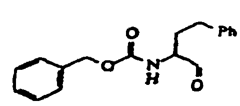 R₁ est H, Z est H, Z₁ est CO₂CH₂CH₃, et R₄ est 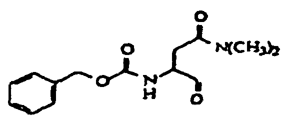 R₁ est H, Z est H, Z₁ est CO₂CH₂CH₃, et R₄ est 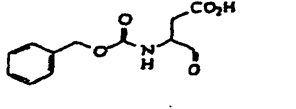 R₁ est H, Z est H, Z₁ est 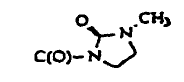 et R₄ est 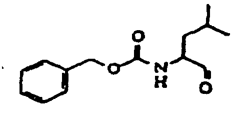 R₁ est H, Z est H, Z₁ est CO₂CH₂CH₃, et R₄ est 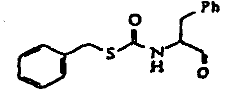 R₁ est H, Z est H, Z₁ est CO₂CH₂CH₃, et R₄ est 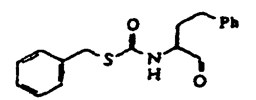 R₁ est H, Z est H, Z₁ est CO₂CH₂C(CH₃)₃ et R₄ est 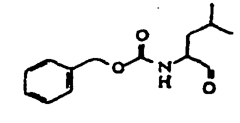 R₁ est H, Z et Z₁ forment ensemble 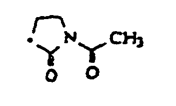 et R₄ est 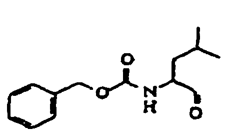 R₁ est H, Z est H, Z₁ est CO₂CH₂CH₃, et R₄ est 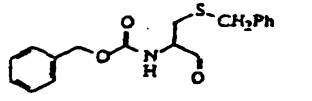 R₁ est H, Z est H, Z₁ est CO₂CH₂CH₃, et R₄ est 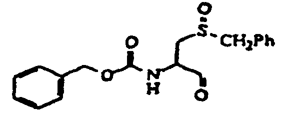 R₁ est H, Z est H, Z₁ est CO₂CH₂CH₃, et R₄ est 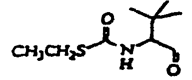 R₁ est H, Z est CH₃, Z₁ est CO₂CH₂CH₃, et R₄ est 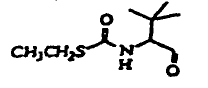 R₁ est H, Z et Z₁ forment ensemble 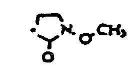 et R₄ est 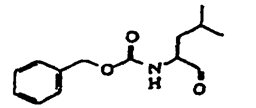 R₁ est H, Z est H, Z₁ est CO₂CH₂CH₃, et R₄ est 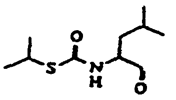 R₁ est H, Z est CH₃, Z₁ est CO₂CH₂CH₃, et R₄ est 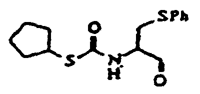 R₁ est H, Z est H, Z₁ est CO₂CH₂CH₃, et R₄ est 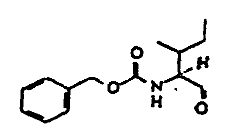 ou R₁ est H, Z est H, Z₁ est CO₂CH₂CH₃, et R₄ est 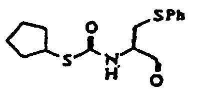 ou un sel ou solvate de celui-ci pharmaceutiquement acceptable.

33. Composé selon la formule (I) ayant la formule IX : 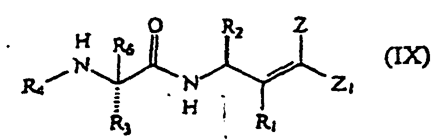 où R₆ est H, R₂ est CH₂CH₂C(O)NH₂, R₁ est H et Z est H, Z₁ est CO₂CH₂CH₃, R₃ est 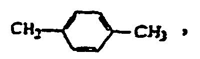 et R₄ est 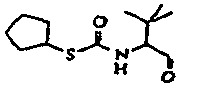 Z est CH₃, Z₁ est CO₂CH₂CH₃, R₃ est 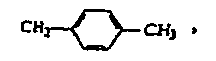 et R₄ est 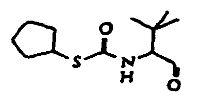 Z est H, Z₁ est CO₂CH₂CH₃, R₃ est 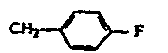 et R₄ est 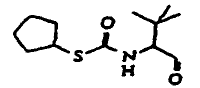 Z est CH₃, Z₁ est CO₂CH₂CH₃, R₃ est 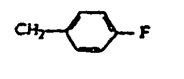 et R₄ est 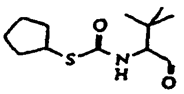 Z est H, Z₁ est CO₂CH₂CH₃, R₃ est CH₂Ph et R₄ est 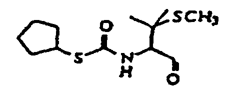 Z est H, Z₁ est CO₂CH₂CH₃, R₃ est CH₂Ph et R₄ est 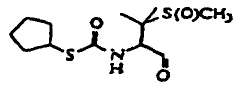 Z est H, Z₁ est CO₂CH₂CH₃, R₃ est CH₂Ph et R₄ est 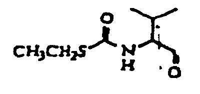 Z et Z₁ forment ensemble 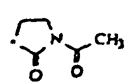 R₃ est 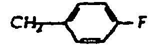 et R₄ est 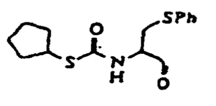 Z est H, Z₁ est CO₂CH₂CH₃, R₃ est CH₂Ph et R₄ est 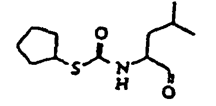 Z est H, Z₁ est CO₂CH₂CH₃, R₃ est 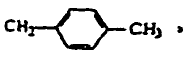 et R₄ est 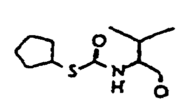 Z est H, Z₁ est CO₂CH₂CH₃, R₃ est 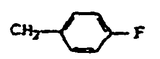 et R₄ est 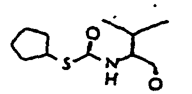 ou Z est H, Z₁ est CO₂CH₂CH₃, R₃ est 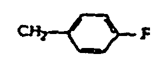 et R₄ est 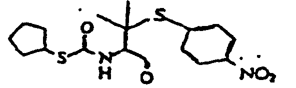 ou un sel ou solvate de celui-ci pharmaceutiquement acceptable.

34. Composition pharmaceutique comprenant :
(a) une quantité thérapeutiquement efficace d'un composé tel que défini dans une quelconque des revendications précédentes, ou d'un sel ou d'un solvate de celui-ci pharmaceutiquement acceptable ; et
(b) un support, diluant, véhicule ou excipient pharmaceutiquement acceptable.

35. Utilisation d'un composé tel que défini dans une quelconque des revendications 1 à 33 ou d'un sel ou d'un solvate de celui-ci pharmaceutiquement acceptable pour la fabrication d'un médicament pour le traitement d'un état pathologique faisant intervenir une activité protéase de picornavirus chez un mammifère.

36. Méthode d'inhibition de l'activité d'une protéase 3C de picornavirus qui comprend la mise en contact de la protéase 3C de picornavirus, dans le but d'inhiber celle-ci, avec une quantité efficace d'un composé tel que défini dans une quelconque des revendications 1 à 33 ou d'un sel ou d'un solvate de celui-ci pharmaceutiquement acceptable.

37. Méthode d'inhibition de l'activité d'une protéase de rhinovirus qui comprend la mise en contact de la protéase de rhinovirus, dans le but d'inhiber celle-ci, avec une quantité efficace d'un composé tel que défini dans une quelconque des revendications 1 à 33 ou d'un sel ou d'un solvate de celui-ci pharmaceutiquement acceptable.

38. Méthode de fabrication d'un composé selon la revendication 1, comprenant la conversion d'un composé de formule Q 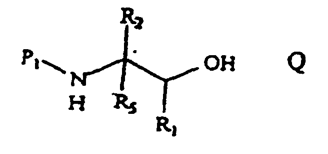 où R₁, R₂ et R₅ sont tels que définis dans la revendication 1, et P₁ est un groupe protecteur, ou d'un sel ou d'un solvate de celui-ci, en un composé de formule I, tel que défini dans la revendication 1, ou en un sel ou solvate de celui-ci pharmaceutiquement acceptable.

39. Méthode selon la revendication 38, où P₁ est un benzyloxycarbonyle ou un t-butoxycarbonyle.

40. Méthode de fabrication d'un composé selon la revendication 1, comprenant la conversion d'un composé de formule B 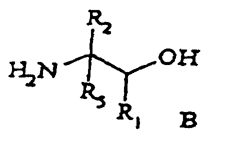 où R₁, R₂ et R₅ sont tels que définis dans la revendication 1, ou d'un sel ou d'un solvate de celui-ci, en un composé de formule I, tel que défini dans la revendication 1, ou en un sel ou solvate de celui-ci pharmaceutiquement acceptable.

41. Méthode de fabrication d'un composé selon la revendication 1, comprenant la conversion d'un composé de formule O 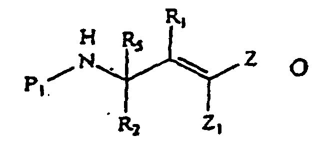 où R₁, R₂, R₅, Z et Z₁ sont tels que définis dans la revendication 1, et P₁ est un groupe protecteur, ou d'un sel ou d'un solvate de celui-ci, en un composé de formule I, tel que défini dans la revendication 1, ou en un sel ou solvate de celui-ci pharmaceutiquement acceptable.

42. Méthode selon la revendication 41, où P₁ est un benzyloxycarbonyle ou un t-butoxycarbonyle.

43. Méthode de fabrication d'un composé selon la revendication 1, comprenant la conversion d'un composé de formule P 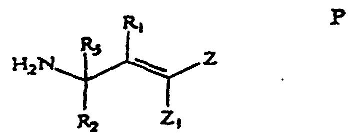 où R₁, R₂, R₅, Z et Z₁ sont tels que définis dans la revendication 1, ou d'un sel ou d'un solvate de celui-ci, en un composé de formule I, tel que défini dans la revendication 1, ou en un sel ou solvate de celui-ci phannaceutiquement acceptable.

44. Composés choisis dans le groupe constitué par :
les composés 46 à 66 et 78 ayant la formule IX : 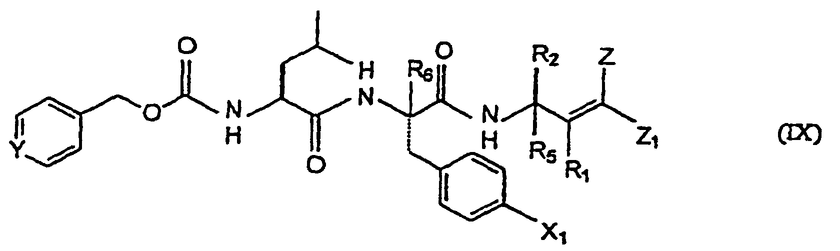
46. R₁ est H ; R₂ est CH₂CH₂C(O)NH₂; R₅, R₆ et X₁ sont H ; Y est CH ; Z est H ; et Z₁ est 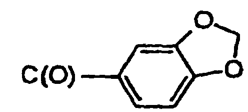
47. R₁ est H ; R₂ est CH₂CH₂C(O)NH₂; R₅, R₆ et X₁ sont H ; Y est CH ; Z est H ; et Z₁ est 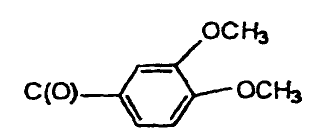
48. R₁ est H ; R₂ est CH₂CH₂C(O)NH₂; R₅, R₆ et X₁ sont H ; Y est CH ; Z est H ; et Z₁ est 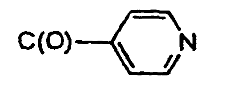
49. R₁ est H ; R₂ est CH₂CH₂C(O)NH₂; R₅, R₆ et X₁ sont H ; Y est CH ; Z est H ; et Z₁ est 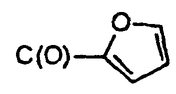
50. R₁ est H ; R₂ est CH₂CH₂C(O)NH₂; R₅, R₆ et X₁ sont H ; Y est CH ; Z est H ; et Z₁ est 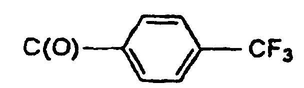
51. R₁ est H ; R₂ est CH₂CH₂C(O)NH₂ ; R₅, R₆ et X₁ sont H ; Y est CH ; Z est H ; et Z₁ est 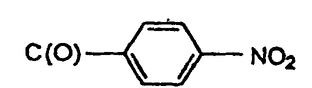
52. R₁ est H ; R₂ est CH₂CH₂C(O)NH₂ ; R₅, R₆ et X₁ sont H ; Y est CH ; Z est H ; et Z₁ est C(O)t-Bu
53. R₁ est H ; R₂ est CH₂CH₂C(O)NH₂ ; R₅ et R₆ sont H ; X₁ est OH ; Y est CH ; Z est H ; et Z₁ est CO₂CH₂CH₃
54. R₁ est H ; R₂ est CH₂CH₂C(O)NH₂ ; R₅, R₆ et X₁ sont H ; Y est CH ; Z est H ; et Z₁ est C(O)C(O)CH₃
55. R₁ est H ; R₂ est CH₂CH₂C(O)NH₂ ; R₅, R₆ et X₁ sont H ; Y est CH ; Z est H ; et Z₁ est C(O)C(O)N(CH₃)₂
56. R₁ est H ; R₂ est CH₂OC(O)NH₂; R₅, R₆ et X₁ sont H ; Y est CH ; Z est H ; et Z₁ est CO₂CH₂CH₃
57. R₁ est H ; R₂ est CH₂CH₂C(O)NH₂ ; R₅, R₆ et X₁ sont H ; Y est CH ; Z est H ; et Z et Z₁ forment ensemble 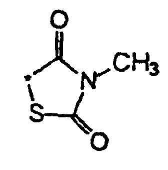 où le S est de préférence en trans par rapport au groupe R₁
58. R₁ est H ; R₂ est CH₂CH₂C(O)NH₂ ; R₅, R₆ et X₁ sont H ; Y est CH ; et Z et Z₁ forment ensemble 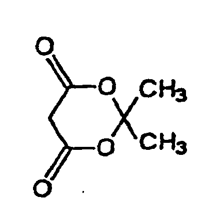
59. R₁ est H ; R₂ est CH₂CH₂C(O)NH₂; R₅, R₆ et X₁ sont H ; Y est CH ; Z est H ; et Z₁ est C(O)NHPh
60. R₁ est H ; R₂ est CH₂CH₂C(O)NH₂ ; R₅, R₆ et X₁ sont H ; Y est CH ; Z est H ; et Z₁ est C(O)N(CH₃)Ph
61. R₁ est H ; R₂ est CH₂CH₂C(O)NH₂; R₅, R₆ et X₁ sont H ; Y est CH ; Z est H ; et Z₁ est 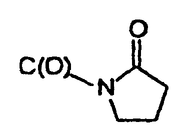
62. R₁ est H ; R₂ est CH₂CH₂C(O)NH₂ ; R₅, R₆ et X₁ sont H ; Y est CH ; Z est H ; et Z₁ est 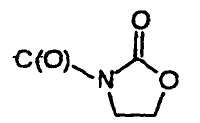
63. R₁, R₅, R₆, X₁ et Z sont H ; Y est CH ; R₂ est CH₂CH₂C(O)NH₂ ; et Z₁ est 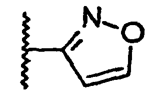
64. R₁, R₅, R₆, X₁ et Z sont H ; Y est CH ; R₂ est CH₂CH₂C(O)NH₂ ; et Z₁ est 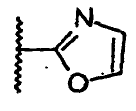
65. R₁, R₅, R₆, X₁ et Z sont H ; Y est CH ; R₂ est CH₂CH₂C(O)NH₂ ; et Z₁ est 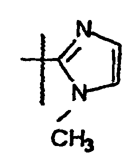
66. R₁, R₅, R₆, X₁ et Z sont H ; Y est CH ; R₂ est CH₂CH₂C(O)NH₂; et Z₁ est 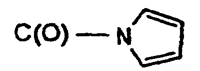
78. R₁, R₅, R₆ et X₁ sont H ; Y est CH ; R₂ est CH₂CH₂C(O)NH₂; Z est CH₂Cl et Z₁ est H ;
les composés 67 à 69 ayant la formule X : 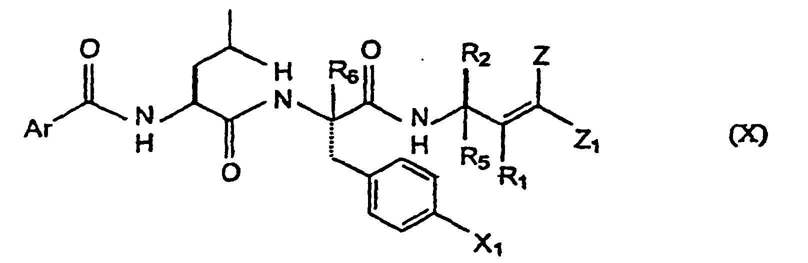
67. R₁, R₅, R₆, X₁ et Z sont H; R₂ est CH₂CH₂C(O)NH₂; Z₁ est CO₂CH₂CH₃ ; et Ar est Ph
68. R₁, R₅, R₆, X₁ et Z sont H ; R₂ est CH₂CH₂C(O)NH₂ ; Z₁ est CO₂CH₃ ; et Ar est 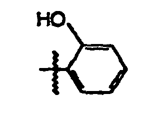
69. R₁, R₅, R₆, X₁ et Z sont H; R₂ est CH₂CH₂C(O)NH₂; Z₁ est CO₂CH₂CH₃ ; et Ar est 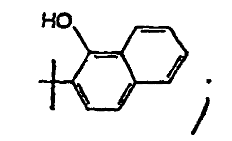
les composés 70 à 73 ayant la formule XI : 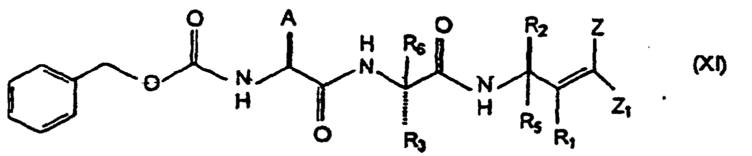
70. R₁, R₅, R₆ et Z sont H ; R₂ est CH₂CH₂C(O)NH₂ ; R₃ est CH₂Ph; Z₁ est CO₂CH₂CH₃ ; et A est 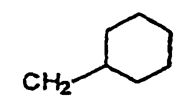
71. R₁, R₅, R₆ et Z sont H ; R₂ est CH₂CH₂C(O)NH₂ ; R₃ est CH₂Ph ; Z₁ est C4₂CH₂CH₃ ; et A est Ph
72. R₁, R₅, R₆ et Z sont H ; R₂ est CH₂CH₂C(O)NH₂; A est CH₂CH(CH₃)₂ ; Z₁ est CO₂CH₂CH₃ ; et R₃ est 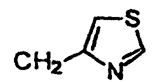
73. R₁, R₅, R₆ et Z sont H ; R₂ est CH₂CH₂C(O)NH₂; A est CH₂CH(CH₃)₂ ; Z₁ est CO₂CH₂CH₃ ; et R₃ est 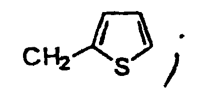
les composés 1, 6, 8 à 10, 15, 20, 23, 38 à 40, 76 et 77 ayant la formule XII : 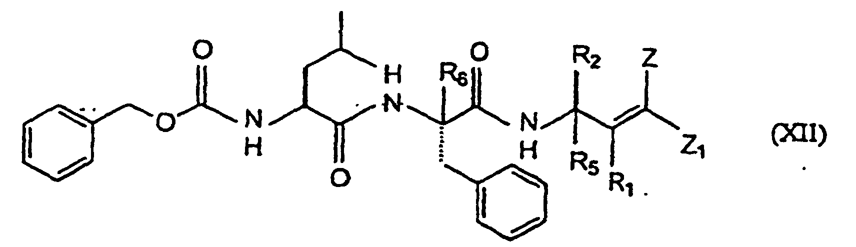
1. R₁ est H ; R₂ est CH₂CH₂CN ; R₅ est H ; R₆ est H ; Z est F et Z₁ est CO₂CH₂CH₃
6. R₁ est H ; R₂ est CH₂CH₂C(O)NH₂ ; R₅ est H ; R₆ est H ; Z est H et Z₁ est C(O)NHCH₂CH₃
8. R₁ est H ; R₂ est CH₂CH₂C(O)NH₂ ; R₅ est H ; R₆ est H ; Z est F et Z₁ est CO₂CH₂CH₃
9. R₁ est H ; R₂ est CH₂CH₂C(O)NH₂ ; R₅ est H ; R₆ est H ; Z est H et Z₁ est SO₂CH₃
10. R₁ est H ; R₂ est CH₂CH₂C(O)NH₂ ; R₅ est H ; R₆ est H ; Z est H et Z₁ est SO₂Ph
15. R₁ est H ; R₂ est CH₂CH₂C(O)NH₂ ; R₅ est H ; R₆ est H ; Z est H et Z₁ est CO₂H
20. R₁ est H ; R₂ est CH₂CH₂C(O)NH₂ ; R₅ est H ; R₆ est H ; Z est H et Z₁ est PO(OCH₂CH₃)₂
23. R₁ est H ; R₂ est CH₂CH₂C(O)NH₂ ; R₅ est H ; R₆ est H ; Z est H et Z₁ est 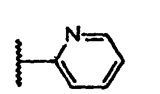
38. R₁ est H ; R₂ est CH₂CH₂C(O)NH₂ ; R₅ est H ; R₆ est H ; Z est H et Z₁ est 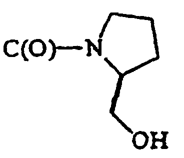
39. R₁ est H ; R₂ est CH₂CH₂C(O)NH₂ ; R₅ est H ; R₆ est H ; Z est H et Z₁ est 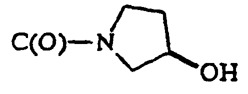
40. R₁ est H ; R₂ est CH₂CH₂C(O)NH₂ ; R₅ est H ; R₆ est H ; Z est H et Z₁ est 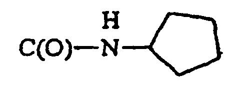
76. R₁ est H ; R₂ est CH₂CH₂C(O)NH₂ ; R₅ est H ; R₆ est H ; Z est H et Z₁ est CH₂OAc
77. R₁ est H ; R₂ est CH₂CH₂C(O)NH₂ ; R₅ est H ; R₆ est H ; Z est H et Z₁ est 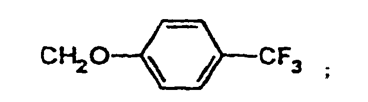
le composé 45 ayant la formule XIII :
45. 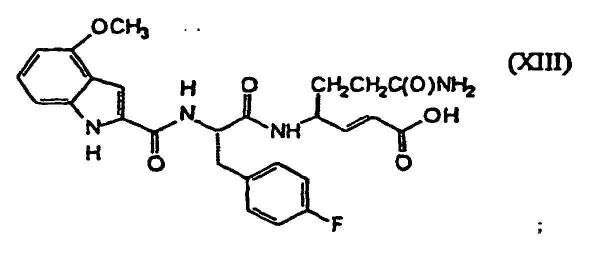
les composés 79 à 97 ayant également la formule III : 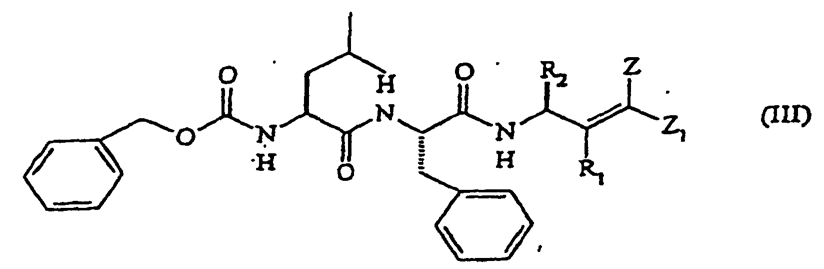
82. R₂ est CH₂CH₂C(O)NH₂, R₁ est H, Z est CH₃, et Z₁ est CO₂CH₂CH₃,
90. R₂ est CH₂CH₂C(O)NH₂, R₁ est H, et Z et Z₁ forment ensemble 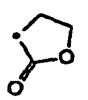 où C=O est de préférence en cis par rapport au groupe R₁
ou bien où R₂ est CH₂CH₂C(O)NH₂, R₁ est H, Z est H et Z₁ est choisi parmi 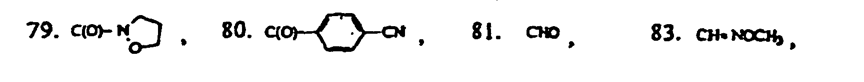 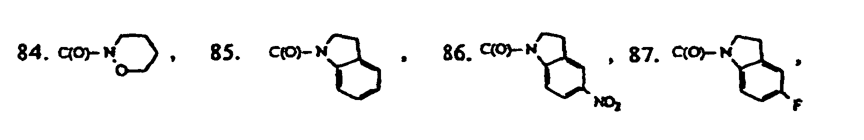 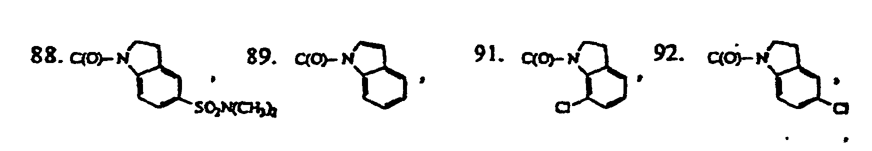 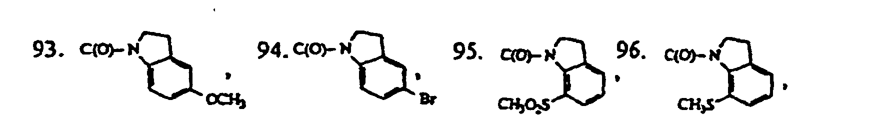 et 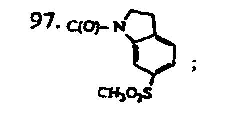
les composés 98 à 121 ayant la formule XIV : 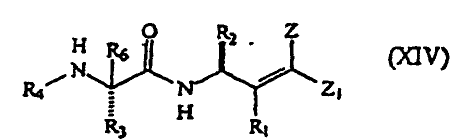 où R₆ est H, R₁ est H, R₂ est CH₂CH₂C(O)NH₂, Z est H, Z₁ est CO₂CH₂CH₃ et
98. R₃ est CH₂Ph et R₄ est 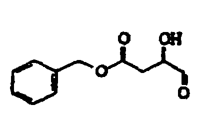
99. R₃ est H et R₄ est 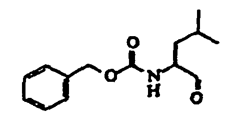
100. R₃ est 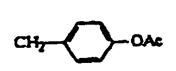 et R₄ est 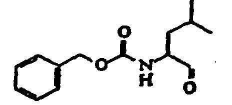
101. R₃ est CH₂Ph et R₄ est 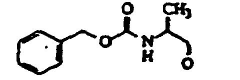
102. R₃ est CH₂Ph et R₄ est 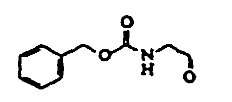
103. R₃ est 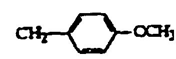 et R₄ est 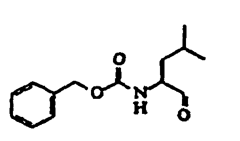
104. R₃ est CH₂Ph et R₄ est 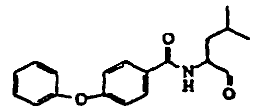
105. R₃ est 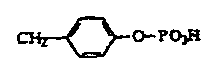 et R₄ est 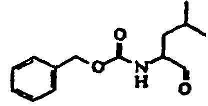
106. R₃ est CH₂Ph et R₄ est 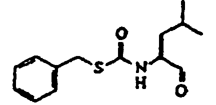
107. R₃ est CH₂Ph et R₄ est 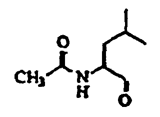
108. R₃ est CH₂CH₃ et R₄ est 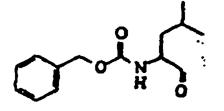
109. R₃ est CH₃ et R₄ est 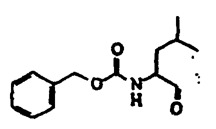
110. R₃ est CH₂Ph et R₄ est 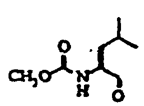
111. R₃ est CH₂Ph et R₄ est 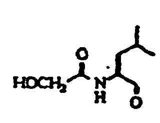
112. R₃ est 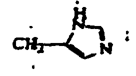 et R₄ est 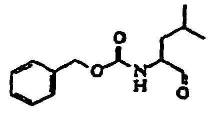
113. R₃ est 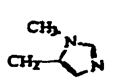 et R₄ est 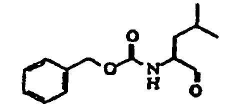
114. R₃ est 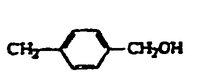 et R₄ est 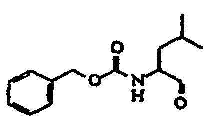
115. R₃ est CH₂Ph et R₄ est 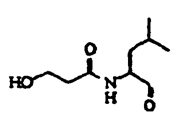
116. R₃ est CH₂Ph et R₄ est 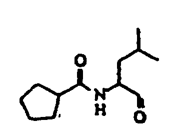
117. R₃ est CH₂Ph et R₄ est 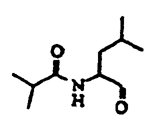
118. R₃ est CH₂Ph et R₄ est 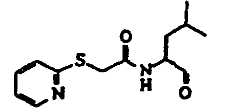
119. R₃ est CH₂Ph et R₄ est 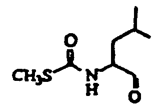
120. R₃ est CH₂Ph et R₄ est 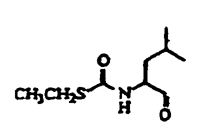
121. R₃ est CH₂CH₂CO₂H et R₄ est 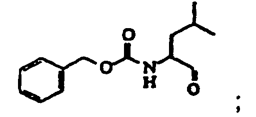
les composés 122 à 130 ayant également la formule XIV : 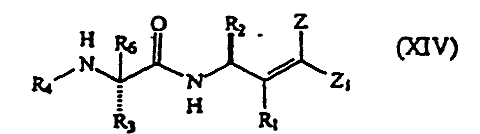 où R₆ est H, R₁ est H, R₃ est CH₂Ph et
122. R₂ est CH₂OC(O)NHC(O)CH₂Cl, Z est H, Z₁ est CO₂CH₂CH₃ et R₄ est 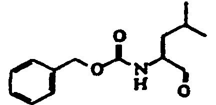
123. R₂ est CH₂CH₂C(O)NH₂, Z est H, Z₁ est CO₂CH₂CH₃ et R₄ est 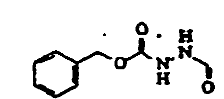
124. R₂ est CH₂CH₂C(O)NH₂, Z est H, Z₁ est 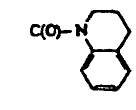 et R₄ est 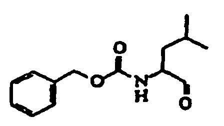
125. R₂ est CH₂CH₂C(O)NH₂, Z est H, Z₁ est NO₂ et R₄ est 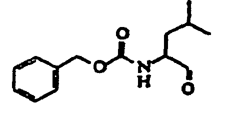
126. R₂ est CH₂CH₂C(O)NH₂, Z est H, Z₁ est 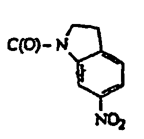 et R₄ est 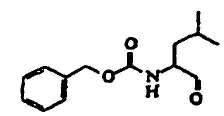
127. R₂ est CH₂CH₂C(O)NH₂, Z est H, Z₁ est 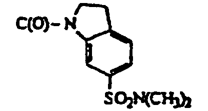 et R₄ est 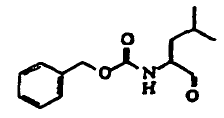
128. R₂ est CH₂CH₂C(O)NH₂, Z est H, Z₁ est 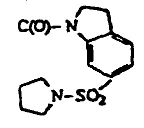 et R₄ est 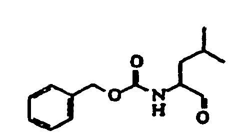
129. R₂ est CH₂CH₂C(O)NH₂, Z est H, Z₁ est CO₂CH₂CH₃ et R₄ est 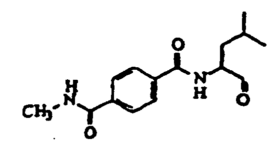
130. R₂ est CH₂CH₂C(O)NH₂, Z et Z₁ forment ensemble 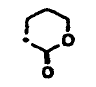 et R₄ est 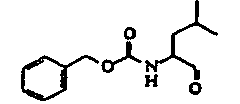 où C=O est de préférence en cis par rapport au groupe R₁ ;
les composés 131 à 145 ayant également la formule XIV : 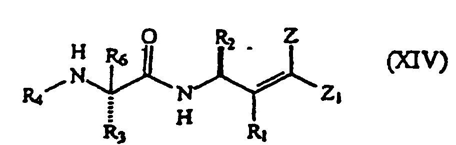 où R₆ est H, R₁ est H, R₂ est CH₂CH₂C(O)NH₂, R₄ est 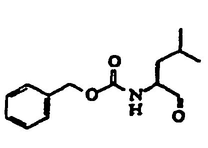 et
131. R₃ est CH₂Ph, Z est H et Z₁ est 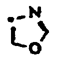
132. R₃ est 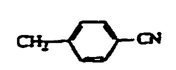 Z est H, Z₁ est CO₂CH₂CH₃
133. R₃ est 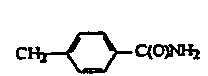 Z est H, Z₁ est CO₂CH₂CH₃
134. R₃ est CH(OH)CH₃, Z est H et Z₁ est CO₂CH₂CH₃
135. R₃ est 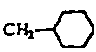 Z est H et Z₁ est CO₂CH₂CH₃
136. R₃ est 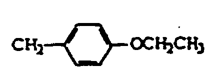 Z est H et Z₁ est CO₂CH₂CH₃
137. R₃ est CH₂CH₂CH₃, Z est H et Z₁ est CO₂CH₂CH₃
138. R₃ est CH₂Ph, Z est H et Z₁ est C(O)N(OH)CH₃
139. R₃ est 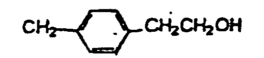 Z est H et Z₁ est CO₂CH₂CH₃
140. R₃ est 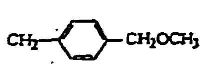 Z est H et Z₁ est CO₂CH₂CH₃
141. R₃ est CH₂CH(CH₃)₂, Z est H et Z₁ est CO₂CH₂CH₃
142. R₃ est CH₂SCH₃, Z est H et Z₁ est CO₂CH₂CH₃
143. R₃ est CH₂SCH₂CH₃, Z est H et Z₁ est CO₂CH₂CH₃
144. R₃ est CH₂Ph, Z est CH₃ et Z₁ est CO₂H
145. R₃ est CH₂Ph, Z est H et Z₁ est 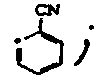
les composés 146 à 155 ayant également la formule XIV : 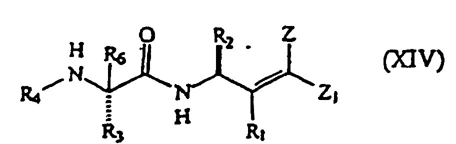 où R₅ est H, R₁ est H, R₂ est CH₂CH₂C(O)NH₂, Z est H et
146. Z₁ est CO₂CH₂CH₃, R₃ est CH₂Ph, et R₄ est 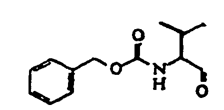
147. Z₁ est CO₂CH₂CH₃, R₃ est CH₂Ph, et R₄ est 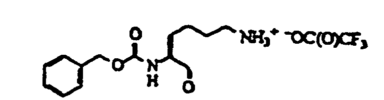
148. Z₁ est CO₂CH₂CH₃, R₃ est CH₂Ph, et R₄ est 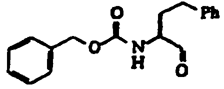
149. Z₁ est CO₂CH₂CH₃, R₃ est CH₂Ph, et R₄ est 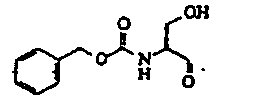
150. Z₁ est 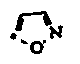 R₃ est CH₂Ph, et R₄ est 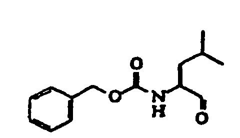
151. Z₁ est CO₂CH₂CH₃, R₃ est CH₂Ph, et R₄ est 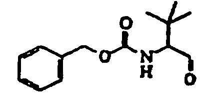
152. Z₁ est CO₂CH₂CH₃, R₃ est CH₂Ph, et R₄ est 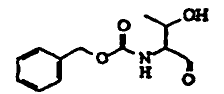
153. Z₁ est CO₂CH₂CH₃, R₃ est CH₂Ph, et R₄ est 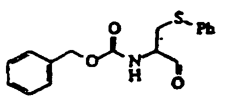
154. Z₁ est CO₂CH₂CH₃, R₃ est CH₂Ph, et R₄ est 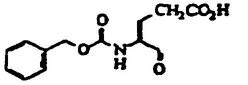
155. Z₁ est CO₂CH₂CH₃, R₃ est 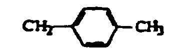 et R₄ est 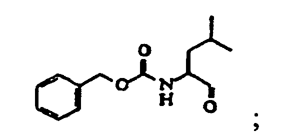
les composés 156 à 173 ayant également la formule XIV : 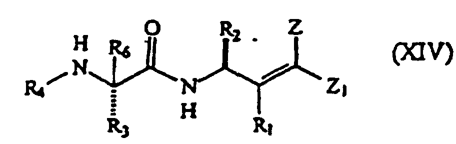 où R₆ est H, R₃ est CH₂Ph, R₂ est CH₂CH₂C(O)NH₂, et
156. R₁ est OH, Z est H, Z₁ est CO₂CH₂CH₃, et R₄ est 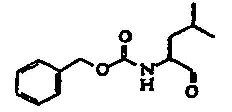
157. R₁ est H, Z est H, Z₁ est CO₂CH₂CH₃, et R₄ est 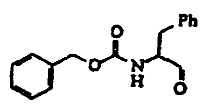
158. R₁ est H, Z est H, Z₁ est CO₂CH₂CH₃, et R₄ est 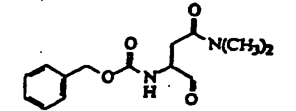
159. R₁ est H, Z est H, Z₁ est CO₂CH₂CH₃, et R₄ est 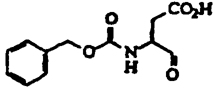
160. R₁ est H, Z est H, Z₁ est 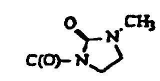 et R₄ est 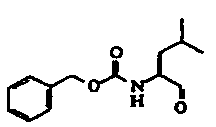
161. R₁ est H, Z est H, Z₁ est CO₂CH₂CH₃, et R₄ est 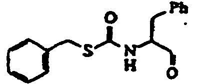
162. R₁ est H, Z est H, Z₁ est CO₂CH₂CH₃, et R₄ est 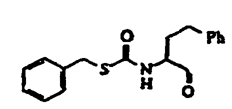
163. R₁ est H, Z est H, Z₁ est CO₂CH₂C(CH₃)₃, et R₄ est 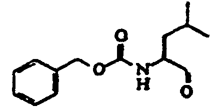
164. R₁ est H, Z et Z₁ forment ensemble 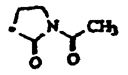 et R₄ est 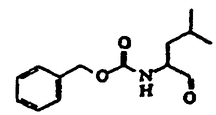 où C=O est de préférence en cis par rapport au groupe R₁
165. R₁ est H, Z est H, Z₁ est CO₂CH₂CH₃, et R₄ est 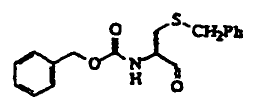
166. R₁ est H, Z est H, Z₁ est CO₂CH₂CH₃, et R₄ est 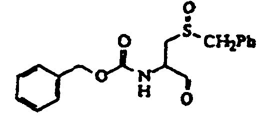
167. R₁ est H, Z est H, Z₁ est CO₂CH₂CH₃, et R₄ est 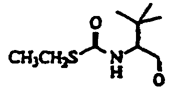
168. R₁ est H, Z est CH₃, Z₁ est CO₂CH₂CH₃, et R₄ est 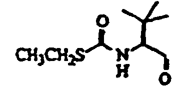
169. R₁ est H, Z et Z₁ forment ensemble 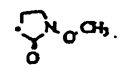 et R₄ est 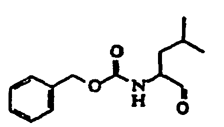 où C=O est de préférence en cis par rapport à R₁
170. R₁ est H, Z est H, Z₁ est CO₂CH₂CH₃, et R₄ est 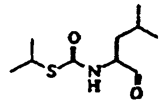
171. R₁ est H, Z est CH₃, Z₁ est CO₂CH₂CH₃, et R₄ est 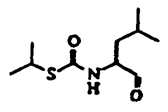
172. R₁ est H, Z est H, Z₁ est CO₂CH₂CH₃, et R₄ est 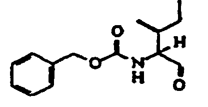
173. R₁ est H, Z est H, Z₁ est CO₂CH₂CH₃, et R₄ est 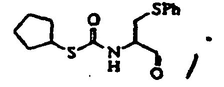
les composés 174 à 188 ayant également la formule XIV : 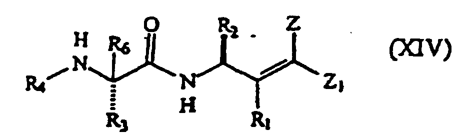 où R₆ est H, R₂ est CH₂CH₂C(O)NH₂, R₁ est H et
174. Z est H, Z₁ est CO₂CH₂CH₃, R₃ est 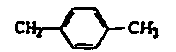 et R₄ est 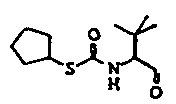
175. Z est CH₃, Z₁ est CO₂CH₂CH₃, R₃ est 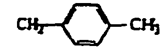 et R₄ est 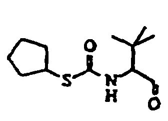
176. Z est H, Z₁ est CO₂CH₂CH₃, R₃ est 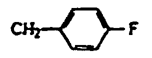 et R₄ est 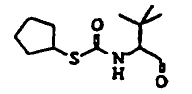
177. Z est CH₃, Z₁ est CO₂CH₂CH₃, R₃ est 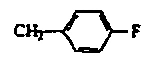 et R₄ est 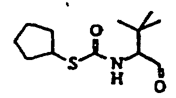
178. Z est H, Z₁ est CO₂CH₂CH₃, R₃ est CH₂Ph, et R₄ est 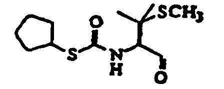
179. Z est H, Z₁ est CO₂CH₂CH₃, R₃ est CH₂Ph, et R₄ est 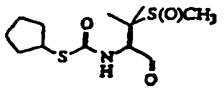
180. Z est H, Z₁ est CO₂CH₂CH₃, R₃ est CH₂Ph, et R₄ est 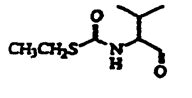
181. Z et Z₁ forment ensemble 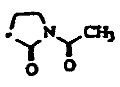 R₃ est 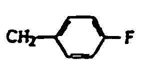 et R₄ est 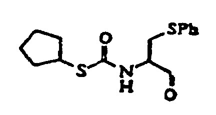 où C=O est de préférence en cis par rapport au groupe R₁
182. Z est H, Z₁ est CO₂CH₂CH₃, R₃ est CH₂Ph, et R₄ est 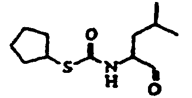
183. Z est H, Z₁ est CO₂CH₂CH₃, R₃ est 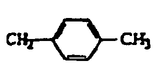 et R₄ est 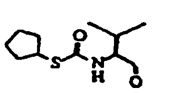
184. Z est H, Z₁ est CO₂CH₂CH₃, R₃ est 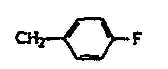 et R₄ est 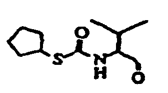
185. Z est H, Z₁ est CO₂CH₂CH₃, R₃ est 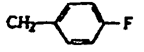 et R₄ est 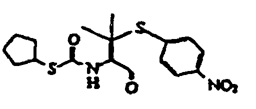
186. Z est H, Z₁ est CO₂CH₂Ph, R₃ est 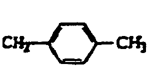 et R₄ est 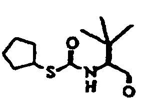
187. Z est CH₃, Z₁ est CO₂CH₂CH₃, R₃ est CH₂Ph, et R₄ est 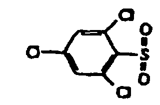
188. Z est H, Z₁ est CO₂CH₂CH₂OCH₃, R₃ est 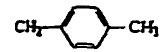 et R₄ est 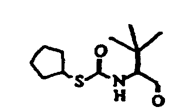
189. R₃ est 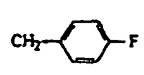 R₄ est 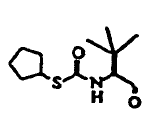 et Z et Z₁ forment ensemble 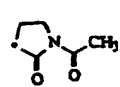 où C=O est de préférence en cis par rapport au groupe R₁
190. Z est H, Z₁ est CO₂CH₂CH₃, R₃ est 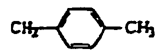 et R₄ est 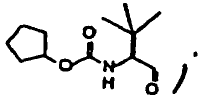
et les composés de formule (III) ci-dessous : 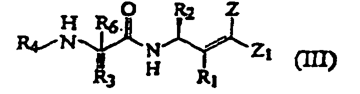 où R₆ est H, R₁ est H, R₃ est CH₂Ph, R₂ est CH₂CH₂C(O)NH₂, Z est H, Z₁ est CO₂CH₂CH₃ et R₄ est choisi parmi les groupes suivants : 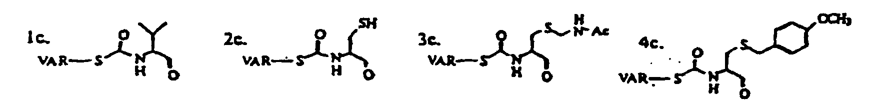 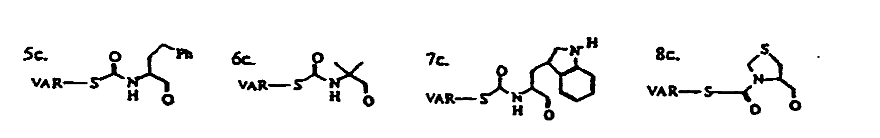 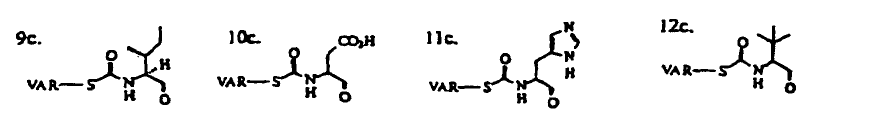 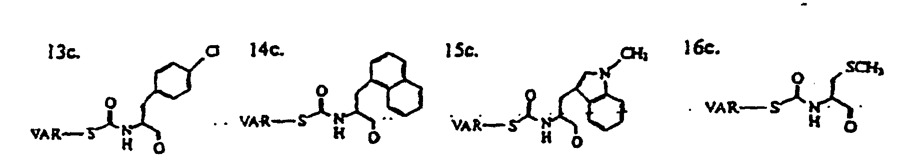 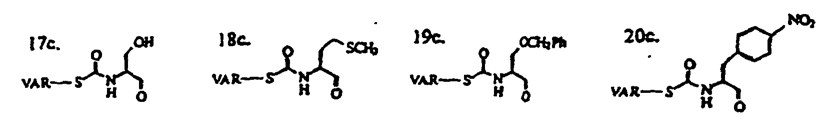 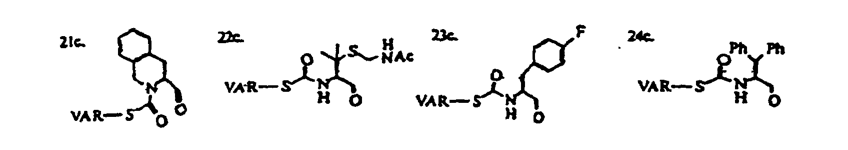 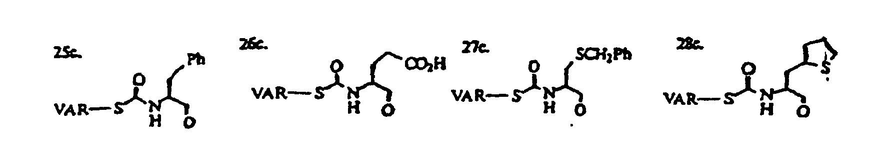 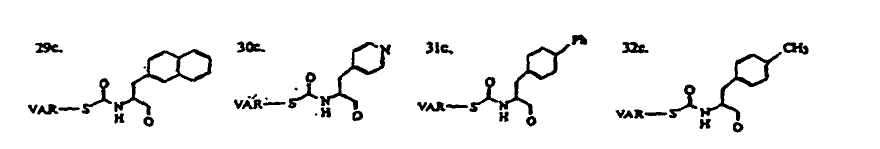 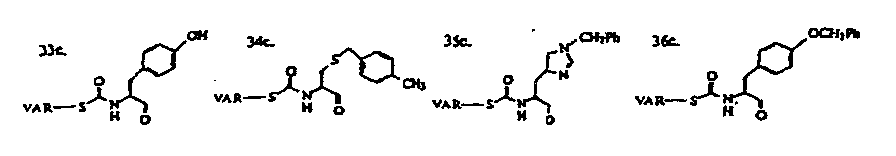 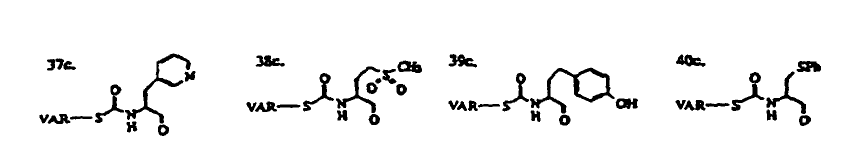 où VAR est choisi parmi -CH₂CH₃, -CH(CH₃)₂, -CH₂CH(CH₃)₂, -CH₂-Ph,
